# EUROPEAN PATENT SPECIFICATION

(11) **EP 1 383 534 B1**
(45) Date of publication and mention of the grant of the patent: **21.03.2007**
(21) Application number: 02712901.4
(22) Date of filing: 08.02.2002
(51) Int. Cl.: A61K 39/116, A61P 31/04

(54) **Chalamydia vaccine based on blebs**
Auf Bläschen basierendes Impfstoff gegen Chlamydia
Vaccin contre Chlamydia se basant sur des papules membranaires

(30) Priority: 08.02.2001 GB 0103169
(43) Date of publication of application: 28.01.2004
(73) Proprietor: GlaxoSmithKline Biologicals S.A., 1330 Rixensart (BE)
(72) Inventor: BERTHET, F-X Jacqueline, GlaxoSmithKline Bio S.A., B-1330 Rixensart (BE); LOBET, Yves, GlaxoSmithKline Biologicals S.A., B-1330 Rixensart (BE); POOLMAN, Jan, GlaxoSmithKline Biologicals S.A., B-1330 Rixensart (BE); VERLANT, Vincent, GlaxoSmithKline Biological S.A., B-1330 Rixensart (BE)
(74) Representative: Dalton, Marcus Jonathan William
(86) International application number: PCT/EP2002/001356
(87) International publication number: WO 2002/062380

(56) References cited:
- WO-A-00/26239
- WO-A-01/09350
- WO-A-01/89535
- ZHOU L ET AL: "ON THE ORIGIN OF MEMBRANE VESICLES IN GRAM-NEGATIVE BACTERIA" FEMS MICROBIOLOGY LETTERS, AMSTERDAM, NL, vol. 163, no. 2, 15 June 1998 (1998-06-15), pages 223-228, XP001007553 ISSN: 0378-1097
- WYLLIE S ET AL: "Single channel analysis of recombinant major outer membrane protein porins from Chlamydia psittaci and Chlamydia pneumoniae" FEBS LETTERS, ELSEVIER SCIENCE PUBLISHERS, AMSTERDAM, NL, vol. 445, no. 1, 19 February 1999 (1999-02-19), pages 192-196, XP004259874 ISSN: 0014-5793

## Description

### FIELD OF THE INVENTION

The present invention relates to the field of Gram-negative bacterial vaccine compositions and their manufacture. More particularly it relates to the field of useful Gram-negative bacterial outer membrane vesicle (or bleb) compositions comprising heterologously expressed *Chlamydia* antigens, and advantageous methods of rendering these compositions more effective and safer as a vaccine.

### BACKGROUND OF THE INVENTION

*Chlamydiae* are obligate intracellular Gram negative bacteria which replicate only in cytoplasmic inclusions of eukaryotic cells. They have a unique developmental cycle which is represented by two major forms, the spore-like elementary body (EB) which is the infectious form transmitted from cell to cell, and the non infectious, metabolically active reticulate body (RB) which replicates within the host-cell.

Of the four known chlamydial species, *Chlamydia trachomatis* and *C*. *pneumoniae* are the important human pathogens. The recently defined species *C*. *pneumoniae* (Grayston 1989) is now recognized as a major cause of respiratory tract infections (Grayston 1993) and data are now growing for an association with atherosclerosis. The association is supported by seroepidemiological studies, studies demonstrating the presence of the bacterium in the atherosclerotic lesions, studies showing *C*. *pneumoniae* capability to replicate in the different cell types present in the atheroclerotic lesions, interventional trials with antibiotics in patients with coronary artery disease and experimental respiratory tract infection in rabbits or apolipoprotein-E deficient mice which leads to inflammatory changes in the aorta (Danesh 1997, Fong 1997, Laitinen 1997, Moazed 1997). Overall, those data implicate C. *pneumoniae* as a causative and/or aggravating factor of atherosclerosis.

*C. trachomatis* is a major human pathogen; transmitted from human to human (there is no known animal reservoir), it causes ocular and genital infections which can result in long term sequelae. Trachoma, a chlamydial ocular infection, is endemic in several developing countries and is the world's leading cause of preventable blindness with millions people affected by the disease. Genital chlamydial infections constitute the most common bacterial sexually transmitted disease (STD) worldwide. In 1996, WHO generated a new set of global estimates for four major STDs drawing an extensive review of the published and unpublished prevalence data (Gerbase 1998). It has been estimated that in 1995, 4 and 5.2 million new cases of *C*. *trachomatis* infection occured in individuals aged 15-49 for North America and Western Europe, respectively; worldwide, *C*. *trachomatis* totalized an estimate of 89.1 million new cases. Collectively, data show higher infection rates in women as compared to men (Washington 1987, Peeling 1995, Cates 1991); higher incidence is found in adolescent and young adults, approximately 70% of the chlamydial infections being reported in the 15-24 years of age group (Peeling 1995).

There is a clear need for effective vaccines against *Chlamydia trachomatis* and *Chlamydia pneumoniae*.

### Outer membrane vesicles (blebs)

Gram-negative bacteria are separated from the external medium by two successive layers of membrane structures. These structures, referred to as the cytoplasmic membrane and the outer membrane (OM), differ both structurally and functionally. The outer membrane plays an important role in the interaction of pathogenic bacteria with their respective hosts. Consequently, the surface exposed bacterial molecules represent important targets for the host immune response, making outer-membrane components attractive candidates in providing vaccine, diagnostic and therapeutics reagents.

Whole cell bacterial vaccines (killed or attenuated) have the advantage of supplying multiple antigens in their natural micro-environment. Drawbacks around this approach are the side effects induced by bacterial components such as endotoxin and peptidoglycan fragments. On the other hand, acellular subunit vaccines containing purified components from the outer membrane may supply only limited protection and may not present the antigens properly to the immune system of the host.

Proteins, phospholipids and lipopolysaccharides are the three major constituents found in the outer-membrane of all Gram-negative bacteria. These molecules are distributed asymmetrically: membrane phospholipids (mostly in the inner leaflet), lipooligosaccharides (exclusively in the outer leaflet) and proteins (inner and outer leaflet lipoproteins, integral or polytopic membrane proteins). For many bacterial pathogens which impact on human health, lipopolysaccharide and outer-membrane proteins have been shown to be immunogenic and amenable to confer protection against the corresponding disease by way of immunization.

The OM of Gram-negative bacteria is dynamic and, depending on the environmental conditions, can undergo drastic morphological transformations. Among these manifestations, the formation of outer-membrane vesicles or "blebs" has been studied and documented in many Gram-negative bacteria (Zhou, L *et al.* 1998. *FEMS Microbiol. Lett.* 163: 223-228). Among these, a non-exhaustive list of bacterial pathogens reported to produce blebs include: *Bordetella pertussis, Borrelia burgdorferi, Brucella melitensis, Brucella ovis, Chlamydia psittaci, Chlamydia trachomatis, Esherichia coli, Haemophilus influenzae, Legionella pneumophila, Neisseria gonorrhoeae, Neisseria meningitidis, Pseudomomas aeruginosa* and *Yersinia enterocolitica.* Although the biochemical mechanism responsible for the production of OM blebs is not fully understood, these outer membrane vesicles have been extensively studied as they represent a powerful methodology in order to isolate outer-membrane protein preparations in their native conformation.

Examples of bacterial species from which bleb vaccines can be made have been reviewed in WO 01/09350. For example, *N*. *meningitidis* serogroup B (menB) excretes outer membrane blebs in sufficient quantities to allow their manufacture on an industrial scale. Such multicomponent outer-membrane protein vaccines from naturally-occurring menB strains have been found to be efficacious in protecting teenagers from menB disease and have become registered in Latin America. An alternative method of preparing outer-membrane vesicles is via the process of detergent extraction of the bacterial cells (EP 11243).

### SUMMARY OF THE INVENTION

The present inventors have found that Gram-negative bacterial blebs are an ideal context to present *Chlamydia* outer membrane proteins. In particular gonococcal blebs are useful in the case of presenting *C*. *trachomatis* OMPs and meningococcal blebs are useful in the case of presenting *C. pneumoniae* OMPs. This is because a) these outer-membrane proteins can integrate into such blebs in a native (or near-native) conformation thus retaining a useful immunological effect; b) blebs (particularly from *Neisseria* strains) can be produced in industrial quantities, c) blebs may be mucosally administered, and d) the combination of *Chlamydia* antigens with native bleb antigens can have important interactions for certain conditions such as salpingitis.

The present invention thus provides advantageous Gram-negative bacterial bleb preparations (derived from bleb-producing bacterial strains listed above, and preferably not derived from Chlamydia) presenting on its surface one or more recombinant (and preferably heterologous) protein antigens from *Chlamydia trachomatis* or *Chlamydia pneumoniae.* Advantagous vaccine formulations and methods of administration are also provided.

### DESCRIPTION OF THE INVENTION

The present invention provides a Gram-negative bacterial bleb not derived from Chlamydia, presenting on its surface the PorB outer membrane protein from *Chlamydia trachomatis*, wherein the combination of the Chlamydia antigen with the native Gram-negative bacterial bleb antigens interact in the prevention or treatment of salpingitis when present in a vaccine formulation.

Also provided is a Gram-negative bleb not derived from Chlamydia, presenting on its surface both the PmpG and MOMP (from one or more serovars) outer membrane proteins from *Chlamydia trachomatis,* wherein the combination of the Chlamydia antigens with the native Gram-negative bacterial bleb antigens interact in the prevention or treatment of salpingitis when present in a vaccine formulation.

Additionally there is provided a Gram-negative bleb produced from *Neisseria meningitidis, Moraxella catharralis* or *Haemophilus influenzae* presenting on its surface a protective antigen from *Chlamydia pneumoniae.*

In the context of this application the term "presenting on its surface" indicates that the *Chlamydia* protein should be exposed to the outer surface of the bleb and tethered to the outer membrane (preferably by being integrated into the outer membrane). Most preferably it should take up its native fold within the heterologous bleb context.

An efficient strategy to modulate the composition of a Bleb preparation in this way is to deliver one or more copies of a DNA segment containing an expression cassette comprising a gene encoding said Chlamydia outer membrane protein into the genome of a Gram-negative bacterium.

A non exhaustive list of preferred bacterial species that could be used as a recipient for such a cassette includes: *Bordetella pertussis, Borrelia burgdorferi, Brucella melitensis, Brucella ovis, Esherichia coli, Haemophilus influenzae, Legionella pneumophila, Neisseria gonorrhoeae, Neisseria meningitidis, Pseudomonas aeruginosa* and *Yersinia enterocolitica. Neisseria meningitidis, Neisseiria gonorrhoeae, Moraxella catarrhalis, Haemophilus influenzae, Pseudomonas aeruginosa,* are more preferred for this purpose, and *Neisseria gonorrhoeae* and *Neisseria meningitidis* are most preferred for making the blebs of this invention. The *Chlamydia* OMPs are expressed heterologously, and in such situations *Chlamydia* strains should not be used to make the blebs of the invention.

The gene(s) contained in the expression cassette may be homologous (or endogenous) (i.e. exist naturally in the genome of the manipulated bacterium) or, preferably, heterologous (i.e. do not exist naturally in the genome of the manipulated bacterium). The introduced expression cassette may consist of unmodified, "natural" promoter/gene/operon sequences or engineered expression cassettes in which the promoter region and/or the coding region or both have been altered. A non-exhaustive list of preferred promoters (preferably strong) that could be used for expression includes the promoters *porA, porB, lbpB, tbpB, p110, lst, hpuAB* from *N. meningitidis* or *N. gonorroheae,* the promoters p2, p5, p4, ompf, p1, ompH, p6, hin47 from *H*. *influenzae,* the promoters ompH, ompG, ompCD, ompE, ompB1, ompB2, ompA of *M. catarrhalis,* the promoter λpL, *lac, tac, araB* of *Escherichia coli* or promoters recognized specifically by bacteriophage RNA polymerase such as the *E*. *coli* bacteriophage T7.

The expression cassette may be delivered and integrated in the bacterial chromosome by means of homologous and/or site specific recombination (as discussed in WO 01/09350). Integrative vectors used to deliver such genes and/or operons can be conditionally replicative or suicide plasmids, bacteriophages, transposons or linear DNA fragments obtained by restriction hydrolysis or PCR amplification. Integration is preferably targeted to chromosomal regions dispensable for growth *in vitro.* A non exhaustive list of preferred loci that can be used to target DNA integration includes the *porA, porB, opa, opc, rmp, omp26, lecA, cps, lgtB* genes of *Neisseiria meningitidis* and *Neisseria gonorrhoeae,* the *P1, P5, hmw1*/*2, IgA-protease, fimE* genes of NTHi; the *lecA1, lecA2, omp106, uspA1, uspA2* genes of *Moraxella catarrhalis.* Alternatively, the expression cassette used to modulate the expression of bleb component(s) can be delivered into a bacterium of choice by means of episomal vectors such as circular/linear replicative plasmids, cosmids, phasmids, lysogenic bacteriophages or bacterial artificial chromosomes. Selection of the recombination event can be selected by means of selectable genetic marker such as genes conferring resistance to antibiotics (for instance kanamycin, erythromycin, chloramphenicol, or gentamycin), genes conferring resistance to heavy metals and/or toxic compounds or genes complementing auxotrophic mutations (for instance *pur*, *leu, met, aro*). Blebs may be made from the resulting modified strain.

The expression of some heterologous proteins in bacterial blebs may require the addition of outer-membrane targeting signal(s). The preferred method to solve this problem is by creating a genetic fusion between a heterologous gene and a gene coding for a resident OMP as a specific approach to target recombinant proteins to blebs. Most preferably, the heterologous gene is fused to the signal peptides sequences of such an OMP.

A particularly preferred application of this invention is the introduction of Chlamydia (trachomatis or pneumoniae) protective antigens (preferably outer membrane proteins) into Gram-negative bacterial blebs not from Chlamydia strains. This has several advantages including the fact that such blebs (and vaccines comprising them) are extremely suitable for mucosal administration, which is beneficial as a mucosal (IgA) immune response against the Chlamydia antigens present in the bleb will be more protective against Chlamydia infections which manifest themselves in the mucosa.

### Chlamydia trachomatis antigens integrated into a Gram negative bacterial bleb

A particularly preferred use is in the field of the prophylaxis or treatment of sexually-transmitted diseaseses (STDs). It is often difficult for practitioners to determine whether the principal cause of a STD is due to gonococcus or *Chlamydia trachomatis* infection. These two organisms are major causes of salpingitis - a disease which can lead to sterility in the host. It would be useful if a STD could be vaccinated against or treated with a combined vaccine effective against disease caused by both organisms. The Major Outer Membrane Protein (MOMP or OMP1 or OMPI) of *C*. *trachomatis* has been shown to be the target of protective antibodies. However, the structural integrity of this integral membrane protein is important for inducing such antibodies. In addition, the epitopes recognised by these antibodies are variable and define more than 10 serovars. The bleb context of the invention allows the proper folding of one or more MOMP or other Chlamydia membrane proteins for vaccine purposes. The engineering of (preferably) a gonococcal strain expressing one or more *C. trachomatis* MOMP serovars and/or one or more other protective Chlamydia OMPs in the outer membrane, and the production of blebs therefrom, produces a single solution to the multiple problems of correctly folded membrane proteins, the presentation of sufficient MOMP serovars and/or other Chlamydia OMPs to protect against a wide spectrum of serovars, and the simultaneous prophylaxis/treatment of gonococcal infection (and consequently the non-requirement of practitioners to initially decide which organism is causing particular clinical symptoms - both organisms can be vaccinated against simultaneously thus allowing the treatment of the STD at a very early stage). Preferred loci for gene insertion in the gonoccocal chromosome are give above. Preferred, protective *C*. *trachomatis* genes that could be incorporated are HMWP, PmpG and those OMPs disclosed in WO 99/28475.

A particularly preferrred embodiment of the invention provides a Gram-negative bacterial bleb (preferably gonococcal) presenting on its surface the PorB outer membrane protein (see below) from *Chlamydia trachomatis.*

### PorB Chlamydia trachomatis serovar D (D/UW-3/Cx) DNA sequence

### Translated amino acid sequence

The presence of PorB in the blebs means that the antigen can be mucosally administered more easily, and provides more effective protection than if administered alone.

The present invention additionally provides a Gram-negative bacterial bleb (preferably gonococcal) presenting on its surface one or more of the following proteins from *Chlamydia trachomatis,* or *C*. *trachomatis* PorB in combination with one or more of the following proteins. It will be clear to a skilled person that instead of the sequences below (and the PorB sequence above), the natural analogue of the sequences from other *C*. *trachomatis* serovars or serotypes could be used, as could genes encoding functional analogues of the proteins comprising insertions, deletions or substitutions from the recited sequences which unaffect the immunological properties of the encoded protein. Preferably a sequence from a serovar D strain should be selected. A bacterial strain capable of producing such a bleb is a further aspect of the invention.

The following *Chlamydia trachomatis* outer membrane proteins (full sequences above) are disclosed for the first time as being useful in a *C*. *trachomatis* vaccine.

### Amino acid sequences:

>gi|6578118|gb|AAC68456.2| predicted Protease containing IRBP and DHR domains [Chlamydia trachomatis]
>gi|6578109|gb|AAC68227.2| CHLPN 76kDa Homolog [Chlamydia trachomatis]
>gi|3328866|gb|AAC68034.1| Sulfite Reductase [Chlamydia trachomatis]
>gi|3328815|gb|A.AC67986.1| hypothetical protein [Chlamydia trachomatis]
>gi|3328587|gb|AAC67774.1| CMP-2-keto-3-deoxcyoctulosolfc acid synthetase [Chlamydia trachomatis]
>gi|3329039|gb|AAC68197.1| Thio:disulfide Interchange Protein [Chlamydia trachomatis]
>gi|3329000|gb|AAC68161.1| Yop proteins translocation lipoprotein J [Chlamydia trachomatis]
>gi|3328905|gb|AAC68071.1| hypothetical protein [Chlamydia trachomatis]
>gi|3328884|gb|4AAC68051.1| Phosphatidate Cytidylytransferase [Chlamydia trachomatis]
>gi|3328855|gb|AAC68022.1| hypothetical protein [Chlamydia trachomatis]
>gi|3328772|gb|AAC67946.1| hypothetical protein [Chlamydia trachomatis]
>gi|3328763|gb|AAC67938.1| O-Sialoglycoprotein Endopeptidase family [Chlamydia trachomatis]
>gi|657802|jb|AC67897.2| ATP Synthase Subunit K [Chlamydia trachomatis]
>gi|3329252|gb|AAC68382.1| S 14 Ribosomal Protein [Chlamydia trachomatis]
>gi|3328987|gb|AAC68150.1| hypothetical protein [Chlamydia trachomatis]
>gi|3328972|gb|AAC68136.1| J Apolipoprotein N-Acetyltransferase [Chlamydia trachomatis]
>gi|3328612|gb|AAC67797.1| Fructose-6-P Phosphotransferase [Chlamydia trachomatis]
>gi|3328517|gb|AAC67709.1| hypothetical protein [Chlamydia trachomatis]
>gi|3328482|gb|AAC67677.1| L28 Ribosomal Protein [Chlamydia trachomatis]
>gi|3328436|gb|AAC67635.1| SS DNA Binding Protein [Chlamydia trachomatis]
>gi|3328411|gb|AAC67611.1| hypothetical protein [Chlamydia trachomatis]

Again, when such blebs are present in a vaccine formulation they may be more protective against *Chlamydia trachomatis* infection than the use of the protein in isolation.

Particularly beneficial pairs of *Chlamydia trachomatis* antigens are further invention. This, is a further aspect a Gram-negative bleb (preferably from gonococcus) is provided presenting on its surface both the PorB and PmpG outer membrane proteins from *Chlamydia trachomatis.* Furthermore, a Gram-negative bleb (preferably from gonococcus) is provided presenting on its surface both the PorB and MOMP (from one or more serovars) outer membrane proteins from *Chlamydia trachomatis.* Lastly, a Gram-negative bleb (preferably from gonococcus) is provided presenting on its surface both the PmpG and MOMP (from one or more serovars) outer membrane proteins from *Chlamydia trachomatis.*

By MOMP (or OMP1 or OMP I) from one or more (1, 2, 3, 4, 5, 6, 7, 8, 9, 10 or more) serovars it is preferred that one or more should be selected from a list serovars consisting of: B, Ba, D, Da, E, L1, L2, L2a, F, G, K, L3, A, C, H, L Ia, & J; more preferably from a list consisting of D, E, F, G, K, H, I, & J. Most preferably one or more MOMPs should at least comprise MOMP from serovar D or E (most preferably D). A further preferred strategy is the selection of one or more MOMP from each of the following 3 serogroups: B-serogroup (consisting of serovars B, Ba, D, Da, E, L1, L2 and L2a, and preferably selected from serovars D, Da, & E); F-G-serogroup (consisting of serovars F and G); and C-serogroup (consisting of servars A, C, H, I, Ia, J, K, and L3, and preferably selected from serovars H, I, Ia, J and K).

Most preferably the genes for the *Chlamydia trachomatis* antigens should be inserted at the PorA locus of *Neisseria* (preferably gonococcus).

Such a prepartion formulated as a vaccine may give enhanced protection to a host against *Chlamydia trachomatis* than when a single antigen is administered.

Preferably the bleb has been derived from a strain (preferably gonococcus) which has been modified to upregulate one or more protective outer membrane antigens (as described below).

Preferably the bleb has been derived from a strain (preferably gonococcus) which has been modified to downregulate one or more immunodominant variable or non-protective outer membrane antigens (as described below).

Preferably the blebs are derived from a strain (preferably gonococcus) which has a detoxified lipid A portion of bacterial LPS, due to the strain having been engineered to reduce or switch off expression of one or more genes which cause LPS to be toxic (preferably selected from the following genes, or homologues there of htrB, msbB and lpxK; see section below).

Preferably the blebs are derived from a strain (preferably gonococcus) which has a detoxified lipid A portion of bacterial LPS, due to the strain having been engineered to express at a higher level of one or more genes producing a gene product that is capable of detoxifying LPS (preferably selected from the following genes, or homologues thereof: pmrA, pmrB, pmrE and pmrF; see section below).

Vaccine compositions comprising the bleb of the invention and a pharmaceutically suitable excipient or carrier is also envisaged. Preferably the vaccine additionally comprises a mucosal adjuvant. Mucosal adjuvants are well known in the art (see Vaccine Design "The subunit and adjuvant approach" (eds Powell M.F. & Newman M.J.) (1995) Plenum Press New York). A preferred mucosal adjuvant is LT2 (or LTII, which can be split into LTIIa and LTIIb - see Martin et al. Infection and Immunity, 2000, 68:281-287). Preferably such vaccines should be formulated and administered as described below in "vaccine formulations".

The content of blebs per dose in the vaccine will typically be in the range 1-100µg, preferably 5-50µg, most typically in the range 5 - 25µg.

Optimal amounts of components for a particular vaccine can be ascertained by standard studies involving observation of appropriate immune responses in subjects. Following an initial vaccination, subjects may receive one or several booster immunisations adequately spaced.

A method of manufacturing a medicament for preventing *Chlamydia trachomatis* infection in a host is also provided comprising the steps of administering an effective amount of the above vaccine to a host in need thereof. Preferably the vaccine is mucosally administered via either a intranasal, oral, intradermal or intravaginal route.

### Chlamydia pneumoniae antigens integrated into a Gram negative bacterial bleb

In a further aspect, the invention provides a Gram-negative bleb presenting on its surface a protective antigen from *Chlamydia pneumoniae*. *Neisseria meningitidis*, *Moraxella catharralis*, and *Haemophilus influenzae* are the species for the production of said bleb. Such protective antigens are preferably one or more of those listed below:
1) Cell Envelope: Membrane Proteins, Lipoproteins and Porins

| Gene: | Protein Function: |
|---|---|
| yaeT | OMP85 homolog |
| 60IM | 60 kD inner membrane protein |
| lgt | prolipoprotein diacylglyceryl transferase |
| crpA | CHLTR 15 kD cysteine-rich protein |
| omcB | 60 kD cysteine-rich outer membrane complex protein |
| omcA | 9 kD cysteine-rich outer membrane complex lipoprotein |
| cutE | apolipoprotein N-acetyltransferase |
| ompA | major outer membrane protein |
| pal | peptidoglycan-associated lipoprotein |
| porB | outer membrane protein analog |

2) Coding Genes (Not in C. trachomatis)

| Gene: | Protein Function: |
|---|---|
| yqff | conserved hypothetical inner membrane protein |
| yxjG | hypothetical protein |
| guaA | GMP synthase |
| guaB | inosine 5'-monophosphate dehydrogenase |
| argR | similarity to arginine repressor |
| CPn0232 | similarity to 5'-methylthioadenosine nucleosidase |
| CPn0251 | conserved hypothetical protein |
| CPn0278 | conserved outer membrane lipoprotein protein/a> |
| CPn0279 | possible ABC transporter permease |
| yxjG | hypothetical protein |
| yqeV | hypothetical protein |
| CPn0486 | hypothetical proline permease |
| CPn0505 | 3-methyladenine DNA glycosylase |
| CPn0562 | CHLPS 43 kDa protein |
| CPn0585 | similarity to CHLPS IncA |
| yvyD | conserved hypothetical protein |
| CPn0608 | uridine 5'-monophosphate synthase |
| CPn0735 | uridine kinase |
| CPn0907 | CutA-like periplasmic divalent cation tolerance protein |
| CPn0927 | CHLPS 43 kDa protein |
| CPn0928 | CHLPS 43 kDa protein |
| CPn0929 | CHLPS 43 kDa protein |
| CPn0980 | similar to S. cerevisiae 52.9 kDa protein |
| bioA | adenosylmethionine-8-amino-7-oxononanoate aminotransferase |
| bioD | dethiobiotin synthetase |
| bioB | biotin synthase |
| CPn1045 | conserved hypothetical membrane protein |
| CPn1046 | tryptophan hydroxylase |

3) *Chlamydia*-Specific Proteins

| Gene: | Protein Function: |
|---|---|
| pmp_1 | polymorphic outer membrane protein |
| pmp_2 | polymorphic outer membrane protein |
| pmp_3 | polymorphic outer membrane protein |
| pmp_3 | polymorphic outer membrane protein |
| pmp_4 | polymorphic outer membrane protein - |
| pmp_4 | polymorphic outer membrane protein |
| pmp_5 | polymorphic outer membrane protein |
| pmp_5 | polymorphic outer membrane protein |
| CPn0133 | CHLPS hypothetical protein |
| CPn0186 | similarity to IncA |
| incB | inclusion membrane protein B |
| incC | inclusion membrane protein C |
| CPn0332 | CHLTR T2 protein |
| ltuB | LtuB protein |
| pmp_6 | polymorphic outer membrane protein |
| pmp_7 | polymorphic outer membrane protein |
| pmp_8 | polymorphic outer membrane protein |
| pmp_9 | polymorphic outer membrane protein |
| pmp_10 | polymorphic outer membrane protein |
| pmp_10 | polymorphic outer membrane protein |
| pmp_11 | polymorphic outer membrane protein |
| pmp_12 | polymorphic outer membrane protein |
| pmp_13 | polymorphic outer membrane protein |
| pmp_14 | polymorphic outer membrane protein |
| pmp_15 | polymorphic outer membrane protein |
| pmp_16 | polymorphic outer membrane protein |
| pmp_17 | polymorphic outer membrane protein |
| pmp_17 | polymorphic outer membrane protein |
| pmp_17 | polymorphic outer membrane protein |
| pmp_18 | polymorphic outer membrane protein |
| pmp_19 | polymorphic outer membrane protein |
| pmp_20 | polymorphic outer membrane protein |
| euo | CHLPS Euo protein |
| CPn0562 | CHLPS 43 kDa protein homolog |
| CPn0585 | similar to CHLPS inclusion membrane protein A |
| CPn0728 | CHLPN 76 kDa protein homolog |
| CPn0729 | CHLPN 76 kDa protein homolog |
| gp6D | CHLTR plasmid protein |
| CPn0927 | CHLPS 43 kDa protein homolog |
| CPn0928 | CHLPS 43 kDa protein homolog |
| CPn0929 | CHLPS 43 kDa protein homolog |
| pmp_21 | polymorphic outer membrane protein |
| ItuA | LtuA protein |

(Full sequence information has been published at the Chlamydia Genome Project web site: http://chlamydia-www.berkeley.edu:4231/index.html).

**Additional Chlamydia genes, and encoded proteins, suitable for expression in a Gram-negative bacteria for OMV vaccine preparation:**

| | | | |
|---|---|---|---|
| **Chlamydia pneumoniae 98kD putative outer membrane protein gene.** | | | **WO200026237** |
| | | | **-A2** |
| | Patent Inventors | *DUNN PL* | |
| | | *OOMEN RP* | |
| | | *MURDIN AD* | |
| **Chlamydia POMP91B precursor gene.** | | | **WO200026239** |
| | | | **-A2** |
| | Patent Inventors | *DUNN PL* | |
| | | *OOMEN RP* | |
| | | *MURDIN AD* | |

| | | | |
|---|---|---|---|
| **Chlamydia antigen CPN100634 full length coding sequence.** | | | **WO200032794** |
| | | | **-A2** |
| | Patent Inventors | *OOMEN RP* | |
| | | *WANG J* | |
| | | *MURDIN AD* | |
| **Chlamydia antigen CPN100634 gene open reading frame.** | | | **WO200032794** |
| | | | **-A2** |
| | Patent Inventors | *OOMEN RP* | |
| | | *WANG J* | |
| | | *MURDIN AD* | |
| **Chlamydia antigen CPN100635 full length coding sequence.** | | | **WO200032794** |
| | | | **-A2** |
| | Patent Inventors | *OOMEN RP* | |
| | | *WANG J* | |
| | | *MURDIN AD* | |
| **Chlamydia antigen CPN100635 gene open reading frame.** | | | **WO200032794** |
| | | | **-A2** |
| | Patent Inventors | *OOMEN RP* | |
| | | *WANG J* | |
| | | *MURDIN AD* | |
| **Chlamydia antigen CPN100638 full length coding sequence.** | | | **WO200032794** |
| | | | **-A2** |
| | Patent Inventors | *OOMEN RP* | |
| | | *WANG J* | |
| | | *MURDIN AD* | |
| **Chlamydia antigen CPN100638 gene open reading frame.** | | | **WO200032794** |
| | | | **-A2** |
| | Patent Inventors | *OOMEN RP* | |
| | | *WANG J* | |
| | | *MURDIN AD* | |
| **Chlamydia antigen CPN100639 full length coding sequence.** | | | **WO200032794** |
| | | | **-A2** |
| | Patent Inventors | *OOMEN RP* | |
| | | *WANG J* | |
| | | *MURDIN AD* | |
| **Chlamydia antigen CPN100639 gene open reading frame.** | | | **WO200032794** |
| | | | **-A2** |
| | Patent Inventors | *OOMEN RP* | |
| | | *WANG J* | |
| | | *MURDIN AD* | |
| **Chlamydia antigen CPN100708 full length coding sequence.** | | | **WO200032794** |
| | | | **-A2** |
| | Patent Inventors | *OOMEN RP* | |
| | | *WANG J* | |
| | | *MURDIN AD* | |
| **Chlamydia antigen CPN100708 gene open reading frame.** | | | **WO200032794** |
| | | | **-A2** |
| | Patent Inventors | *OOMEN RP* | |
| | | *WANG J* | |
| | | *MURDIN AD* | |
| **C. pneumoniae ATP/ADP translocase coding sequence.** | | | **WO200039157** |
| | | | **-A1** |
| | Patent Inventors | *DUNN P* | |
| | | *OOMEN RP* | |
| | | *WANG J* | |
| | | *MURDIN AD* | |
| **Chlamydia pneumoniae 98 kDa outer membrane protein CPN100640 gene.** | | | **WO200032784** |
| | | | **-A1** |
| | Patent Inventors | *DUNN P* | |

| | | | |
|---|---|---|---|
| | | *OOMEN RP* | |
| | | *WANG J* | |
| | | *MURDIN AD* | |
| **Chlamydia pneumoniae 98 kDa outer membrane protein coding region.** | | | **WO200032784** |
| | | | **-A1** |
| | Patent Inventors | *DUNN P* | |
| | | *OOMEN RP* | |
| | | *WANG J* | |
| | | *MURDIN AD* | |
| **DNA encoding a 9 kDa cysteine-rich membrane protein.** | | | **WO200053764** |
| | | | **-A1** |
| | Patent Inventors | *DUNN P* | |
| | | *OOMEN RP* | |
| | | *WANG J* | |
| | | *MURDIN AD* | |
| **DNA encoding a 60 kDa cysteine-rich membrane protein.** | | | **WO200055326** |
| | | | **-A1** |
| | Patent Inventors | *DUNN P* | |
| | | *OOMEN RP* | |
| | | *WANG J* | |
| | | *MURDIN AD* | |
| **A 9 kDa cysteine-rich membrane protein.** | | | **WO200053764** |
| | | | **-A1** |
| | Patent Inventors | *DUNN P* | |
| | | *OOMEN RP* | |
| | | *WANG J* | |
| | | *MURDIN AD* | |
| **A 60 kDa cysteine-rich membrane protein of Chlamydia pneumoniae.** | | | **WO200055326** |
| | | | **-A1** |
| | Patent Inventors | *DUNN P* | |
| | | *OOMEN RP* | |
| | | *WANG J* | |
| | | *MURDIN AD* | |
| **C. pneumoniae mip (outer membrane protein).** | | | **WO200006741** |
| | | | **-A1** |
| | Patent Inventors | *DUNN PL* | |
| | | *OOMEN RP* | |
| | | *MURDIN AD* | |
| **C. pneumoniae mip (outer membrane protein) truncated protein.** | | | **WO200006741** |
| | | | **-A1** |
| | Patent Inventors | *DUNN PL* | |
| | | *OOMEN RP* | |
| | | *MURDIN AD* | |
| **C. pneumoniae omp protein sequence.** | | | **WO200006743** |
| | | | **-A2** |
| | Patent Inventors | *DUNN PL* | |
| | | *OOMEN RP* | |
| | | *MURDIN AD* | |
| **C. pneumoniae omp protein truncated sequence.** | | | **WO200006743** |
| | | | **-A2** |
| | Patent Inventors | *DUNN PL* | |
| | | *OOMEN RP* | |
| | | *MURDIN AD* | |
| **Amino acid sequence of the CPN100111 polypeptide.** | | | **WO200011183** |
| | | | **-A2** |
| | Patent Inventors | *OOMEN RP* | |
| | | *MURDIN AD* | |
| **Amino acid sequence of the CPN100224 polypeptide.** | | | **WO200011183** |
| | | | **-A2** |

| | | | |
|---|---|---|---|
| | Patent Inventors | *OOMEN RP* | |
| | | *MURDIN AD* | |
| **Amino acid sequence of the CPN100230 polypeptide.** | | | **WO200011183** |
| | | | **-A2** |
| | Patent Inventors | *OOMEN RP* | |
| | | *MURDIN AD* | |
| **Amino acid sequence of the CPN100231 polypeptide.** | | | **WO200011183** |
| | | | **-A2** |
| | Patent Inventors | *OOMEN RP* | |
| | | *MURDIN AD* | |
| **Amino acid sequence of the CPN100232 polypeptide.** | | | **WO200011183** |
| | | | **-A2** |
| | Patent Inventors | *OOMEN RP* | |
| | | *MURDIN AD* | |
| **Amino acid sequence of the CPN100233 polypeptide.** | | | **WO200011183** |
| | | | **-A2** |
| | Patent Inventors | *OOMEN RP* | |
| | | *MURDIN AD* | |
| **Amino acid sequence of the CPN100394 polypeptide.** | | | **WO200011183** |
| | | | **-A2** |
| | Patent Inventors | *OOMEN RP* | |
| | | *MURDIN AD* | |
| **Amino acid sequence of the CPN100395 polypeptide.** | | | **WO200011183** |
| | | | **-A2** |
| | Patent Inventors | *OOMEN RP* | |
| | | *MURDIN AD* | |
| **Amino acid sequence of the POMP91A protein of Chlamydia pneumoniae.** | | | **WO200011180** |
| | | | **-A1** |
| | Patent Inventors | *DUNN PL* | |
| | | *OOMEN RP* | |
| | | *MURDIN AD* | |
| **Chlamydia pneumoniae antigen CPN100202 protein sequence.** | | | **WO200006739** |
| | | | **-A2** |
| | Patent Inventors | *OOMEN RP* | |
| | | *MURDIN AD* | |
| **Chlamydia pneumoniae antigen CPN100149 protein SEQ ID NO:2.** | | | **WO200006740** |
| | | | **-A1** |
| | Patent Inventors | *OOMEN RP* | |
| | | *MURDIN AD* | |
| **Chlamydia pneumoniae antigen CPN100605 protein SEQ ID NO:2.** | | | **WO200006742** |
| | | | **-A2** |
| | Patent Inventors | *OOMEN RP* | |
| | | *MURDIN AD* | |
| **Chlamydia antigen CPN100634.** | | | **WO200032794** |
| | | | **-A2** |
| | Patent Inventors | *OOMEN RP* | |
| | | *WANG J* | |
| | | *MURDIN AD* | |
| **Chlamydia antigen CPN100635.** | | | **WO200032794** |
| | | | **-A2** |
| | Patent Inventors | *OOMEN RP* | |
| | | *WANG J* | |
| | | *MURDIN AD* | |
| **Mature Chlamydia antigen CPN100635.** | | | **WO200032794** |
| | | | **-A2** |
| | Patent Inventors | *OOMEN RP* | |
| | | *WANG J* | |
| | | *MURDIN AD* | |
| **Chlamydia antigen CPN100638.** | | | **WO200032794** |

| | | | |
|---|---|---|---|
| | | | **-A2** |
| | Patent Inventors | *OOMEN RP* | |
| | | *WANG J* | |
| | | *MURDlN AD* | |
| **Chlamydia antigen CPN100639.** | | | **WO200032794** |
| | | | **-A2** |
| | Patent Inventors | *OOMEN RP* | |
| | | *WANG J* | |
| | | *MURDlN AD* | |
| **Chlamydia antigen CPN100708.** | | | **WO200032794** |
| | | | **-A2** |
| | Patent Inventors | *OOMEN RP* | |
| | | *WANG J* | |
| | | *MURDIN AD* | |
| **C. pneumoniae ATP/ADP translocase protein sequence.** | | | **WO200039157** |
| | | | **-A1** |
| | Patent Inventors | *DUNN P* | |
| | | *OOMEN RP* | |
| | | *WANG J* | |
| | | *MURDIN AD* | |
| **Chlamydia pneumoniae 98kD putative outer membrane protein.** | | | **WO200026237** |
| | | | **-A2** |
| | Patent Inventors | *DUNN PL* | |
| | | *OOMEN RP* | |
| | | *MURDIN AD* | |
| **Chlamydia POMP91B precursor protein.** | | | **WO200026239** |
| | | | **-A2** |
| | Patent Inventors | *DUNN PL* | |
| | | *OOMEN RP* | |
| | | *MURDIN AD* | |
| **Chlamydia pneumoniae 98 kDa outer membrane protein CPN100640.** | | | **WO200032784** |
| | | | **-A1** |
| | Patent Inventors | *DUNN P* | |
| | | *OOMEN RP* | |
| | | *WANG J* | |
| | | *MURDIN AD* | |
| **Chlamydia pneumoniae processed 98 kDa outer membrane protein CPN100640.** | | | **WO200032784** |
| | | | **-A1** |
| | Patent Inventors | *DUNN P* | |
| | | *OOMEN RP* | |
| | | *WANG J* | |
| | | *MURDIN AD* | |
| **C. pneumoniae mip (outer membrane protein) encoding DNA.** | | | **WO200006741** |
| | | | **-A1** |
| | Patent Inventors | *DUNN PL* | |
| | | *OOMEN RP* | |
| | | *MURDIN AD* | |
| **C. pneumoniae omp protein encoding DNA.** | | | **WO200006743** |
| | | | **-A2** |
| | Patent Inventors | *DUNN PL* | |
| | | *OOMEN RP* | |
| | | *MURDIN AD* | |
| **DNA encoding the CPN100111 polypeptide.** | | | **WO200011183** |
| | | | **-A2** |
| | Patent Inventors | *OOMEN RP* | |
| | | *MURDIN AD* | |
| **DNA encoding the CPNI00224 polypeptide.** | | | **WO200011183** |
| | | | **-A2** |
| | Patent Inventors | *OOMEN RP* | |

| | | | |
|---|---|---|---|
| | | *MURDIN AD* | |
| **DNA encoding the CPN100230 polypeptide.** | | | **WO200011183** |
| | | | **-A2** |
| | Patent Inventors | *OOMEN RP* | |
| | | *MURDIN AD* | |
| **DNA encoding the CPN100231 polypeptide.** | | | **WO200011183** |
| | | | **-A2** |
| | Patent Inventors | *OOMEN RP* | |
| | | *MURDIN AD* | |
| **DNA encoding the CPN100232 polypeptide.** | | | **WO200011183** |
| | | | **-A2** |
| | Patent Inventors | *OOMEN RP* | |
| | | *MURDIN AD* | |
| **DNA encoding the CPN100233 polypeptide.** | | | **WO200011183** |
| | | | **-A2** |
| | Patent Inventors | *OOMEN RP* | |
| | | *MURDIN AD* | |
| **DNA encoding the CPN100394 polypeptide.** | | | **WO200011183** |
| | | | **-A2** |
| | Patent Inventors | *OOMEN RP* | |
| | | *MURDIN AD* | |
| **DNA encoding the CPN100395 polypeptide.** | | | **WO200011183** |
| | | | **-A2** |
| | Patent Inventors | *OOMEN RP* | |
| | | *MURDIN AD* | |
| **Nucleotide sequence of the POMP91A gene of Chlamydia pneumoniae.** | | | **WO200011180** |
| | | | **-A1** |
| | Patent Inventors | *DUNN PL* | |
| | | *OOMEN RP* | |
| | | *MURDIN AD* | |
| **Chlamydia pneumoniae antigen CPN100202 nucleotide sequence.** | | | **WO200006739** |
| | | | **-A2** |
| | Patent Inventors | *OOMEN RP* | |
| | | *MURDIN AD* | |
| **Chlamydia pneumoniae antigen CPN100149 protein encoding DNA SEQ ID NO:1.** | | | **WO200006740** |
| | | | **-A1** |
| | Patent Inventors | *OOMEN RP* | |
| | | *MURDIN AD* | |
| **Chlamydia pneumoniae antigen CPN100605 protein encoding DNA SEQ ID NO:1.** | | | **WO200006742** |
| | | | **-A2** |
| | Patent Inventors | *OOMEN RP* | |
| | | *MURDIN AD* | |

When such blebs are present in a vaccine formulation they may be more protective against *Chlamydia pneumoniae* infection than the use of the protein/antigen in isolation.

Particularly beneficial pairs of *Chlamydia pneumoniae* antigens have also been found. Thus in a further aspect a Gram-negative bleb (preferably from meningococcus) is provided presenting on its surface both the PorB and MOMP outer membrane proteins from *Chlamydia pneumoniae.* Furthermore, a Gram-negative bleb (preferably from meningococcus) is provided presenting on its surface both MOMP and one or more Pmp outer membrane proteins from *Chlamydia pneumoniae.* A Gram-negative bleb (preferably from meningococcus) is additionally provided presenting on its surface both the PorB and one or more Pmp outer membrane proteins from *Chlamydia pneumoniae.* A Gram-negative bleb (preferably from meningococcus) is also provided presenting on its surface both the PorB and Npt1 proteins from *Chlamydia pneumoniae.* A Gram-negative bleb (preferably from meningococcus) is additionally provided presenting on its surface both the Npt1 and one or more Pmp proteins from *Chlamydia pneumoniae.* Lastly, a Gram-negative bleb (preferably from meningococcus) is provided presenting on its surface both the Npt1 and MOMP proteins from *Chlamydia pneumoniae.*

Such prepartions formulated as a vaccine can give enhanced protection to a host against *Chlamydia* than when a single antigen is administered.

Preferably the bleb has been derived from a strain which has been modified to upregulate one or more protective outer membrane antigens (see below; for instance for meningocococcal protective outer membrane antigens see section "Neisserial bleb preparations" for those antigens that should preferably be upregulated).

Preferably the bleb has been derived from a strain which has been modified to downregulate one or more immunodominant variable or non-protective outer membrane antigens (as described below; for instance for meningocococcal variable/non-protective outer membrane antigens see section "Neisserial bleb preparations" for those antigens that should preferably be downregulated).

Preferably the blebs are derived from a strain which has a detoxified lipid A portion of bacterial LPS, due to the strain having been engineered to reduce or switch off expression of one or more genes which cause LPS to be toxic (preferably selected from the following genes, or homologues thereof htrB, msbB and lpxK; see section below).

Preferably the blebs are derived from a strain which has a detoxified lipid A portion of bacterial LPS, due to the strain having been engineered to express at a higher level of one or more genes producing a gene product that is capable of detoxifying LPS (preferably selected from the following genes, or homologues thereof pmrA, pmrB, pmrE and pmrF; see section below).

Vaccine compositions comprising the bleb of the invention and a pharmaceutically suitable excipient or carrier are also envisaged. Preferably the vaccine additionally comprising a mucosal adjuvant. Mucosal adjuvants are well known in the art (see Vaccine Design "The subunit and adjuvant approach" (eds Powell M.F. & Newman M.J.) (1995) Plenum Press New York). A preferred mucosal adjuvant is LT2 (or LTII, which can be split into LTIIa and LTIIb - see Martin et al. Infection and Immunity, 2000, 68:281-287). Preferably such vaccines should be formulated and administered as described below in "Vaccine formulations".

The content of blebs per dose in the vaccine will typically be in the range 1-100µg, preferably 5-50µg, most typically in the range 5 - 25µg.

Optimal amounts of components for a particular vaccine can be ascertained by standard studies involving observation of appropriate immune responses in subj ects. Following an initial vaccination, subjects may receive one or several booster immunisations adequately spaced.

The efficacy of a *C*. *pneumoniae* vaccine can be evaluated in a mouse model of infection such as the one described by Murdin et al., 2000, J. Infect. Dis. 181 (suppl 3):S5444-51. The protection elicited by a vaccine formulation can be assessed by reduction of the bacterial load in the lung after a challenge infection with C. *pneumoniae.*

A method of manufacturing a medicament for preventing *Chlamydia pneumoniae* infection in a host is also provided comprising the steps of administering an effective amount of the above vaccine to a host in need thereof. Preferably the vaccine is mucosally administered via either an intranasal, intradermal or oral route.

### Further improvements in the bacteria and blebs of the invention

The Gram-negative bacterium may be further genetically engineered by one or more processes selected from the following group: (a) a process of down-regulating expression of immunodominant variable or non-protective antigens, (b) a process of upregulating expression of protective OMP antigens, (c) a process of down-regulating a gene involved in rendering the lipid A portion of LPS toxic, (d) a process of upregulating a gene involved in rendering the lipid A portion of LPS less toxic, and (e) a process of down-regulating synthesis of an antigen which shares a structural similarity with a human structure and may be capable of inducing an auto-immune response in humans. These processes are described in detail in WO 01/09350.

Such bleb vaccines of the invention are designed to focus the immune response on a few protective (preferably conserved) antigens or epitopes - formulated in a multiple component vaccine. Where such antigens are integral OMPs, the outer membrane vesicles of bleb vaccines will ensure their proper folding. This invention provides methods to optimize the OMP and LPS composition of OMV (bleb) vaccines by deleting immunodominant variable as well as non protective OMPs, by creating conserved OMPs by deletion of variable regions, by upregulating expression of protective OMPs, and by eliminating control mechanisms for expression (such as iron restriction) of protective OMPs. In addition the reduction in toxicity of lipid A by modification of the lipid portion or by changing the phosphoryl composition whilst retaining its adjuvant activity or by masking it is provided. Each of these new methods of improvement individually improve the bleb vaccine, however a combination of one or more of these methods work in conjunction so as to produce an optimised engineered bleb vaccine which is immuno-protective and non-toxic - particularly suitable for paediatric use.

### (a) a process of down-regulating expression of immunodominant variable or non-protective antigens

Many surface antigens are variable among bacterial strains and as a consequence are protective only against a limited set of closely related strains. The reduction in expression, or, preferably, the deletion of the gene(s) encoding variable surface protein(s) which results in a bacterial strain producing blebs which, when administered in a vaccine, have a stronger potential for cross-reactivity against various strains due to a higher influence exerted by conserved proteins (retained on the outer membranes) on the vaccinee's immune system is described. Examples of such variable antigens include: for *Neisseria -* pili (PilC) which undergoes antigenic variations, PorA, Opa, TbpB, FrpB; for *H*. *influenzae -* P2, P5, pilin, IgA1-protease; and for *Moraxella -* CopB, OMP106.

Other types of gene that could be down-regulated or switched off are genes which, *in vivo,* can easily be switched on (expressed) or off by the bacterium. As outer membrane proteins encoded by such genes are not always present on the bacteria, the presence of such proteins in the bleb preparations can also be detrimental to the effectiveness of the vaccine for the reasons stated above. A preferred example to down-regulate or delete is *Neisseria* Opc protein. Anti-Opc immunity induced by an Opc containing bleb vaccine would only have limited protective capacity as the infecting organism could easily become Opc⁻- *H. influenzae* HgpA and HgpB are other examples of such proteins.

In process a), these variable or non-protective genes are down-regulated in expression, or terminally switched off. This has the surprising advantage of concentrating the immune system on better antigens that are present in low amounts on the outer surface of blebs.

The strain can be engineered in this way by a number of strategies including transposon insertion to disrupt the coding region or promoter region of the gene, or point mutations or deletions to achieve a similar result. Homologous recombination may also be used to delete a gene from a chromosome (where sequence X comprises part (preferably all) of the coding sequence of the gene of interest). It may additionally be used to change its strong promoter for a weaker (or no) promoter. All these techniques are described in WO 01/09350.

### (b) a process of upregulating expression of protective OMP antigens

This may be done by inserting a copy of such a protective OMP into the genome (preferably by homologous recombination), or by upregulating expression of the native gene by replacing the native promoter for a stronger promoter, or inserting a strong promoter upstream of the gene in question (also by homologous recombination). Such methods can be accomplished using the techniques described in WO 01/09350.

Such methods are particularly useful for enhancing the production of immunologically relevant Bleb components such as outer-membrane proteins and lipoproteins (preferably conserved OMPs, usually present in blebs at low concentrations).

### (c) a process of down-regulating a gene involved in rendering the lipid A portion of LPS toxic

The toxicity of bleb vaccines presents one of the largest problems in the use of blebs in vaccines. A method of genetically detoxifying the LPS present in Blebs is disclosed. Lipid A is the primary component of LPS responsible for cell activation. Many mutations in genes involved in this pathway lead to essential phenotypes. However, mutations in the genes responsible for the terminal modifications steps lead to temperature-sensitive (*htrB*) or permissive (*msbB*) phenotypes. Mutations resulting in a decreased (or no) expression of these genes result in altered toxic activity of lipid A. Indeed, the non-lauroylated (htrB mutant) [also defined by the resulting LPS lacking both secondary acyl chains] or non-myristoylated (msbB mutant) [also defined by the resulting LPS lacking only a single secondary acyl chain] lipid A are less toxic than the wild-type lipid A. Mutations in the lipid A 4'-kinase encoding gene (*lpxk*) also decreases the toxic activity of lipid A.

Process c) thus involves either the deletion of part (or preferably all) of one or more of the above open reading frames or promoters. Alternatively, the promoters could be replaced with weaker promoters. Preferably the homologous recombination techniques are used to carry out the process. Preferably the methods described in WO 01/09350 are used. The sequences of the htrB and msbB genes from *Neisseria meningitidis B, Moraxella catarrhalis,* and *Haemophilus influenzae* are provided in WO Ol/09350 for this purpose.

### (d) a process of upregulating a gene involved in rendering the lipid A portion of LPS less toxic

LPS toxic activity could also be altered by introducing mutations in genes/loci involved in polymyxin B resistance (such resistance has been correlated with addition of aminoarabinose on the 4' phosphate of lipid A). These genes/loci could be *pmrE* that encodes a UDP-glucose dehydrogenase, or a region of antimicrobial peptide-resistance genes common to many enterobacteriaciae which could be involved in aminoarabinose synthesis and transfer. The gene *pmrF* that is present in this region encodes a dolico1-phosphate manosyl transferase (Gunn J.S., Kheng, B.L., Krueger J., Kim K.,Guo L., Hackett M., Miller S.I. 1998. *Mol. Microbiol.* 27: 1171-1182).

Mutations in the PhoP-PhoQ regulatory system, which is a phospho-relay two component regulatory system (f. i. PhoP constitutive phenotype, PhoP^{c}), or low Mg⁺⁺, environmental or culture conditions (that activate the PhoP-PhoQ regulatory system) lead to the addition of aminoarabinose on the 4'-phosphate and 2-hydroxymyristate replacing myristate (hydroxylation of myristate). This modified lipid A displays reduced ability to stimulate E-selectin expression by human endothelial cells and TNF-α secretion from human monocytes.

Process d) involves the upregulation of these genes using a strategy as described in WO 01/09350.

### (e) a process of down-regulating synthesis of an antigen which shares a structural similarity with a human structure and may be capable of inducing an auto-immune response in humans

The isolation of bacterial outer-membrane blebs from encapsulated Gram-negative bacteria often results in the co-purification of capsular polysaccharide. In some cases, this "contaminant" material may prove useful since polysaccharide may enhance the immune response conferred by other bleb components. In other cases however, the presence of contaminating polysaccharide material in bacterial bleb preparations may prove detrimental to the use of the blebs in a vaccine. For instance, it has been shown at least in the case of *N. meningitidis* that the serogroup B capsular polysaccharide does not confer protective immunity and is susceptible to induce an adverse auto-immune response in humans. Consequently, process e) is the engineering of the bacterial strain for bleb production such that it is free of capsular polysaccharide. The blebs will then be suitable for use in humans. A particularly preferred example of such a bleb preparation is one from *N. meningitidis* serogroup B devoid of capsular polysaccharide.

This may be achieved by using modified bleb production strains in which the genes necessary for capsular biosynthesis and/or export have been impaired as described in WO 01/09350. A preferred method is the deletion of some or all of the *Neisseria meningitidis cps* genes required for polysaccharide biosynthesis and export. For this purpose, the replacement plasmid pMF121 (described in Frosh et al. 1990, *Mol*. *Microbiol.* 4:1215-1218) can be used to deliver a mutation deleting the *c̅psCAD* (+ *galE*) gene cluster. Alternatively the *siaD* gene could be deleted, or down-regulated in expression (the meningococcal *siaD* gene encodes alpha-2,3-sialyltransferase, an enzyme required for capsular polysaccharide and LOS synthesis). Such mutations may also remove host-similar structures on the saccharide portion of the LPS of the bacteria.

### Combinations of methods a) - e)

It may be appreciated that one or more of the above processes may be used to produce a modified strain from which to make improved bleb preparations of the invention. Preferably one such process is used, more preferably two or more (2, 3, 4, or 5) of the processes are used in order to manufacture the bleb vaccine. As each additional method is used in the manufacture of the bleb vaccine, each improvement works in conjunction with the other methods used in order to make an optimised engineered bleb preparation.

A preferred meningococcal (particularly *N. meningitidis* B) bleb preparation comprises the use of processes b), c) and e) (optionally combined with process a)). Such bleb preparations are safe (no structures similar to host structures), non-toxic, and structured such that the host immune response will be focused on high levels of protective (and preferably conserved) antigens. All the above elements work together in order to provide an optimised bleb vaccine.

Similarly for *M catarrhalis*, non-typeable *H*. *influenzae*, gonococcus, and non serotype B meningococcal strains (e.g. serotype A, C, Y or W), preferred bleb preparations comprise the use of processes b) and c), optionally combined with process a).

### Preferred Neisserial bleb preparations

One or more of the following genes (encoding protective antigens) are preferred for upregulation via process b) when carried out on a Neisserial strain, including gonococcus, and meningococcus (particularly *N*. *meningitidis* B): NspA (WO 96/29412), Hsf-like (WO 99/31132), Hap (PCT/EP99/02766), PorA, PorB, OMP85 (WO 00/23595), PilQ (PCT/EP99/03603), PldA (PCTlEP99/06718), FipB (WO 96/31618), TbpA (US 5,912,336), TbpB, FrpA/FrpC (WO 92/01460), LbpA,/LbpB (PCT/EP98/05117), FhaB (WO 98/02547), HasR (PCT/EP99/05989), lipo02 (PCT/EP99/08315), Tbp2 (WO 99/57280), MltA (WO 99/57280), and ctrA (PCT/EP00/00135). They are also preferred as genes which may be heterologously introduced into other Gram-negative bacteria.

One or more of the following genes are preferred for downregulation via process a): PorA, PorB, PilC, TbpA, TbpB, LbpA, LbpB, Opa, and Opc (most preferably PorA).

One or more of the following genes are preferred for downregulation via process c): htrB, msbB and lpxK (most preferably msbB which removes only a single secondary acyl chain from the LPS molecule).

One or more of the following genes are preferred for upregulation via process d): pmrA, pmrB, pmrE, and pmrF.

One or more of the following genes are preferred for downregulation via process e): galE, siaA, siaB, siaC, siaD,'" ctrA, ctrB, ctrC, and ctrD (the genes are described in described in WO 01/09350).

Many of the above open reading frames and upstream regions are described in WO 01/09350.

Preferred gonococcal genes to upregulate via process b) include one or more of the following:

Preferred gonococcal genes to downregulate via process a) include one or more of the following:

### Preferred Pseudomonas aeruginosa bleb preparations

One or more of the following genes (encoding protective antigens) are preferred for upregulation via process b): PcrV, OprF, OprI. They are also preferred as genes which may be heterologously introduced into other Gram-negative bacteria.

### Preferred Moraxella catarrhalis bleb preparations

One or more of the following genes (encoding protective antigens) are preferred for upregulation via process b): OMP106 (WO 97/41731 & WO 96/34960), HasR (PCT/EP99/03824), PilQ (PCT/EP99/03823), OMP85 (PCT/EP00/01468), lipo06 (GB 9917977.2), lipo10 (GB 9918208.1), lipoll (GB 9918302.2), lipo18 (GB 9918038.2), P6 (PCT/EP99/03038), ompCD, CopB (Helminen ME, et al (1993) Infect. Immun. 61:2003-2010), D15 (PCT/EP99/03822), OmplA1 (PCT/EP99/06781), Hly3 (PCT/EP99/03257), LbpA and LbpB (WO 98/55606), TbpA and TbpB (WO 97/13785 & WO 97/32980), OmpE, UspA1 and UspA2 (WO 93/03761), FhaB (WO 99/58685) and Omp21. They are also preferred as genes which may be heterologously introduced into other Gram-negative bacteria.

One or more of the following genes are preferred for downregulation via process a): CopB, OMP106, OmpB1, TbpA, TbpB, LbpA, and LbpB.

One or more of the following genes are preferred for downregulation via process c): htrB, msbB and lpxK (most preferably msbB).

One or more of the following genes are preferred for upregulation via process d): pmrA, pmrB, pmrE, and pmrF.

Many of the above open reading frames and upstream regions are described in WO 01/09350.

### Preferred Haemophilus influenzae bleb preparations

One or more of the following genes (encoding protective antigens) are preferred for upregulation via process b): D15 (WO 94/12641), P6 (EP 281673), TbpA, TbpB, P2, P5 (WO 94/26304), OMP26 (WO 97/01638), HMW1, HMW2, HMW3, HMW4, Hia, Hsf, Hap, Hin47, Iomp1457 (GB 0025493.8), YtfN (GB 0025488.8), VirG (GB 0026002.6), Iomp1681 (GB 0025998.6), OstA (GB 0025486.2) and Hif (all genes in this operon should be upregulated in order to upregulate pilin). They are also preferred as genes which may be heterologously introduced into other Gram-negative bacteria.

One or more of the following genes are preferred for downregulation via process a): P2, P5, Hif, IgA1-protease, HgpA; HgpB, HMW1, HMW2, Hxu, TbpA, and TbpB.

One or more of the following genes are preferred for downregulation via process c): htrB, msbB and lpxK (most preferably msbB).

One or more of the following genes are preferred for upregulation via process d): pmrA, pmrB, pmrE, and pmrF.

Many of the above open reading frames and upstream regions are described in WO 01/09350.

### Preparations of membrane vesicles (blebs) of the invention

The manufacture of bleb preparations from any of the aforementioned modified strains may be achieved by harvesting blebs naturally shed by the bacteria, or by any of the methods well known to a skilled person (e.g. as disclosed in EP 301992, US 5,597,572, EP 11243 or US 4,271,147). For Neisseria, the method described in the Example below is preferably used

Preferably, the preparation of membrane vesicles is capable of being filtered through a 0.22 µm membrane.

A sterile (preferably homogeneous) preparation of membrane vesicles obtainable by passing the membrane vesicles through a 0.22 µm membrane is also envisaged.

### Vaccine Formulations

A preferred embodiment of the invention is the formulation of the bleb preparations of the invention in a vaccine which may also comprise a pharmaceutically acceptable excipient.

Vaccine preparation is generally described in Vaccine Design ("The subunit and adjuvant approach" (eds Powell M.F. & Newman M.J.) (1995) Plenum Press New York).

The bleb preparations of the present invention may be adjuvanted in the vaccine formulation of the invention. Suitable adjuvants include an aluminium salt such as aluminum hydroxide gel (alum) or aluminium phosphate, but may also be a salt of calcium (particularly calcium carbonate), iron or zinc, or may be an insoluble suspension of acylated tyrosine, or acylated sugars, cationically or anionically derivatised polysaccharides, or polyphosphazenes.

Suitable Thl adjuvant systems that may be used include, Monophosphoryl lipid A, particularly 3-de-O-acylated monophosphoryl lipid A, and a combination of monophosphoryl lipid A, preferably 3-de-O-acylated monophosphoryl lipid A (3D-MPL) together with an aluminium salt. An enhanced system involves the combination of a monophosphoryl lipid A and a saponin derivative particularly the combination of QS21 and 3D-MPL as disclosed in WO 94/00153, or a less reactogenic composition where the QS21 is quenched with cholesterol as disclosed in WO96/33739. A particularly potent adjuvant formulation involving QS21 3D-MPL and tocopherol in an oil in water emulsion is described in WO95/17210 and is a preferred formulation.

The vaccine may comprise a saponin, more preferably QS21. It may also comprise an oil in water emulsion and tocopherol. Unmethylated CpG containing oligo nucleotides (WO 96/02555) are also preferential inducers of a TH1 response and are suitable for use in the present invention.

The vaccine preparation of the present invention may be used to protect or treat a mammal susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts.

The amount of antigen in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-100µg of protein antigen, preferably 5-50µg, and most typically in the range 5 - 25µg.

An optimal amount for a particular vaccine can be ascertained by standard studies involving observation of appropriate immune responses in subjects. Following an initial vaccination, subjects may receive one or several booster immunisations adequately spaced.

### Ghost or Killed Whole cell vaccines

The inventors envisage that the above modified bacterial strains may not only be useful in generating bleb preparations useful in vaccines - they may also be easily used to make ghost or killed whole cell preparations and vaccines (with identical advantages). Methods of making ghost preparations (empty cells with intact envelopes) from Gram-negative strains are well known in the art (see for example WO 92/01791). Methods of killing whole cells to make inactivated cell preparations for use in vaccines are also well known. The terms 'bleb preparations' and 'bleb vaccines' as well as the processes described throughout this document are therefore applicable to the terms 'ghost preparation' and 'ghost vaccine', and 'killed whole cell preparation' and 'killed whole cell vaccine', respectively, for the purposes of this invention.

### EXAMPLES

The examples below are carried out using standard techniques, which are well known and routine to those of skill in the art, except where otherwise described in detail. The examples are illustrative, but do not limit the invention.

### Example 1: Previously reported examples

Examples describing: Construction of a *Neisseiria meningitidis* serogroup B strain lacking capsular polysaccharides; Construction of versatile gene delivery vectors (the pCMK series) targeting integration in the *porA* locus of *Neisseiria meningitidis;* Construction of a *Neisseiria meningitidis* serogroup B strain lacking both capsular polysaccharides and the major immunodominant antigen PorA; Up-regulation of the NspA outer membrane protein production in blebs derived from a recombinant *Neisseiria meningitidis* serogroup B strain lacking functional *porA* and *cps* genes; Up-regulation of the D15/Omp85 outer membrane protein antigen in blebs derived from a recombinant *Neisseiria meningitidis* serogroup B strain lacking functional *cps* genes but expressing PorA; Construction of versatile promoter delivery vectors; Fermentation processes for producing recombinant blebs; Identification of bacterial promoters suitable for up-regulation antigens-coding genes; Up-regulation of the *N*. *meningitidis* serogroup B *Omp85* gene by promoter replacement; Up-regulation of the Hsf protein antigen in a recombinant *Neisseiria meningitidis* serogroup B strain lacking functional *cps* genes but expressing PorA; *Expression of the Green Fluorescent Protein in a recombinant Neisseria meningitidis serogroup B strain lacking functional cps genes but expressing PorA;* Up-regulation of the *N. meningitidis* serogroup B *NspA* gene by promoter replacement; Up-regulation of the *N. meningitidis* serogroup B *pldA (omplA)* gene by promoter replacement; Up-regulation of the *N. meningitidis* serogroup B *tbpA* gene by promoter replacement; Up-regulation of the *N. meningitidis* serogroup B *pilQ* gene by promoter replacement; Construction of a *kanR*/*sacB* cassette for introducing "clean", unmarked mutations in the *N. meningitidis* chromosome; Use of small recombinogenic sequences (43bp) to allow homologous recombination in the chromosome of *Neisseria meningitidis;* Active protection of mice immunized with WT and recombinant *Neisseria meningitidis* blebs; and Immunogenicity of recombinant blebs measured by whole cell & specific ELISA methods have been described in WO 01/09350.

### Example 2: Gonococcal blebs expressing Chlamydia trachomatis proteins on its surface for use in a vaccine composition

Both *Chlamydia trachomatis* and *N. gonorrhoeae* cause sexually transmitted diseases, including urethritis, cervicitis, salpingitis and pelvic inflammatory disease. Mixed infection with both CT and GC does occur. Therefore, in the design of a vaccine targeting one, or more of these diseases, the possibility to afford protection against both organisms with one single formulation creates a technical advantage.

### Protection against N. gono.

A *N. gonorrhoeae* OMV vaccine can be obtained from bleb producing strain(s) in which the expression of one or several genes have been up and/or down regulated. A list of genes encoding *N. gonorrhoeae* proteins for which it is particularly useful to up/down regulate expression is provided above.

A successful vaccine for the prevention of infection by N. gono may require more than one of the following elements: generation of serum and/or mucosal antibodies to facilitate complement mediated killing of the gonococcus, and/or to enhance phagocytosis and microbial killing by leukocytes such as polymorphonuclear leukocytes, and/or to prevent attachment of the gonococci to the host tissues; induction of a cell mediated immune response may also participate to protection.

The potential of a bleb gono vaccine preparation can be evaluated by analyzing the induced immune response for serum and/or mucosal antibodies that have antiadherence, and/or opsonizing properties, and/or bactericidal activity, as described by others (McChesney D et al, Infect. Immun. 36: 1006, 1982; Boslego J et al: Efficacy trialof a purified gonococcl pilus vaccine, in Program and Abstracts of the 24^{th} Interscience Conference on Antimicrobial Agents and Chemotherapy, Whashington, American Society for Microbiology, 1984; Siegel M et al, J. Infect. Dis 145: 300, 1982; de la Pas, Microbiology, 141 (Pt4): 913-20, 1995).

A mouse model of genital infection by N. gono has recently been described (Plante M, J. Infect. Dis., 182: 848-55, 2000). The efficiency of a bleb gono vaccine could also be evaluated by its ability to prevent or to reduce colonization by N. gono in this mouse model of infection.

### Protection against CT

A GC/CT bleb vaccine can be obtained from a strain expressing one or several Chlamydia genes, preferably selected from the above list of genes encoding predicted outer membrane proteins.

Other genes of interest for overexpression in Neisseria are *C*. *trachomatis* genes for which no homolog has been found in *C*. *pneumoniae.* Such a set of genes has been described in Richard S. ; p:9-27, Stephens Stephens Ed. ASM Press, Washington DC, Chlamydia: Intracellular Biology, Pathogenesis, and Immunity ISBN: 1-55581-155-8 pages: 380.

Most preferred combinations of *Chlamydia trachomatis* genes are as follows: Major outer membrane protein MOMP (from one or several different serovars) and the Outer membrane Protein Analog (also known as PorB), MOMP (from one or several different serovars) and the Putative Outer Membrane Protein G (pmpG); & PorB and pmpG.

Although the immunity to CT is not fully understood, there is evidence that Ab play a role in protection. Ab to CT in genital fluids have been associated with immunity to CT (Brunham RC, Infect Immun. 1983 Mar;39(3):1491-4.). A protective role of serum antibody in immunity to chlamydial genital infection has also been shown (Rank RG, Infect Immun. 1989 Jan;57(1):299-301.). Antibodies, e.g. MOMP specific antibodies, have been shown to be capable to neutralize CT infection in vitro and in vivo (Caldwell et al. 1982 Infect. Immun. 38: 745-54, Lucero et al, 1985, Infect. Immun. 50: 595-97, Zhang et al. 1987 J. Immunol. 138: 575-581). The MOMP surface antigen of CT has been shown to bear non linear surface epitopes which are target of neutralizing antibodies (Fan J, J. Infect Dis 1997, 176(3):713-21).

Thus, an important objective in the design of a protective chlamydia vaccine includes the identification of formulation(s) of the CT antigens able to optimize the induction of a chlamydia specific antibody responses. Optimization of the Ab response includes targeting to the genital mucosa, and/or presentation of properly folded Chlamydia antigens, and/or combination of several antibody targets.

Mucosal targeting of the immune response to Chlamydia antigen can be achieved by mucosal administration of the vaccine. Intranasal administration of a outer membrane vesicle vaccine can induce persistent local mucosal antibodies and serum antibodies with strong bactericidal activity in humans.

For certain B cell epitopes, such as non linear epitopes, the presentation of the antigen to the immune system in a properly folded manner is critical. A bleb vaccine prepared from a strain expressing Chlamydia antigen(s) offers to chlamydia OMP an outer membrane environment which can be critical to maintain these antigens in a properly folded structure.

Combination of several antibody targets can create an increased efficacy by tackling the infection at different steps of the life cycle of the bacteria, such as adhesion to the host cell, internalization by the host cell and/or interference with further steps of the intracellular development.

The induction and recruitement of Th1 cells into the local genital mucosae are important for immunity against Chlamydia. Thus, an important objective in designing a protective anti-chlamydia vaccine includes the identification of formulation(s) of CT antigen(s) able to optimize the induction of chlamydia speicific Thl cells, and preferably recruitment of these cells into the genital mucosae. A bleb vaccine prepared from a strain expressing chlamydia antigen(s) can induce a chlamydia specific CMI response. Antigen-specific T-cell responses can be induced in humans after intranasal immunization with an outer membrane vesicle vaccine.

A particular advantage of a GC/CT bleb vaccine is its capability to induce both Ab and CMI responses.

The efficacy of the GC/CT bleb vaccine can be evaluated by its ability to elicit Ag or Chlamydia-specific Ab and/or CMI responses. Ab responses can be evaluated by classical techniques such as ELISA or western blot. Preferably, the induced antibodies can neutralize the infectivity of Chlamydia in an in vitro assay (Byrne G. et al. (J Infect Dis. 1993 Aug;168(2):415-20). Preferably, the CMI response is biased toward the Thl phenotype. A Thl biased immune response can be assessed by elevated antigen-specific IgG2a/ IgG1 ratios in mice (Snapper et al. 1987, Science 236:944-47). Elevated ratio of Th1/Th2 cytokine, e.g. elevated IFN-gamma/IL-5, ratio upon in vitro restimulation of immune T cells with the antigen(s) can also indicate such a biased Th1 response.

The ability of the formulation to elicit Ag specific mucosal Ab is of particular interest, and can be demonstrated by detection of antibodies, such as IgG and/or IgA in mucosal fluids, such as genital tract secretions, vaginal lavages. To this end, certain route of administration of the vaccine may be particularly desired such as intranasal, oral, intravaginal, intradermal, deliveries.

The efficacy of the GC/CT bleb vaccine can be evaluated by its ability to induce protection against a Chlamydia challenge in animal model(s). Examples of such animal models have been described in the literature: genital infection with MoPn in mice (Barron et al. J. Infect. Dis. 1143:63-66), genital infection with human strains in mice (Igietseme et al.2000, Infect. Immun. 68:6798-806, Tuffrey et al. 1992 J. Gen. Microbiol. 138: 1707-1715), Tuffrey), genital infection with GPIC strain in guinea pigs (Rank et al. 1992 Am. J. Pathol. 140:927-936). Protection against infection can be assessed by reduction of shed Chlamydia from the infected site and/or reduction of the histopathological reactions after a challenge infection in immunized animals.

The advantage of combining two or more Chlamydia antigens (as described above) can be evaluated by one or more of the following techniques:
➢ Ability to elicit a multi-target Ab and/or T cell protective response
➢ Ability to elicit Ab titers in an in vitro neutralizing assay, and/or neutralizing Ab against multiple strains (antigenically distinct)
➢ Ability to elicit a protective immune response against Chlamydia in a mouse model of genital infection as assessed by reduced shedding of bacteria and/or pathology after challenge.

### Example 3: Expression of heterologous antigens (Clamydia trachomatis MOMP and PorB) in blebs derived from a recombinant Neisseiria meningitidis serogroup B strain lacking functional porA and cps genes.

Other genes of interest for over-expression in *Neisseria* are *Chlamydia trachomatis* genes for which no homologue has been found in *Chlamydia pneumoniae.* Among those, the major outer membrane protein (MOMP) and the outer membrane protein analog (PorB) have been shown to play a protective role against chlamydial genital infection. Optimization of the Ab response could be achieved by presentation of properly folded proteins.

MenB bleb vesicles may be used as delivery vectors to express heterologous membrane protein antigens under the control of the engineered *porA-lacO* promoter described in WO 01/09350. Expressed in the bleb context, recombinant MOMP and PorB from *Chlamydia trachomatis* serovar D and K can be correctly folded in the membrane and exposed at the surface. *Neisseiria meningitidis* strains lacking functional *cps* genes are advantageously used as recipient strains to express the heterologous antigens (WO 01/09350).

### PCR amplifications of the genes coding for MOMP (Chlamydia trachomatis).

Murine McCoy cells (ATCC) infected either, with *Chlamydia trachomatis* Serovar K (UW31-CX-serK), or Serovar D (UW31-CX-serD), were lysed in 400µl of lysis buffer: 50mM KCl, 10mM Tris-HCl pH 8.3, 2.5mM MgCl2, 0.45% Nonidet P40, 0.45% Tween 20 containing 60µg/ml proteinase K, 3 hours at 56°C. Ten µl of the lysate were used as template to amplify the corresponding genes. The gene coding for MOMP (Serovar K) (SEQID N° 1 below) was PCR amplified using the CYK/OMP/5/NDE and CYKD/OMP/3BG oligonucleotide primers (see table 1). The gene coding for MOMP (Serovar D) (SEQID N°2 below) was PCR amplified using the CYD/OMP/5/NRU and CYKD/OMP/3BG oligonucleotide primers (see table 1). The conditions used for PCR amplification were those described by the supplier (HiFi DNA polymerase, Boehringer Mannheim, GmbH). Thermal cycling was the following: 25 times (94°C 1min., 52°C 1min., 72°C 3min.) and 1 time (72°C 10min., 4°C up to recovery). The corresponding amplicons (1194bp) were digested with either *Nde*I/*Bgl*II or *Nru*I/*Bgl*II restriction enzymes and can be cloned in the corresponding restriction sites of pCMK (+) delivery vector (as described in WO 01/09350).

### PCR amplifications of the genes coding for PorB (Chlamydia trachomatis).

Murine McCoy cells (ATCC) infected either, with *Chlamydia trachomatis* Serovar K (UW31-CX-serK), or Serovar D (UW31-CX-serD), were lysed in 400 µl of lysis buffer: 50mM KCl, 10mM Tris-HCl pH 8.3, 2.5mM MgCl2, 0.45% Nonidet P40, 0.45% Tween 20 containing 60µg/ml proteinase K, 3 hours at 56°C. Ten µl of the lysate were used as template to amplified the corresponding genes.

PorB sequences are highly conserved amongst serovar D and K (SEQID N°3 below). The same primers were used to amplify the corresponding genes in both serovars: CYD/PORB/5/NRU and CYD/PORB/3/BG (see table 1). The conditions used for PCR amplification were those described by the supplier (HiFi DNA polymerase, Boehringer Mannheim, GmbH). Thermal cycling was the following: 25 times (94°C 1min., 52°C 1min., 72°C 3min.) and 1 time (72°C 10min., 4°C up to recovery). The corresponding amplicons (1035bp) were digested with *Nru*I/*Bgl*II restriction enzymes and can be cloned in the corresponding restriction sites of pCMK (+) delivery vector (as described in WO 01/09350).

### Transformation

Linearized recombinant pCMK plasmids can be transformed within a *Neisseria meningitidis* serogroup B strain lacking functional *cps* genes (described in WO 01/09350). Integration resulting from a double crossing-over between the pCMK vectors and the chromosomal *porA* locus can be selected by a combination of PCR and Western Blot screening as described in WO 01/09350.

**Table 1: Oligonucleotides used in this work**

| Oligonucleotides | Sequence | Remark(s) |
|---|---|---|
| CYK/OMP/5/NDE | 5' -GGG AAT CCA TAT GAA AAA ACT CTT GAA ATC GG-3' | *Nde*I cloning Site |
| CYKD/OMP/3/BG | 5' -GGA AGA TCT TTA GAA GCG GAA TTG TGC AT-3' | *Bgl* II cloning site |
| CYD/OMP/5/NRU | 5'-CTG CAG AAT CGC GAA TGA AAA AAC TCT TGA AAT CGG-3' | *Nru*I cloning site |
| CYD/POR/5/NRU | 5'-CTG CAG AAT CGC GAA TGA GTA GCA AGC TAG TGA AC-3' | *Nru*I cloning site |
| CYD/POR/3/BG | 5'- AGG AGA TCT TTA GAA TTG GAA TCC TCC GG-3' | *Bgl* II cloning site |

### SEQID N°1:

### Nucleotide sequence of DNA coding for Chlamydia trachomatis MOMP serovar K protein.

### SEQID N°2:

### Nucleotide sequence of DNA coding for Chlamydia trachomatis MOMP serovar D protein.

### SEQID N°3:

### Nucleotide sequence of DNA coding for Chlamydia trachomatis PorB serovar D protein.

### Example 4: Isolation and purification of blebs from meningococci devoid of capsular polysaccharide

Recombinant blebs can be purified as described below. The cell paste (42gr) is suspended in 211 ml of 0.1M Tris-Cl buffer pH 8.6 containing 10 mM EDTA and 0.5% Sodium Deoxycholate (DOC). The ratio of buffer to biomass should be 5/1 (V/W). The biomass is extracted by magnetic stirring for 30 minutes at room temperature. Total extract is then centrifuged at 20,000g for 30 minutes at 4°C (13,000 rpm in a JA-20 rotor, Beckman J2-HS centrifuge). The pellet should be discarded. The supernatant is ultracentrifuged at 125,000g for 2 hours at 4°C (40,000 rpm in a 50.2Ti rotor, Beckman L8-70M ultracentrifuge) in order to concentrate vesicles. The supernatant should be discarded. The pellet is gently suspended in 25 ml of 50 mM Tris-Cl buffer pH 8.6 containing 2 mM EDTA, 1.2% DOC and 20% sucrose. After a second ultracentrifugation step at 125,000g for 2 hours at 4°C, vesicles are gently suspended in 44 ml of 3% sucrose and stored at 4°C. All solutions used for bleb extraction and purification contained 0.01% thiomersalate. As illustrated in WO 01/019350, this procedure yields protein preparations highly enriched in outer-membrane proteins.

### Example 5: Models for testing protection against gonococcal and C. trachomatis infection

This can be done as described above in Example 2. In addition Whittum-Hudson et al. (Vaccine 2001 Jul 16;19(28-29):4061-71) "The anti-idiotypic antibody to chlamydial glycolipid exoantigen (GLXA) protects mice against genital infection with a human biovar of Chlamydia trachomatis" is a vaginal inoculation model for C. trachomatis which can also be used to test vaccine efficacy.

## Claims

1. A Gram-negative bacterial bleb not derived from Chlamydia, presenting on its surface the PorB outer membrane protein from *Chlamydia trachomatis*, wherein the combination of the Chlamydia antigen with the native Gram-negative bacterial bleb antigens interact in the prevention or treatment of salpingitis when present in a vaccine formulation.

2. The Gram-negative bleb of claim 1 further presenting on its surface the PmpG outer membrane proteins from *Chlamydia trachomatis.*

3. The Gram-negative bleb of claim 1 further presenting on its surface MOMP from one or more serovars from *Chlamydia trachomatis.*

4. A Gram-negative bleb not derived from Chlamydia, presenting on its surface both the PmpG and MOMP (from one or more serovars) outer membrane proteins from *Chlamydia trachomatis,* wherein the combination of the Chlamydia antigens with the native Gram-negative bacterial bleb antigens interact in the prevention or treatment of salpingitis when present in a vaccine formulation.

5. The bleb of claims 1-4 which are gonococcal blebs.

6. The bleb of claim 5 which has been derived from a gonococcal strain which has been modified to upregulate one or more protective gonococcal outer membrane antigens.

7. The bleb of claims 5 and 6 derived from a gonococcal strain which has been modified to downregulate one or more immunodominant variable or non-protective gonococcal outer membrane antigens.

8. The bleb of claims 5-7 derived from a strain which has a detoxified lipid A portion of bacterial LPS, due to the strain having been engineered to reduce or switch off expression of one or more genes selected from the group consisting of: htrB, msbB and lpxk.

9. The bleb of claims 5-8 wherein the bleb preparation is derived from a strain which has a detoxified lipid A portion of bacterial LPS, due to the strain having been engineered to express at a higher level one or more genes selected from the group consisting of: pmrA, pmrB, pmrE and pmrF.

10. A vaccine composition comprising the bleb of claims 1-9 and a pharmaceutically suitable excipient or carrier.

11. The vaccine of claim 10, additionally comprising a mucosal adjuvant.

12. Use of the blets of claims 1-9 in the manufacture of a vaccine for preventing *Chlamydia trachomatis* infection in a host comprising the steps of administering an effective amount of the vaccine of claim 10 or 11 to a host in need thereof.

13. The use of claim 12 where in the vaccine is mucosally administered via either a intranasal, oral, or intravaginal route.

14. A Gram-negative bleb produced from *Neisseria meningitidis, Moraxella catharralis* or *Haemophilus influenzae* presenting on its surface a protective antigen from *Chlamydia pneumoniae.*

15. The Gram-negative bleb of claim 14 presenting on its surface a PorB outer membrane protein from *Chlamydia pneumoniae.*

16. The Gram-negative bleb of claim 15 further presenting on its surface MOMP outer membrane protein from *Chlamydia pneumoniae.*

17. The Gram-negative bleb of claim 15 further presenting on its surface one or more Pmp outer membrane proteins from *Chlamydia pneumoniae.*

18. The Gram-negative bleb of claim 15 further presenting on its surface Npt1 protein from *Chlamydia pneumoniae*.

19. The Gram-negative bleb of claim 14 presenting on its surface one or more Pmp proteins from *Chlamydia pneumoniae.*

20. The Gram-negative bleb of claim 19 further presenting on its surface Npt1 protein from *Chlamydia pneumoniae*

21. The Gram-negative bleb of claim 19 further presenting on its surface MOMP protein from *Chlamydia pneumoniae.*

22. The Gram-negative bleb of claim 14 presenting on its surface MOMP protein from Chlamydia pneumoniae.

23. The Gram-negative bleb of claim 22 further presenting on its surface Npt1 protein from *Chlamydia pneumoniae.*

24. The bleb of claims 14-23 which are meningococcal blebs.

25. The bleb of claim 24 derived from a meningococcal strain that has been modified to upregulate one or more protective meningococcal outer membrane antigens.

26. The bleb of claim 24 or 25 derived from a meningococcal strain that has been modified to downregulate one or more immunodominant variable or non-protective meningococcal outer membrane antigens.

27. The bleb of claims 24-26 derived from a strain which has a detoxified lipid A portion of bacterial LPS, due to the strain having been engineered to reduce or switch off expression of one or more genes selected from the group consisting of:
htrB, msbB and lpxK.

28. The bleb of claims 24-27 wherein the bleb preparation is derived from a strain which has a detoxified lipid A portion of bacterial LPS, due to the strain having been engineered to express at a higher level one or more genes selected from the group consisting of: pmrA, pmrB, pmrE and pmrF.

29. A vaccine composition comprising the bleb of claims 14-28 and a pharmaceutically suitable excipient or carrier.

30. The vaccine of claim 29, additionally comprising a mucosal adjuvant.

31. Use of blets of claims 14-28 in the manufacture of a vaccine for preventing *Chlamydia pneumoniae* infection in a host comprising the steps of administering an effective amount of the vaccine of claim 29 or 30 to a host in need thereof.

32. The use of claim 31 where in the vaccine is mucosally administered via either an intranasal, or oral route.

## Patentansprüche

1. Gram-negatives bakterielles Bläschen, das nicht aus Chlamydia stammt und auf seiner Oberfläche das PorB-Außenmembranprotein von Chlamydia trachomatis präsentiert, worin die Kombination des Chlamydia-Antigens mit den nativen Gram-negativen bakteriellen Bläschenantigenen in der Prävention oder Behandlung von Salpingitis, bei Vorhandensein in einer Impfstofformulierung, wechselwirkt.

2. Gram-negatives Bläschen gemäß Anspruch 1, das ferner auf seiner Oberfläche die PmpG-Außenmembranproteine von Chlamydia trachomatis präsentiert.

3. Gram-negatives Bläschen gemäß Anspruch 1, das ferner auf seiner Oberfläche MOMP aus einem oder mehreren Serovars von Chlamydia trachomatis präsentiert.

4. Gram-negatives Bläschen, das nicht aus Chlamydia stammt und auf seiner Oberfläche sowohl die PmpG- als auch MOMP- (aus einem oder mehreren Serovars) Außenmembranproteine von Chlamydia trachomatis präsentiert, worin die Kombination der Chlamydia-Antigene mit den nativen Gram-negativen bakteriellen Bläschenantigenen in der Prävention oder Behandlung von Salpingitis, bei Vorhandensein in einer Impfstofformulierung, wechselwirkt.

5. Bläschen gemäß Ansprüchen 1 bis 4, die Gonokokkenbläschen sind.

6. Bläschen gemäß Anspruch 5, das aus einem Gonokokkenstamm stammt, der zur Aufregulierung eines oder mehrerer Gonokokken-Außenmembran-Schutzantigene modifiziert wurde.

7. Bläschen gemäß Ansprüchen 5 und 6, das aus einem Gonokokkenstamm stammt, der zur Abregulierung eines oder mehrerer immundominanter variabler oder nichtschützender Gonokokken-Außenmembranantigene modifiziert wurde.

8. Bläschen gemäß Ansprüchen 5 bis 7, das aus einem Stamm stammt, der einen entgifteten Lipid A-Anteil von bakteriellem LPS aufgrund der Tatsache hat, daß der Stamm verändert wurde, um die Expression eines oder mehrerer Gene zu reduzieren oder auszuschalten, die aus der Gruppe ausgewählt sind, die aus htrB, msbB und lpxK besteht.

9. Bläschen gemäß Ansprüchen 5 bis 8, worin die Bläschenzubereitung aus einem Stamm stammt, der einen entgifteten Lipid A-Anteil von bakteriellem LPS aufgrund der Tatsache hat, daß der Stamm verändert wurde, um ein oder mehrere Gene mit höherer Stärke zu exprimieren, die aus der Gruppe ausgewählt sind, die aus pmrA, pmrB, pmrE und pmrF besteht.

10. Impfstoffzusammensetzung, die das Bläschen gemäß Ansprüchen 1 bis 9 und einen pharmazeutisch geeigneten Exzipienten oder Träger umfaßt.

11. Impfstoff gemäß Anspruch 10, der zusätzlich einen mukosalen Hilfsstoff umfaßt.

12. Verwendung des Bläschens gemäß Ansprüchen 1 bis 9 in der Herstellung eines Impfstoffs zur Prävention von Chlamydia trachomatis-Infektion in einem Wirt, umfassend die Schritte der Verabreichung einer wirksamen Menge des Impfstoffs gemäß Anspruch 10 oder 11 an einen Wirt, der dessen bedarf.

13. Verwendung gemäß Anspruch 12, worin der Impfstoff mukosal über einen entweder intranasalen, oralen oder intravaginalen Weg verabreicht wird.

14. Gram-negatives Bläschen, das von Neisseria meningitidis, Moraxella catharralis oder Haemophilus influenzae erzeugt wird und auf seiner Oberfläche ein Schutzantigen von Chlamydia pneumoniae präsentiert.

15. Gram-negatives Bläschen gemäß Anspruch 14, das auf seiner Oberfläche ein PorB-Außenmembranprotein von Chlamydia pneumoniae präsentiert.

16. Gram-negatives Bläschen gemäß Anspruch 15, das ferner auf seiner Oberfläche MOMP-Außenmembranprotein von Chlamydia pneumoniae präsentiert.

17. Gram-negatives Bläschen gemäß Anspruch 15, das ferner auf seiner Oberfläche ein oder mehrere Pmp-Außenmembranproteine von Chlamydia pneumoniae präsentiert.

18. Gram-negatives Bläschen gemäß Anspruch 15, das ferner auf seiner Oberfläche Npt1-Protein von Chlamydia pneumoniae präsentiert.

19. Gram-negatives Bläschen gemäß Anspruch 14, das auf seiner Oberfläche ein oder mehrere Pmp-Proteine von Chlamydia pneumoniae präsentiert.

20. Gram-negatives Bläschen gemäß Anspruch 19, das ferner auf seiner Oberfläche Npt1-Protein von Chlamydia pneumoniae präsentiert.

21. Gram-negatives Bläschen gemäß Anspruch 19, das ferner auf seiner Oberfläche MOMP-Protein von Chlamydia pneumoniae präsentiert.

22. Gram-negatives Bläschen gemäß Anspruch 14, das auf seiner Oberfläche MOMP-Protein von Chlamydia pneumoniae präsentiert.

23. Gram-negatives Bläschen gemäß Anspruch 22, das ferner auf seiner Oberfläche Npt1-Protein von Chlamydia pneumoniae präsentiert.

24. Bläschen gemäß Ansprüchen 14 bis 23, die Meningokokkenblälschen sind.

25. Bläschen gemäß Anspruch 24, das aus einem Meningokokkenstamm stammt, der zur Aufregulierung eines oder mehrerer Meningokokken-Außenmembran-Schutzantigene modifiziert wurde.

26. Bläschen gemäß Anspruch 24 oder 25, das aus einem Meningokokkenstamm stammt, der zur Abregulierung eines oder mehrerer immundominanter variabler oder nichtschützender Meningokokken-Außenmembranantigene modifiziert wurde.

27. Bläschen gemäß Ansprüchen 24 bis 26, das aus einem Stamm stammt, der einen entgifteten Lipid A-Anteil von bakteriellem LPS aufgrund der Tatsache hat, daß der Stamm verändert wurde, um die Expression eines oder mehrerer Gene zu reduzieren oder auszuschalten, die aus der Gruppe ausgewählt sind, die aus htrB, msbB und lpxK besteht.

28. Bläschen gemäß Ansprüchen 24 bis 27, worin die Bläschenzubereitung aus einem Stamm stammt, der einen entgifteten Lipid A-Anteil von bakteriellem LPS aufgrund der Tatsache hat, daß der Stamm verändert wurde, um ein oder mehrere Gene mit höherer Stärke zu exprimieren, die aus der Gruppe ausgewählt sind, die aus pmrA, pmrB, pmrE und pmrF besteht.

29. Impfstoffzusammensetzung, die das Bläschen gemäß Ansprüchen 14 bis 28 und einen pharmazeutisch geeigneten Exzipienten oder Träger umfaßt.

30. Impfstoff gemäß Anspruch 29, der zusätzlich einen mukosalen Hilfsstoff umfaßt.

31. Verwendung von Bläschen gemäß Ansprüchen 14 bis 28 in der Herstellung eines Impfstoffs zur Prävention von Chlamydia pneumoniae-Infektion in einem Wirt, umfassend die Schritte der Verabreichung einer wirksamen Menge des Impfstoffs gemäß Anspruch 29 oder 30 an einen Wirt, der dessen bedarf.

32. Verwendung gemäß Anspruch 31, worin der Impfstoff mukosal über einen entweder intranasalen oder oralen Weg verabreicht wird.

## Revendications

1. Vésicule de bactérie Gram-négative non dérivée de Chlamydia, présentant sur sa surface la protéine de la membrane externe PorB de Chlamydia trachomatis, dans laquelle la combinaison de l'antigène de Chlamydia avec les antigènes de vésicules de bactéries Gram-négatives natifs interagissent dans la prévention ou le traitement de la salpingite lorsqu'ils sont présents dans une formulation vaccinale.

2. Vésicule de bactérie Gram-négative selon la revendication 1, présentant en outre sur sa surface les protéines de la membrane externe PmpG de Chlamydia trachomatis.

3. Vésicule de bactérie Gram-négative selon la revendication 1, présentant en outre sur sa surface les MOMP d'un ou plusieurs sérotypes de Chlamydia trachomatis.

4. Vésicule de bactérie Gram-négative non dérivée de Chlamydia, présentant sur sa surface à la fois les protéines de la membrane externe PmpG et MOMP (d'un ou plusieurs sérotypes) de Chlamydia trachomatis, dans laquelle la combinaison des antigènes de Chlamydia avec les antigènes de vésicules de bactéries Gram-négatives natifs interagissent dans la prévention ou le traitement de la salpingite lorsqu'ils sont présents dans une formulation vaccinale.

5. Vésicule selon les revendications 1 à 4 qui sont des vésicules gonococciques.

6. Vésicule selon la revendication 5, dérivée d'une souche gonococcique qui a été modifiée pour réguler de manière positive un ou plusieurs antigènes gonococciques protecteurs de la membrane externe.

7. Vésicule selon les revendications 5 et 6, dérivée d'une souche gonococcique qui a été modifiée pour réguler de manière négative un ou plusieurs antigènes gonococciques variables ou non-protecteurs immunodominants de la membrane externe.

8. Vésicule selon les revendications 5 à 7, dérivée d'une souche qui a une portion détoxifiée du lipide A du LPS bactérien, du fait que la souche a été modifiée pour réduire ou arrêter l'expression d'un ou plusieurs gènes choisis dans le groupe constitué de : htrB, msbB et lpxK.

9. Vésicule selon les revendications 5 à 8, dans laquelle la préparation de la vésicule est dérivée d'une souche qui a une portion détoxifiée du lipide A du LPS bactérien, du fait que la souche a été modifiée pour exprimer à un niveau plus élevé un ou plusieurs gènes choisis dans le groupe constitué de : pmrA, pmrB, pmrE et pmrF.

10. Composition vaccinale comprenant la vésicule selon les revendications 1 à 9 et un excipient ou vecteur pharmaceutiquement approprié.

11. Vaccin selon la revendication 10, comprenant en outre un adjuvant muqueux.

12. Utilisation d'une vésicule selon les revendications 1 à 9 dans la fabrication d'un vaccin pour prévenir l'infection par Chlamydia trachomatis dans un hôte comprenant les étapes d'administration d'une quantité efficace du vaccin selon la revendication 10 ou 11 à un hôte qui en a besoin.

13. Utilisation selon la revendication 12, dans laquelle le vaccin est administré par voie muqueuse via une voie intranasale, orale ou intravaginale.

14. Vésicule de bactérie Gram-négative produite à partir de Neisseria meningitidis, Moraxella catharralis ou Haemophilus influenzae présentant sur sa surface un antigène protecteur de Chlamydia pneumoniae.

15. Vésicule de bactérie Gram-négative selon la revendication 14, présentant sur sa surface la protéine de la membrane externe PorB de Chlamydia pneumoniae.

16. Vésicule de bactérie Gram-négative selon la revendication 15, présentant en outre sur sa surface la protéine MOMP de la membrane externe de Chlamydia pneumoniae.

17. Vésicule de bactérie Gram-négative selon la revendication 15, présentant en outre sur sa surface une ou plusieurs protéines Pmp de la membrane externe de Chlamydia pneumoniae.

18. Vésicule de bactérie Gram-négative selon la revendication 15, présentant en outre sur sa surface la protéine Nptl de Chlamydia pneumoniae.

19. Vésicule de bactérie Gram-négative selon la revendication 14, présentant sur sa surface une ou plusieurs protéines Pmp de Chlamydia pneumoniae.

20. Vésicule de bactérie Gram-négative selon la revendication 19, présentant en outre sur sa surface la protéine Nptl de Chlamydia pneumoniae.

21. Vésicule de bactérie Gram-négative selon la revendication 19, présentant en outre sur sa surface la protéine MOMP de Chlamydia pneumoniae.

22. Vésicule de bactérie Gram-négative selon la revendication 14, présentant sur sa surface la protéine MOMP de Chlamydia pneumoniae.

23. Vésicule de bactérie Gram-négative selon la revendication 22, présentant en outre sur sa surface la protéine Nptl de Chlamydia pneumoniae.

24. Vésicule selon les revendications 14 à 23 qui sont des vésicules méningococciques.

25. Vésicule selon la revendication 24 dérivée d'une souche méningococcique qui a été modifiée pour réguler de manière positive un ou plusieurs antigènes protecteurs meningococciques de la membrane externe.

26. Vésicule selon la revendication 24 ou 25 dérivée d'une souche méningococcique qui a été modifiée pour réguler de manière négative un ou plusieurs antigènes méningococciques variables ou non-protecteurs immunodominants de la membrane externe.

27. Vésicule selon les revendications 24 à 26 dérivée d'une souche qui a une partie détoxifiée du lipide A du LPS bactérien, du fait que la souche a été modifiée pour réduire ou arrêter l'expression d'un ou plusieurs gènes choisis dans le groupe constitué de : htrB, msbB et lpxK.

28. Vésicule selon les revendications 24 à 27, dans laquelle la préparation de la vésicule est dérivée d'une souche qui a une partie détoxifiée du lipide A du LPS bactérien, du fait que la souche a été modifiée pour exprimer à un niveau plus élevé un ou plusieurs gènes choisis dans le groupe constitué de : pmrA, pmrB, pmrE et pmrF.

29. Composition vaccinale comprenant la vésicule selon les revendications 14 à 28 et un excipient ou vecteur pharmaceutiquement approprié.

30. Vaccin selon la revendication 29, comprenant en outre un adjuvant muqueux.

31. Utilisation d'une vésicule selon les revendications 14 à 28 dans la fabrication d'un vaccin pour prévenir l'infection par Chlamydia pneumoniae dans un hôte comprenant les étapes d'administration d'une quantité efficace du vaccin selon la revendication 29 ou 30 à un hôte qui en a besoin.

32. Utilisation selon la revendication 31, dans laquelle le vaccin est administré par voie muqueuse via une voie intranasale ou orale.


### SEQUENCE LISTING

<110> SmithKline Beecham Biologicals s.a.
<120> Vaccine Composition
<130> B45261
<160> 108
<170> FastSEQ for Windows Version 4.0
<210> 1
   <211> 1023
   <212> DNA
   <213> Chlamydia trachomatis
<400> 1
   atgagtagca agctagtgaa ctatctccgt ttgactttcc tatctttttt agggatcgca 60
   tctacttcat tagacgctat gcctgcgggg aatccggcgt ttccagtcat cccggggatt 120
   aatattgaac agaaaaatgc ctgttctttc gatttatgta attcttatga tgtactatcc 180
   gcactgtccg gtaacctgaa gctctgcttc tgcggagatt atatcttttc agaagaagct 240
   caggtaaaag atgtccctgt cgttacctct gtgacaacag ctggggttgg tccttctcct 300
   gatattactt cgacaaccaa aacgcgaaat ttcgatctcg tgaactgtaa tctcaataca 360
   aactgtgtag ctgtagcttt ttcccttcct gatcgttcgc tgagcgcgat tcctctgttt 420
   gatgtgagtt tcgaagtgaa agtaggagga ctgaaacaat actaccgcct tcccatgaat 480
   gcctatcgag acttcacctc ggaacctctc aattctgaat cagaagttac ggacgggatg 540
   attgaagtac agtccaatta cggatttgtt tgggatgtta gcttgaaaaa agtcatatgg 600
   aaagatggcg tttcctttgt aggcgtcggt gcagactatc gccatgcttc ttgccctatt 660
   gactacatca ttgcaaacag tcaagctaat ccagaagtat tcatcgctga ctcggatggg 720
   aaactgaact tcaaggagtg gagtgtctgc gtaggtctta ctacctatgt gaatgactac 780
   gttcttcctt acttagcgtt ttctataggg agtgtttctc gccaagctcc ggacgacagc 840
   ttcaaaaaat tagaagatcg cttcactaac ctcaaattta aagttcgtaa aattaccagc 900
   tctcatcgtg gaaacatctg catcggagcg acaaactatg tcgccgataa cttcttctac 960
   aacgtagaag gaagatgggg aagccagcgc gctgtgaacg tctccggagg attccaattc 1020
   taa                                                               1023
<210> 2
   <211> 340
   <212> PRT
   <213> Chlamydia trachomatis
<400> 2
   Met Ser Ser Lys Leu Val Asn Tyr Leu Arg Leu Thr Phe Leu Ser Phe
   1 5 10 15
   Leu Gly Ile Ala Ser Thr Ser Leu Asp Ala Met Pro Ala Gly Asn Pro
   20 25 30
   Ala Phe Pro Val Ile Pro Gly Ile Asn Ile Glu Gln Lys Asn Ala Cys
   35 40 45
   Ser Phe Asp Leu Cys Asn Ser Tyr Asp Val Leu Ser Ala Leu Ser Gly
   50 55 60
   Asn Leu Lys Leu Cys Phe Cys Gly Asp Tyr Ile Phe Ser Glu Glu Ala
   65 70 75 80
   Gln Val Lys Asp Val Pro Val Val Thr Ser Val Thr Thr Ala Gly Val
   85 90 95
   Gly Pro Ser Pro Asp Ile Thr Ser Thr Thr Lys Thr Arg Asn Phe Asp
   100 105 110
   Leu Val Asn Cys Asn Leu Asn Thr Asn Cys Val Ala Val Ala Phe Ser
   115 120 125
   Leu Pro Asp Arg Ser Leu Ser Ala Ile Pro Leu Phe Asp Val Ser Phe
   130 135 140
   Glu Val Lys Val Gly Gly Leu Lys Gln Tyr Tyr Arg Leu Pro Met Asn
   145 150 155 160
   Ala Tyr Arg Asp Phe Thr Ser Glu Pro Leu Asn Ser Glu Ser Glu Val
   165 170 175
   Thr Asp Gly Met Ile Glu Val Gln Ser Asn Tyr Gly Phe Val Trp Asp
   180 185 190
   Val Ser Leu Lys Lys Val Ile Trp Lys Asp Gly Val Ser Phe Val Gly
   195 200 205
   Val Gly Ala Asp Tyr Arg His Ala Ser Cys Pro Ile Asp Tyr Ile Ile
   210 215 220
   Ala Asn Ser Gln Ala Asn Pro Glu Val Phe Ile Ala Asp Ser Asp Gly
   225 230 235 240
   Lys Leu Asn Phe Lys Glu Trp Ser Val Cys Val Gly Leu Thr Thr Tyr
   245 250 255
   Val Asn Asp Tyr Val Leu Pro Tyr Leu Ala Phe Ser Ile Gly Ser Val
   260 265 270
   Ser Arg Gln Ala Pro Asp Asp Ser Phe Lys Lys Leu Glu Asp Arg Phe
   275 280 285
   Thr Asn Leu Lys Phe Lys Val Arg Lys Ile Thr Ser Ser His Arg Gly
   290 295 300
   Asn Ile Cys Ile Gly Ala Thr Asn Tyr Val Ala Asp Asn Phe Phe Tyr
   305 310 315 320
   Asn Val Glu Gly Arg Trp Gly Ser Gln Arg Ala Val Asn Val Ser Gly
   325 330 335
   Gly Phe Gln Phe
   340
<210> 3
   <211> 601
   <212> PRT
   <213> Chlamydia trachomatis
<400> 3
   Met Lys Met Asn Arg Ile Trp Leu Leu Leu Leu Thr Phe Ser Ser Ala
   1               5                   10                  15
   Ile His Ser Pro Val Gln Gly Glu Ser Leu Val Cys Lys Asn Ala Leu
               20                  25                  30
   Gln Asp Leu Ser Phe Leu Glu His Leu Leu Gln Val Lys Tyr Ala Pro
           35                  40                  45
   Lys Thr Trp Lys Glu Gln Tyr Leu Gly Trp Asp Leu Val Gln Ser Ser
       50                  55                  60
   Val Ser Ala Gln Gln Lys Leu Arg Thr Gln Glu Asn Pro Ser Thr Ser
   65                  70                  75                  80
   Phe Cys Gln Gln Val Leu Ala Asp Phe Ile Gly Gly Leu Asn Asp Phe
                   85                  90                  95
   His Ala Gly Val Thr Phe Phe Ala Ile Glu Ser Ala Tyr Leu Pro Tyr
               100                 105                 110
   Thr Val Gln Lys Ser Ser Asp Gly Arg Phe Tyr Phe Val Asp Ile Met
           115                 120                 125
   Thr Phe Ser Ser Glu Ile Arg Val Gly Asp Glu Leu Leu Glu Val Asp
       130                 135                 140
   Gly Ala Pro Val Gln Asp Val Leu Ala Thr Leu Tyr Gly Ser Asn His
   145                 150                 155                 160
   Lys Gly Thr Ala Ala Glu Glu Ser Ala Ala Leu Arg Thr Leu Phe Ser
                   165                 170                 175
   Arg Met Ala Ser Leu Gly His Lys Val Pro Ser Gly Arg Thr Thr Leu
               180                 185                 190
   Lys Ile Arg Arg Pro Phe Gly Thr Thr Arg Glu Val Arg Val Lys Trp
           195                 200                 205
   Arg Tyr Val Pro Glu Gly Val Gly Asp Leu Ala Thr Ile Ala Pro Ser
       210                 215                 220
   Ile Arg Ala Pro Gln Leu Gln Lys Ser Met Arg Ser Phe Phe Pro Lys
   225                 230                 235                 240
   Lys Asp Asp Ala Phe His Arg Ser Ser Ser Leu Phe Tyr Ser Pro Met
                   245                 250                 255
   Val Pro His Phe Trp Ala Glu Leu Arg Asn His Tyr Ala Thr Ser Gly
               260                 265                 270
   Leu Lys Ser Gly Tyr Asn Ile Gly Ser Thr Asp Gly Phe Leu Pro Val
           275                     280                     285
   Ile Gly Pro Val Ile Trp Glu Ser Glu Gly Leu Phe Arg Ala Tyr Ile
       290                     295                     300
   Ser Ser Val Thr Asp Gly Asp Gly Lys Ser His Lys Val Gly Phe Leu
   305                     310                     315                     320
   Arg Ile Pro Thr Tyr Ser Trp Gln Asp Met Glu Asp Phe Asp Pro Ser
                   325                     330                     335
   Gly Pro Pro Pro Trp Glu Glu Phe Ala Lys Ile Ile Gln Val Phe Ser
               340                     345                     350
   Ser Asn Thr Glu Ala Leu Ile Ile Asp Gln Thr Asn Asn Pro Gly Gly
           355                     360                     365
   Ser Val Leu Tyr Leu Tyr Ala Leu Leu Ser Met Leu Thr Asp Arg Pro
       370                     375                     380
   Leu Glu Leu Pro Lys His Arg Met Ile Leu Thr Gln Asp Glu Val Val
   385                     390                     395                     400
   Asp Ala Leu Asp Trp Leu Thr Leu Leu Glu Asn Val Asp Thr Asn Val
                   405                     410                     415
   Glu Ser Arg Leu Ala Leu Gly Asp Asn Met Glu Gly Tyr Thr Val Asp
               420                     425                     430
   Leu Gln Val Ala Glu Tyr Leu Lys Ser Phe Gly Arg Gln Val Leu Asn
           435                     440                     445
   Cys Trp Ser Lys Gly Asp Ile Glu Leu Ser Thr Pro Ile Pro Leu Phe
       450                     455                     460
   Gly Phe Glu Lys Ile His Pro His Pro Arg Val Gln Tyr Ser Lys Pro
   465                     470                     475                     480
   Ile Cys Val Leu Ile Asn Glu Gln Asp Phe Ser Cys Ala Asp Phe Phe
                   485                     490                     495
   Pro Val Val Leu Lys Asp Asn Asp Arg Ala Leu Ile Val Gly Thr Arg
               500                     505                     510
   Thr Ala Gly Ala Gly Gly Phe Val Phe Asn Val Gln Phe Pro Asn Arg
           515                     520                     525
   Thr Gly Ile Lys Thr Cys Ser Leu Thr Gly Ser Leu Ala Val Arg Glu
       530                     535                     540
   His Gly Ala Phe Ile Glu Asn Ile Gly Val Glu Pro His Ile Asp Leu
   545                     550                     555                     560
   Pro Phe Thr Ala Asn Asp Ile Arg Tyr Lys Gly Tyr Ser Glu Tyr Leu
                   565                     570                     575
   Asp Lys Val Lys Lys Leu Val Cys Gln Leu Ile Asn Asn Asp Gly Thr
               580                     585                     590
   Ile Ile Leu Ala Glu Asp Gly Ser Phe
           595                     600
<210> 4
   <211> 1806
   <212> DNA
   <213> Chlamydia trachomatis
<400> 4
   atgaaaatga ataggatttg gctattactg cttacctttt cttctgccat acattctcct 60
   gtacaaggag aaagcttggt ttgcaagaat gctcttcaag atttgagttt tttagagcat 120
   ttattacagg ttaaatatgc tcctaaaaca tggaaagagc aatacttagg atgggatctt 180
   gttcaaagct ccgtttctgc acagcagaag cttcgtacac aagaaaatcc atcaacaagt 240
   ttttgccagc aggtccttgc tgattttatc ggaggattaa atgactttca cgctggagta 300
   actttctttg cgatagaaag tgcttacctt ccttataccg tacaaaaaag tagtgacggc 360
   cgtttctact ttgtagatat catgactttt tcttcagaga tccgtgttgg agatgagttg 420
   ctagaggtgg atggggcgcc tgtccaagat gtactcgcta ctctatatgg aagcaatcac 480
   aaagggactg cagctgaaga gtcggctgct ttaagaacac tattttctcg catggcctct 540
   ttagggcaca aagtaccttc tgggcgcact actttaaaga ttcgtcgtcc ttttggtact 600
   acgagagaag ttcgtgtgaa atggcgttat gttcctgaag gtgtaggaga tttggctacc 660
   atagctcctt ctatcagggc tccacagtta cagaaatcga tgagaagctt tttccctaag 720
   aaagatgatg cgtttcatcg gtctagttcg ctattctact ctccaatggt tccgcatttt 780
   tgggcagagc ttcgcaatca ttatgcaacg agtggtttga aaagcgggta caatattggg 840
   agtaccgatg ggtttctccc tgtcattggg cctgttatat gggagtcgga gggtcttttc 900
   cgcgcttata tttcttcggt gactgatggg gatggtaaga gccataaagt aggatttcta 960
   agaattccta catatagttg gcaggacatg gaagattttg atccttcagg accgcctcct 1020
   tgggaagaat ttgctaagat tattcaagta ttttcttcta atacagaagc tttgattatc 1080
   gaccaaacga acaacccagg tggtagtgtc ctttatcttt atgcactgct ttccatgttg 1140
   acagaccgtc ctttagaact tcctaaacat agaatgattc tgactcagga tgaagtggtt 1200
   gatgctttag attggttaac cctgttggaa aacgtagaca caaacgtgga gtctcgcctt 1260
   gctctgggag acaacatgga aggatatact gtggatctac aggttgccga gtatttaaaa 1320
   agctttggac gtcaagtatt gaattgttgg agtaaagggg atatcgagtt atcaacgcct 1380
   attcctcttt ttggttttga gaagattcat ccacatcctc gagttcaata ctctaaaccg 1440
   atttgtgttt tgatcaatga gcaagacttt tcttgtgctg acttcttccc tgtagttttg 1500
   aaagacaatg atcgagctct tattgttggt actcgaacag ctggagctgg aggatttgtc 1560
   tttaatgtgc agttcccaaa tagaactgga ataaaaactt gttctttaac aggatcatta 1620
   gctgttagag agcatggtgc cttcattgag aacatcggag tcgaaccgca tatcgatctg 1680
   ccttttacag cgaatgatat tcgctataaa ggctattccg agtatcttga taaggtcaaa 1740
   aaattggttt gtcagctgat caataacgac ggtaccatta ttcttgcgga agatggtagt 1800
   ttttaa                                                            1806
<210> 5
   <211> 432
   <212> PRT
   <213> Chlamydia trachomatis
<400> 5
   Met Lys Lys Tyr Phe Tyr Lys Gly Phe Val Gly Ala Leu Leu Leu Ala
   1               5                   10                  15
   Cys Gly Ser Thr Asn Leu Ala Phe Ala Gln Ala Ser Ser Met Asp Ser
               20                  25                  30
   Gln Leu Trp Ser Val Glu Asp Leu Asp Ser Tyr Leu Ser Ser Lys Gly
           35                  40                  45
   Phe Val Glu Thr Arg Lys Arg Asp Gly Val Leu Arg Leu Ala Gly Asp
       50                  55                  60
   Val Arg Ala Arg Trp Ile Tyr Ala Lys Glu Asp Leu Glu Thr Thr Gln
   65                  70                  75                  80
   Thr Pro Ala Lys Pro Met Leu Pro Thr Asn Arg Tyr Arg Ser Glu Phe
                   85                  90                  95
   Asn Leu Tyr Val Asp Tyr Thr Ala Ala Asn Ser Trp Met Thr Ser Lys
               100                 105                 110
   Met Asn Trp Val Thr Ile Ala Gly Gly Glu Ser Ser Ala Ala Gly Leu
           115                 120                 125
   Asp Ile Asn Arg Ala Phe Leu Gly Tyr Arg Phe Tyr Lys Asn Pro Glu
       130                 135                 140
   Thr Gln Ala Glu Val Phe Ala Glu Ile Gly Arg Ser Gly Leu Gly Asp
   145                 150                 155                 160
   Ile Phe Asp Ser Asp Val Gln Phe Asn Ser Asn Phe Asp Gly Ile His
                   165                 170                 175
   Leu Tyr Ala Ala Arg Arg Ile Ser Glu Lys Leu Pro Phe Thr Met Ile
               180                 185                 190
   Val His Gly Gly Pro Phe Val Val Asn Met Ala Glu Lys Glu Tyr Ala
           195                 200                 205
   Trp Val Val Glu Ala Ile Leu Asn Lys Leu Pro Gly Asn Phe Val Val
       210                 215                 220
   Lys Thr Ser Val Val Asp Trp Asn Thr Leu Thr Ala Lys Thr Asn Asp
   225                 230                 235                 240
   Pro Ala Asp Ala Ser Ala Ala Gln Pro Ala Lys Pro Asn Thr Lys Tyr
                   245                 250                 255
   Asp Tyr Leu Val Trp Gln Trp Leu Val Gly Lys Ser Thr Ala Met Pro
               260                 265                 270
   Trp Phe Asn Gly Gln Thr Lys Asn Leu Tyr Thr Tyr Gly Ala Tyr Leu
           275                 280                 285
   Phe Asn Pro Leu Ala Glu Ile Pro Glu Asn Trp Lys Gln Ser Thr Thr
       290                 295                 300
   Pro Thr Thr Lys Ile Thr Asn Gly Lys Glu Asn His Ala Trp Phe Ile
   305                 310                 315                 320
   Gly Cys Ser Leu Gly Gly Val Arg Arg Ala Gly Asp Trp Ser Ala Thr
                   325                 330                 335
   Val Arg Tyr Glu Tyr Val Glu Ala Leu Ala Ile Pro Glu Ile Asp Val
               340                 345                 350
   Ala Gly Ile Gly Arg Gly Asn Gln Met Lys Tyr Trp Phe Ala Gln Ala
           355                 360                 365
   Ile Lys Gln Gly Leu Asp Pro Lys Glu Ser Asn Gly Phe Thr Asn Tyr
       370                 375                 380
   Lys Gly Val Ser Tyr Gln Phe Val Met Gly Leu Thr Asp Ser Val Ser
   385                 390                 395                 400
   Phe Arg Ala Tyr Ala Ala Tyr Ser Lys Pro Ala Asn Asp Asn Leu Gly
                   405                 410                 415
   Ser Asp Phe Thr Tyr Arg Lys Tyr Asp Leu Gly Leu Ile Ser Ser Phe
               420                 425                 430
<210> 6
   <211> 1299
   <212> DNA
   <213> Chlamydia trachomatis
<400> 6
   atgaaaaaat acttttataa agggtttgta ggcgcgcttt tattagcttg tgggtctaca 60
   aacttggctt ttgcgcaggc tagttcgatg gatagccagc tatggtctgt tgaagattta 120
   gattcttatt tgagttccaa aggttttgtc gagactcgta agcgcgatgg agttctacgt 180
   ttagctggag atgtccgcgc tcgatggatt tatgcaaaag aggatcttga gacaactcag 240
   actcctgcta aacctatgtt acctaccaat cggtatcgta gtgaattcaa tttgtatgtg 300
   gattacaccg ctgctaatag ttggatgact tcgaaaatga attgggtaac gattgctggc 360
   ggagaatctt ctgcagcagg gttagatatt aatcgtgcct tcttaggata ccgattctac 420
   aaaaacccag aaacgcaagc agaagtattt gcagagattg gtcgctctgg attgggagat 480
   atttttgatt ccgacgttca gtttaatagt aatttcgacg gaattcattt atacgctgcg 540
   cgacgtatta gtgagaaact tcctttcacc atgattgttc atggtggtcc ttttgtcgtg 600
   aatatggcag agaaagagta tgcttgggtc gtggaagcta ttttgaataa actcccagga 660
   aatttcgttg tgaaaacgag tgttgttgac tggaatacgt taacagcaaa aacgaatgat 720
   ccagcagacg caagcgctgc acaaccagct aaacctaata ccaagtacga ttatttagta 780
   tggcaatggt tggttgggaa gagcacagct atgccatggt ttaatggaca aacaaaaaat 840
   ctttacactt acggagccta tctctttaat ccattagcgg aaataccaga gaactggaaa 900
   caatcaacaa ctcctacaac caaaattaca aatggtaagg aaaaccatgc ttggttcatc 960
   ggctgctctc taggcggtgt tcgacgagct ggagactggt ctgcaacagt tcgttatgag 1020
   tatgttgaag ctttagcgat tccagaaatt gatgtcgcgg gtattggtcg cggaaaccaa 1080
   atgaaatatt ggtttgctca agctatcaaa caaggattgg atcctaaaga atctaacggc 1140
   tttactaact ataaaggagt ttcctatcag tttgttatgg gtctgacaga ttcggtttct 1200
   ttccgagctt atgctgctta ttctaagcct gctaacgata accttggtag cgacttcacc 1260
   tatcgtaagt atgacctagg tttaatttct tcattctaa                       1299
<210> 7
   <211> 878
   <212> PRT
   <213> Chlamydia trachomatis
<400> 7
   Met Arg Pro Asp His Met Asn Phe Cys Cys Leu Cys Ala Ala Ile Leu
   1               5                   10                  15
   Ser Ser Thr Ala Val Leu Phe Gly Gln Asp Pro Leu Gly Glu Thr Ala
               20                  25                  30
   Leu Leu Thr Lys Asn Pro Asn His Val Val Cys Thr Phe Phe Glu Asp
           35                  40                  45
   Cys Thr Met Glu Ser Leu Phe Pro Ala Leu Cys Ala His Ala Ser Gln
       50                  55                  60
   Asp Asp Pro Leu Tyr Val Leu Gly Asn Ser Tyr Cys Trp Phe Val Ser
   65                  70                  75                  80
   Lys Leu His Ile Thr Asp Pro Lys Glu Ala Leu Phe Lys Glu Lys Gly
                   85                  90                  95
   Asp Leu Ser Ile Gln Asn Phe Arg Phe Leu Ser Phe Thr Asp Cys Ser
               100                 105                 110
   Ser Lys Glu Ser Ser Pro Ser Ile Ile His Gln Lys Asn Gly Gln Leu
           115                 120                 125
   Ser Leu Arg Asn Asn Gly Ser Met Ser Phe Cys Arg Asn His Ala Glu
       130                 135                 140
   Gly Ser Gly Gly Ala Ile Ser Ala Asp Ala Phe Ser Leu Gln His Asn
   145                 150                 155                 160
   Tyr Leu Phe Thr Ala Phe Glu Glu Asn Ser Ser Lys Gly Asn Gly Gly
   165 170 175
   Ala Ile Gln Ala Gln Thr Phe Ser Leu Ser Arg Asn Val Ser Pro Ile
   180 185 190
   Ser Phe Ala Arg Asn Arg Ala Asp Leu Asn Gly Gly Ala Ile Cys Cys
   195 200 205
   Ser Asn Leu Ile Cys Ser Gly Asn Val Asn Pro Leu Phe Phe Thr Gly
   210 215 220
   Asn Ser Ala Thr Asn Gly Gly Ala Ile Cys Cys Ile Ser Asp Leu Asn
   225 230 235 240
   Thr Ser Glu Lys Gly Ser Leu Ser Leu Ala Cys Asn Gln Glu Thr Leu
   245 250 255
   Phe Ala Ser Asn Ser Ala Lys Glu Lys Gly Gly Ala Ile Tyr Ala Lys
   260 265 270
   His Met Val Leu Arg Tyr Asn Gly Pro Val Ser Phe Ile Asn Asn Ser
   275 280 285
   Ala Lys Ile Gly Gly Ala Ile Ala Ile Gln Ser Gly Gly Ser Leu Ser
   290 295 300
   Ile Leu Ala Gly Glu Gly Ser Val Leu Phe Gln Asn Asn Ser Gln Arg
   305 310 315 320
   Thr Ser Asp Gln Gly Leu Val Arg Asn Ala Ile Tyr Leu Glu Lys Asp
   325 330 335
   Ala Ile Leu Ser Ser Leu Glu Ala Arg Asn Gly Asp Ile Leu Phe Phe
   340 345 350
   Asp Pro Ile Val Gln Glu Ser Ser Ser Lys Glu Ser Pro Leu Pro Ser
   355 360 365
   Ser Leu Gln Ala Ser Val Thr Ser Pro Thr Pro Ala Thr Ala Ser Pro
   370 375 380
   Leu Val Ile Gln Thr Ser Ala Asn Arg Ser Val Ile Phe Ser Ser Glu
   385 390 395 400
   Arg Leu Ser Glu Glu Glu Lys Thr Pro Asp Asn Leu Thr Ser Gln Leu
   405 410 415
   Gln Gln Pro Ile Glu Leu Lys Ser Gly Arg Leu Val Leu Lys Asp Arg
   420 425 430
   Ala Val Leu Ser Ala Pro Ser Leu Ser Gln Asp Pro Gln Ala Leu Leu
   435 440 445
   Ile Met Glu Ala Gly Thr Ser Leu Lys Thr Ser Ser Asp Leu Lys Leu
   450 455 460
   Ala Thr Leu Ser Ile Pro Leu His Ser Leu Asp Thr Glu Lys Ser Val
   465 470 475 480
   Thr Ile His Ala Pro Asn Leu Ser Ile Gln Lys Ile Phe Leu Ser Asn
   485 490 495
   Ser Gly Asp Glu Asn Phe Tyr Glu Asn Val Glu Leu Leu Ser Lys Glu
   500 505 510
   Gln Asn Asn Ile Pro Leu Leu Thr Leu Ser Lys Glu Gln Ser His Leu
   515 520 525
   His Leu Pro Asp Gly Asn Leu Ser Ser His Phe Gly Tyr Gln Gly Asp
   530 535 540
   Trp Thr Phe Ser Trp Lys Asp Ser Asp Glu Gly His Ser Leu Ile Ala
   545 550 555 560
   Asn Trp Thr Pro Lys Asn Tyr Val Pro His Pro Glu Arg Gln Ser Thr
   565 570 575
   Leu Val Ala Asn Thr Leu Trp Asn Thr Tyr Ser Asp Met Gln Ala Val
   580 585 590
   Gln Ser Met Ile Asn Thr Ile Ala His Gly Gly Ala Tyr Leu Phe Gly
   595 600 605
   Thr Trp Gly Ser Ala Val Ser Asn Leu Phe Tyr Ala His Asp Ser Ser
   610 615 620
   Gly Lys Pro Ile Asp Asn Trp His His Arg Ser Leu Gly Tyr Leu Phe
   625 630 635 640
   Gly Ile Ser Thr His Ser Leu Asp Asp His Ser Phe Cys Leu Ala Ala
   645 650 655
   Gly Gln Leu Leu Gly Lys Ser Ser Asp Ser Phe Ile Thr Ser Thr Glu
   660 665 670
   Thr Thr Ser Tyr Ile Ala Thr Val Gln Ala Gln Leu Ala Thr Pro Leu
   675 680 685
   Met Lys Ile Ser Ala Gln Ala Cys Tyr Asn Glu Ser Ile His Glu Leu
   690 695 700
   Lys Thr Lys Tyr Arg Ser Phe Ser Lys Glu Gly Phe Gly Ser Trp His
   705 710 715 720
   Ser Val Ala Val Ser Gly Glu Val Cys Ala Ser Ile Pro Ile Val Ser
   725 730 735
   Asn Gly Ser Gly Leu Phe Ser Ser Phe Ser Ile Phe Ser Lys Leu Gln
   740 745 750
   Gly Phe Ser Gly Thr Gln Asp Gly Phe Glu Glu Ser Ser Gly Glu Ile
   755 760 765
   Arg Ser Phe Ser Ala Ser Ser Phe Arg Asn Ile Ser Leu Pro Met Gly
   770 775 780
   Ile Thr Phe Glu Lys Lys Ser Gln Lys Thr Arg Asn Tyr Tyr Tyr Phe
   785 790 795 800
   Leu Gly Ala Tyr Ile Gln Asp Leu Lys Arg Asp Val Glu Ser Gly Pro
   805 810 815
   Val Val Leu Leu Lys Asn Ala Val Ser Trp Asp Ala Pro Met Ala Asn
   820 825 830
   Leu Asp Ser Arg Ala Tyr Met Phe Arg Leu Thr Asn Gln Arg Ala Leu
   835 840 845
   His Arg Leu Gln Thr Leu Leu Asn Val Ser Tyr Val Leu Arg Gly Gln
   850 855 860
   Ser His Ser Tyr Ser Leu Asp Leu Gly Thr Thr Tyr Arg Phe
   865 870 875
<210> 8
   <211> 2637
   <212> DNA
   <213> Chlamydia trachomatis
<400> 8
   atgcgacctg atcatatgaa cttctgttgt ctatgtgctg ctattttgtc atccacagcg 60
   gtcctctttg gccaggatcc cttaggtgaa accgccctcc tcactaaaaa tcctaatcat 120
   gtcgtctgta catttttgga ggactgtacc atggagagcc tctttcctgc tctttgtgct 180
   catgcatcac aagatgatcc tttgtatgta cttggaaatt cctactgttg gttcgtatct 240
   aaactccata tcacggaccc caaagaggct ctttttaaag aaaaaggaga tctttccatt 300
   caaaattttc gcttcctttc cttcacagat tgctcttcca aggaaagctc tccttctatt 360
   attcatcaaa agaatggtca gttatccttg cgcaataatg gtagcatgag tttctgtcga 420
   aatcatgctg aaggctctgg aggagccatc tctgcggatg ccttttctct acaacacaac 480
   tatcttttca cagcttttga agagaattct tctaaaggaa atggcggagc cattcaggct 540
   caaaccttct ctttatctag aaatgtgtcg cctatttctt tcgcccgtaa tcgtgcggat 600
   ttaaatggcg gcgctatttg ctgtagtaat cttatttgtt cagggaatgt aaaccctctc 660
   tttttcactg gaaactccgc cacgaatgga ggcgctattt gttgtatcag cgatctaaac 720
   acctcagaaa aaggctctct ctctcttgct tgtaaccaag aaacgctatt tgcaagcaat 780
   tctgctaaag aaaaaggcgg ggctatttat gccaagcaca tggtattgcg ttataacggt 840
   cctgtttcct tcattaacaa cagcgctaaa ataggtggag ctatcgccat ccagtccgga 900
   gggagtctct ctatccttgc aggtgaagga tctgttctgt tccagaataa ctcccaacgc 960
   acctccgacc aaggtctagt aagaaacgcc atctacttag agaaagatgc gattctttct 1020
   tccttagaag ctcgcaacgg agatattctt ttctttgatc ctattgtaca agaaagtagc 1080
   agcaaagaat cgcctcttcc ctcctctttg caagccagcg tgacttctcc caccccagcc 1140
   accgcatctc ctttagttat tcagacaagt gcaaaccgtt cagtgatttt ctcgagcgaa 1200
   cgtctttctg aagaagaaaa aactcctgat aacctcactt cccaactaca gcagcctatc 1260
   gaactgaaat ccggacgctt agttttaaaa gatcgcgctg tcctttccgc gccttctctc 1320
   tctcaggatc ctcaagctct cctcattatg gaagcgggaa cttctttaaa aacttcctct 1380
   gatttgaagt tagctacgct aagtattccc cttcattcct tagatactga aaaaagcgta 1440
   actatccacg cccctaacct ttctatccaa aagatcttcc tctctaattc tggagatgag 1500
   aatttttatg aaaatgtaga gcttctcagt aaagagcaaa acaatattcc tctccttact 1560
   ctctctaaag agcaatctca tttacatctt cctgatggga acctctcttc tcactttgga 1620
   tatcaaggag attggacttt ttcttggaaa gattctgatg aagggcattc tctgattgct 1680
   aattggacgc ctaaaaacta tgtgcctcat ccagaacgtc aatctacact cgttgcgaac 1740
   actctttgga acacctattc cgatatgcaa gctgtgcagt cgatgattaa tacaatagcg 1800
   cacggaggag cctatctatt tggaacgtgg ggatctgctg tttctaattt attctatgct 1860
   cacgacagct ctgggaaacc tatcgataat tggcatcata gaagccttgg ctacctattc 1920
   ggtatcagta ctcacagttt agatgaccat tctttctgct tggctgcagg acaattactc 1980
   gggaaatcgt ccgattcctt tattacgtct acagaaacga cctcctatat agctactgta 2040
   caagcgcaac tcgctacccc tctaatgaaa atctctgcac aggcatgcta taatgaaagt 2100
   atccatgagc taaaaacaaa atatcgctcc ttctctaaag aaggattcgg atcctggcat 2160
   agcgttgcag tatccggaga agtgtgcgca tcgattccta ttgtatccaa tggttccgga 2220
   ctgttcagct ccttctctat tttctctaaa ctgcaaggat tttcaggaac acaggacggt 2280
   tttgaggaga gttcgggaga gattcggtcc ttttctgcca gctctttcag aaatatttca 2340
   cttcctatgg gaataacatt tgaaaaaaaa tcccaaaaaa cacgaaacta ctattacttt 2400
   ctgggagcct acatccaaga cctaaaacgt gatgtggaat cgggacctgt agtgttactc 2460
   aaaaatgccg tctcctggga tgctcctatg gcgaacttgg attcgcgagc ctacatgttc 2520
   aggcttacga atcaaagagc tctgcataga cttcagacgc tgttaaatgt gtcttacgta 2580
   ctgcgcgggc aaagccatag ttactccctg gatctgggga ccacttacag gttctag    2637
<210> 9
   <211> 1013
   <212> PRT
   <213> Chlamydia trachomatis
<400> 9
   Met Gln Thr Ser Phe His Lys Phe Phe Leu Ser Met Ile Leu Ala Tyr
   1 5 10 15
   Ser Cys Cys Ser Leu Ser Gly Gly Gly Tyr Ala Ala Glu Ile Met Ile
   20 25 30
   Pro Gln Gly Ile Tyr Asp Gly Glu Thr Leu Thr Val Ser Phe Pro Tyr
   35 40 45
   Thr Val Ile Gly Asp Pro Ser Gly Thr Thr Val Phe Ser Ala Gly Glu
   50 55 60
   Leu Thr Leu Lys Asn Leu Asp Asn Ser Ile Ala Ala Leu Pro Leu Ser
   65 70 75 80
   Cys Phe Gly Asn Leu Leu Gly Ser Phe Thr Val Leu Gly Arg Gly His
   85 90 95
   Ser Leu Thr Phe Glu Asn Ile Arg Thr Ser Thr Asn Gly Ala Ala Leu
   100 105 110
   Ser Asp Ser Ala Asn Ser Gly Leu Phe Thr Ile Glu Gly Phe Lys Glu
   115 120 125
   Leu Ser Phe Ser Asn Cys Asn Ser Leu Leu Ala Val Leu Pro Ala Ala
   130 135 140
   Thr Thr Asn Asn Gly Ser Gln Thr Pro Thr Thr Thr Ser Thr Pro Ser
   145 150 155 160
   Asn Gly Thr Ile Tyr Ser Lys Thr Asp Leu Leu Leu Leu Asn Asn Glu
   165 170 175
   Lys Phe Ser Phe Tyr Ser Asn Leu Val Ser Gly Asp Gly Gly Ala Ile
   180 185 190
   Asp Ala Lys Ser Leu Thr Val Gln Gly Ile Ser Lys Leu Cys Val Phe
   195 200 205
   Gln Glu Asn Thr Ala Gln Ala Asp Gly Gly Ala Cys Gln Val Val Thr
   210 215 220
   Ser Phe Ser Ala Met Ala Asn Glu Ala Pro Ile Ala Phe Ile Ala Asn
   225 230 235 240
   Val Ala Gly Val Arg Gly Gly Gly Ile Ala Ala Val Gln Asp Gly Gln
   245 250 255
   Gln Gly Val Ser Ser Ser Thr Ser Thr Glu Asp Pro Val Val Ser Phe
   260 265 270
   Ser Arg Asn Thr Ala Val Glu Phe Asp Gly Asn Val Ala Arg Val Gly
   275 280 285
   Gly Gly Ile Tyr Ser Tyr Gly Asn Val Ala Phe Leu Asn Asn Gly Lys
   290 295 300
   Thr Leu Phe Leu Asn Asn Val Ala Ser Pro Val Tyr Ile Ala Ala Glu
   305 310 315 320
   Gln Pro Thr Asn Gly Gln Ala Ser Asn Thr Ser Asp Asn Tyr Gly Asp
   325 330 335
   Gly Gly Ala Ile Phe Cys Lys Asn Gly Ala Gln Ala Ala Gly Ser Asn
   340 345 350
   Asn Ser Gly Ser Val Ser Phe Asp Gly Glu Gly Val Val Phe Phe Ser
   355 360 365
   Ser Asn Val Ala Ala Gly Lys Gly Gly Ala Ile Tyr Ala Lys Lys Leu
   370 375 380
   Ser Val Ala Asn Cys Gly Pro Val Gln Phe Leu Gly Asn Ile Ala Asn
   385                 390                 395                 400
   Asp Gly Gly Ala Ile Tyr Leu Gly Glu Ser Gly Glu Leu Ser Leu Ser
                   405                 410                 415
   Ala Asp Tyr Gly Asp Ile Ile Phe Asp Gly Asn Leu Lys Arg Thr Ala
               420                 425                 430
   Lys Glu Asn Ala Ala Asp Val Asn Gly Val Thr Val Ser Ser Gln Ala
           435                 440                 445
   Ile Ser Met Gly Ser Gly Gly Lys Ile Thr Thr Leu Arg Ala Lys Ala
       450                 455                 460
   Gly His Gln Ile Leu Phe Asn Asp Pro Ile Glu Met Ala Asn Gly Asn
   465                 470                 475                 480
   Asn Gln Pro Ala Gln Ser Ser Glu Pro Leu Lys Ile Asn Asp Gly Glu
                   485                 490                 495
   Gly Tyr Thr Gly Asp Ile Val Phe Ala Asn Gly Asn Ser Thr Leu Tyr
               500                 505                 510
   Gln Asn Val Thr Ile Glu Gln Gly Arg Ile Val Leu Arg Glu Lys Ala
           515                 520                 525
   Lys Leu Ser Val Asn Ser Leu Ser Gln Thr Gly Gly Ser Leu Tyr Met
       530                 535                 540
   Glu Ala Gly Ser Thr Leu Asp Phe Val Thr Pro Gln Pro Pro Gln Gln
   545                 550                 555                 560
   Pro Pro Ala Ala Asn Gln Leu Ile Thr Leu Ser Asn Leu His Leu Ser
                   565                 570                 575
   Leu Ser Ser Leu Leu Ala Asn Asn Ala Val Thr Asn Pro Pro Thr Asn
               580                 585                 590
   Pro Pro Ala Gln Asp Ser His Pro Ala Ile Ile Gly Ser Thr Thr Ala
           595                 600                 605
   Gly Ser Val Thr Ile Ser Gly Pro Ile Phe Phe Glu Asp Leu Asp Asp
       610                 615                 620
   Thr Ala Tyr Asp Arg Tyr Asp Trp Leu Gly Ser Asn Gln Lys Ile Asp
   625                 630                 635                 640
   Val Leu Lys Leu Gln Leu Gly Thr Gln Pro Ser Ala Asn Ala Pro Ser
                   645                 650                 655
   Asp Leu Thr Leu Gly Asn Glu Met Pro Lys Tyr Gly Tyr Gln Gly Ser
               660                 665                 670
   Trp Lys Leu Ala Trp Asp Pro Asn Thr Ala Asn Asn Gly Pro Tyr Thr
           675                 680                 685
   Leu Lys Ala Thr Trp Thr Lys Thr Gly Tyr Asn Pro Gly Pro Glu Arg
       690                 695                 700
   Val Ala Ser Leu Val Pro Asn Ser Leu Trp Gly Ser Ile Leu Asp Ile
   705                 710                 715                 720
   Arg Ser Ala His Ser Ala Ile Gln Ala Ser Val Asp Gly Arg Ser Tyr
                   725                 730                 735
   Cys Arg Gly Leu Trp Val Ser Gly Val Ser Asn Phe Phe Tyr His Asp
               740                 745                 750
   Arg Asp Ala Leu Gly Gln Gly Tyr Arg Tyr Ile Ser Gly Gly Tyr Ser
           755                 760                 765
   Leu Gly Ala Asn Ser Tyr Phe Gly Ser Ser Met Phe Gly Leu Ala Phe
       770                 775                 780
   Thr Glu Val Phe Gly Arg Ser Lys Asp Tyr Val Val Cys Arg Ser Asn
   785                 790                 795                 800
   His His Ala Cys Ile Gly Ser Val Tyr Leu Ser Thr Lys Gln Ala Leu
                   805                 810                 815
   Cys Gly Ser Tyr Leu Phe Gly Asp Ala Phe Ile Arg Ala Ser Tyr Gly
               820                 825                 830
   Phe Gly Asn Gln His Met Lys Thr Ser Tyr Thr Phe Ala Glu Glu Ser
           835                 840                 845
   Asp Val Arg Trp Asp Asn Asn Cys Leu Val Gly Glu Ile Gly Val Gly
       850                 855                 860
   Leu Pro Ile Val Ile Thr Pro Ser Lys Leu Tyr Leu Asn Glu Leu Arg
   865                 870                 875                 880
   Pro Phe Val Gln Ala Glu Phe Ser Tyr Ala Asp His Glu Ser Phe Thr
                   885                 890                 895
   Glu Glu Gly Asp Gln Ala Arg Ala Phe Arg Ser Gly His Leu Met Asn
               900                 905                 910
   Leu Ser Val Pro Val Gly Val Lys Phe Asp Arg Cys Ser Ser Thr His
   915 920 925
   Pro Asn Lys Tyr Ser Phe Met Gly Ala Tyr Ile Cys Asp Ala Tyr Arg
   930 935 940
   Thr Ile Ser Gly Thr Gln Thr Thr Leu Leu Ser His Gln Glu Thr Trp
   945 950 955 960
   Thr Thr Asp Ala Phe His Leu Ala Arg His Gly Val Ile Val Arg Gly
   965 970 975
   Ser Met Tyr Ala Ser Leu Thr Ser Asn Ile Glu Val Tyr Gly His Gly
   980 985 990
   Arg Tyr Glu Tyr Arg Asp Thr Ser Arg Gly Tyr Gly Leu Ser Ala Gly
   995 1000 1005
   Ser Lys Val Arg Phe
   1010
<210> 10
   <211> 3042
   <212> DNA
   <213> Chlamydia trachomatis
<400> 10
   atgcaaacgt ctttccataa gttctttctt tcaatgattc tagcttattc ttgctgctct 60
   ttaagtgggg gggggtatgc agcagaaatc atgattcctc aaggaattta cgatggggag 120
   acgttaactg tatcatttcc ctatactgtt ataggagatc cgagtgggac tactgttttt 180
   tctgcaggag agttaacgtt aaaaaatctt gacaattcta ttgcagcttt gcctttaagt 240
   tgttttggga acttattagg gagttttact gttttaggga gaggacactc gttgactttc 300
   gagaacatac ggacttctac aaatggagct gcactaagtg acagcgctaa tagcgggtta 360
   tttactattg agggttttaa agaattatct ttttccaatt gcaactcatt acttgccgta 420
   ctgcctgctg caacgactaa taatggtagc cagactccga cgacaacatc tacaccgtct 480
   aatggtacta tttattctaa aacagatctt ttgttactca ataatgagaa gttctcattc 540
   tatagtaatt tagtctctgg agatggggga gctatagatg ctaagagctt aacggttcaa 600
   ggaattagca agctttgtgt cttccaagaa aatactgctc aagctgatgg gggagcttgt 660
   caagtagtca ccagtttctc tgctatggct aacgaggctc ctattgcctt tatagcgaat 720
   gttgcaggag taagaggggg agggattgct gctgttcagg atgggcagca gggagtgtca 780
   tcatctactt caacagaaga tccagtagta agtttttcca gaaatactgc ggtagagttt 840
   gatgggaacg tagcccgagt aggaggaggg atttactcct acgggaacgt tgctttcctg 900
   aataatggaa aaaccttgtt tctcaacaat gttgcttctc ctgtttacat tgctgctgag 960
   caaccaacaa atggacaggc ttctaatacg agtgataatt acggagatgg aggagctatc 1020
   ttctgtaaga atggtgcgca agcagcagga tccaataact ctggatcagt ttcctttgat 1080
   ggagagggag tagttttctt tagtagcaat gtagctgctg ggaaaggggg agctatttat 1140
   gccaaaaagc tctcggttgc taactgtggc cctgtacaat tcttagggaa tatcgctaat 1200
   gatggtggag cgatttattt aggagaatct ggagagctca gtttatctgc tgattatgga 1260
   gatattattt tcgatgggaa tcttaaaaga acagccaaag agaatgctgc cgatgttaat 1320
   ggcgtaactg tgtcctcaca agccatttcg atgggatcgg gagggaaaat aacgacatta 1380
   agagctaaag cagggcatca gattctcttt aatgatccca tcgagatggc aaacggaaat 1440
   aaccagccag cgcagtcttc cgaacctcta aaaattaacg atggtgaagg atacacaggg 1500
   gatattgttt ttgctaatgg aaacagtact ttgtaccaaa atgttacgat agagcaagga 1560
   aggattgttc ttcgtgaaaa ggcaaaatta tcagtgaatt ctctaagtca gacaggtggg 1620
   agtctgtata tggaagctgg gagtacattg gattttgtaa ctccacaacc accacaacag 1680
   cctcctgccg ctaatcagtt gatcacgctt tccaatctgc atttgtctct ttcttctttg 1740
   ttagcaaaca atgcagttac gaatcctcct accaatcctc cagcgcaaga ttctcatcct 1800
   gcaatcattg gtagcacaac tgctggttct gttacaatta gtgggcctat cttttttgag 1860
   gatttggatg atacagctta tgataggtat gattggctag gttctaatca aaaaatcgat 1920
   gtcctgaaat tacagttagg gactcagccc tcagctaatg ccccatcaga tttgactcta 1980
   gggaatgaga tgcctaagta tggctatcaa ggaagctgga agcttgcgtg ggatcctaat 2040
   acagcaaata atggtcctta tactctgaaa gctacatgga ctaaaactgg gtataatcct 2100
   gggcctgagc gagtagcttc tttggttcca aatagtttat ggggatccat tttagatata 2160
   cgatctgcgc attcagcaat tcaagcaagt gtggatgggc gctcttattg tcgaggatta 2220
   tgggtttctg gagtttcgaa tttcttctat catgaccgcg atgctttagg tcagggatat 2280
   cggtatatta gtgggggtta ttccttagga gcaaactcct actttggatc atcgatgttt 2340
   ggtctagcat ttaccgaagt atttggtaga tctaaagatt atgtagtgtg tcgttccaat 2400
   catcatgctt gcataggatc cgtttatcta tctaccaaac aagctttatg tggatcctat 2460
   ttgttcggag atgcgtttat ccgtgctagc tacgggtttg ggaaccagca tatgaaaacc 2520
   tcatacacat ttgcagagga gagcgatgtt cgttgggata ataactgtct ggttggagag 2580
   attggagtgg gattaccgat tgtgattact ccatctaagc tctatttgaa tgagttgcgt 2640
   cctttcgtgc aagctgagtt ttcttatgcc gatcatgaat cttttacaga ggaaggcgat 2700
   caagctcggg cattcaggag tggacatctc atgaatctat cagttcctgt tggagtaaaa 2760
   tttgatcgat gttctagtac acaccctaat aaatatagct ttatgggggc ttatatctgt 2820
   gatgcttatc gcaccatctc tgggactcag acaacactcc tatcccatca agagacatgg 2880
   acaacagatg cctttcattt ggcaagacat ggagtcatag ttagagggtc tatgtatgct 2940
   tctctaacaa gcaatataga agtatatggc catggaagat atgagtatcg agatacttct 3000
   cgaggttatg gtttgagtgc aggaagtaaa gtccggttct aa                   3042
<210> 11
   <211> 1034
   <212> PRT
   <213> Chlamydia trachomatis
<400> 11
   Met Ile Lys Arg Thr Ser Leu Ser Phe Ala Cys Leu Ser Phe Phe Tyr
   1               5                   10                  15
   Leu Ser Thr Ile Ser Ile Leu Gln Ala Asn Glu Thr Asp Thr Leu Gln
               20                  25                  30
   Phe Arg Arg Phe Thr Phe Ser Asp Arg Glu Ile Gln Phe Val Leu Asp
           35                  40                  45
   Pro Ala Ser Leu Ile Thr Ala Gln Asn Ile Val Leu Ser Asn Leu Gln
       50                  55                  60
   Ser Asn Gly Thr Gly Ala Cys Thr Ile Ser Gly Asn Thr Gln Thr Gln
   65                  70                  75                  80
   Ile Phe Ser Asn Ser Val Asn Thr Thr Ala Asp Ser Gly Gly Ala Phe
                   85                  90                  95
   Asp Met Val Thr Thr Ser Phe Thr Ala Ser Asp Asn Ala Asn Leu Leu
               100                 105                 110
   Phe Cys Asn Asn Tyr Cys Thr His Asn Lys Gly Gly Gly Ala Ile Arg
           115                 120                 125
   Ser Gly Gly Pro Ile Arg Phe Leu Asn Asn Gln Asp Val Leu Phe Tyr
       130                 135                 140
   Asn Asn Ile Ser Ala Gly Ala Lys Tyr Val Gly Thr Gly Asp His Asn
   145                 150                 155                 160
   Glu Lys Asn Arg Gly Gly Ala Leu Tyr Ala Thr Thr Ile Thr Leu Thr
                   165                 170                 175
   Gly Asn Arg Thr Leu Ala Phe Ile Asn Asn Met Ser Gly Asp Cys Gly
               180                 185                 190
   Gly Ala Ile Ser Ala Asp Thr Gln Ile Ser Ile Thr Asp Thr Val Lys
           195                 200                 205
   Gly Ile Leu Phe Glu Asn Asn His Thr Leu Asn His Ile Pro Tyr Thr
       210                 215                 220
   Gln Ala Glu Asn Met Ala Arg Gly Gly Ala Ile Cys Ser Arg Arg Asp
   225                 230                 235                 240
   Leu Cys Ser Ile Ser Asn Asn Ser Gly Pro Ile Val Phe Asn Tyr Asn
                   245                 250                 255
   Gln Gly Gly Lys Gly Gly Ala Ile Ser Ala Thr Arg Cys Val Ile Asp
               260                 265                 270
   Asn Asn Lys Glu Arg Ile Ile Phe Ser Asn Asn Ser Ser Leu Gly Trp
           275                 280                 285
   Ser Gln Ser Ser Ser Ala Ser Asn Gly Gly Ala Ile Gln Thr Thr Gln
       290                 295                 300
   Gly Phe Thr Leu Arg Asn Asn Lys Gly Ser Ile Tyr Phe Asp Ser Asn
   305                 310                 315                 320
   Thr Ala Thr His Ala Gly Gly Ala Ile Asn Cys Gly Tyr Ile Asp Ile
                   325                 330                 335
   Arg Asp Asn Gly Pro Val Tyr Phe Leu Asn Asn Ser Ala Ala Trp Gly
               340                 345                 350
   Ala Ala Phe Asn Leu Ser Lys Pro Arg Ser Ala Thr Asn Tyr Ile His
           355                 360                 365
   Thr Gly Thr Gly Asp Ile Val Phe Asn Asn Asn Val Val Phe Thr Leu
       370                 375                 380
   Asp Gly Asn Leu Leu Gly Lys Arg Lys Leu Phe His Ile Asn Asn Asn
   385                 390                 395                 400
   Glu Ile Thr Pro Tyr Thr Leu Ser Leu Gly Ala Lys Lys Asp Thr Arg
                   405                 410                 415
   Ile Tyr Phe Tyr Asp Leu Phe Gln Trp Glu Arg Val Lys Glu Asn Thr
   420 425 430
   Ser Asn Asn Pro Pro Ser Pro Thr Ser Arg Asn Thr Ile Thr Val Asn
   435 440 445
   Pro Glu Thr Glu Phe Ser Gly Ala Val Val Phe Ser Tyr Asn Gln Met
   450 455 460
   Ser Ser Asp Ile Arg Thr Leu Met Gly Lys Glu His Asn Tyr Ile Lys
   465 470 475 480
   Glu Ala Pro Thr Thr Leu Lys Phe Gly Thr Leu Ala Ile Glu Asp Asp
   485 490 495
   Ala Glu Leu Glu Ile Phe Asn Ile Pro Phe Thr Gln Asn Pro Thr Ser
   500 505 510
   Leu Leu Ala Leu Gly Ser Gly Ala Thr Leu Thr Val Gly Lys His Gly
   515 520 525
   Lys Leu Asn Ile Thr Asn Leu Gly Val Ile Leu Pro Ile Ile Leu Lys
   530 535 540
   Glu Gly Lys Ser Pro Pro Cys Ile Arg Val Asn Pro Gln Asp Met Thr
   545 550 555 560
   Gln Asn Thr Gly Thr Gly Gln Thr Pro Ser Ser Thr Ser Ser Ile Ser
   565 570 575
   Thr Pro Met Ile Ile Phe Asn Gly Arg Leu Ser Ile Val Asp Glu Asn
   580 585 590
   Tyr Glu Ser Val Tyr Asp Ser Met Asp Leu Ser Arg Gly Lys Ala Glu
   595 600 605
   Gln Leu Ile Leu Ser Ile Glu Thr Thr Asn Asp Gly Gln Leu Asp Ser
   610 615 620
   Asn Trp Gln Ser Ser Leu Asn Thr Ser Leu Leu Ser Pro Pro His Tyr
   625 630 635 640
   Gly Tyr Gln Gly Leu Trp Thr Pro Asn Trp Ile Thr Thr Thr Tyr Thr
   645 650 655
   Ile Thr Leu Asn Asn Asn Ser Ser Ala Pro Thr Ser Ala Thr Ser Ile
   660 665 670
   Ala Glu Gln Lys Lys Thr Ser Glu Thr Phe Thr Pro Ser Asn Thr Thr
   675 680 685
   Thr Ala Ser Ile Pro Asn Ile Lys Ala Ser Ala Gly Ser Gly Ser Gly
   690 695 700
   Ser Ala Ser Asn Ser Gly Glu Val Thr Ile Thr Lys His Thr Leu Val
   705 710 715 720
   Val Asn Trp Ala Pro Val Gly Tyr Ile Val Asp Pro Ile Arg Arg Gly
   725 730 735
   Asp Leu Ile Ala Asn Ser Leu Val His Ser Gly Arg Asn Met Thr Met
   740 745 750
   Gly Leu Arg Ser Leu Leu Pro Asp Asn Ser Trp Phe Ala Leu Gln Gly
   755 760 765
   Ala Ala Thr Thr Leu Phe Thr Lys Gln Gln Lys Arg Leu Ser Tyr His
   770 775 780
   Gly Tyr Ser Ser Ala Ser Lys Gly Tyr Thr Val Ser Ser Gln Ala Ser
   785 790 795 800
   Gly Ala His Gly His Lys Phe Leu Leu Ser Phe Ser Gln Ser Ser Asp
   805 810 815
   Lys Met Lys Glu Lys Glu Thr Asn Asn Arg Leu Ser Ser Arg Tyr Tyr
   820 825 830
   Leu Ser Ala Leu Cys Phe Glu His Pro Met Phe Asp Arg Ile Ala Leu
   835 840 845
   Ile Gly Ala Ala Ala Cys Asn Tyr Gly Thr His Asn Met Arg Ser Phe
   850 855 860
   Tyr Gly Thr Lys Lys Ser Ser Lys Gly Lys Phe His Ser Thr Thr Leu
   865 870 875 880
   Gly Ala Ser Leu Arg Cys Glu Leu Arg Asp Ser Met Pro Leu Arg Ser
   885 890 895
   Ile Met Leu Thr Pro Phe Ala Gln Ala Leu Phe Ser Arg Thr Glu Pro
   900 905 910
   Ala Ser Ile Arg Glu Ser Gly Asp Leu Ala Arg Leu Phe Thr Leu Glu
   915 920 925
   Gln Ala His Thr Ala Val Val Ser Pro Ile Gly Ile Lys Gly Ala Tyr
   930 935 940
   Ser Ser Asp Thr Trp Pro Thr Leu Ser Trp Glu Met Glu Leu Ala Tyr
   945 950 955 960
   Gln Pro Thr Leu Tyr Trp Lys Arg Pro Leu Leu Asn Thr Leu Leu Ile
   965 970 975
   Gln Asn Asn Gly Ser Trp Val Thr Thr Asn Thr Pro Leu Ala Lys His
   980 985 990
   Ser Phe Tyr Gly Arg Gly Ser His Ser Leu Lys Phe Ser His Leu Lys
   995 1000 1005
   Leu Phe Ala Asn Tyr Gln Ala Glu Val Ala Thr Ser Thr Val Ser His
   1010 1015 1020
   Tyr Ile Asn Ala Gly Gly Ala Leu Val Phe
   1025 1030
<210> 12
   <211> 3105
   <212> DNA
   <213> Chlamydia trachomatis
<400> 12
   atgattaaaa gaacttctct atcctttgct tgcctcagtt ttttttatct ttcaactata 60
   tccattttgc aagctaatga aacggatacg ctacagttcc ggcgatttac tttttcggat 120
   agagagattc agttcgtcct agatcccgcc tctttaatta ccgcccaaaa catcgtttta 180
   tctaatttac agtcaaacgg aaccggagcc tgtaccattt caggcaatac gcaaactcaa 240
   atcttttcta attccgttaa caccaccgca gattctggtg gagcctttga tatggttact 300
   acctcattca cggcctctga taatgctaat ctactcttct gcaacaacta ctgcacacat 360
   aataaaggcg gaggagctat tcgttccgga ggacctattc gattcttaaa taatcaagac 420
   gtgctttttt ataataacat atcggcaggg gctaaatatg ttggaacagg agatcacaac 480
   gaaaaaaata ggggcggtgc gctttatgca actactatca ctttgacagg gaatcgaact 540
   cttgccttta ttaacaatat gtctggagac tgcggtggag ccatctctgc tgacactcaa 600
   atatcaataa ctgataccgt taaaggaatt ttatttgaaa acaatcacac gctcaatcat 660
   ataccgtaca cgcaagctga aaatatggca cgaggaggag caatctgtag tagaagagac 720
   ttgtgctcaa tcagcaataa ttctggtccc atagttttta actataacca aggcgggaaa 780
   ggtggagcta ttagcgctac ccgatgtgtt attgacaata acaaagaaag aatcatcttt 840
   tcaaacaata gttccctggg atggagccaa tcttcttctg caagtaacgg aggagccatt 900
   caaacgacac aaggatttac tttacgaaat aataaaggct ctatctactt cgacagcaac 960
   actgctacac acgccggggg agccattaac tgtggttaca ttgacatccg agataacgga 1020
   cccgtctatt ttctaaataa ctctgctgcc tggggagcgg cctttaattt atcgaaacca 1080
   cgttcagcga caaattatat ccatacaggg acaggcgata ttgtttttaa taataacgtt 1140
   gtctttactc ttgacggtaa tttattaggg aaacggaaac tttttcatat taataataat 1200
   gagataacac catatacatt gtctctcggc gctaaaaaag atactcgtat ctatttttat 1260
   gatcttttcc aatgggagcg tgttaaagaa aatactagca ataacccacc atctcctacc 1320
   agtagaaaca ccattaccgt taacccggaa acagagtttt ctggagctgt tgtgttctcc 1380
   tacaatcaaa tgtctagtga catacgaact ctgatgggta aagaacacaa ttacattaaa 1440
   gaagccccaa ctactttaaa attcggaacg ctagccatag aagatgatgc agaattagaa 1500
   atcttcaata tcccgtttac ccaaaatccg actagccttc ttgctttagg aagcggcgct 1560
   acgctgactg ttggaaagca cggtaagctc aatattacaa atcttggtgt tattttaccc 1620
   attattctca aagaggggaa gagtccgcct tgtattcgcg tcaacccaca agatatgacc 1680
   caaaatactg gtaccggcca aactccatca agcacaagta gtataagcac tccaatgatt 1740
   atctttaatg ggcgcctctc aattgtagac gaaaattatg aatcagtcta cgacagtatg 1800
   gacctctcca gagggaaagc agaacaacta attctatcca tagaaaccac taatgatggg 1860
   caattagact ccaattggca aagttctctg aatacttctc tactctctcc tccacactat 1920
   ggctatcaag gtctatggac tcctaattgg ataacaacaa cctataccat cacgcttaat 1980
   aataattctt cagctccaac atctgctacc tccatcgctg agcagaaaaa aactagtgaa 2040
   acttttactc ctagtaacac aactacagct agtatcccta atattaaagc ttccgcagga 2100
   tcaggctctg gatcggcttc caattcagga gaagttacga ttaccaaaca tacccttgtt 2160
   gtaaactggg caccagtcgg ctacatagta gatcctattc gtagaggaga tctgatagcc 2220
   aatagcttag tacattcagg aagaaacatg accatgggct tacgatcatt actcccggat 2280
   aactcttggt ttgctttgca aggagctgca acaacattat ttacaaaaca acaaaaacgt 2340
   ttgagttatc atggctactc ttctgcatca aaggggtata ccgtctcttc tcaagcatca 2400
   ggagctcatg gtcataagtt tcttctttcc ttctcccagt catctgataa gatgaaagaa 2460
   aaagaaacaa ataaccgcct ttcttctcgt tactatcttt ctgctttatg tttcgaacat 2520
   cctatgtttg atcgcattgc tcttatcgga gcagcagctt gcaattatgg aacacataac 2580
   atgcggagtt tctatggaac taaaaaatct tctaaaggga aatttcactc tacaacctta 2640
   ggagcttctc ttcgctgtga actacgcgat agtatgcctt tacgatcaat aatgctcacc 2700
   ccatttgctc aggctttatt ctctcgaaca gaaccagctt ctatccgaga aagcggtgat 2760
   ctagctagat tatttacatt agagcaagcc catactgccg ttgtctctcc aataggaatc 2820
   aaaggagctt attcttctga tacatggcca acactctctt gggaaatgga actagcttac 2880
   caacccaccc tctactggaa acgtcctcta ctcaacacac tattaatcca aaataacggt 2940
   tcttgggtca ccacaaatac cccattagct aaacattcct tttatgggag aggttctcac 3000
   tccctcaaat tttctcatct gaaactattt gctaactatc aagcagaagt ggctacttcc 3060
   actgtctcac actacatcaa tgcaggagga gctctggtct tttaa                3105
<210> 13
   <211> 964
   <212> PRT
   <213> Chlamydia trachomatis
<400> 13
   Met Lys Lys Ala Phe Phe Phe Phe Leu Ile Gly Asn Ser Leu Ser Gly
   1               5                   10                  15
   Leu Ala Arg Glu Val Pro Ser Arg Ile Phe Leu Met Pro Asn Ser Val
               20                  25                  30
   Pro Asp Pro Thr Lys Glu Ser Leu Ser Asn Lys Ile Ser Leu Thr Gly
           35                  40                  45
   Asp Thr His Asn Leu Thr Asn Cys Tyr Leu Asp Asn Leu Arg Tyr Ile
       50                  55                  60
   Leu Ala Ile Leu Gln Lys Thr Pro Asn Glu Gly Ala Ala Val Thr Ile
   65                  70                  75                  80
   Thr Asp Tyr Leu Ser Phe Phe Asp Thr Gln Lys Glu Gly Ile Tyr Phe
                   85                  90                  95
   Ala Lys Asn Leu Thr Pro Glu Ser Gly Gly Ala Ile Gly Tyr Ala Ser
               100                 105                 110
   Pro Asn Ser Pro Thr Val Glu Ile Arg Asp Thr Ile Gly Pro Val Ile
           115                 120                 125
   Phe Glu Asn Asn Thr Cys Cys Arg Leu Phe Thr Trp Arg Asn Pro Tyr
       130                 135                 140
   Ala Ala Asp Lys Ile Arg Glu Gly Gly Ala Ile His Ala Gln Asn Leu
   145                 150                 155                 160
   Tyr Ile Asn His Asn His Asp Val Val Gly Phe Met Lys Asn Phe Ser
                   165                 170                 175
   Tyr Val Gln Gly Gly Ala Ile Ser Thr Ala Asn Thr Phe Val Val Ser
               180                 185                 190
   Glu Asn Gln Ser Cys Phe Leu Phe Met Asp Asn Ile Cys Ile Gln Thr
           195                 200                 205
   Asn Thr Ala Gly Lys Gly Gly Ala Ile Tyr Ala Gly Thr Ser Asn Ser
       210                 215                 220
   Phe Glu Ser Asn Asn Cys Asp Leu Phe Phe Ile Asn Asn Ala Cys Cys
   225                 230                 235                 240
   Ala Gly Gly Ala Ile Phe Ser Pro Ile Cys Ser Leu Thr Gly Asn Arg
                   245                 250                 255
   Gly Asn Ile Val Phe Tyr Asn Asn Arg Cys Phe Lys Asn Val Glu Thr
               260                 265                 270
   Ala Ser Ser Glu Ala Ser Asp Gly Gly Ala Ile Lys Val Thr Thr Arg
           275                 280                 285
   Leu Asp Val Thr Gly Asn Arg Gly Arg Ile Phe Phe Ser Asp Asn Ile
       290                 295                 300
   Thr Lys Asn Tyr Gly Gly Ala Ile Tyr Ala Pro Val Val Thr Leu Val
   305                 310                 315                 320
   Asp Asn Gly Pro Thr Tyr Phe Ile Asn Asn Ile Ala Asn Asn Lys Gly
                   325                 330                 335
   Gly Ala Ile Tyr Ile Asp Gly Thr Ser Asn Ser Lys Ile Ser Ala Asp
               340                 345                 350
   Arg His Ala Ile Ile Phe Asn Glu Asn Ile Val Thr Asn Val Thr Asn
           355                 360                 365
   Ala Asn Gly Thr Ser Thr Ser Ala Asn Pro Pro Arg Arg Asn Ala Ile
       370                 375                 380
   Thr Val Ala Ser Ser Ser Gly Glu Ile Leu Leu Gly Ala Gly Ser Ser
   385                 390                 395                 400
   Gln Asn Leu Ile Phe Tyr Asp Pro Ile Glu Val Ser Asn Ala Gly Val
                   405                 410                 415
   Ser Val Ser Phe Asn Lys Glu Ala Asp Gln Thr Gly Ser Val Val Phe
               420                 425                 430
   Ser Gly Ala Thr Val Asn Ser Ala Asp Phe His Gln Arg Asn Leu Gln
           435                 440                 445
   Thr Lys Thr Pro Ala Pro Leu Thr Leu Ser Asn Gly Phe Leu Cys Ile
       450                 455                 460
   Glu Asp His Ala Gln Leu Thr Val Asn Arg Phe Thr Gln Thr Gly Gly
   465                 470                 475                 480
   Val Val Ser Leu Gly Asn Gly Ala Val Leu Ser Cys Tyr Lys Asn Gly
                   485                 490                 495
   Thr Gly Asp Ser Ala Ser Asn Ala Ser Ile Thr Leu Lys His Ile Gly
               500                 505                 510
   Leu Asn Leu Ser Ser Ile Leu Lys Ser Gly Ala Glu Ile Pro Leu Leu
           515                 520                 525
   Trp Val Glu Pro Thr Asn Asn Ser Asn Asn Tyr Thr Ala Asp Thr Ala
       530                 535                 540
   Ala Thr Phe Ser Leu Ser Asp Val Lys Leu Ser Leu Ile Asp Asp Tyr
   545                 550                 555                 560
   Gly Asn Ser Pro Tyr Glu Ser Thr Asp Leu Thr His Ala Leu Ser Ser
                   565                 570                 575
   Gln Pro Met Leu Ser Ile Ser Glu Ala Ser Asp Asn Gln Leu Gln Ser
               580                 585                 590
   Glu Asn Ile Asp Phe Ser Gly Leu Asn Val Pro His Tyr Gly Trp Gln
           595                 600                 605
   Gly Leu Trp Thr Trp Gly Trp Ala Lys Thr Gln Asp Pro Glu Pro Ala
       610                 615                 620
   Ser Ser Ala Thr Ile Thr Asp Pro Gln Lys Ala Asn Arg Phe His Arg
   625                 630                 635                 640
   Thr Leu Leu Leu Thr Trp Leu Pro Ala Gly Tyr Val Pro Ser Pro Lys
                   645                 650                 655
   His Arg Ser Pro Leu Ile Ala Asn Thr Leu Trp Gly Asn Met Leu Leu
               660                 665                 670
   Ala Thr Glu Ser Leu Lys Asn Ser Ala Glu Leu Thr Pro Ser Gly His
           675                 680                 685
   Pro Phe Trp Gly Ile Thr Gly Gly Gly Leu Gly Met Met Val Tyr Gln
       690                 695                 700
   Asp Pro Arg Glu Asn His Pro Gly Phe His Met Arg Ser Ser Gly Tyr
   705                 710                 715                 720
   Ser Ala Gly Met Ile Ala Gly Gln Thr His Thr Phe Ser Leu Lys Phe
                   725                 730                 735
   Ser Gln Thr Tyr Thr Lys Leu Asn Glu Arg Tyr Ala Lys Asn Asn Val
               740                 745                 750
   Ser Ser Lys Asn Tyr Ser Cys Gln Gly Glu Met Leu Phe Ser Leu Gln
           755                 760                 765
   Glu Gly Phe Leu Leu Thr Lys Leu Val Gly Leu Tyr Ser Tyr Gly Asp
       770                 775                 780
   His Asn Cys His His Phe Tyr Thr Gln Gly Glu Asn Leu Thr Ser Gln
   785                 790                 795                 800
   Gly Thr Phe Arg Ser Gln Thr Met Gly Gly Ala Val Phe Phe Asp Leu
                   805                 810                 815
   Pro Met Lys Pro Phe Gly Ser Thr His Ile Leu Thr Ala Pro Phe Leu
               820                 825                 830
   Gly Ala Leu Gly Ile Tyr Ser Ser Leu Ser His Phe Thr Glu Val Gly
           835                 840                 845
   Ala Tyr Pro Arg Ser Phe Ser Thr Lys Thr Pro Leu Ile Asn Val Leu
       850                 855                 860
   Val Pro Ile Gly Val Lys Gly Ser Phe Met Asn Ala Thr His Arg Pro
   865                 870                 875                 880
   Gln Ala Trp Thr Val Glu Leu Ala Tyr Gln Pro Val Leu Tyr Arg Gln
                   885                 890                 895
   Glu Pro Gly Ile Ala Ala Gln Leu Leu Ala Ser Lys Gly Ile Trp Phe
               900                 905                 910
   Gly Ser Gly Ser Pro Ser Ser Arg His Ala Met Ser Tyr Lys Ile Ser
           915                 920                 925
   Gln Gln Thr Gln Pro Leu Ser Trp Leu Thr Leu His Phe Gln Tyr His
       930                 935                 940
   Gly Phe Tyr Ser Ser Ser Thr Phe Cys Asn Tyr Leu Asn Gly Glu Ile
   945                 950                 955                 960
   Ala Leu Arg Phe
<210> 14
   <211> 2895
   <212> DNA
   <213> Chlamydia trachomatis
<400> 14
   atgaaaaaag cgtttttctt tttccttatc ggaaactccc tatcaggact agctagagag 60
   gttccttcta gaatctttct tatgcccaac tcagttccag atcctacgaa agagtcgcta 120
   tcaaataaaa ttagtttgac aggagacact cacaatctca ctaactgcta tctcgataac 180
   ctacgctaca tactggctat tctacaaaaa actcccaatg aaggagctgc tgtcacaata 240
   acagattacc taagcttttt tgatacacaa aaagaaggta tttattttgc aaaaaatctc 300
   acccctgaaa gtggtggtgc gattggttat gcgagtccca attctcctac cgtggagatt 360
   cgtgatacaa taggtcctgt aatctttgaa aataatactt gttgcagact atttacatgg 420
   agaaatcctt atgctgctga taaaataaga gaaggcggag ccattcatgc tcaaaatctt 480
   tacataaatc ataatcatga tgtggtcgga tttatgaaga acttttctta tgtccaagga 540
   ggagccatta gtaccgctaa tacctttgtt gtgagcgaga atcagtcttg ttttctcttt 600
   atggacaaca tctgtattca aactaataca gcaggaaaag gtggcgctat ctatgctgga 660
   acgagcaatt cttttgagag taataactgc gatctcttct tcatcaataa cgcctgttgt 720
   gcaggaggag cgatcttctc ccctatctgt tctctaacag gaaatcgtgg taacatcgtt 780
   ttctataaca atcgctgctt taaaaatgta gaaacagctt cttcagaagc ttctgatgga 840
   ggagcaatta aagtaactac tcgcctagat gttacaggca atcgtggtag gatctttttt 900
   agtgacaata tcacaaaaaa ttatggcgga gctatttacg ctcctgtagt taccctagtg 960
   gataatggcc ctacctactt tataaacaat atcgccaata ataagggggg cgctatctat 1020
   atagacggaa ccagtaactc caaaatttct gccgaccgcc atgctattat ttttaatgaa 1080
   aatattgtga ctaatgtaac taatgcaaat ggtaccagta cgtcagctaa tcctcctaga 1140
   agaaatgcaa taacagtagc aagctcctct ggtgaaattc tattaggagc agggagtagc 1200
   caaaatttaa ttttttatga tcctattgaa gttagcaatg caggggtctc tgtgtccttc 1260
   aataaggaag ctgatcaaac aggctctgta gtattttcag gagctactgt taattctgca 1320
   gattttcatc aacgcaattt acaaacaaaa acacctgcac cccttactct cagtaatggt 1380
   tttctatgta tcgaagatca tgctcagctt acagtgaatc gattcacaca aactgggggt 1440
   gttgtttctc ttgggaatgg agcagttctg agttgctata aaaatggtac aggagattct 1500
   gctagcaatg cctctataac actgaagcat attggattga atctttcttc cattctgaaa 1560
   agtggtgctg agattccttt attgtgggta gagcctacaa ataacagcaa taactataca 1620
   gcagatactg cagctacctt ttcattaagt gatgtaaaac tctcactcat tgatgactac 1680
   gggaactctc cttatgaatc cacagatctg acccatgctc tgtcatcaca gcctatgcta 1740
   tctatttctg aagctagcga taaccagcta caatcagaaa atatagattt ttcgggacta 1800
   aatgtccctc attatggatg gcaaggactt tggacttggg gctgggcaaa aactcaagat 1860
   ccagaaccag catcttcagc aacaatcact gatccacaaa aagccaatag atttcataga 1920
   accttactac taacatggct tcctgccggg tatgttccta gcccaaaaca cagaagtccc 1980
   ctcatagcta acaccttatg ggggaatatg ctgcttgcaa cagaaagctt aaaaaatagt 2040
   gcagagctga cacctagtgg tcatcctttc tggggaatta caggaggagg actaggcatg 2100
   atggtttacc aagatcctcg agaaaatcat cctggattcc atatgcgctc ttccggatac 2160
   tctgcgggga tgatagcagg gcagacacac accttctcat tgaaattcag tcagacctac 2220
   accaaactca atgagcgtta cgcaaaaaac aacgtatctt ctaaaaatta ctcatgccaa 2280
   ggagaaatgc tcttctcatt gcaagaaggt ttcttgctga ctaaattagt tgggctttac 2340
   agctatggag accataactg tcaccatttc tatactcaag gagaaaatct aacatctcaa 2400
   gggacgttcc gcagtcaaac gatgggaggt gctgtctttt ttgatctccc tatgaaaccc 2460
   tttggatcaa cgcatatact gacagctccc tttttaggtg ctcttggtat ttattctagc 2520
   ctgtctcact ttactgaggt gggagcctat ccgcgaagct tttctacaaa gactcctttg 2580
   atcaatgtcc tagtccctat tggagttaaa ggtagcttta tgaatgctac ccacagacct 2640
   caagcctgga ctgtagaatt ggcataccaa cccgttctgt atagacaaga accagggatc 2700
   gcagcccagc tcctagccag taagggtatt tggttcggta gtggaagccc ctcatcgcgt 2760
   catgccatgt cctataaaat ctcacagcaa acacaacctt tgagttggtt aactctccat 2820
   ttccagtatc atggattcta ctcctcttca accttctgta attatctcaa tggggaaatt 2880
   gctctgcgat tctag                                                 2895
<210> 15
   <211> 1531
   <212> PRT
   <213> Chlamydia trachomatis
<400> 15
   Met Ser Ser Glu Lys Asp Ile Lys Ser Thr Cys Ser Lys Phe Ser Leu
   1               5                   10                  15
   Ser Val Val Ala Ala Ile Leu Ala Ser Val Ser Gly Leu Ala Ser Cys
               20                  25                  30
   Val Asp Leu His Ala Gly Gly Gln Ser Val Asn Glu Leu Val Tyr Val
           35                  40                  45
   Gly Pro Gln Ala Val Leu Leu Leu Asp Gln Ile Arg Asp Leu Phe Val
       50                  55                  60
   Gly Ser Lys Asp Ser Gln Ala Glu Gly Gln Tyr Arg Leu Ile Val Gly
   65                  70                  75                  80
   Asp Pro Ser Ser Phe Gln Glu Lys Asp Ala Asp Thr Leu Pro Gly Lys
                   85                  90                  95
   Val Glu Gln Ser Thr Leu Phe Ser Val Thr Asn Pro Val Val Phe Gln
               100                 105                 110
   Gly Val Asp Gln Gln Asp Gln Val Ser Ser Gln Gly Leu Ile Cys Ser
           115                 120                 125
   Phe Thr Ser Ser Asn Leu Asp Ser Pro Arg Asp Gly Glu Ser Phe Leu
       130                 135                 140
   Gly Ile Ala Phe Val Gly Asp Ser Ser Lys Ala Gly Ile Thr Leu Thr
   145                 150                 155                 160
   Asp Val Lys Ala Ser Leu Ser Gly Ala Ala Leu Tyr Ser Thr Glu Asp
                   165                 170                 175
   Leu Ile Phe Glu Lys Ile Lys Gly Gly Leu Glu Phe Ala Ser Cys Ser
               180                 185                 190
   Ser Leu Glu Gln Gly Gly Ala Cys Ala Ala Gln Ser Ile Leu Ile His
           195                 200                 205
   Asp Cys Gln Gly Leu Gln Val Lys His Cys Thr Thr Ala Val Asn Ala
       210                 215                 220
   Glu Gly Ser Ser Ala Asn Asp His Leu Gly Phe Gly Gly Gly Ala Phe
   225                 230                 235                 240
   Phe Val Thr Gly Ser Leu Ser Gly Glu Lys Ser Leu Tyr Met Pro Ala
                   245                 250                 255
   Gly Asp Met Val Val Ala Asn Cys Asp Gly Ala Ile Ser Phe Glu Gly
               260                 265                 270
   Asn Ser Ala Asn Phe Ala Asn Gly Gly Ala Ile Ala Ala Ser Gly Lys
           275                 280                 285
   Val Leu Phe Val Ala Asn Asp Lys Lys Thr Ser Phe Ile Glu Asn Arg
       290                 295                 300
   Ala Leu Ser Gly Gly Ala Ile Ala Ala Ser Ser Asp Ile Ala Phe Gln
   305                 310                 315                 320
   Asn Cys Ala Glu Leu Val Phe Lys Gly Asn Cys Ala Ile Gly Thr Glu
                   325                 330                 335
   Asp Lys Gly Ser Leu Gly Gly Gly Ala Ile Ser Ser Leu Gly Thr Val
               340                 345                 350
   Leu Leu Gln Gly Asn His Gly Ile Thr Cys Asp Lys Asn Glu Ser Ala
           355                 360                 365
   Ser Gln Gly Gly Ala Ile Phe Gly Lys Asn Cys Gln Ile Ser Asp Asn
       370                 375                 380
   Glu Gly Pro Val Val Phe Arg Asp Ser Thr Ala Cys Leu Gly Gly Gly
   385                 390                 395                 400
   Ala Ile Ala Ala Gln Glu Ile Val Ser Ile Gln Asn Asn Gln Ala Gly
                   405                 410                 415
   Ile Ser Phe Glu Gly Gly Lys Ala Ser Phe Gly Gly Gly Ile Ala Cys
               420                 425                 430
   Gly Ser Phe Ser Ser Ala Gly Gly Ala Ser Val Leu Gly Thr Ile Asp
           435                 440                 445
   Ile Ser Lys Asn Leu Gly Ala Ile Ser Phe Ser Arg Thr Leu Cys Thr
       450                 455                 460
   Thr Ser Asp Leu Gly Gln Met Glu Tyr Gln Gly Gly Gly Ala Leu Phe
   465                 470                 475                 480
   Gly Glu Asn Ile Ser Leu Ser Glu Asn Ala Gly Val Leu Thr Phe Lys
                   485                 490                 495
   Asp Asn Ile Val Lys Thr Phe Ala Ser Asn Gly Lys Ile Leu Gly Gly
               500                 505                 510
   Gly Ala Ile Leu Ala Thr Gly Lys Val Glu Ile Thr Asn Asn Ser Glu
           515                 520                 525
   Gly Ile Ser Phe Thr Gly Asn Ala Arg Ala Pro Gln Ala Leu Pro Thr
   530 535 540
   Gln Glu Glu Phe Pro Leu Phe Ser Lys Lys Glu Gly Arg Pro Leu Ser
   545 550 555 560
   Ser Gly Tyr Ser Gly Gly Gly Ala Ile Leu Gly Arg Glu Val Ala Ile
   565 570 575
   Leu His Asn Ala Ala Val Val Phe Glu Gln Asn Arg Leu Gln Cys Ser
   580 585 590
   Glu Glu Glu Ala Thr Leu Leu Gly Cys Cys Gly Gly Gly Ala Val His
   595 600 605
   Gly Met Asp Ser Thr Ser Ile Val Gly Asn Ser Ser Val Arg Phe Gly
   610 615 620
   Asn Asn Tyr Ala Met Gly Gln Gly Val Ser Gly Gly Ala Leu Leu Ser
   625 630 635 640
   Lys Thr Val Gln Leu Ala Gly Asn Gly Ser Val Asp Phe Ser Arg Asn
   645 650 655
   Ile Ala Ser Leu Gly Gly Gly Ala Leu Gln Ala Ser Glu Gly Asn Cys
   660 665 670
   Glu Leu Val Asp Asn Gly Tyr Val Leu Phe Arg Asp Asn Arg Gly Arg
   675 680 685
   Val Tyr Gly Gly Ala Ile Ser Cys Leu Arg Gly Asp Val Val Ile Ser
   690 695 700
   Gly Asn Lys Gly Arg Val Glu Phe Lys Asp Asn Ile Ala Thr Arg Leu
   705 710 715 720
   Tyr Val Glu Glu Thr Val Glu Lys Val Glu Glu Val Glu Pro Ala Pro
   725 730 735
   Glu Gln Lys Asp Asn Asn Glu Leu Ser Phe Leu Gly Arg Ala Glu Gln
   740 745 750
   Ser Phe Ile Thr Ala Ala Asn Gln Ala Leu Phe Ala Ser Glu Asp Gly
   755 760 765
   Asp Leu Ser Pro Glu Ser Ser Ile Ser Ser Glu Glu Leu Ala Lys Arg
   770 775 780
   Arg Glu Cys Ala Gly Gly Ala Ile Phe Ala Lys Arg Val Arg Ile Val
   785 790 795 800
   Asp Asn Gln Glu Ala Val Val Phe Ser Asn Asn Phe Ser Asp Ile Tyr
   805 810 815
   Gly Gly Ala Ile Phe Thr Gly Ser Leu Arg Glu Glu Asp Lys Leu Asp
   820 825 830
   Gly Gln Ile Pro Glu Val Leu Ile Ser Gly Asn Ala Gly Asp Val Val
   835 840 845
   Phe Ser Gly Asn Ser Ser Lys Arg Asp Glu His Leu Pro His Thr Gly
   850 855 860
   Gly Gly Ala Ile Cys Thr Gln Asn Leu Thr Ile Ser Gln Asn Thr Gly
   865 870 875 880
   Asn Val Leu Phe Tyr Asn Asn Val Ala Cys Ser Gly Gly Ala Val Arg
   885 890 895
   Ile Glu Asp His Gly Asn Val Leu Leu Glu Ala Phe Gly Gly Asp Ile
   900 905 910
   Val Phe Lys Gly Asn Ser Ser Phe Arg Ala Gln Gly Ser Asp Ala Ile
   915 920 925
   Tyr Phe Ala Gly Lys Glu Ser His Ile Thr Ala Leu Asn Ala Thr Glu
   930 935 940
   Gly His Ala Ile Val Phe His Asp Ala Leu Val Phe Glu Asn Leu Glu
   945 950 955 960
   Glu Arg Lys Ser Ala Glu Val Leu Leu Ile Asn Ser Arg Glu Asn Pro
   965 970 975
   Gly Tyr Thr Gly Ser Ile Arg Phe Leu Glu Ala Glu Ser Lys Val Pro
   980 985 990
   Gln Cys Ile His Val Gln Gln Gly Ser Leu Glu Leu Leu Asn Gly Ala
   995 1000 1005
   Thr Leu Cys Ser Tyr Gly Phe Lys Gln Asp Ala Gly Ala Lys Leu Val
   1010 1015 1020
   Leu Ala Ala Gly Ala Lys Leu Lys Ile Leu Asp Ser Gly Thr Pro Val
   1025 1030 1035 1040
   Gln Gln Gly His Ala Ile Ser Lys Pro Glu Ala Glu Ile Glu Ser Ser
   1045 1050 1055
   Ser Glu Pro Glu Gly Ala His Ser Leu Trp Ile Ala Lys Asn Ala Gln
   1060 1065 1070
   Thr Thr Val Pro Met Val Asp Ile His Thr Ile Ser Val Asp Leu Ala
   1075 1080 1085
   Ser Phe Ser Ser Ser Gln Gln Glu Gly Thr Val Glu Ala Pro Gln Val
   1090 1095 1100
   Ile Val Pro Gly Gly Ser Tyr Val Arg Ser Gly Glu Leu Asn Leu Glu
   1105 1110 1115 1120
   Leu Val Asn Thr Thr Gly Thr Gly Tyr Glu Asn His Ala Leu Leu Lys
   1125 1130 1135
   Asn Glu Ala Lys Val Pro Leu Met Ser Phe Val Ala Ser Gly Asp Glu
   1140 1145 1150
   Ala Ser Ala Glu Ile Ser Asn Leu Ser Val Ser Asp Leu Gln Ile His
   1155 1160 1165
   Val Val Thr Pro Glu Ile Glu Glu Asp Thr Tyr Gly His Met Gly Asp
   1170 1175 1180
   Trp Ser Glu Ala Lys Ile Gln Asp Gly Thr Leu Val Ile Ser Trp Asn
   1185 1190 1195 1200
   Pro Thr Gly Tyr Arg Leu Asp Pro Gln Lys Ala Gly Ala Leu Val Phe
   1205 1210 1215
   Asn Ala Leu Trp Glu Glu Gly Ala Val Leu Ser Ala Leu Lys Asn Ala
   1220 1225 1230
   Arg Phe Ala His Asn Leu Thr Ala Gln Arg Met Glu Phe Asp Tyr Ser
   1235 1240 1245
   Thr Asn Val Trp Gly Phe Ala Phe Gly Gly Phe Arg Thr Leu Ser Ala
   1250 1255 1260
   Glu Asn Leu Val Ala Ile Asp Gly Tyr Lys Gly Ala Tyr Gly Gly Ala
   1265 1270 1275 1280
   Ser Ala Gly Val Asp Ile Gln Leu Met Glu Asp Phe Val Leu Gly Val
   1285 1290 1295
   Ser Gly Ala Ala Phe Leu Gly Lys Met Asp Ser Gln Lys Phe Asp Ala
   1300 1305 1310
   Glu Val Ser Arg Lys Gly Val Val Gly Ser Val Tyr Thr Gly Phe Leu
   1315 1320 1325
   Ala Gly Ser Trp Phe Phe Lys Gly Gln Tyr Ser Leu Gly Glu Thr Gln
   1330 1335 1340
   Asn Asp Met Lys Thr Arg Tyr Gly Val Leu Gly Glu Ser Ser Ala Ser
   1345 1350 1355 1360
   Trp Thr Ser Arg Gly Val Leu Ala Asp Ala Leu Val Glu Tyr Arg Ser
   1365 1370 1375
   Leu Val Gly Pro Val Arg Pro Thr Phe Tyr Ala Leu His Phe Asn Pro
   1380 1385 1390
   Tyr Val Glu Val Ser Tyr Ala Ser Met Lys Phe Pro Gly Phe Thr Glu
   1395 1400 1405
   Gln Gly Arg Glu Ala Arg Ser Phe Glu Asp Ala Ser Leu Thr Asn Ile
   1410 1415 1420
   Thr Ile Pro Leu Gly Met Lys Phe Glu Leu Ala Phe Ile Lys Gly Gln
   1425 1430 1435 1440
   Phe Ser Glu Val Asn Ser Leu Gly Ile Ser Tyr Ala Trp Glu Ala Tyr
   1445 1450 1455
   Arg Lys Val Glu Gly Gly Ala Val Gln Leu Leu Glu Ala Gly Phe Asp
   1460 1465 1470
   Trp Glu Gly Ala Pro Met Asp Leu Pro Arg Gln Glu Leu Arg Val Ala
   1475 1480 1485
   Leu Glu Asn Asn Thr Glu Trp Ser Ser Tyr Phe Ser Thr Val Leu Gly
   1490 1495 1500
   Leu Thr Ala Phe Cys Gly Gly Phe Thr Ser Thr Asp Ser Lys Leu Gly
   1505 1510 1515 1520
   Tyr Glu Ala Asn Thr Gly Leu Arg Leu Ile Phe
   1525 1530
<210> 16
   <211> 4596
   <212> DNA
   <213> Chlamydia trachomatis
<400> 16
   atgagttccg agaaagatat aaaaagcacc tgttctaagt tttctttgtc tgtagtagca 60
   gctatccttg cctctgttag cgggttagct agttgcgtag atcttcatgc tggaggacag 120
   tctgtaaatg agctggtata tgtaggccct caagcggttt tattgttaga ccaaattcga 180
   gatctattcg ttgggtctaa agatagtcag gctgaaggac agtataggtt aattgtagga 240
   gatccaagtt ctttccaaga gaaagatgcg gatactcttc ccgggaaggt agagcaaagt 300
   actttgttct cagtaaccaa tcccgtggtt ttccaaggtg tggaccaaca ggatcaagtc 360
   tcttcccaag ggttaatttg tagttttacg agcagcaacc ttgattctcc tcgtgacgga 420
   gaatcttttt taggtattgc ttttgttggg gatagtagta aggctggaat cacattaact 480
   gacgtgaaag cttctttgtc tggagcggct ttatattcta cagaagatct tatctttgaa 540
   aagattaagg gtggattgga atttgcatca tgttcttctc tagaacaggg gggagcttgt 600
   gcagctcaaa gtattttgat tcatgattgt caaggattgc aggttaaaca ctgtactaca 660
   gccgtgaatg ctgaggggtc tagtgcgaat gatcatcttg gatttggagg aggcgctttc 720
   tttgttacgg gttctctttc tggagagaaa agtctctata tgcctgcagg agatatggta 780
   gttgcgaatt gtgatggggc tatatctttt gaaggaaaca gcgcgaactt tgctaatgga 840
   ggagcgattg ctgcctctgg gaaagtgctt tttgtcgcta atgataaaaa gacttctttt 900
   atagagaacc gagctttgtc tggaggagcg attgcagcct cttctgatat tgcctttcaa 960
   aactgcgcag aactagtttt caaaggcaat tgtgcaattg gaacagagga taaaggttct 1020
   ttaggtggag gggctatatc ttctctaggc accgttcttt tgcaagggaa tcacgggata 1080
   acttgtgata agaatgagtc tgcttcgcaa ggaggcgcca tttttggcaa aaattgtcag 1140
   atttctgaca acgaggggcc agtggttttc agagatagta cagcttgctt aggaggaggc 1200
   gctattgcag ctcaagaaat tgtttctatt cagaacaatc aggctgggat ttccttcgag 1260
   ggaggtaagg ctagtttcgg aggaggtatt gcgtgtggat ctttttcttc cgcaggtggt 1320
   gcttctgttt tagggaccat tgatatttcg aagaatttag gcgcgatttc gttctctcgt 1380
   actttatgta cgacctcaga tttaggacaa atggagtacc agggaggagg agctctattt 1440
   ggtgaaaata tttctctttc tgagaatgct ggtgtgctca cctttaaaga caacattgtg 1500
   aagacttttg cttcgaatgg gaaaattctg ggaggaggag cgattttagc tactggtaag 1560
   gtggaaatta ctaataattc cgaaggaatt tcttttacag gaaatgcgag agctccacaa 1620
   gctcttccaa ctcaagagga gtttccttta ttcagcaaaa aagaagggcg accactctct 1680
   tcaggatatt ctgggggagg agcgatttta ggaagagaag tagctattct ccacaacgct 1740
   gcagtagtat ttgagcaaaa tcgtttgcag tgcagcgaag aagaagcgac attattaggt 1800
   tgttgtggag gaggcgctgt tcatgggatg gatagcactt cgattgttgg caactcttca 1860
   gtaagatttg gtaataatta cgcaatggga caaggagtct caggaggagc tcttttatct 1920
   aaaacagtgc agttagctgg gaatggaagc gtcgattttt ctcgaaatat tgctagtttg 1980
   ggaggaggag ctcttcaagc ttctgaagga aattgtgagc tagttgataa cggctatgtg 2040
   ctattcagag ataatcgagg gagggtttat gggggtgcta tttcttgctt acgtggagat 2100
   gtagtcattt ctggaaacaa gggtagagtt gaatttaaag acaacatagc aacacgtctt 2160
   tatgtggaag aaactgtaga aaaggttgaa gaggtagagc cagctcctga gcaaaaagac 2220
   aataatgagc tttctttctt agggagagca gaacagagtt ttattactgc agctaatcaa 2280
   gctcttttcg catctgaaga tggggattta tcacctgagt catccatttc ttctgaagaa 2340
   cttgcgaaaa gaagagagtg tgctggagga gctatttttg caaaacgggt tcgtattgta 2400
   gataaccaag aggccgttgt attctcgaat aacttctctg atatttatgg cggcgccatt 2460
   tttacaggtt ctcttcgaga agaggataag ttagatgggc aaatccctga agtcttgatc 2520
   tcaggcaatg caggggatgt tgttttttcc ggaaattcct cgaagcgtga tgagcatctt 2580
   cctcatacag gtgggggagc catttgtact caaaatttga cgatttctca gaatacaggg 2640
   aatgttctgt tttataacaa cgtggcctgt tcgggaggag ctgttcgtat agaggatcat 2700
   ggtaatgttc ttttagaagc ttttggagga gatattgttt ttaaaggaaa ttcttctttc 2760
   agagcacaag gatccgatgc tatctatttt gcaggtaaag aatcgcatat tacagccctg 2820
   aatgctacgg aaggacatgc tattgttttc cacgacgcat tagtttttga aaatctagaa 2880
   gaaaggaaat ctgctgaagt attgttaatc aatagtcgag aaaatccagg ttacactgga 2940
   tctattcgat ttttagaagc agaaagtaaa gttcctcaat gtattcatgt acaacaagga 3000
   agccttgagt tgctaaatgg agccacatta tgtagttatg gttttaaaca agatgctgga 3060
   gctaagttgg tattggctgc tggagctaaa ctgaagattt tagattcagg aactcctgta 3120
   caacaagggc atgctatcag taaacctgaa gcagaaatcg agtcatcttc tgaaccagag 3180
   ggtgcacatt ctctttggat tgcgaagaat gctcaaacaa cagttcctat ggttgatatc 3240
   catactattt ctgtagattt agcctccttc tcttctagtc aacaggaggg gacagtagaa 3300
   gctcctcagg ttattgttcc tggaggaagt tatgttcgat ctggagagct taatttggag 3360
   ttagttaaca caacaggtac tggttatgaa aatcatgctt tattgaagaa tgaggctaaa 3420
   gttccattga tgtctttcgt tgcttctggt gatgaagctt cagccgaaat cagtaacttg 3480
   tcggtttctg atttacagat tcatgtagta actccagaga ttgaagaaga cacatacggc 3540
   catatgggag attggtctga ggctaaaatt caagatggaa ctcttgtcat tagttggaat 3600
   cctactggat atcgattaga tcctcaaaaa gcaggggctt tagtatttaa tgcattatgg 3660
   gaagaagggg ctgtcttgtc tgctctgaaa aatgcacgct ttgctcataa tctcactgct 3720
   cagcgtatgg aattcgatta ttctacaaat gtgtggggat tcgcctttgg tggtttccga 3780
   actctatctg cagagaatct ggttgctatt gatggataca aaggagctta tggtggtgct 3840
   tctgctggag tcgatattca attgatggaa gattttgttc taggagttag tggagctgct 3900
   ttcctaggta aaatggatag tcagaagttt gatgcggagg tttctcggaa gggagttgtt 3960
   ggttctgtat atacaggatt tttagctgga tcctggttct tcaaaggaca atatagcctt 4020
   ggagaaacac agaacgatat gaaaacgcgt tatggagtac taggagagtc gagtgcttct 4080
   tggacatctc gaggagtact ggcagatgct ttagttgaat accgaagttt agttggtcct 4140
   gtgagaccta ctttttatgc tttgcatttc aatccttatg tcgaagtatc ttatgcttct 4200
   atgaaattcc ctggctttac agaacaagga agagaagcgc gttcttttga agacgcttcc 4260
   cttaccaata tcaccattcc tttagggatg aagtttgaat tggcgttcat aaaaggacag 4320
   ttttcagagg tgaactcttt gggaataagt tatgcatggg aagcttatcg aaaagtagaa 4380
   ggaggcgcgg tgcagctttt agaagctggg tttgattggg agggagctcc aatggatctt 4440
   cctagacagg agctgcgtgt cgctctggaa aataatacgg aatggagttc ttacttcagc 4500
   acagtcttag gattaacagc tttttgtgga ggatttactt ctacagatag taaactagga 4560
   tatgaggcga atactggatt gcgattgatc ttttaa                          4596
<210> 17
   <211> 340
   <212> PRT
   <213> Chlamydia trachomatis
<400> 17
   Met Ser Ser Lys Leu Val Asn Tyr Leu Arg Leu Thr Phe Leu Ser Phe
   1 5 10 15
   Leu Gly Ile Ala Ser Thr Ser Leu Asp Ala Met Pro Ala Gly Asn Pro
   20 25 30
   Ala Phe Pro Val Ile Pro Gly Ile Asn Ile Glu Gln Lys Asn Ala Cys
   35 40 45
   Ser Phe Asp Leu Cys Asn Ser Tyr Asp Val Leu Ser Ala Leu Ser Gly
   50 55 60
   Asn Leu Lys Leu Cys Phe Cys Gly Asp Tyr Ile Phe Ser Glu Glu Ala
   65 70 75 80
   Gln Val Lys Asp Val Pro Val Val Thr Ser Val Thr Thr Ala Gly Val
   85 90 95
   Gly Pro Ser Pro Asp Ile Thr Ser Thr Thr Lys Thr Arg Asn Phe Asp
   100 105 110
   Leu Val Asn Cys Asn Leu Asn Thr Asn Cys Val Ala Val Ala Phe Ser
   115 120 125
   Leu Pro Asp Arg Ser Leu Ser Ala Ile Pro Leu Phe Asp Val Ser Phe
   130 135 140
   Glu Val Lys Val Gly Gly Leu Lys Gln Tyr Tyr Arg Leu Pro Met Asn
   145 150 155 160
   Ala Tyr Arg Asp Phe Thr Ser Glu Pro Leu Asn Ser Glu Ser Glu Val
   165 170 175
   Thr Asp Gly Met Ile Glu Val Gln Ser Asn Tyr Gly Phe Val Trp Asp
   180 185 190
   Val Ser Leu Lys Lys Val Ile Trp Lys Asp Gly Val Ser Phe Val Gly
   195 200 205
   Val Gly Ala Asp Tyr Arg His Ala Ser Cys Pro Ile Asp Tyr Ile Ile
   210 215 220
   Ala Asn Ser Gln Ala Asn Pro Glu Val Phe Ile Ala Asp Ser Asp Gly
   225 230 235 240
   Lys Leu Asn Phe Lys Glu Trp Ser Val Cys Val Gly Leu Thr Thr Tyr
   245 250 255
   Val Asn Asp Tyr Val Leu Pro Tyr Leu Ala Phe Ser Ile Gly Ser Val
   260 265 270
   Ser Arg Gln Ala Pro Asp Asp Ser Phe Lys Lys Leu Glu Asp Arg Phe
   275 280 285
   Thr Asn Leu Lys Phe Lys Val Arg Lys Ile Thr Ser Ser His Arg Gly
   290 295 300
   Asn Ile Cys Ile Gly Ala Thr Asn Tyr Val Ala Asp Asn Phe Phe Tyr
   305 310 315 320
   Asn Val Glu Gly Arg Trp Gly Ser Gln Arg Ala Val Asn Val Ser Gly
   325 330 335
   Gly Phe Gln Phe
   340
<210> 18
   <211> 1023
   <212> DNA
   <213> Chlamydia trachomatis
<400> 18
   atgagtagca agctagtgaa ctatctccgt ttgactttcc tatctttttt agggatcgca 60
   tctacttcat tagacgctat gcctgcgggg aatccggcgt ttccagtcat cccggggatt 120
   aatattgaac agaaaaatgc ctgttctttc gatttatgta attcttatga tgtactatcc 180
   gcactgtccg gtaacctgaa gctctgcttc tgcggagatt atatcttttc agaagaagct 240
   caggtaaaag atgtccctgt cgttacctct gtgacaacag ctggggttgg tccttctcct 300
   gatattactt cgacaaccaa aacgcgaaat ttcgatctcg tgaactgtaa tctcaataca 360
   aactgtgtag ctgtagcttt ttcccttcct gatcgttcgc tgagcgcgat tcctctgttt 420
   gatgtgagtt tcgaagtgaa agtaggagga ctgaaacaat actaccgcct tcccatgaat 480
   gcctatcgag acttcacctc ggaacctctc aattctgaat cagaagttac ggacgggatg 540
   attgaagtac agtccaatta cggatttgtt tgggatgtta gcttgaaaaa agtcatatgg 600
   aaagatggcg tttcctttgt aggcgtcggt gcagactatc gccatgcttc ttgccctatt 660
   gactacatca ttgcaaacag tcaagctaat ccagaagtat tcatcgctga ctcggatggg 720
   aaactgaact tcaaggagtg gagtgtctgc gtaggtctta ctacctatgt gaatgactac 780
   gttcttcctt acttagcgtt ttctataggg agtgtttctc gccaagctcc ggacgacagc 840
   ttcaaaaaat tagaagatcg cttcactaac ctcaaattta aagttcgtaa aattaccagc 900
   tctcatcgtg gaaacatctg catcggagcg acaaactatg tcgccgataa cttcttctac 960
   aacgtagaag gaagatgggg aagccagcgc gctgtgaacg tctccggagg attccaattc 1020
   taa                                                               1023
<210> 19
   <211> 350
   <212> PRT
   <213> Chlamydia trachomatis
<400> 19
   Met Ser Leu Phe Ser Lys Phe Lys Ala Gln Trp Met Phe Leu His Ser
   1               5                   10                  15
   Arg Glu Leu Cys Ser Ser Thr Ser Asp Ile Gly Asn Thr Cys Ser Asp
               20                  25                  30
   Pro Val Phe Gln Val Leu Cys Asn Pro Val Arg Ser Glu Ile Ser Tyr
           35                  40                  45
   Lys Val Gly Asp Ser Leu Gly Val Phe Pro Thr Asn Pro Ser Ile Leu
       50                  55                  60
   Val Asp Ser Val Leu Asp Ala Leu Gln Tyr Gly Pro Arg Ser Pro Val
   65                  70                  75                  80
   Val Ser Arg His Ala Asp Ser Val Leu Pro Leu His Glu Phe Leu Thr
                   85                  90                  95
   Ser Tyr Val Asp Leu Asp Lys Ile Pro Lys Ser Leu Arg Pro Phe Phe
               100                 105                 110
   Pro Gly Asp Leu Asp Asp Thr Trp Ser Leu Ala Glu Ala Ile Leu Val
           115                 120                 125
   Tyr Gln Pro Arg Ile Pro Phe Glu Glu Phe Ile Arg Ser Ala Met Pro
       130                 135                 140
   Leu Leu Pro Arg Phe Tyr Ser Ile Ala Ser Ser Pro Thr Cys Ser His
   145                 150                 155                 160
   Gly Lys Leu Glu Leu Leu Val Arg Cys Val Ser Phe Gln Gly Lys Thr
                   165                 170                 175
   Gln Leu Arg Tyr Gly Leu Cys Ser Ala Phe Leu Cys Lys Asp Leu Gln
               180                 185                 190
   Glu Gly Glu Ser Phe Arg Gly Phe Ile Gln Pro Thr Arg His Phe Thr
           195                 200                 205
   Leu Glu Gln Lys Asn Phe Gly Lys Pro Leu Ile Met Ile Gly Ala Gly
       210                 215                 220
   Thr Gly Ile Ala Pro Tyr Lys Gly Phe Leu Gln His Arg Ile Tyr His
   225                 230                 235                 240
   Gln Asp Val Gly Ser Asn Ile Leu Phe Phe Gly Glu Arg Phe Glu Lys
                   245                 250                 255
   Ser Asn Phe Tyr Tyr Arg Asp Phe Leu Gln Glu Leu Ile Val Ser Gly
               260                 265                 270
   Lys Leu Gln Leu Phe Thr Ala Phe Ser Arg Asp Ser Glu Ser Lys Leu
           275                 280                 285
   Tyr Val Gln Asn Val Ile Glu Gln Gln Lys Glu Leu Ile Gln Glu Val
       290                 295                 300
   Tyr Glu Gln Glu Ala Phe Phe Phe Val Cys Gly Lys Lys Ile Leu Gly
   305                 310                 315                 320
   Thr Glu Val Lys Arg Ala Leu Glu Gln Ile Leu Gly Pro Lys Ala Val
                   325                 330                 335
   Arg Glu Leu Ile Ala Gln Lys Arg Leu Val Ser Asp Val Tyr
               340                 345                 350
<210> 20
   <211> 1053
   <212> DNA
   <213> Chlamydia trachomatis
<400> 20
   atgtctttat tttctaaatt caaagctcag tggatgtttt tacattcacg tgagctttgt 60
   tcttccacat cggatattgg gaatacttgt tcggatcctg tttttcaggt tttatgtaat 120
   ccggttcgtt ctgagatttc ctataaagtt ggggattctt tgggggtatt cccaacaaat 180
   ccttccatat tagtcgattc agttctagat gctttacagt atggccccag gtctcctgtc 240
   gtatctcggc atgcagattc tgttctccct cttcacgaat ttcttactag ttacgtagac 300
   ttagataaaa ttccaaaatc gttaagacct tttttcccag gggatttaga cgatacctgg 360
   tctttagctg aagctatttt ggtttaccag ccgcgtattc cttttgaaga gtttattcgg 420
   agtgcgatgc ctttattgcc tcgattttat tctatagctt cttctccaac atgttctcat 480
   gggaagctag agttgctcgt gcgctgtgtt agtttccaag gtaaaacgca gctgcgctat 540
   ggattatgtt cggctttttt atgtaaggac ttacaagagg gagagtcttt tcgtgggttt 600
   atacaaccga cgcggcattt tactttggag cagaaaaatt ttgggaaacc tttaattatg 660
   atcggagcag ggacaggtat cgctccgtac aaagggttct tacaacatcg aatataccat 720
   caggacgtag gctccaatat tctattcttt ggagagcgtt ttgagaaaag taacttctat 780
   taccgggatt ttctccagga gctgatcgtt tcaggaaaac tccagttatt cacagccttt 840
   tccagagatt ccgagtctaa attgtatgtt cagaatgtta tagagcaaca aaaagaactt 900
   atacaagaag tctacgaaca agaagctttc tttttgtttt gtgggaaaaa aatccttggt 960
   acggaagtta aacgtgcttt agagcagata ttaggtccta aggcggtacg agagctgatt 1020
   gcacagaaga gactagtttc agacgtatac taa                             1053
<210> 21
   <211> 1770
   <212> PRT
   <213> Chlamydia trachomatis
<400> 21
   Met Lys Phe Met Ser Ala Thr Ala Val Phe Ala Ala Ala Leu Ser Ser
   1               5                   10                  15
   Val Thr Glu Ala Ser Ser Ile Gln Asp Gln Ile Lys Asn Thr Asp Cys
               20                  25                  30
   Asn Val Ser Lys Leu Gly Tyr Ser Thr Ser Gln Ala Phe Thr Asp Met
           35                  40                  45
   Met Leu Ala Asp Asn Thr Glu Tyr Arg Ala Ala Asp Ser Val Ser Phe
       50                  55                  60
   Tyr Asp Phe Ser Thr Ser Ser Arg Leu Pro Arg Lys His Leu Ser Ser
   65                  70                  75                  80
   Ser Ser Glu Ala Ser Pro Thr Thr Glu Gly Val Ser Ser Ser Ser Ser
                   85                  90                  95
   Gly Glu Thr Asp Glu Lys Thr Glu Glu Glu Leu Asp Asn Gly Gly Ile
               100                 105                 110
   Ile Tyr Ala Arg Glu Lys Leu Thr Ile Ser Glu Ser Gln Asp Ser Leu
           115                 120                 125
   Ser Asn Gln Ser Ile Glu Leu His Asp Asn Ser Ile Phe Phe Gly Glu
       130                 135                 140
   Gly Glu Val Ile Phe Asp His Arg Val Ala Leu Lys Asn Gly Gly Ala
   145                 150                 155                 160
   Ile Tyr Gly Glu Lys Glu Val Val Phe Glu Asn Ile Lys Ser Leu Leu
                   165                 170                 175
   Val Glu Val Asn Ile Ala Val Glu Lys Gly Gly Ser Val Tyr Ala Lys
               180                 185                 190
   Glu Arg Val Ser Leu Glu Asn Val Thr Glu Ala Thr Phe Ser Ser Asn
   195 200 205
   Gly Gly Glu Gln Gly Gly Gly Gly Ile Tyr Ser Glu Gln Asp Met Leu
   210 215 220
   Ile Ser Asp Cys Asn Asn Val His Phe Gln Gly Asn Ala Ala Gly Ala
   225 230 235 240
   Thr Ala Val Lys Gln Cys Leu Asp Glu Glu Met Ile Val Leu Leu Ala
   245 250 255
   Glu Cys Val Asp Ser Leu Ser Glu Asp Thr Leu Asp Ser Thr Pro Glu
   260 265 270
   Thr Glu Gln Thr Glu Ser Asn Gly Asn Gln Asp Gly Ser Ser Glu Thr
   275 280 285
   Glu Asp Thr Gln Val Ser Glu Ser Pro Glu Ser Thr Pro Ser Pro Asp
   290 295 300
   Asp Val Leu Gly Lys Gly Gly Gly Ile Tyr Thr Glu Lys Ser Leu Thr
   305 310 315 320
   Ile Thr Gly Ile Thr Gly Thr Ile Asp Phe Val Ser Asn Ile Ala Thr
   325 330 335
   Asp Ser Gly Ala Gly Val Phe Thr Lys Glu Asn Leu Ser Cys Thr Asn
   340 345 350
   Thr Asn Ser Leu Gln Phe Leu Lys Asn Ser Ala Gly Gln His Gly Gly
   355 360 365
   Gly Ala Tyr Val Thr Gln Thr Met Ser Val Thr Asn Thr Thr Ser Glu
   370 375 380
   Ser Ile Thr Thr Pro Pro Leu Ile Gly Glu Val Ile Phe Ser Glu Asn
   385 390 395 400
   Thr Ala Lys Gly His Gly Gly Gly Ile Cys Thr Asn Lys Leu Ser Leu
   405 410 415
   Ser Asn Leu Lys Thr Val Thr Leu Thr Lys Asn Ser Ala Lys Glu Ser
   420 425 430
   Gly Gly Ala Ile Phe Thr Asp Leu Ala Ser Ile Pro Ile Thr Asp Thr
   435 440 445
   Pro Glu Ser Ser Thr Pro Ser Ser Ser Ser Pro Ala Ser Thr Pro Glu
   450 455 460
   Val Val Ala Ser Ala Lys Ile Asn Arg Phe Phe Ala Ser Thr Ala Lys
   465 470 475 480
   Pro Ala Ala Pro Ser Leu Thr Glu Ala Glu Ser Asp Gln Thr Asp Gln
   485 490 495
   Thr Glu Thr Ser Asp Thr Asn Ser Asp Ile Asp Val Ser Ile Glu Asn
   500 505 510
   Ile Leu Asn Val Ala Ile Asn Gln Asn Thr Ser Ala Lys Lys Gly Gly
   515 520 525
   Ala Ile Tyr Gly Lys Lys Ala Lys Leu Ser Arg Ile Asn Asn Leu Glu
   530 535 540
   Leu Ser Gly Asn Ser Ser Gln Asp Val Gly Gly Gly Leu Cys Leu Thr
   545 550 555 560
   Glu Ser Val Glu Phe Asp Ala Ile Gly Ser Leu Leu Ser His Tyr Asn
   565 570 575
   Ser Ala Ala Lys Glu Gly Gly Ala Ile His Ser Lys Thr Val Thr Leu
   580 585 590
   Ser Asn Leu Lys Ser Thr Phe Thr Phe Ala Asp Asn Thr Val Lys Ala
   595 600 605
   Ile Val Glu Ser Thr Pro Glu Ala Pro Glu Glu Ile Pro Pro Val Glu
   610 615 620
   Gly Glu Glu Ser Thr Ala Thr Glu Asp Pro Asn Ser Asn Thr Glu Gly
   625 630 635 640
   Ser Ser Ala Asn Thr Asn Leu Glu Gly Ser Gln Gly Asp Thr Ala Asp
   645 650 655
   Thr Gly Thr Gly Asp Val Asn Asn Glu Ser Gln Asp Thr Ser Asp Thr
   660 665 670
   Gly Asn Ala Glu Ser Glu Glu Gln Leu Gln Asp Ser Thr Gln Ser Asn
   675 680 685
   Glu Glu Asn Thr Leu Pro Asn Ser Asn Ile Asp Gln Ser Asn Glu Asn
   690 695 700
   Thr Asp Glu Ser Ser Asp Ser His Thr Glu Glu Ile Thr Asp Glu Ser
   705 710 715 720
   Val Ser Ser Ser Ser Glu Ser Gly Ser Ser Thr Pro Gln Asp Gly Gly
                   725                 730                 735
   Ala Ala Ser Ser Gly Ala Pro Ser Gly Asp Gln Ser Ile Ser Ala Asn
               740                 745                 750
   Ala Cys Leu Ala Lys Ser Tyr Ala Ala Ser Thr Asp Ser Ser Pro Val
           755                 760                 765
   Ser Asn Ser Ser Gly Ser Glu Glu Pro Val Thr Ser Ser Ser Asp Ser
       770                 775                 780
   Asp Val Thr Ala Ser Ser Asp Asn Pro Asp Ser Ser Ser Ser Gly Asp
   785                 790                 795                 800
   Ser Ala Gly Asp Ser Glu Glu Pro Thr Glu Pro Glu Ala Gly Ser Thr
                   805                 810                 815
   Thr Glu Thr Leu Thr Leu Ile Gly Gly Gly Ala Ile Tyr Gly Glu Thr
               820                 825                 830
   Val Lys Ile Glu Asn Phe Ser Gly Gln Gly Ile Phe Ser Gly Asn Lys
           835                 840                 845
   Ala Ile Asp Asn Thr Thr Glu Gly Ser Ser Ser Lys Ser Asp Val Leu
       850                 855                 860
   Gly Gly Ala Val Tyr Ala Lys Thr Leu Phe Asn Leu Asp Ser Gly Ser
   865                 870                 875                 880
   Ser Arg Arg Thr Val Thr Phe Ser Gly Asn Thr Val Ser Ser Gln Ser
                   885                 890                 895
   Thr Thr Gly Gln Val Ala Gly Gly Ala Ile Tyr Ser Pro Thr Val Thr
               900                 905                 910
   Ile Ala Thr Pro Val Val Phe Ser Lys Asn Ser Ala Thr Asn Asn Ala
           915                 920                 925
   Asn Asn Thr Thr Asp Thr Gln Arg Lys Asp Thr Phe Gly Gly Ala Ile
       930                 935                 940
   Gly Ala Thr Ser Ala Val Ser Leu Ser Gly Gly Ala His Phe Leu Glu
   945                 950                 955                 960
   Asn Val Ala Asp Leu Gly Ser Ala Ile Gly Leu Val Pro Gly Thr Gln
                   965                 970                 975
   Asn Thr Glu Thr Val Lys Leu Glu Ser Gly Ser Tyr Tyr Phe Glu Lys
               980                 985                 990
   Asn Lys Ala Leu Lys Arg Ala Thr Ile Tyr Ala Pro Val Val Ser Ile
           995                 1000                1005
   Lys Ala Tyr Thr Ala Thr Phe Asn Gln Asn Arg Ser Leu Glu Glu Gly
       1010                1015                1020
   Ser Ala Ile Tyr Phe Thr Lys Glu Ala Ser Ile Glu Ser Leu Gly Ser
   1025                1030                1035                1040
   Val Leu Phe Thr Gly Asn Leu Val Thr Leu Thr Leu Ser Thr Thr Thr
                   1045                1050                1055
   Glu Gly Thr Pro Ala Thr Thr Ser Gly Asp Val Thr Lys Tyr Gly Ala
               1060                1065                1070
   Ala Ile Phe Gly Gln Ile Ala Ser Ser Asn Gly Ser Gln Thr Asp Asn
           1075                1080                1085
   Leu Pro Leu Lys Leu Ile Ala Ser Gly Gly Asn Ile Cys Phe Arg Asn
       1090                1095                1100
   Asn Glu Tyr Arg Pro Thr Ser Ser Asp Thr Gly Thr Ser Thr Phe Cys
   1105                1110                1115                1120
   Ser Ile Ala Gly Asp Val Lys Leu Thr Met Gln Ala Ala Lys Gly Lys
                   1125                1130                1135
   Thr Ile Ser Phe Phe Asp Ala Ile Arg Thr Ser Thr Lys Lys Thr Gly
               1140                1145                1150
   Thr Gln Ala Thr Ala Tyr Asp Thr Leu Asp Ile Asn Lys Ser Glu Asp
           1155                1160                1165
   Ser Glu Thr Val Asn Ser Ala Phe Thr Gly Thr Ile Leu Phe Ser Ser
       1170                1175                1180
   Glu Leu His Glu Asn Lys Ser Tyr Ile Pro Gln Asn Val Val Leu His
   1185                1190                1195                1200
   Ser Gly Ser Leu Val Leu Lys Pro Asn Thr Glu Leu His Val Ile Ser
                   1205                1210                1215
   Phe Glu Gln Lys Glu Gly Ser Ser Leu Val Met Thr Pro Gly Ser Val
               1220                1225                1230
   Leu Ser Asn Gln Thr Val Ala Asp Gly Ala Leu Val Ile Asn Asn Met
           1235                1240                1245
   Thr Ile Asp Leu Ser Ser Val Glu Lys Asn Gly Ile Ala Glu Gly Asn
   1250 1255 1260
   Ile Phe Thr Pro Pro Glu Leu Arg Ile Ile Asp Thr Thr Thr Gly Gly
   1265 1270 1275 1280
   Ser Gly Gly Thr Pro Ser Thr Asp Ser Glu Ser Asn Gln Asn Ser Asp
   1285 1290 1295
   Asp Thr Glu Glu Gln Asn Asn Asn Asp Ala Ser Asn Gln Gly Glu Ser
   1300 1305 1310
   Ala Asn Gly Ser Ser Ser Pro Ala Val Ala Ala Ala His Thr Ser Arg
   1315 1320 1325
   Thr Arg Asn Phe Ala Ala Ala Ala Thr Ala Thr Pro Thr Thr Thr Pro
   1330 1335 1340
   Thr Ala Thr Thr Thr Thr Ser Asn Gln Val Ile Leu Gly Gly Glu Ile
   1345 1350 1355 1360
   Lys Leu Ile Asp Pro Asn Gly Thr Phe Phe Gln Asn Pro Ala Leu Arg
   1365 1370 1375
   Ser Asp Gln Gln Ile Ser Leu Leu Val Leu Pro Thr Asp Ser Ser Lys
   1380 1385 1390
   Met Gln Ala Gln Lys Ile Val Leu Thr Gly Asp Ile Ala Pro Gln Lys
   1395 1400 1405
   Gly Tyr Thr Gly Thr Leu Thr Leu Asp Pro Asp Gln Leu Gln Asn Gly
   1410 1415 1420
   Thr Ile Ser Val Leu Trp Lys Phe Asp Ser Tyr Arg Gln Trp Ala Tyr
   1425 1430 1435 1440
   Val Pro Arg Asp Asn His Phe Tyr Ala Asn Ser Ile Leu Gly Ser Gln
   1445 1450 1455
   Met Leu Met Val Thr Val Lys Gln Gly Leu Leu Asn Asp Lys Met Asn
   1460 1465 1470
   Leu Ala Arg Phe Glu Glu Val Ser Tyr Asn Asn Leu Trp Ile Ser Gly
   1475 1480 1485
   Leu Gly Thr Met Leu Ser Gln Val Gly Thr Pro Thr Ser Glu Glu Phe
   1490 1495 1500
   Thr Tyr Tyr Ser Arg Gly Ala Ser Val Ala Leu Asp Ala Lys Pro Ala
   1505 1510 1515 1520
   His Asp Val Ile Val Gly Ala Ala Phe Ser Lys Met Ile Gly Lys Thr
   1525 1530 1535
   Lys Ser Leu Lys Arg Glu Asn Asn Tyr Thr His Lys Gly Ser Glu Tyr
   1540 1545 1550
   Ser Tyr Gln Ala Ser Val Tyr Gly Gly Lys Pro Phe His Phe Val Ile
   1555 1560 1565
   Asn Lys Lys Thr Glu Lys Ser Leu Pro Leu Leu Leu Gln Gly Val Ile
   1570 1575 1580
   Ser Tyr Gly Tyr Ile Lys His Asp Thr Val Thr His Tyr Pro Thr Ile
   1585 1590 1595 1600
   Arg Glu Arg Asn Lys Gly Glu Trp Glu Asp Leu Gly Trp Leu Thr Ala
   1605 1610 1615
   Leu Arg Val Ser Ser Val Leu Arg Thr Pro Ala Gln Gly Asp Thr Lys
   1620 1625 1630
   Arg Ile Thr Val Tyr Gly Glu Leu Glu Tyr Ser Ser Ile Arg Gln Lys
   1635 1640 1645
   Gln Phe Thr Glu Thr Glu Tyr Asp Pro Arg Tyr Phe Asp Asn Cys Thr
   1650 1655 1660
   Tyr Arg Asn Leu Ala Ile Pro Met Gly Leu Ala Phe Glu Gly Glu Leu
   1665 1670 1675 1680
   Ser Gly Asn Asp Ile Leu Met Tyr Asn Arg Phe Ser Val Ala Tyr Met
   1685 1690 1695
   Leu Ser Ile Tyr Arg Asn Ser Pro Thr Cys Lys Tyr Gln Val Leu Ser
   1700 1705 1710
   Ser Gly Glu Gly Gly Glu Ile Ile Cys Gly Val Pro Thr Arg Asn Ser
   1715 1720 1725
   Ala Arg Gly Glu Tyr Ser Thr Gln Leu Tyr Leu Gly Pro Leu Trp Thr
   1730 1735 1740
   Leu Tyr Gly Ser Tyr Thr Ile Glu Ala Asp Ala His Thr Leu Ala His
   1745 1750 1755 1760
   Met Met Asn Cys Gly Ala Arg Met Thr Phe
   1765 1770
<210> 22
   <211> 5313
   <212> DNA
   <213> Chlamydia trachomatis
<400> 22
   atgaaattta tgtcagctac tgctgtattt gctgcagcac tctcctccgt tactgaggcg 60
   agctcgatcc aagatcaaat aaagaatacc gactgcaatg ttagcaaatt aggatattca 120
   acttctcaag catttactga tatgatgcta gcagacaaca cagagtatcg agctgctgat 180
   agtgtttcat tctatgactt ttcgacatct tccagattac ctagaaaaca tcttagtagt 240
   agtagtgaag cttctccaac gacagaagga gtgtcttcat cttcatctgg agaaactgat 300
   gagaaaacag aagaagaact agacaatggc ggaatcattt atgctagaga gaaactaact 360
   atctcagaat ctcaggactc tctctctaat caaagcatag aactccatga caatagtatt 420
   ttcttcggag aaggtgaagt tatctttgat cacagagttg ccctcaaaaa cggaggagct 480
   atttatggag agaaagaggt agtctttgaa aacataaaat ctctactagt agaagtaaat 540
   atcgcggtcg agaaaggggg tagcgtctat gcaaaagaac gagtatcttt agaaaatgtt 600
   accgaagcaa ccttctcctc caatggtggg gaacaaggtg gtggtggaat ctattcagaa 660
   caggatatgt taatcagtga ttgcaacaat gtacatttcc aagggaatgc tgcaggagca 720
   acagcagtaa aacaatgtct ggatgaagaa atgatcgtat tgctcgcaga atgcgttgat 780
   agcttatccg aagatacact ggatagcact ccagaaacgg aacagactga gtcaaatgga 840
   aatcaagacg gttcgtctga aacagaagat acacaagtat cagaatcacc agaatcaact 900
   cctagccccg acgatgtttt aggtaaaggt ggtggtatct atacagaaaa atctttgacc 960
   atcactggaa ttacagggac tatagatttt gtcagtaaca tagctaccga ttctggagca 1020
   ggtgtattca ctaaagaaaa cttgtcttgc accaacacga atagcctaca gtttttgaaa 1080
   aactcggcag gtcaacatgg aggaggagcc tacgttactc aaaccatgtc tgttactaat 1140
   acaactagtg aaagtataac tactccccct ctcataggag aagtgatttt ctctgaaaat 1200
   acagctaaag ggcacggtgg tggtatctgc actaacaaac tttctttatc taatttaaaa 1260
   acggtgactc tcactaaaaa ctctgcaaag gagtctggag gagctatttt tacagatctg 1320
   gcgtctatac caataacaga taccccagaa tcttctaccc cctcttcctc ctcgcctgca 1380
   agcactcctg aagtagttgc ttctgctaaa ataaatcgat tctttgcctc tacggcaaaa 1440
   ccggcagccc cttctctaac agaggctgag tctgatcaaa cggatcaaac agaaacttct 1500
   gatactaata gcgatataga cgtgtcgatt gagaacattt tgaatgtcgc tatcaatcaa 1560
   aacacttctg cgaaaaaagg aggggctatt tacgggaaaa aagctaaact ttcccgtatt 1620
   aacaatcttg aactttcagg gaattcatcc caggatgtag gaggaggtct ctgtttaact 1680
   gaaagcgtag aatttgatgc aattggatcg ctcttatccc actataactc tgctgctaaa 1740
   gaaggtgggg ctattcattc taaaacggtt actctatcta acctcaagtc taccttcact 1800
   tttgcagata acactgttaa agcaatagta gaaagcactc ctgaagctcc agaagagatt 1860
   cctccagtag aaggagaaga gtctacagca acagaagatc caaattctaa tacagaagga 1920
   agttcggcta acactaacct tgaaggatct caaggggata ctgctgatac agggactggt 1980
   gatgttaaca atgagtctca agacacatca gatactggaa acgctgaatc tgaagaacaa 2040
   ctacaagatt ctacacaatc taatgaagaa aatacccttc ccaatagtaa tattgatcaa 2100
   tctaacgaaa acacagacga atcatctgat agccacactg aggaaataac tgacgagagt 2160
   gtctcatcgt cctctgaaag tggatcatct actcctcaag atggaggagc agcttcttca 2220
   ggggctccct caggagatca atctatctct gcaaacgctt gtttagctaa aagctatgct 2280
   gcgagtactg atagctcccc cgtatctaat tcttcaggtt cagaagagcc tgtcacttct 2340
   tcttcagatt cagacgttac tgcatcttct gataatccag actcttcctc atctggagat 2400
   agcgctggag actctgaaga accgactgag ccagaagctg gttctacaac agaaactctt 2460
   actttaatag gaggaggtgc tatctatgga gaaactgtta agattgagaa cttctctggc 2520
   caaggaatat tttctggaaa caaagctatc gataacacca cagaaggctc ctcttccaaa 2580
   tctgacgtcc tcggaggtgc ggtctatgct aaaacattgt ttaatctcga tagcgggagc 2640
   tctagacgaa ctgtcacctt ctccgggaat actgtctctt ctcaatctac aacaggtcag 2700
   gttgctggag gagctatcta ctctcctact gtaaccattg ctactcctgt agtattttct 2760
   aaaaactctg caacaaacaa tgctaataac actacagata ctcagagaaa agacaccttt 2820
   ggaggagcta tcggagctac ttctgctgtt tctctatcag gaggggctca tttcttagaa 2880
   aacgttgctg acctcggatc tgctattggg ttggtgccag gcacacaaaa tacagaaaca 2940
   gtgaaattag agtctggctc ctactacttt gaaaaaaata aagctttaaa acgagctact 3000
   atttacgcac ctgtcgtttc cattaaagcc tatactgcga catttaacca aaacagatct 3060
   ctagaagaag gaagcgcgat ttactttaca aaagaagcat ctattgagtc tttaggctct 3120
   gttctcttca caggaaactt agtaacccta acgctaagca caactacaga aggcacacca 3180
   gccacaacct caggagatgt aacaaaatat ggtgctgcta tctttggaca aatagcaagc 3240
   tcaaacggat ctcagacgga taaccttccc ctgaaactca ttgcttcagg aggaaatatt 3300
   tgtttccgaa acaatgaata ccgtcctact tcttctgata ccggaacctc tactttctgt 3360
   agtattgcgg gagatgttaa attaaccatg caagctgcaa aagggaaaac gatcagtttc 3420
   tttgatgcaa tccggacctc tactaagaaa acaggtacac aggcaactgc ctacgatact 3480
   ctcgatatta ataaatctga ggattcagaa actgtaaact ctgcgtttac aggaacgatt 3540
   ctgttctcct ctgaattaca tgaaaataaa tcctatattc cacaaaacgt agttctacac 3600
   agtggatctc ttgtattgaa gccaaatacc gagcttcatg ttatttcttt tgagcagaaa 3660
   gaaggctctt ctctcgttat gacacctgga tctgttcttt cgaaccagac tgttgctgat 3720
   ggagctttgg tcataaataa catgaccatt gatttatcca gcgtagagaa aaatggtatt 3780
   gctgaaggaa atatctttac tcctccagaa ttgagaatca tagacactac tacaggtgga 3840
   agcggtggaa ccccatctac agatagtgaa agtaaccaga atagtgatga taccgaggag 3900
   caaaataata atgacgcctc gaatcaagga gaaagcgcga atggatcgtc ttctcctgca 3960
   gtagctgctg cacacacatc tcgtacaaga aactttgccg ctgcagctac agccacacct 4020
   acgacaacac caacggctac aactacaaca agcaaccaag taatcctagg aggagaaatt 4080
   aaactcatcg atcctaatgg gaccttcttc cagaaccctg cattaagatc cgaccaacaa 4140
   atctccttgt tagtgctccc tacagactca tcaaaaatgc aagctcagaa aatagtactg 4200
   acgggtgata ttgctcctca gaaaggatat acaggaacac tcactctgga tcctgatcaa 4260
   ctacaaaatg gaacgatctc agtgctctgg aaatttgact cttatagaca atgggcttat 4320
   gtacctagag acaatcattt ctatgcgaac tcgattctgg gatctcaaat gttaatggtc 4380
   acagtcaaac aaggcttgct caacgataaa atgaatctag ctcgctttga ggaagttagc 4440
   tataacaacc tgtggatatc aggactagga acgatgctat cgcaagtagg aacacctact 4500
   tctgaagaat tcacttatta cagcagagga gcttctgttg ccttagatgc taaaccagcc 4560
   catgatgtga ttgttggagc tgcatttagt aagatgatcg ggaaaacaaa atccttgaaa 4620
   agagagaata actacactca caaaggatcc gaatattctt accaagcatc ggtatacgga 4680
   ggcaaaccat tccactttgt aatcaataaa aaaacggaaa aatcgctacc gctattgtta 4740
   caaggagtca tctcttacgg atatatcaaa catgatacag tgactcacta tccaacgatc 4800
   cgtgaacgaa acaaaggaga atgggaagac ttaggatggc tgacagctct ccgtgtctcc 4860
   tctgtcttaa gaactcctgc acaaggggat actaaacgta tcactgttta cggagaattg 4920
   gaatactcca gtatccgtca gaaacaattc acagaaacag aatacgatcc tcgttacttc 4980
   gacaactgca cctatagaaa cttagcaatt cctatggggt tagcattcga aggagagctc 5040
   tctggtaacg atattttgat gtacaacaga ttctctgtag catacatgct atcaatctat 5100
   cgaaattctc caacatgcaa ataccaagtg ctctcttcag gagaaggcgg agaaattatt 5160
   tgtggagtac cgacaagaaa ctcagctcgc ggagaataca gcacgcagct gtacctggga 5220
   cctttgtgga ctctgtatgg atcctacacg atagaagcag acgcacatac actagctcat 5280
   atgatgaact gcggtgctcg tatgacattc taa 5313
<210> 23
   <211> 408
   <212> PRT
   <213> Chlamydia trachomatis
<400> 23
   Met Met Lys Pro Leu Arg Phe Gly Tyr Phe Phe Cys Ala Ile Tyr Phe
    1               5                  10                  15
   Thr Leu Leu Gln Ala Ala Phe Ala Lys Glu Pro Asn Ser Cys Pro Asp
               20                  25                  30
   Cys Gln Asn Asn Trp Lys Glu Val Thr His Thr Asp Gln Leu Pro Glu
           35                  40                  45
   Asn Ile Ile His Ala Asp Asp Ala Cys Tyr His Ser Gly Tyr Val Gln
       50                  55                  60
   Ala Leu Ile Asp Met His Phe Leu Asp Ser Cys Cys Gln Val Ile Val
   65                  70                  75                  80
   Glu Asn Gln Thr Ala Tyr Leu Phe Ser Leu Pro Thr Asp Asp Val Thr
                   85                  90                  95
   Arg Asn Ala Ile Ile Asn Leu Ile Lys Asp Leu Pro Phe Ile His Ser
               100                 105                 110
   Val Glu Ile Cys Gln Ala Ser Tyr Gln Thr Cys His His Gln Gly Pro
           115                 120                 125
   His Gly Lys Thr Ser Leu Pro Glu Gln Arg Ser Phe Cys Thr Lys Val
       130                 135                 140
   Cys Gly Lys Glu Ala Ile Trp Leu Pro Gln Asn Thr Ile Leu Phe Ser
   145                 150                 155                 160
   Pro Leu Val Ala Asp Pro Arg Gln Ala Thr Asn Ser Ala Gly Ile Arg
                   165                 170                 175
   Phe Asn Asp Glu Val Leu Gly Lys Arg Val Gly Ser Ala Thr Phe Gly
               180                 185                 190
   Gly Asp Phe Ile Phe Leu Arg Leu Phe Asp Ile Ser Arg Phe His Gly
           195                 200                 205
   Asp Met Asp Ile Gly Leu Gln Gly Ala Val Phe Ser Val Phe Asp Leu
       210                 215                 220
   Asp His Pro Glu Ala Cys Met Val Asn Ser Asp Phe Phe Val Ala Ala
   225                 230                 235                 240
   Leu Cys Asn Phe Ala Val Asn Lys Trp Ser Tyr Arg Phe Arg Leu Trp
                   245                 250                 255
   His Leu Ser Ser His Leu Gly Asp Glu Phe Ile Leu Ala Asn Gln Leu
               260                 265                 270
   Pro Pro Lys Lys Arg Tyr Asn Arg Ser Asp Glu Ala Val Asp Phe Phe
           275                 280                 285
   Ala Ser Phe Arg Tyr Thr Pro Gln Ile Arg Val Tyr Gly Gly Ile Gly
       290                 295                 300
   Tyr Ile Ile Ser Arg Asp Leu Thr Phe Pro Glu Asp Pro Leu Tyr Phe
   305                 310                 315                 320
   Glu Gly Gly Ile Glu Leu Arg Pro Phe Gly Leu Arg Glu Asp Asn Leu
                   325                 330                 335
   His Ala Gln Pro Val Phe Ala Met His Phe Arg Phe Trp Glu Glu His
               340                 345                 350
   Asp Phe Ser Ile Asp Gln Thr Tyr Ile Val Gly Met Glu Trp Ser Lys
           355                 360                 365
   Phe Gln Asp Val Gly Arg Lys Val Arg Ala Val Leu Glu Tyr His Gln
       370                 375                 380
   Gly Phe Ser His Glu Gly Gln Phe Val Arg Glu Glu Cys Asp Tyr Tyr
   385                 390                 395                 400
   Gly Phe Arg Leu Ser Tyr Gly Phe
                   405
<210> 24
   <211> 1227
   <212> DNA
   <213> Chlamydia trachomatis
<400> 24
   atgatgaaac ctctacgttt cggttatttc ttttgcgcaa tctattttac tttgttacag 60
   gcagcgtttg ctaaagaacc gaattcttgt cccgactgcc agaataattg gaaagaagtc 120
   acccacacgg atcaactccc agaaaacatc attcatgctg atgatgcttg ttatcactct 180
   ggttatgtac aggctctcat tgatatgcat ttcttagata gctgctgcca ggtcatcgtt 240
   gaaaaccaaa ctgcttactt attttctctt cctacagatg atgttacgcg caacgccatt 300
   atcaacctaa ttaaagacct tccattcatt cactccgtag aaatctgcca agcatcctat 360
   caaacctgtc atcatcaagg ccctcatgga aagacttctc ttccagaaca acgttctttc 420
   tgtacaaagg tctgtggaaa agaagctatt tggttaccac agaataccat cctattctcg 480
   cctcttgtag cagatcctag acaagcaact aatagtgcag gtatccgttt taacgacgaa 540
   gtcttaggaa aacgtgttgg ctctgctacc ttcggtggag atttcatctt cttacgatta 600
   tttgatatct cccgattcca tggagacatg gatattggtc tccaaggagc tgtattctct 660
   gttttcgacc tggatcatcc agaagcttgc atggtcaact ctgacttttt tgtcgccgct 720
   ttgtgcaact ttgcagtgaa caaatggagc taccgcttca gactatggca tctttcttct 780
   catcttggcg acgaatttat tcttgccaac cagttacctc ctaaaaaacg ttataatcga 840
   agcgatgaag ccgtcgattt ctttgcttct tttcgttaca ctccacagat ccgtgtttat 900
   ggaggtattg ggtatatcat tagtcgagat ttaacattcc ctgaagatcc tctttacttt 960
   gaaggaggta tcgaactacg tcctttcgga ttacgggaag acaaccttca tgcccaaccc 1020
   gtctttgcta tgcattttcg cttttgggaa gagcatgact tttctataga ccaaacttat 1080
   atagtaggca tggagtggtc caaattccag gatgtaggga gaaaagtgcg cgctgtattg 1140
   gaataccacc aaggtttctc ccacgaagga caatttgtcc gagaagaatg cgattattat 1200
   ggctttcgat taagttatgg cttctag                                    1227
<210> 25
   <211> 792
   <212> PRT
   <213> Chlamydia trachomatis
<400> 25
   Met Leu Gly Ile Arg Lys Lys Thr Ile Leu Gln Leu Ala Val Leu Leu
   1               5                   10                  15
   Leu Leu Thr Phe Ser Arg Ser Ser Phe Cys Ser Thr Ser Glu Gly Arg
               20                  25                  30
   Met Val Val Glu Ser Ile Thr Ile Thr Thr Gln Gly Glu Asn Thr Gln
           35                  40                  45
   Asn Lys Arg Ala Ile Pro Lys Ile Lys Thr Lys Gln Gly Thr Leu Phe
   50 55 60
   Ser Gln Ala Asp Phe Asp Glu Asp Leu Arg Thr Leu Ser Lys Asp Phe
   65 70 75 80
   Asp Arg Val Glu Pro Ile Val Glu Phe Arg Asn Gly Gln Ala Val Ile
   85 90 95
   Ser Leu Ile Leu Thr Ala Lys Pro Val Ile Arg Glu Ile Asn Ile Ser
   100 105 110
   Gly Asn Glu Ala Ile Pro Thr His Lys Ile Leu Lys Thr Leu Glu Leu
   115 120 125
   Tyr Lys Asn Asp Leu Phe Asp Arg Glu Leu Phe Phe Lys Asn Phe Asp
   130 135 140
   Ala Leu Arg Thr Leu Tyr Leu Lys Arg Gly Tyr Tyr Asp Ser Gln Leu
   145 150 155 160
   Ser Tyr Ser His Asn His Asn Glu Lys Glu Gly Phe Ile Asp Ile Ser
   165 170 175
   Ile Glu Ile Lys Glu Gly Arg His Gly Arg Ile Lys Lys Leu Thr Ile
   180 185 190
   Ser Gly Ile Thr Arg Thr Glu Ala Ser Asp Leu Gly Asp Ile Val Leu
   195 200 205
   Thr Lys Gln Tyr Ser Thr Thr Thr Ser Trp Phe Thr Gly Ala Gly Val
   210 215 220
   Tyr His Pro Asp Met Val Glu Gln Asp Leu Phe Ala Ile Thr Asn Tyr
   225 230 235 240
   Phe Gln Asn Lys Gly Tyr Ala Asp Ala Lys Val Ser Lys Glu Val Ser
   245 250 255
   Thr Asp Ala Lys Gly Asn Ile Thr Leu Leu Ile Val Val Asp Lys Gly
   260 265 270
   Pro Leu Tyr Thr Leu Gly His Val His Ile Glu Gly Phe Thr Ala Leu
   275 280 285
   Ser Lys Arg Leu Leu Asp Lys Gln Leu Leu Val Gly Pro Asn Ser Leu
   290 295 300
   Tyr Cys Pro Asp Lys Ile Trp Thr Gly Ala Gln Lys Ile Arg Ser Ala
   305 310 315 320
   Tyr Ala Arg Tyr Gly Tyr Val Asn Thr Asn Val Asp Val Ser Phe Ser
   325 330 335
   Ala His Pro Thr Leu Pro Val Tyr Asp Val Thr Tyr Arg Val Ser Glu
   340 345 350
   Gly Ser Pro Tyr Lys Ile Gly Leu Ile Lys Ile Lys Gly Asn Thr His
   355 360 365
   Thr Lys His Asp Val Ile Leu His Glu Thr Ser Leu Phe Pro Gly Asp
   370 375 380
   Thr Phe Asp Arg Leu Lys Leu Glu Gly Thr Glu Thr Arg Leu Arg Asn
   385 390 395 400
   Thr Gly Tyr Phe Lys Ser Val Ser Val Tyr Thr Val Arg Ser Gln Leu
   405 410 415
   Asp Pro Leu Asp Ser Asn Asp Leu Tyr Arg Asp Val Phe Ile Glu Val
   420 425 430
   Lys Glu Thr Glu Thr Gly Asn Leu Gly Leu Phe Leu Gly Phe Ser Ser
   435 440 445
   Ile Asp His Leu Phe Gly Gly Ala Glu Ile Ala Glu Ser Asn Phe Asp
   450 455 460
   Leu Phe Gly Ala Arg Asn Phe Leu Lys Lys Gly Phe Lys Ser Leu Arg
   465 470 475 480
   Gly Gly Gly Glu Tyr Leu Phe Leu Lys Ala Asn Leu Gly Asp Lys Val
   485 490 495
   Thr Asp Tyr Thr Val Lys Trp Thr Lys Pro His Phe Leu Asn Thr Pro
   500 505 510
   Trp Ile Leu Gly Val Glu Leu Asp Lys Ser Ile Asn Lys Ala Leu Ser
   515 520 525
   Lys Asp Tyr Ser Val Asp Thr Tyr Gly Gly Asn Ile Ser Thr Thr Tyr
   530 535 540
   Ile Leu Asn Asp Lys Leu Lys Tyr Gly Met Tyr Tyr Arg Gly Ser Gln
   545 550 555 560
   Thr Ser Leu Ser Leu Arg Lys Lys Thr Ser Ser Ser Asn Arg Pro Gly
   565 570 575
   Pro Asp Leu Asp Ser Asn Lys Gly Phe Val Ser Ala Ala Gly Leu Asn
   580 585 590
   Val Leu Tyr Asp Ser Ile Asp Asn Pro Arg Lys Pro Thr Met Gly Ile
   595 600 605
   Arg Ser Ser Leu Asn Phe Glu Leu Ser Gly Leu Gly Gly Thr Tyr Gln
   610 615 620
   Phe Thr Lys Leu Thr Ala Ser Gly Ser Ile Tyr Arg Leu Leu Thr Lys
   625 630 635 640
   Lys Gly Val Leu Lys Val Arg Ala Glu Ala Lys Phe Ile Lys Pro Phe
   645 650 655
   Gly Thr Thr Thr Ala Gln Gly Ile Pro Val Ser Glu Arg Phe Phe Leu
   660 665 670
   Gly Gly Glu Thr Thr Val Arg Gly Tyr Lys Pro Phe Ile Ile Gly Pro
   675 680 685
   Lys Phe Ser Pro Thr Glu Pro Gln Gly Gly Leu Ser Ser Leu Leu Leu
   690 695 700
   Thr Glu Glu Phe Gln Tyr Pro Leu Ile Ser Gln Pro Cys Ile Asn Ala
   705 710 715 720
   Phe Val Phe Leu Asp Ser Gly Phe Ile Gly Ile Glu Glu Tyr Thr Ile
   725 730 735
   Arg Leu Lys Asp Leu Cys Ser Ser Ala Gly Phe Gly Leu Arg Phe Asp
   740 745 750
   Met Met Asn Asn Val Pro Ile Met Leu Gly Trp Gly Trp Pro Phe Arg
   755 760 765
   Pro Thr Glu Ile Leu Asn Asn Glu Lys Ile Asp Val Ser Gln Arg Phe
   770 775 780
   Phe Phe Ala Leu Gly Gly Val Phe
   785 790
<210> 26
   <211> 2379
   <212> DNA
   <213> Chlamydia trachomatis
<400> 26
   atgcttggaa tacgcaaaaa aacgattctg caactcgctg ttttactgtt gctcaccttt 60
   tcacgaagtt ctttctgttc aacttcagaa ggacgtatgg tcgtagagtc tatcaccatt 120
   acgactcaag gagagaatac tcaaaataaa cgagctattc ctaaaataaa aacaaagcag 180
   gggacgttgt tctctcaagc agattttgat gaagatctaa gaacactttc gaaagatttt 240
   gatcgagtag agcctatcgt agagtttcgt aatggacaag ctgtgatctc tctgattctg 300
   acggcaaaac ctgttatcag agagatcaat atttcaggaa atgaagctat ccccactcat 360
   aaaattctga aaactttaga gctttataaa aatgatcttt ttgatcggga attattcttt 420
   aaaaattttg atgcgctaag aactctttat ttgaaacgag ggtactacga ttctcaactc 480
   tcctattctc ataatcataa tgagaaagag ggctttatcg atatttccat cgagattaaa 540
   gaaggacgtc acggtcgcat aaaaaaatta acgatttcgg gaattacgcg aacagaagca 600
   tcagacttag gtgacattgt tttaactaaa caatactcca caacaacgag ctggttcact 660
   ggtgccggag tgtatcatcc ggacatggta gagcaagact tatttgctat cacaaattac 720
   ttccaaaata aaggatatgc tgatgctaaa gtaagcaaag aggtctctac agatgctaaa 780
   ggaaacatta ctttgcttat cgttgtagac aaaggacctt tatacacatt aggtcacgta 840
   catatagaag gattcacagc gttatccaaa agactgctcg ataaacaact attggttgga 900
   cctaactcct tatattgccc agataaaatt tggactggag cacaaaagat tcgtagcgca 960
   tacgctagat atggctacgt gaacactaac gttgatgtct ccttctcagc gcaccccact 1020
   ctacctgttt acgatgttac ctatcgagtg agtgaaggat ctccctacaa aatcgggtta 1080
   attaaaatca aagggaacac tcatactaag catgatgtga ttttgcatga gactagtctt 1140
   ttccctggag acacttttga tagattaaaa ctggaaggta cagagactcg tttacgcaac 1200
   accggctact ttaaaagtgt aagtgtctat acggttcgtt cccaattaga tcctcttgat 1260
   tctaacgacc tttatcgaga tgtttttatt gaagtcaaag agactgaaac aggaaatctt 1320
   gggctattct taggattcag ctccattgac catttatttg gaggggcaga aattgcagaa 1380
   agcaactttg atttatttgg agcccgaaac tttctcaaaa aaggattcaa atctttaaga 1440
   ggtggtggag aatacctctt cctaaaagct aatttaggag ataaggtcac cgattacact 1500
   gttaaatgga cgaaaccaca cttcttaaat accccttgga ttcttggagt agaattagat 1560
   aaatcaatta ataaagcttt atcaaaagac tactctgtgg atacctatgg agggaatatc 1620
   agtaccacct acattcttaa cgataagtta aaatatggga tgtattaccg tggtagccaa 1680
   acaagcttaa gtttgcgcaa aaaaacgtcc agctctaata gacctggacc agatttagat 1740
   agtaataaag gatttgtttc cgcagcggga ctcaatgttc tctatgattc tattgataat 1800
   cctagaaaac ctactatggg aatccgcagc tccttaaact ttgaattatc tggtttaggc 1860
   ggaacttacc aatttactaa actaacagct agtggttcta tctatcgctt attaactaaa 1920
   aaaggtgttt tgaaagtccg tgcagaagct aagtttatca aacctttcgg aacaacaact 1980
   gcacaaggca ttcctgtcag cgaacggttc ttcttaggag gtgaaaccac tgttcgcggt 2040
   tacaaacctt ttattattgg accgaaattt tctcctactg aaccacaagg aggcttgtct 2100
   tccctactat taacagaaga atttcaatat cctttgattt ctcaaccttg cattaatgcc 2160
   tttgtatttc tagattccgg attcattggg atagaagagt acactattcg cctgaaagac 2220
   ctttgcagta gcgccggatt tggtctacgc tttgatatga tgaataatgt gccaattatg 2280
   ctaggctggg gttggccgtt ccgcccaaca gaaatcctca ataatgaaaa aattgatgta 2340
   tctcaaagat tcttttttgc cttgggagga gtattctag 2379
<210> 27
   <211> 254
   <212> PRT
   <213> Chlamydia trachomatis
<400> 27
   Met Phe Ala Phe Leu Thr Ser Lys Lys Val Gly Ile Leu Pro Ser Arg
   1 5 10 15
   Trp Gly Ser Ser Arg Phe Pro Gly Lys Pro Leu Ala Lys Ile Leu Gly
   20 25 30
   Lys Thr Leu Val Gln Arg Ser Tyr Glu Asn Ala Leu Ser Ser Gln Ser
   35 40 45
   Leu Asp Cys Val Val Val Ala Thr Asp Asp Gln Arg Ile Phe Asp His
   50 55 60
   Val Val Glu Phe Gly Gly Leu Cys Val Met Thr Ser Thr Ser Cys Ala
   65 70 75 80
   Asn Gly Thr Glu Arg Val Glu Glu Val Val Ser Arg His Phe Pro Gln
   85 90 95
   Ala Glu Ile Val Val Asn Ile Gln Gly Asp Glu Pro Cys Leu Ser Pro
   100 105 110
   Thr Val Ile Asp Gly Leu Val Ser Thr Leu Glu Asn Asn Pro Ala Ala
   115 120 125
   Asp Met Val Thr Ser Val Thr Glu Thr Thr Asp Pro Glu Ala Ile Leu
   130 135 140
   Thr Asp His Lys Val Lys Cys Val Phe Asp Lys Asn Gly Lys Ala Leu
   145 150 155 160
   Tyr Phe Ser Arg Ser Ala Ile Pro His Asn Phe Lys His Pro Thr Pro
   165 170 175
   Ile Tyr Leu His Ile Gly Val Tyr Ala Phe Arg Lys Ala Phe Leu Ser
   180 185 190
   Glu Tyr Val Lys Ile Pro Pro Ser Ser Leu Ser Leu Ala Glu Asp Leu
   195 200 205
   Glu Gln Leu Arg Val Leu Glu Ile Gly Arg Ser Ile Tyr Val His Val
   210 215 220
   Val Gln Asn Ala Thr Gly Pro Ser Val Asp Tyr Pro Glu Asp Ile Thr
   225 230 235 240
   Lys Val Glu Gln Tyr Leu Leu Cys Leu Ser Lys Ala Ser Phe
   245 250
<210> 28
   <211> 765
   <212> DNA
   <213> Chlamydia trachomatis
<400> 28
   gtgtttgcgt ttttaaccag caaaaaagtc ggcattctcc cctctagatg gggaagctcc 60
   cgcttccccg gaaaacctct agcaaaaatt ctagggaaaa cccttgttca aagatcctat 120
   gaaaatgcct taagcagtca atctctagat tgcgttgttg tggcaacaga tgatcaacga 180
   atttttgacc atgtcgttga atttgggggg ctctgtgtca tgactagcac atcttgcgct 240
   aacggaactg agcgagtaga agaggttgtg tctcgacatt ttcctcaagc agagattgtt 300
   gtgaacatcc aaggagacga gccctgttta tctcctaccg tcatagatgg gcttgtgagc 360
   acgctagaga acaatcctgc tgcagatatg gtcacatctg ttacagaaac aacagacccc 420
   gaagcgatat tgacagatca caaagtgaag tgtgttttcg ataagaatgg caaagctctt 480
   tactttagca gaagcgctat tcctcacaac tttaaacacc caacgcctat ttatctgcat 540
   attggtgttt atgcttttag aaaagctttt ctaagtgaat atgttaaaat tcctccttcc 600
   tcgttaagcc tagccgaaga tcttgaacaa ttacgagtat tagaaatagg tcgttctatc 660
   tacgttcatg tcgttcagaa tgcaacgggc ccttctgttg attatcccga agatataacc 720
   aaagtggagc agtatttatt atgtctttca aaagcatctt tttga 765
<210> 29
   <211> 692
   <212> PRT
   <213> Chlamydia trachomatis
<400> 29
   Met Ile Arg Gln Trp Tyr Gly Phe Phe Leu Cys Leu Leu Phe Ser Tyr
   1 5 10 15
   Thr Ser Cys Phe Gly Val Glu Glu Asn Ser Gly Arg Ala Thr Pro Thr
   20 25 30
   Val Glu Leu Val Ser Glu Ser Glu Gln Ala Val Glu Gly Glu Val Leu
   35 40 45
   Arg Ile Gly Val Leu Ile Ala Ile Pro Glu Gly Glu His Ile Tyr Trp
   50 55 60
   Lys Asn Pro Gly Lys Leu Gly Met Pro Leu Arg Ile Ser Trp Asp Leu
   65 70 75 80
   Pro Ser Gly Cys Arg Leu Leu Glu Glu His Trp Pro Thr Pro Glu Ile
   85 90 95
   Phe Glu Glu Asp Gly Val Val Tyr Phe Gly Tyr Lys His Ser Thr Met
   100 105 110
   Val Val Ala Asp Ile Arg Val Ser Lys Glu Ile Glu Thr Arg Pro Leu
   115 120 125
   Glu Ile Lys Ala Gln Val Glu Trp Leu Ser Cys Gly Ala Ser Cys Leu
   130 135 140
   Pro Gly Ser Ser Ser Arg Val Leu Val Ile Pro Ile Asp Gln Gly Pro
   145 150 155 160
   Leu Ile Pro Asn Ser Lys Glu Thr Phe Thr Phe Ser Arg Ala Leu Ala
   165 170 175
   Ala Gln Pro Arg Pro Leu Asp Ala Ala Ile Lys Ile Ser Tyr Gln Pro
   180 185 190
   Asp Gly Leu Asp Val Phe Val Pro Ala Gly Lys Ala Asp Arg Ala Thr
   195 200 205
   Gln Ala Trp Phe Ile Ala Glu Asn Thr Arg Asp Phe Ala Tyr Ala Gln
   210 215 220
   Glu Val Pro Leu Glu Gln Ala Thr Thr Tyr Ile Trp Lys Leu Lys His
   225 230 235 240
   Pro Glu Gly Asn Met Pro Lys Gly Ile Gly Leu Ser Gly Ile Leu Ile
   245 250 255
   Phe Lys Asp Asp Ala Gly Lys Val Val Ala Ser Tyr Gln Val Glu Glu
   260 265 270
   Asn Gln Val Glu Gln Leu Ser Ala Leu Ser Trp Arg Phe Leu Ser Ile
   275 280 285
   Leu Leu Met Ala Phe Ile Gly Gly Ile Leu Leu Asn Ile Met Pro Cys
   290 295 300
   Val Leu Pro Leu Ile Thr Leu Lys Val Phe Ser Leu Ile Lys Ser Ala
   305 310 315 320
   Ala Asp His His Ser Ser Ser Val Ile Gly Gly Ile Gly Phe Thr Leu
   325 330 335
   Gly Ala Ile Val Ser Phe Trp Gly Leu Ala Phe Cys Ala Phe Leu Leu
   340 345 350
   Lys Val Leu Gly Gln Asn Ile Gly Trp Gly Phe Gln Leu Gln Glu Pro
   355 360 365
   Met Phe Val Ala Val Leu Ile Ile Val Phe Phe Leu Phe Ala Leu Ser
   370 375 380
   Ser Leu Gly Val Phe Glu Met Gly Ile Ile Cys Leu Ser Leu Gly Lys
   385 390 395 400
   Lys Leu Gln Glu Glu Gly Gly Ala Ser Val Arg Lys Asn Gln Ile Trp
   405 410 415
   Gly Ala Phe Phe Asn Gly Met Leu Thr Thr Leu Val Thr Thr Pro Cys
   420 425 430
   Thr Gly Pro Phe Leu Gly Ser Val Phe Gly Leu Val Met Ala Val Ser
   435 440 445
   Phe Val Lys Gln Leu Ala Ile Phe Thr Ala Ile Gly Leu Gly Met Ala
   450 455 460
   Ser Pro Tyr Leu Leu Phe Ala Ser Phe Pro Lys Met Leu Ala Ile Leu
   465 470 475 480
   Pro Lys Pro Gly Pro Trp Met Ser Thr Phe Lys Gln Leu Thr Gly Phe
   485 490 495
   Met Leu Leu Ala Thr Ala Thr Trp Leu Ile Trp Ile Phe Gly Val Glu
   500 505 510
   Thr Ser Ala Thr Ala Val Thr Ile Leu Leu Val Gly Leu Trp Leu Ala
   515 520 525
   Ala Val Gly Ala Trp Ile Leu Gly Arg Trp Gly Thr Leu Val Ser Pro
   530 535 540
   Arg Asn Gln Arg Leu Leu Ala Ser Val Val Phe Ile Phe Cys Ile Leu
   545 550 555 560
   Ser Ser Leu Val Ile Thr Ser Ile Gly Val Arg Tyr Phe Asp Glu Asn
   565 570 575
   Val Pro Pro Ala His Ser Ser Asp Trp Gln Ser Phe Ser Pro Glu Lys
   580 585 590
   Leu Ala Asp Leu Arg Glu Lys Gly Ile Pro Val Phe Val Asn Phe Thr
   595 600 605
   Ala Lys Trp Cys Leu Thr Cys Gln Leu Asn Lys Pro Leu Leu His Ala
   610 615 620
   Asn Met Gln Ala Phe Ala Ala Lys Gly Val Val Thr Leu Glu Ala Asp
   625 630 635 640
   Trp Thr Lys Lys Asp Pro Lys Ile Thr Glu Glu Leu Ala Arg Leu Gly
   645 650 655
   Arg Ala Ser Val Pro Ser Tyr Val Tyr Tyr Pro Ala Gly Asn Lys Ala
   660 665 670
   Pro Leu Ile Leu Pro Glu Arg Leu Ser Gln Ser Ala Leu Glu Glu Met
   675 680 685
   Val Phe Ser Gln
   690
<210> 30
   <211> 2079
   <212> DNA
   <213> Chlamydia trachomatis
<400> 30
   atgattcggc aatggtatgg atttttcttt tgcttgctgt tcagctatac gtcttgtttt 60
   ggtgtagaag aaaatagtgg aagagctacg cctacagtag aacttgttag tgaaagcgaa 120
   caagctgttg aaggagaagt gcttcgtatc ggagtattga ttgctattcc agaaggagag 180
   catatctact ggaaaaatcc agggaagctt ggaatgcctt tgcgcatttc ttgggatttg 240
   ccatcaggat gtaggttgct ggaggaacat tggccgactc cagagatttt cgaagaggat 300
   ggggttgttt attttggtta taaacattct acaatggtgg ttgcggatat tcgcgtttct 360
   aaagagatag aaacgcgtcc attggagata aaagcgcaag ttgaatggtt gtcttgcggt 420
   gcatcttgtc tcccaggttc ttcgtcaagg gttcttgtga ttcctataga tcaggggccg 480
   ttaattccta atagtaaaga gacattcact ttttcccgtg cgttagcggc tcaacctcga 540
   cctttggatg ctgccataaa gatttcttat cagcctgatg gcttagatgt ttttgtgcca 600
   gcagggaaag cggatcgggc aacccaggca tggttcattg ctgaaaacac gcgagatttt 660
   gcttatgctc aagaggttcc tcttgagcaa gcgactacgt acatatggaa gttgaaacat 720
   cctgaaggaa atatgcctaa gggtattggg ttgtcgggga ttcttatatt caaggatgat 780
   gcagggaaag tagtcgcttc gtatcaagta gaagagaatc aagtcgaaca gctttcggca 840
   ttgagctgga ggtttctctc tattcttctt atggctttca ttggtggaat cttattaaac 900
   atcatgccct gtgtattgcc tctgattact ttgaaagtat ttagtttaat taaatcggcg 960
   gcagatcacc attcttcctc tgtgattgga gggattgggt ttactttagg ggctattgta 1020
   agcttttggg gactcgcttt ttgtgcgttt ttgttaaagg ttttagggca aaatattgga 1080
   tggggattcc agcttcaaga acccatgttt gttgccgttt taattattgt cttcttctta 1140
   tttgctctga gttcgttagg cgtttttgag atgggaataa tttgtctgag cctagggaaa 1200
   aaattgcaag aagagggagg ggcatcggta aggaagaatc agatctgggg agcttttttc 1260
   aatgggatgt tgactaccct ggttacaact ccttgcactg ggccttttct tggctctgta 1320
   tttggattag ttatggcagt gtcttttgtt aagcagctgg caatttttac tgctatagga 1380
   ttaggaatgg caagtcccta tctattattt gcttcttttc cgaagatgct agccatttta 1440
   cctaaacctg gtccttggat gagtacgttt aaacagttga ctgggtttat gttgcttgct 1500
   actgcaactt ggcttatctg gatttttggg gtagagacga gtgcaaccgc tgtaactatt 1560
   cttcttgtag gattgtggtt ggctgctgta ggtgcatgga ttctagggag atggggaacc 1620
   cttgtatctc cgcgtaatca gcggcttctt gcttccgttg tattcatttt ctgtatttta 1680
   agttccttag tgattacctc tataggtgtc cgttattttg atgagaacgt ccctcctgca 1740
   catagctctg attggcaatc tttttctccc gaaaagctag ctgatttacg cgaaaaaggg 1800
   attccagttt ttgtaaattt cactgcaaag tggtgtttaa cgtgtcaact caataagcct 1860
   cttcttcatg ccaatatgca agcttttgct gctaagggcg tagttacttt agaagcagat 1920
   tggacgaaaa aagatccaaa aattacagaa gaactcgctc gtttaggccg agccagtgta 1980
   ccttcttatg tgtattaccc tgcggggaac aaagctccgc ttattcttcc agaaagatta 2040
   tcgcaatctg ctttggaaga gatggttttt tctcagtag                       2079
<210> 31
   <211> 326
   <212> PRT
   <213> Chlamydia trachomatis
<400> 31
   Met Phe Arg Tyr Thr Leu Ser Arg Ser Leu Phe Phe Ile Leu Ala Leu
    1               5                  10                  15
   Phe Phe Cys Ser Ala Cys Asp Ser Arg Ser Met Ile Thr His Gly Leu
               20                  25                  30
   Ser Gly Arg Asp Ala Asn Glu Ile Val Val Leu Leu Val Ser Lys Gly
           35                  40                  45
   Val Ala Ala Gln Lys Val Pro Gln Ala Ala Ser Ser Thr Gly Gly Ser
       50                  55                  60
   Gly Glu Gln Leu Trp Asp Ile Ser Val Pro Ala Ala Gln Ile Thr Glu
   65                  70                  75                  80
   Ala Leu Ala Ile Leu Asn Gln Ala Gly Leu Pro Arg Met Lys Gly Thr
                   85                  90                  95
   Ser Leu Leu Asp Leu Phe Ala Lys Gln Gly Leu Val Pro Ser Glu Met
               100                 105                 110
   Gln Glu Lys Ile Arg Tyr Gln Glu Gly Leu Ser Glu Gln Met Ala Thr
           115                 120                 125
   Thr Ile Arg Lys Met Asp Gly Ile Val Asp Ala Ser Val Gln Ile Ser
       130                 135                 140
   Phe Ser Pro Glu Glu Glu Asp Gln Arg Pro Leu Thr Ala Ser Val Tyr
   145                 150                 155                 160
   Ile Lys His Arg Gly Val Leu Asp Asn Pro Asn Ser Ile Met Val Ser
                   165                 170                 175
   Lys Ile Lys Arg Leu Val Ala Ser Ala Val Pro Gly Leu Cys Pro Glu
               180                 185                 190
   Asn Val Ser Val Val Ser Asp Arg Ala Ser Tyr Ser Asp Ile Thr Ile
           195                 200                 205
   Asn Gly Pro Trp Gly Leu Ser Asp Glu Met Asn Tyr Val Ser Val Trp
       210                 215                 220
   Gly Ile Ile Leu Ala Lys His Ser Leu Thr Lys Phe Arg Leu Val Phe
   225                 230                 235                 240
   Tyr Phe Leu Ile Leu Leu Leu Phe Ile Leu Ser Cys Gly Leu Leu Trp
                   245                 250                 255
   Val Ile Trp Lys Thr His Thr Leu Ile Ser Ala Leu Gly Gly Thr Lys
               260                 265                 270
   Gly Phe Phe Asp Pro Ala Pro Tyr Ser Gln Leu Ser Phe Thr Gln Asn
           275                 280                 285
   Lys Pro Ala Pro Lys Glu Thr Pro Gly Ala Ala Glu Gly Ala Glu Ala
       290                 295                 300
   Gln Thr Ala Ser Glu Gln Pro Ser Lys Glu Asn Ala Glu Lys Gln Glu
   305                 310                 315                 320
   Glu Asn Asn Glu Asp Ala
                   325
<210> 32
   <211> 981
   <212> DNA
   <213> Chlamydia trachomatis
<400> 32
   atgtttcgtt atactctttc tcgatcctta tttttcattt tggctctttt cttctgctcg 60
   gcttgtgata gtcgttccat gattacacac ggcttgtcag gacgtgatgc taatgaaatc 120
   gtagtgcttc tagtcagtaa aggggtcgct gcacagaaag ttccccaagc agcgtcctca 180
   acaggaggat ctggagaaca actctgggat atttcggttc ctgcagcaca aattacagag 240
   gctctagcta ttctgaacca agctgggctt ccaagaatga aaggaaccag ccttcttgat 300
   ctattcgcta aacaagggct ggtcccttct gaaatgcaag aaaaaatccg ctaccaagaa 360
   ggtctttcag aacaaatggc tacgaccatt agaaagatgg acggtatcgt cgatgcgagc 420
   gtacagattt ccttttctcc tgaagaagaa gatcaacggc cgctaacagc ctctgtatat 480
   atcaaacaca gaggggtatt agacaaccct aacagtatta tggtgtctaa gattaaacgt 540
   ttagttgcga gtgctgtccc aggactatgt cccgagaacg tttccgtagt cagtgaccga 600
   gcttcttata gtgacattac tattaatggc ccttggggac tctccgatga aatgaattat 660
   gtttctgtat gggggatcat tctagctaag cattccctta ctaaattccg ccttgttttc 720
   tatttcttaa ttctccttct cttcattctt tcctgtgggc tactctgggt catttggaaa 780
   acacacacac tgatttctgc tctgggtgga acaaaaggat tctttgatcc tgctccttac 840
   tcacagctct ctttcactca gaataagcca gctccaaaag aaactcctgg agcagcagaa 900
   ggtgcagaag cgcaaaccgc ttcggaacaa ccctctaaag aaaacgcaga aaaacaagaa 960
   gagaataacg aggacgctta a                                           981
<210> 33
   <211> 200
   <212> PRT
   <213> Chlamydia trachomatis
<400> 33
   Met Glu Lys Arg Gly Val Ile Val His Ile Leu Val Cys Leu Leu Thr
    1               5                  10                  15
   Ile Phe Gly Thr Phe Ser Leu Pro Ala Phe Gly Ala His Phe Leu Ala
               20                  25                  30
   Glu Glu Glu Gln Phe Tyr Met Asp Arg Phe Val Phe Ser Gly Gln Tyr
           35                  40                  45
   Pro Asp Met Glu Thr Met Glu Ile His Ala Glu Arg Lys Lys Arg Val
       50                  55                  60
   Gln Phe Asp Val Thr Gly Ser Phe Pro Lys Leu Glu Ser Val Val Tyr
   65                  70                  75                  80
   Lys Gly Ser Phe Gly Leu Leu Arg Ser Lys Ile Lys Gly Glu Cys Pro
                   85                  90                  95
   Glu Leu Ser Ser Val Asn Leu Ser Cys Thr Ser Cys Arg Met Asp Leu
               100                 105                 110
   Asp Phe Arg Gly Glu Trp Lys Lys Asn Ala Ser Ile Tyr Ile Arg Asn
           115                 120                 125
   Glu Gln Glu Pro Ile Thr Ile Met Leu Pro Lys Asp Ile Gly Val Val
       130                 135                 140
   Val Tyr Thr Gln Val Asp Met Asn Ser Lys Val Val Ala Glu Gly Ser
   145                 150                 155                 160
   Leu Ile Lys Arg Gly Arg Gly Phe Trp Lys Lys Thr Phe Arg Asn Ser
                   165                 170                 175
   Leu Val Gly Glu Ser Pro Val Thr Leu Thr Phe His Val Glu Thr Arg
               180                 185                 190
   Asn Gly Gly Val Ile Phe Leu Arg
           195                 200
<210> 34
   <211> 603
   <212> DNA
   <213> Chlamydia trachomatis
<400> 34
   atggagaaga gaggcgttat tgtgcatata ctagtttgtt tgttgacaat cttcggaacg 60
   ttcagtttac ccgctttcgg cgcgcatttt ctcgcggaag aagagcagtt ttatatggat 120
   cggtttgttt tctctgggca gtatccagat atggaaacta tggaaatcca tgcagaaaga 180
   aaaaaacgtg tacaatttga tgtgacggga agcttcccta agttggagag cgtggtttat 240
   aagggatctt ttggattgct gcgttcgaaa ataaagggag agtgtccaga actgtcttct 300
   gtaaatcttt cttgtacctc ctgcagaatg gatttagatt ttcgagggga gtggaaaaag 360
   aatgcgtcta tttatattcg taatgagcaa gagccaatta caattatgtt gcctaaagac 420
   attggtgtag ttgtctatac gcaggttgat atgaatagta aagtagttgc agagggatca 480
   ctaatcaaga gaggaagagg tttttggaag aaaacttttc ggaattcttt ggtaggagaa 540
   tcccctgtga cgctaacttt tcatgtagag actcgtaatg gaggagttat ttttctccgt 600
   tag                                                               603
<210> 35
   <211> 305
   <212> PRT
   <213> Chlamydia trachomatis
<400> 35
   Met Phe Asp Ser Asp His Asn Ser Ile Phe Gln Ser Asp Leu Cys Gln
   1 5 10 15
   Arg Leu Val Val His Ser Ile Leu Leu Thr Phe Leu Val Ile Leu Leu
   20 25 30
   Cys Thr Ser Leu Tyr Pro Ser Ser Ala Phe Ile Val Gly Leu Leu Ser
   35 40 45
   Ser Ala Cys Ala Ala Leu Gly Thr Tyr Glu Met Gly Ala Met Val Arg
   50 55 60
   Ile Lys Phe Pro Phe Ser Phe Thr Arg Tyr Ser Ala Leu Gly Ser Ala
   65 70 75 80
   Ile Phe Ile Ala Leu Thr Cys Leu Thr Ala Arg Cys Lys Met Cys Phe
   85 90 95
   Pro Glu His Ile Asp Leu Leu Pro Trp Phe Phe Leu Phe Phe Trp Thr
   100 105 110
   Ile Arg Leu Val Phe Lys Ser Arg His Tyr Lys Leu Gly Pro Ile Gly
   115 120 125
   Ser Thr Gly Leu Ala Leu Phe Cys Met Leu Tyr Val Ser Val Pro Ile
   130 135 140
   Arg Leu Phe Leu His Ile Leu Tyr Gly Phe Val His Thr Asp Thr Pro
   145 150 155 160
   Phe Val Gly Ile Trp Trp Ala Ile Phe Leu Ile Ala Thr Thr Lys Ser
   165 170 175
   Ser Asp Ile Phe Gly Tyr Phe Phe Gly Lys Ala Phe Gly Lys Lys Arg
   180 185 190
   Ile Ala Pro Val Ile Ser Pro Asn Lys Thr Val Val Gly Phe Ile Ala
   195 200 205
   Gly Cys Cys Gly Ser Ile Leu Val Ser Leu Leu Phe Tyr Ser His Leu
   210 215 220
   Pro Lys Ala Phe Ala Asp Gln Ile Ala Val Pro Trp Ile Leu Ile Ala
   225 230 235 240
   Leu Gly Thr Val Leu Gly Val Ser Gly Phe Phe Gly Asp Ile Ile Glu
   245 250 255
   Ser Thr Phe Lys Arg Asp Ala Gln Ile Lys Asn Ser Ser Asp Leu Glu
   260 265 270
   Ser Ile Gly Gly Met Leu Asp Val Leu Asp Ser Leu Leu Leu Ser Thr
   275 280 285
   Pro Ile Val Tyr Ala Ile Leu Leu Ile Thr Gln Asn Arg Thr Phe Leu
   290 295 300
   Gly
   305
<210> 36
   <211> 918
   <212> DNA
   <213> Chlamydia trachomatis
<400> 36
   atgttcgatt cggatcataa ttccattttt caaagcgatt tgtgtcagcg tctggtcgtc 60
   cactcgattc ttcttacttt ccttgtcatt cttctctgta catctttata tcccagctca 120
   gcctttattg tagggcttct ttcctccgct tgcgcagctc taggaacata tgagatgggg 180
   gctatggtta gaatcaagtt tccattttct ttcacacgct atagtgcatt aggatccgct 240
   attttcattg ctctgacctg tcttacagct cgttgtaaaa tgtgttttcc agagcatata 300
   gacctacttc cttggttctt tctcttcttt tggacgattc gcttagtatt taaaagtcgc 360
   cattataaac ttggtcccat aggctcaact gggctcgcgt tgttttgtat gctttatgta 420
   tcagtcccta tccgcttgtt cctccacatt ttgtatgggt ttgtgcatac cgatactcca 480
   tttgtaggaa tttggtgggc gatttttctt atcgctacaa caaaaagctc tgatattttt 540
   ggttacttct ttggaaaagc ttttgggaaa aaacgcattg caccagtcat tagcccgaac 600
   aaaacagtag taggcttcat tgctggttgc tgtggatcta tcttggttag ccttcttttc 660
   tactcccatc ttcctaaagc ctttgctgat cagattgcgg tgccttggat cctcattgct 720
   ttaggtactg tgttgggcgt tagtggattc tttggagaca ttatcgaatc tacgttcaaa 780
   cgggatgcac agatcaagaa cagcagtgat ctggagtcta tcggaggaat gctagatgtg 840
   ctagactcct tgcttctttc gactcctatc gtttacgcta tcctccttat cactcaaaat 900
   aggacatttt taggatga 918
<210> 37
   <211> 621
   <212> PRT
   <213> Chlamydia trachomatis
<400> 37
   Met Arg Arg Ser Val Cys Tyr Val Thr Pro Ser Val Ala Arg Ala Gly
   1 5 10 15
   Gln Ile Ser Thr Trp Arg Phe Glu Tyr Ser Ser Ala Asn Phe Leu Pro
   20 25 30
   Glu Gly Thr Leu Leu Lys Phe Asp Leu Gly Ile Asp Gly Arg Pro Ile
   35 40 45
   Asp Trp Glu Ile Pro Ser Thr Asp Leu Ser Gln Pro Cys Asn Thr Ile
   50 55 60
   Tyr Leu Glu Thr Pro Ser Glu Asp Ile Val Ala Ala Lys Ala Val Tyr
   65 70 75 80
   Ala Pro Gly Gly Tyr Ile Pro Thr Phe Glu Phe Thr Leu Pro Cys Asp
   85 90 95
   Val Glu Ala Gly Asp Thr Phe Ser Ile Ile Leu Gly Ser Ser Pro Asn
   100 105 110
   Phe Pro Gln Glu Asp Ser Ser Gly Asn Gly Ala Gln Leu Phe Thr Gln
   115 120 125
   Arg Arg Lys Pro Phe Ser Leu Tyr Val Asp Pro Ser Gly Lys Gly Ser
   130 135 140
   Phe Glu Asp Pro Asp Ile Phe Thr Met Asp Ile Arg Gly Asn Val Leu
   145 150 155 160
   Lys Asn Ile Arg Ile Phe Ala Pro Ser Tyr Val Ile Lys Asn Lys Arg
   165 170 175
   Phe Asp Ile Thr Val Arg Phe Glu Asp Glu Phe Gly Asn Leu Thr Asn
   180 185 190
   Phe Ser Pro Glu Glu Thr His Ile Glu Leu Ser Tyr Glu His Leu Arg
   195 200 205
   Glu Asn Leu Asn Trp Gln Leu Phe Ile Pro Glu Thr Gly Phe Val Ile
   210 215 220
   Leu Pro Asn Leu Tyr Phe Asn Glu Pro Gly Ile Tyr Arg Ile Gln Leu
   225 230 235 240
   Arg Asn Gln Ala Thr Lys Glu Val Phe Thr Ser Ala Pro Ile Lys Cys
   245 250 255
   Phe Ala Glu Thr Ser Ser His Leu Leu Trp Gly Leu Leu His Gly Glu
   260 265 270
   Ser Glu Arg Val Asp Ser Glu Gly Asn Ile Glu Ser Cys Leu Arg Tyr
   275 280 285
   Phe Arg Asp Asp Cys Ala Leu Asn Phe Phe Ala Thr Ser Ser Phe Glu
   290 295 300
   Ile Gln Asp Gly Leu Thr Pro Glu Thr Ile Lys Thr Ile Asn Gln Thr
   305 310 315 320
   Val Ala Asp Phe Asn Glu Glu Asp Arg Phe Ile Ala Leu Ser Gly Ala
   325 330 335
   Gln Tyr Leu Ser Glu Glu Pro Gly Glu Gly Ile Arg Glu Val Leu Leu
   340 345 350
   Met Lys Glu Pro Lys Ser Pro Gly Lys His Lys Glu Cys Lys Leu Phe
   355 360 365
   Pro Leu Ser Lys Leu Tyr Lys Gln Ser Thr Ser His Glu Leu Ile Ser
   370 375 380
   Ile Pro Ser Phe Thr Ala Ser Lys Lys Phe Gly Tyr Asn Phe Asn Asn
   385 390 395 400
   Phe His Pro Glu Phe Glu Arg Val Val Glu Ile Tyr Asn Ala Trp Gly
                   405                 410                 415
   Cys Ser Glu Arg Thr Glu Ala Glu Gly Asn Pro Phe Pro Ile Lys Gly
               420                 425                 430
   Ser Ile Asp Ser Glu Asn Pro Glu Gly Thr Val Leu Ser Ala Leu Lys
           435                 440                 445
   Arg Asn Leu Arg Phe Gly Phe Val Ala Gly Gly Leu Asp Asp Arg Asn
       450                 455                 460
   Leu Tyr Asn His Phe Phe Asp Ser Asp Gln Gln Gln Tyr Ser Pro Gly
   465                 470                 475                 480
   Leu Thr Ala Val Ile Cys Asn Lys Tyr Ser Arg Asp Ser Leu Leu Glu
                   485                 490                 495
   Ala Leu Tyr Gln Arg Gln Cys Tyr Ala Thr Thr Gly Gln Arg Ile Ile
               500                 505                 510
   Val Asn Phe Gln Ile Thr Ser Ala Pro Met Gly Ser Glu Leu Ser Thr
           515                 520                 525
   Ala Ile Lys Pro Gly Leu Val Ile Asn Arg His Ile Ser Gly Tyr Val
       530                 535                 540
   Ala Gly Thr Ala Lys Ile Ala Ser Ile Glu Ile Ile Arg Asn Glu Asp
   545                 550                 555                 560
   Ile Leu His Thr Phe His Pro Asp Gly Asn Asn Phe Glu Tyr Glu Tyr
                   565                 570                 575
   Asp Asp Leu Ser Pro Phe Ala Gln Val Thr Leu Lys Asp Pro Gln Asn
               580                 585                 590
   Gly Ala Pro Phe Ala Phe Tyr Tyr Leu Arg Val Thr Gln Glu Asn Gly
           595                 600                 605
   Ala Met Ala Trp Ser Ser Pro Ile Trp Ile Asp Leu Asn
       610                 615                 620
<210> 38
   <211> 1866
   <212> DNA
   <213> Chlamydia trachomatis
<400> 38
   atgcgcagat ctgtttgtta cgttactcct tcagttgcta gggctggtca aatttctacc 60
   tggcgtttcg aatattcttc agctaatttc cttcccgaag gcacattgct aaaatttgac 120
   ctgggaatag acggacgccc tatagactgg gagattcctt ctacagatct ttctcaacca 180
   tgtaatacaa tttatttaga aacgccttcc gaggatattg tggctgcaaa agctgtgtat 240
   gctcccggag gctatatccc tactttcgaa tttactctcc cttgtgatgt ggaagctggg 300
   gacactttct ctattattct tggctcctct cccaacttcc ctcaagagga ctcttcaggt 360
   aatggtgctc aattatttac tcaacgccgt aaacctttct ctctttatgt tgacccatca 420
   gggaaaggat cttttgaaga tcccgatatc ttcacaatgg atatcagagg aaatgtatta 480
   aaaaatatcc ggatttttgc tccttcttat gtgatcaaaa acaaacgctt tgatattaca 540
   gtgcgcttcg aagatgaatt tgggaactta accaatttct ccccagaaga gacccatatc 600
   gagctttcgt acgaacatct tcgcgaaaac ctcaattggc aattgttcat ccctgaaaca 660
   ggctttgtga tccttccaaa cctgtatttc aatgaaccag gtatttatcg tattcaacta 720
   cgcaatcaag caacaaaaga ggtctttaca tcagcgccta tcaaatgttt tgcagaaacc 780
   tcctctcatc ttttgtgggg gcttctacat ggagaatctg aacgtgtcga ctctgaaggt 840
   aatatcgagt cttgcttgcg ttattttcgt gatgactgcg cgttaaactt ttttgcaaca 900
   tcctctttcg aaattcaaga tggcctgacc ccagaaacca ttaaaaccat taaccaaacc 960
   gttgctgatt ttaatgaaga agatcgtttc attgccttat ccggagcaca gtacctttct 1020
   gaagagcctg gcgagggaat tcgtgaagta ttgctgatga aggaacccaa atccccaggg 1080
   aaacataaag aatgcaaact atttccttta tctaagctat ataagcaatc aactagtcat 1140
   gagttaatct caatccccag cttcactgct tcaaagaaat ttggatacaa ttttaataat 1200
   ttccatcctg aatttgaaag agttgttgaa atttataatg cctggggatg ctctgaaaga 1260
   actgaagctg aaggaaaccc tttccctatt aaaggttcta tcgactcaga aaatccagag 1320
   ggaactgttc tatctgcttt aaagagaaac ctgcgttttg gattcgtagc cggtggtctt 1380
   gatgatagaa atctatacaa tcactttttt gattccgatc aacagcaata ctcccctgga 1440
   ttaacagctg tgatctgcaa taaatattct cgggattcct tactcgaggc attataccaa 1500
   cgacaatgct atgctacaac cggccaaaga attatcgtga atttccagat tacatctgct 1560
   cctatgggct ccgaactctc cacagccatt aaaccagggc tcgtgatcaa tagacatatt 1620
   tcgggatatg tagcaggaac tgccaagatt gcgtcgatcg aaatcatccg caatgaggat 1680
   attctccata ccttccaccc agatggaaat aactttgagt atgagtacga cgatctctct 1740
   ccttttgcac aagtcactct aaaagatcct caaaatggag ctccttttgc tttttactac 1800
   ttacgagtca ctcaagagaa tggagctatg gcttggagct ctcctatttg gatagatctt 1860
   aactaa                                                            1866
<210> 39
   <211> 697
   <212> PRT
   <213> Chlamydia trachomatis
<400> 39
   Met Lys Arg Phe Phe Pro Leu Phe Ile Gly Val Leu Leu Ala His Thr
   1 5 10 15
   Leu Pro Ser Glu Gly Leu Ser His Gln Gln Ala Val Gln Lys Lys Ile
   20 25 30
   Ser Tyr Leu Ser His Phe Lys Gly Ile Thr Gly Ile Met Asp Val Glu
   35 40 45
   Asp Gly Val Leu His Ile His Asp Asp Leu Arg Leu Gln Ala Asn Lys
   50 55 60
   Ala Tyr Val Glu Asn Arg Thr Asp Cys Gly Ile Lys Ile Val Ala His
   65 70 75 80
   Gly Asn Val Met Val Asn Tyr Arg Gly Lys Ile Leu Ile Cys Asp Tyr
   85 90 95
   Leu Glu Tyr Tyr Glu Asp Thr Asp Ser Cys Leu Leu Thr Asn Gly Arg
   100 105 110
   Cys Ser Leu Tyr Pro Trp Phe Ile Gly Gly Ser Thr Ile Thr Ile Ser
   115 120 125
   Pro Ser Ser Ile Ile Ile His Lys Gly Tyr Ile Ser Thr Ser Glu Gly
   130 135 140
   Pro Gln Lys His Ile Cys Leu Ser Gly Asp Tyr Leu Lys Tyr Ser Ser
   145 150 155 160
   Asp Ser Val Leu Ser Met Gly Pro Ser Arg Leu Ser Ile Cys Asn Thr
   165 170 175
   Pro Val Leu Leu Leu Pro Gln Ile Ser Ile Met Pro Met Glu Ile Pro
   180 185 190
   Lys Pro Pro Ile Thr Phe Arg Gly Gly Ser Gly Gly Phe Leu Gly Ser
   195 200 205
   Tyr Leu Gly Val Ser Tyr Ser Pro Ile Ser Lys Lys His Cys Ser Thr
   210 215 220
   Thr Leu Phe Leu Asp Gly Phe Phe Lys His Gly Ile Gly Leu Gly Tyr
   225 230 235 240
   Asn Met Arg Phe Ser Ser Gln Glu Asn Pro Ser Asn Ala Ile Asn Ile
   245 250 255
   Lys Ser Tyr Tyr Ala His Arg Leu Ala Ile Asp Ser Ser Gly Ala Lys
   260 265 270
   Asp Arg Tyr Arg Leu His Gly Asp Phe Thr Phe Ser Lys Glu Arg Ala
   275 280 285
   His Leu Ala Gly Glu Phe His Leu Ser Asp Ser Trp Glu Thr Val Val
   290 295 300
   Asp Ile Phe Pro Asn Asn Phe Ser Leu Lys Asn Thr Gly Pro Thr Glu
   305 310 315 320
   Val Ser Leu Ser Trp Arg Asp Asn Asn Leu Phe Gly Lys Met Thr Ser
   325 330 335
   Ser Val Lys Val Asn Ser Phe Gln Asn Val Lys Gln Glu Leu Pro Gln
   340 345 350
   Ala Ile Leu His His Arg Pro Val Arg Ile Arg Arg Ser Arg Ile Phe
   355 360 365
   Leu Glu Asn Arg Leu Glu Ala Gly Phe Leu Asp Phe His Phe Ser Ser
   370 375 380
   Asn Ile Pro Gly Ser Asn Phe Ser Ser Trp Arg Phe Ser Ser Ala His
   385 390 395 400
   Lys Val Tyr Arg Gly Leu Val Leu Pro Ile Gly Thr Leu Thr Pro Ser
   405 410 415
   Leu Ser Gly Thr Ala Ile Tyr Tyr Thr Arg Met Leu Ser Pro Asn Ala
   420 425 430
   Ala His Cys Gln Leu Ser Gly Ser Leu Ser Phe Asp Tyr Arg Val Ala
   435 440 445
   Leu Gln Lys Glu Tyr Arg His Ala Arg His Ile Val Glu Pro Phe Cys
           450                 455                 460
   Ser Phe Leu Lys Thr Thr Arg Pro Val Leu Ser Ser Asp Glu Pro His
   465                 470                 475                 480
   Ile Phe Ser Ile Lys Asp Ala Phe His Ser Ile Asn Leu Leu His Val
                   485                 490                 495
   Gly Leu Glu Ser Lys Val Leu Asn Lys His Ser Thr Pro Ser His Leu
               500                 505                 510
   Lys Leu Trp Thr Thr Tyr Ile Phe Asp Glu Pro His Ala Lys Asp Thr
           515                 520                 525
   Phe Pro Lys Thr Ala Cys Trp Phe Ser Leu Pro Leu Thr Leu Gln Asn
       530                 535                 540
   Thr Leu Ser Leu Asp Ala Glu Trp Ile Trp Lys Lys Ser Arg Trp Asp
   545                 550                 555                 560
   His Leu Asn Val Ile Trp Glu Trp Ile Leu Asn Asp Asn Leu Gly Leu
                   565                 570                 575
   Thr Leu Glu Phe Leu His Arg Ser Lys Tyr Gly Phe Ile Lys Cys Ala
               580                 585                 590
   Lys Asp Asn Tyr Thr Leu Asp Val Ser Arg Ser Leu Asp Thr Leu Leu
           595                 600                 605
   Ala Ser Pro Leu Ser Asp Arg Arg Asn Leu Ile Thr Gly Lys Leu Phe
       610                 615                 620
   Val Arg Pro His Pro His Trp Asn Tyr Asn Leu Asn Leu Arg Tyr Gly
   625                 630                 635                 640
   Trp His Arg Pro Asp Ser Pro Ser Tyr Leu Glu Tyr Gln Met Ile Leu
                   645                 650                 655
   Gly His Lys Ile Phe Glu His Trp Gln Leu Phe Ser Val Tyr Glu Lys
               660                 665                 670
   Arg Glu Ala Asp Lys Arg Cys Phe Phe Tyr Leu Lys Leu Asp Lys Arg
           675                 680                 685
   Lys Gln Lys His Arg His Pro Phe Gly
       690                 695
<210> 40
   <211> 2094
   <212> DNA
   <213> Chlamydia trachomatis
<400> 40
   gtgaaacgat ttttcccact ttttattgga gtgctgctcg cgcacacttt gccgtcagaa 60
   ggtctttctc atcaacaagc tgtccaaaaa aaaatttctt atctgagcca ttttaaaggc 120
   attacaggaa ttatggatgt tgaggatggg gtattacata tccatgatga tctacgtctt 180
   caagccaata aagcctatgt tgaaaatcgc acggattgtg ggatcaaaat cgttgctcat 240
   ggcaacgtta tggtcaatta tcgcgggaaa attttaatct gtgattatct tgagtactat 300
   gaagatacag attcttgttt actcaccaat ggccgctgtt cgttataccc atggttcatt 360
   ggaggatcca ctataacgat ctcaccatct tctattatca ttcataaagg gtatatctcg 420
   acttctgaag gtcctcagaa acatatttgt ttatccggag attatttaaa atactcttca 480
   gacagcgtat tatctatggg accctcacgt ctttctatct gtaatacgcc tgtgttattg 540
   cttcctcaaa tctccattat gcctatggag attcctaagc ctccgattac ttttcgaggt 600
   gggagtggag gatttctggg atcctactta ggtgttagtt attcccctat atctaaaaag 660
   cattgttcta cgactttgtt cttggatggt ttttttaaac atggaatagg tctcggctat 720
   aacatgcgct tttcctctca ggaaaatcca agcaatgcca taaatattaa aagctattac 780
   gcacatcgat tagctattga ttcatcagga gcaaaagatc gctatcgatt acatggagac 840
   ttcacttttt ccaaagaacg agcccatctt gctggtgaat tccatttaag tgatagctgg 900
   gaaacagttg tggatatctt cccaaataac ttctctttaa aaaatacagg ccctacagaa 960
   gttagcctat catggcgcga taacaattta tttgggaaaa tgacttcctc tgtcaaagtc 1020
   aactcctttc aaaatgttaa acaagaattg cctcaagcaa ttcttcatca ccgaccagta 1080
   cgtatcaggc gctctcgcat tttcctagag aatcgcttag aagctggttt tttagatttt 1140
   catttcagta gtaatattcc aggctctaac ttctcatcat ggaggttctc atccgctcac 1200
   aaagtctacc gtgggcttgt tcttcctata ggaacgttaa ccccttcgct atctggaact 1260
   gctatctact atacccgcat gctctcccca aatgcagccc attgtcaatt atctggatcg 1320
   ctatcttttg attatcgcgt tgctttacaa aaagaatatc ggcatgcaag acatattgta 1380
   gagccttttt gctccttttt aaaaaccact cgtcctgtat tatcctctga tgagcctcat 1440
   attttctcga ttaaagatgc ttttcactct atcaaccttc tacatgtagg attggagtca 1500
   aaagtcttaa acaaacattc cactccttcg catctgaaac tatggacgac ctatatcttt 1560
   gatgaacctc acgctaagga cactttccct aaaactgctt gctggttctc tcttcctctt 1620
   acactccaaa atactttatc cttagatgcg gaatggattt ggaaaaaaag ccgatgggat 1680
   catctcaatg taatctggga atggattttg aatgataatc tcggtcttac tttagaattt 1740
   ttacatagaa gtaagtatgg ctttattaag tgcgctaaag ataactacac actcgatgta 1800
   agccgatctt tagacacatt actagcctct cctctttccg atcgaagaaa tttgattact 1860
   ggcaaacttt ttgttcgtcc acatcctcat tggaattata atcttaatct tcgttatgga 1920
   tggcatcgtc cagactctcc atcctattta gaataccaga tgattctggg tcataaaatc 1980
   tttgagcact ggcagctatt ctctgtctac gaaaaacgtg aagctgataa gcgctgcttc 2040
   ttttatctaa aattagataa acgaaaacag aaacaccgcc atccttttgg ataa       2094
<210> 41
   <211> 1016
   <212> PRT
   <213> Chlamydia trachomatis
<400> 41
   Met Pro Phe Ser Leu Arg Ser Thr Ser Phe Cys Phe Leu Ala Cys Leu
   1 5 10 15
   Cys Ser Tyr Ser Tyr Gly Phe Ala Ser Ser Pro Gln Val Leu Thr Pro
   20 25 30
   Asn Val Thr Thr Pro Phe Lys Gly Asp Asp Val Tyr Leu Asn Gly Asp
   35 40 45
   Cys Ala Phe Val Asn Val Tyr Ala Gly Ala Glu Asn Gly Ser Ile Ile
   50 55 60
   Ser Ala Asn Gly Asp Asn Leu Thr Ile Thr Gly Gln Asn His Thr Leu
   65 70 75 80
   Ser Phe Thr Asp Ser Gln Gly Pro Val Leu Gln Asn Tyr Ala Phe Ile
   85 90 95
   Ser Ala Gly Glu Thr Leu Thr Leu Lys Asp Phe Ser Ser Leu Met Phe
   100 105 110
   Ser Lys Asn Val Ser Cys Gly Glu Lys Gly Met Ile Ser Gly Lys Thr
   115 120 125
   Val Ser Ile Ser Gly Ala Gly Glu Val Ile Phe Trp Asp Asn Ser Val
   130 135 140
   Gly Tyr Ser Pro Leu Ser Ile Val Pro Ala Ser Thr Pro Thr Pro Pro
   145 150 155 160
   Ala Pro Ala Pro Ala Pro Ala Ala Ser Ser Ser Leu Ser Pro Thr Val
   165 170 175
   Ser Asp Ala Arg Lys Gly Ser Ile Phe Ser Val Glu Thr Ser Leu Glu
   180 185 190
   Ile Ser Gly Val Lys Lys Gly Val Met Phe Asp Asn Asn Ala Gly Asn
   195 200 205
   Phe Gly Thr Val Phe Arg Gly Asn Ser Asn Asn Asn Ala Gly Ser Gly
   210 215 220
   Gly Ser Gly Ser Ala Thr Thr Pro Ser Phe Thr Val Lys Asn Cys Lys
   225 230 235 240
   Gly Lys Val Ser Phe Thr Asp Asn Val Ala Ser Cys Gly Gly Gly Val
   245 250 255
   Val Tyr Lys Gly Thr Val Leu Phe Lys Asp Asn Glu Gly Gly Ile Phe
   260 265 270
   Phe Arg Gly Asn Thr Ala Tyr Asp Asp Leu Gly Ile Leu Ala Ala Thr
   275 280 285
   Ser Arg Asp Gln Asn Thr Glu Thr Gly Gly Gly Gly Gly Val Ile Cys
   290 295 300
   Ser Pro Asp Asp Ser Val Lys Phe Glu Gly Asn Lys Gly Ser Ile Val
   305 310 315 320
   Phe Asp Tyr Asn Phe Ala Lys Gly Arg Gly Gly Ser Ile Leu Thr Lys
   325 330 335
   Glu Phe Ser Leu Val Ala Asp Asp Ser Val Val Phe Ser Asn Asn Thr
   340 345 350
   Ala Glu Lys Gly Gly Gly Ala Ile Tyr Ala Pro Thr Ile Asp Ile Ser
   355 360 365
   Thr Asn Gly Gly Ser Ile Leu Phe Glu Arg Asn Arg Ala Ala Glu Gly
   370 375 380
   Gly Ala Ile Cys Val Ser Glu Ala Ser Ser Gly Ser Thr Gly Asn Leu
   385 390 395 400
   Thr Leu Ser Ala Ser Asp Gly Asp Ile Val Phe Ser Gly Asn Met Thr
   405 410 415
   Ser Asp Arg Pro Gly Glu Arg Ser Ala Ala Arg Ile Leu Ser Asp Gly
   420 425 430
   Thr Thr Val Ser Leu Asn Ala Ser Gly Leu Ser Lys Leu Ile Phe Tyr
   435 440 445
   Asp Pro Val Val Gln Asn Asn Ser Ala Ala Gly Ala Ser Thr Pro Ser
   450 455 460
   Pro Ser Ser Ser Ser Met Pro Gly Ala Val Thr Ile Asn Gln Ser Gly
   465 470 475 480
   Asn Gly Ser Val Ile Phe Thr Ala Glu Ser Leu Thr Pro Ser Glu Lys
   485 490 495
   Leu Gln Val Leu Asn Ser Thr Ser Asn Phe Pro Gly Ala Leu Thr Val
   500 505 510
   Ser Gly Gly Glu Leu Val Val Thr Glu Gly Ala Thr Leu Thr Thr Gly
   515 520 525
   Thr Ile Thr Ala Thr Ser Gly Arg Val Thr Leu Gly Ser Gly Ala Ser
   530 535 540
   Leu Ser Ala Val Ala Gly Ala Ala Asn Asn Asn Tyr Thr Cys Thr Val
   545 550 555 560
   Ser Lys Leu Gly Ile Asp Leu Glu Ser Phe Leu Thr Pro Asn Tyr Lys
   565 570 575
   Thr Ala Ile Leu Gly Ala Asp Gly Thr Val Thr Val Asn Ser Gly Ser
   580 585 590
   Thr Leu Asp Leu Val Met Glu Ser Glu Ala Glu Val Tyr Asp Asn Pro
   595 600 605
   Leu Phe Val Gly Ser Leu Thr Ile Pro Phe Val Thr Leu Ser Ser Ser
   610 615 620
   Ser Ala Ser Asn Gly Val Thr Lys Asn Ser Val Thr Ile Asn Asp Ala
   625 630 635 640
   Asp Ala Ala His Tyr Gly Tyr Gln Gly Ser Trp Ser Ala Asp Trp Thr
   645 650 655
   Lys Pro Pro Leu Ala Pro Asp Ala Lys Gly Met Val Pro Pro Asn Thr
   660 665 670
   Asn Asn Thr Leu Tyr Leu Thr Trp Arg Pro Ala Ser Asn Tyr Gly Glu
   675 680 685
   Tyr Arg Leu Asp Pro Gln Arg Lys Gly Glu Leu Val Pro Asn Ser Leu
   690 695 700
   Trp Val Ala Gly Ser Ala Leu Arg Thr Phe Thr Asn Gly Leu Lys Glu
   705 710 715 720
   His Tyr Val Ser Arg Asp Val Gly Phe Val Ala Ser Leu His Ala Leu
   725 730 735
   Gly Asp Tyr Ile Leu Asn Tyr Thr Gln Asp Asp Arg Asp Gly Phe Leu
   740 745 750
   Ala Arg Tyr Gly Gly Phe Gln Ala Thr Ala Ala Ser His Tyr Glu Asn
   755 760 765
   Gly Ser Ile Phe Gly Val Ala Phe Gly Gln Leu Tyr Gly Gln Thr Lys
   770 775 780
   Ser Arg Met Tyr Tyr Ser Lys Asp Ala Gly Asn Met Thr Met Leu Ser
   785 790 795 800
   Cys Phe Gly Arg Ser Tyr Val Asp Ile Lys Gly Thr Glu Thr Val Met
   805 810 815
   Tyr Trp Glu Thr Ala Tyr Gly Tyr Ser Val His Arg Met His Thr Gln
   820 825 830
   Tyr Phe Asn Asp Lys Thr Gln Lys Phe Asp His Ser Lys Cys His Trp
   835 840 845
   His Asn Asn Asn Tyr Tyr Ala Phe Val Gly Ala Glu His Asn Phe Leu
   850 855 860
   Glu Tyr Cys Ile Pro Thr Arg Gln Phe Ala Arg Asp Tyr Glu Leu Thr
   865 870 875 880
   Gly Phe Met Arg Phe Glu Met Ala Gly Gly Trp Ser Ser Ser Thr Arg
   885 890 895
   Glu Thr Gly Ser Leu Thr Arg Tyr Phe Ala Arg Gly Ser Gly His Asn
   900 905 910
   Met Ser Leu Pro Ile Gly Ile Val Ala His Ala Val Ser His Val Arg
   915 920 925
   Arg Ser Pro Pro Ser Lys Leu Thr Leu Asn Met Gly Tyr Arg Pro Asp
   930 935 940
   Ile Trp Arg Val Thr Pro His Cys Asn Met Glu Ile Ile Ala Asn Gly
   945 950 955 960
   Val Lys Thr Pro Ile Gln Gly Ser Pro Leu Ala Arg His Ala Phe Phe
   965 970 975
   Leu Glu Val His Asp Thr Leu Tyr Ile His His Phe Gly Arg Ala Tyr
   980 985 990
   Met Asn Tyr Ser Leu Asp Ala Arg Arg Arg Gln Thr Ala His Phe Val
   995 1000 1005
   Ser Met Gly Leu Asn Arg Ile Phe
   1010 1015
<210> 42
   <211> 3051
   <212> DNA
   <213> Chlamydia trachomatis
<400> 42
   atgccttttt ctttgagatc tacatcattt tgttttttag cttgtttgtg ttcctattcg 60
   tatggattcg cgagctctcc tcaagtgtta acacctaatg taaccactcc ttttaagggg 120
   gacgatgttt acttgaatgg agactgcgct tttgtcaatg tctatgcagg ggcagagaac 180
   ggctcaatta tctcagctaa tggcgacaat ttaacgatta ccggacaaaa ccatacatta 240
   tcatttacag attctcaagg gccagttctt caaaattatg ccttcatttc agcaggagag 300
   acacttactc tgaaagattt ttcgagtttg atgttctcga aaaatgtttc ttgcggagaa 360
   aagggaatga tctcagggaa aaccgtgagt atttccggag caggcgaagt gattttttgg 420
   gataactctg tggggtattc tcctttgtct attgtgccag catcgactcc aactcctcca 480
   gcaccagcac cagctcctgc tgcttcaagc tctttatctc caacagttag tgatgctcgg 540
   aaagggtcta ttttttctgt agagactagt ttggagatct caggcgtcaa aaaaggggtc 600
   atgttcgata ataatgccgg gaattttgga acagtttttc gaggtaatag taataataat 660
   gctggtagtg ggggtagtgg gtctgctaca acaccaagtt ttacagttaa aaactgtaaa 720
   gggaaagttt ctttcacaga taacgtagcc tcctgtggag gcggagtagt ctacaaagga 780
   actgtgcttt tcaaagacaa tgaaggaggc atattcttcc gagggaacac agcatacgat 840
   gatttaggga ttcttgctgc tactagtcgg gatcagaata cggagacagg aggcggtgga 900
   ggagttattt gctctccaga tgattctgta aagtttgaag gcaataaagg ttctattgtt 960
   tttgattaca actttgcaaa aggcagaggc ggaagcatcc taacgaaaga attctctctt 1020
   gtagcagatg attcggttgt ctttagtaac aatacagcag aaaaaggcgg tggagctatt 1080
   tatgctccta ctatcgatat aagcacgaat ggaggatcga ttctatttga aagaaaccga 1140
   gctgcagaag gaggcgccat ctgcgtgagt gaagcaagct ctggttcaac tggaaatctt 1200
   actttaagcg cttctgatgg ggatattgtt ttttctggga atatgacgag tgatcgtcct 1260
   ggagagcgca gcgcagcaag aatcttaagt gatggaacga ctgtttcttt aaatgcttcc 1320
   ggactatcga agctgatctt ttatgatcct gtagtacaaa ataattcagc agcgggtgca 1380
   tcgacaccat caccatcttc ttcttctatg cctggtgctg tcacgattaa tcagtccggt 1440
   aatggatctg tgatttttac cgccgagtca ttgactcctt cagaaaaact tcaagttctt 1500
   aactctactt ctaacttccc aggagctctg actgtgtcag gaggggagtt ggttgtgacg 1560
   gaaggagcta ccttaactac tgggaccatt acagccacct ctggacgagt gactttagga 1620
   tccggagctt cgttgtctgc cgttgcaggt gctgcaaata ataattatac ttgtacagta 1680
   tctaagttgg ggattgattt agaatccttt ttaactccta actataagac ggccatactg 1740
   ggtgcggatg gaacagttac tgttaacagc ggctctactt tagacctagt gatggagagt 1800
   gaggcagagg tatatgataa tccgcttttt gtgggatcgc tgacaattcc ttttgttact 1860
   ctatcttcta gtagtgctag taacggagtt acaaaaaatt ctgtcactat taatgatgca 1920
   gacgctgcgc actatgggta tcaaggctct tggtctgcag attggacgaa accgcctctg 1980
   gctcctgatg ctaaggggat ggtacctcct aataccaata acactctgta tctgacatgg 2040
   agacctgctt cgaattacgg tgaatatcga ctggatcctc agagaaaggg agaactagta 2100
   cccaactctc tttgggtagc gggatctgca ttaagaacct ttactaatgg tttgaaagaa 2160
   cactatgttt ctagagatgt tggatttgta gcatctctgc atgctctcgg ggattatatt 2220
   ttgaattata cgcaagatga tcgggatggc tttttagcta gatatggggg attccaggcg 2280
   accgcagcct cccattatga aaatgggtca atatttggag tggcttttgg acaactctat 2340
   ggtcagacaa agagcagaat gtattactct aaagatgctg ggaacatgac gatgttgtcc 2400
   tgtttcggaa gaagttacgt agatattaaa ggaacagaaa ctgttatgta ttgggagacg 2460
   gcttatggct attctgtgca cagaatgcat acgcagtatt ttaatgacaa aacgcagaag 2520
   ttcgatcatt cgaaatgtca ttggcacaac aataactatt atgcgtttgt gggtgccgag 2580
   cataatttct tagagtactg cattcctact cgtcagttcg ctagagatta tgagcttaca 2640
   gggtttatgc gttttgaaat ggccggagga tggtccagtt ctacacgaga aactggctcc 2700
   ctaactagat atttcgctcg cgggtcaggg cataatatgt cgcttccaat aggaattgta 2760
   gctcatgcag tttctcatgt gcgaagatct cctccttcta aactgacact aaatatggga 2820
   tatagaccag acatttggcg tgtcactcca cattgcaata tggaaattat tgctaacgga 2880
   gtgaagacac ctatacaagg atctccgctg gcacggcatg ccttcttctt agaagtgcat 2940
   gatactttgt atattcatca ttttggaaga gcctatatga actattcgct ggatgctcgt 3000
   cgtcgacaaa cggcacattt tgtatccatg ggcttgaata gaatctttta a          3051
<210> 43
   <211> 553
   <212> PRT
   <213> Chlamydia trachomatis
<400> 43
   Met Arg Ile Gly Asp Pro Met Asn Lys Leu Ile Arg Arg Ala Val Thr
   1               5                   10                  15
   Ile Phe Ala Val Thr Ser Val Ala Ser Leu Phe Ala Ser Gly Val Leu
               20                  25                  30
   Glu Thr Ser Met Ala Glu Ser Leu Ser Thr Asn Val Ile Ser Leu Ala
           35                  40                  45
   Asp Thr Lys Ala Lys Asp Asn Thr Ser His Lys Ser Lys Lys Ala Arg
       50                  55                  60
   Lys Asn His Ser Lys Glu Thr Pro Val Asp Arg Lys Glu Val Ala Pro
   65                  70                  75                  80
   Val His Glu Ser Lys Ala Thr Gly Pro Lys Gln Asp Ser Cys Phe Gly
                   85                  90                  95
   Arg Met Tyr Thr Val Lys Val Asn Asp Asp Arg Asn Val Glu Ile Thr
               100                 105                 110
   Gln Ala Val Pro Glu Tyr Ala Thr Val Gly Ser Pro Tyr Pro Ile Glu
           115                 120                 125
   Ile Thr Ala Thr Gly Lys Arg Asp Cys Val Asp Val Ile Ile Thr Gln
       130                 135                 140
   Gln Leu Pro Cys Glu Ala Glu Phe Val Arg Ser Asp Pro Ala Thr Thr
   145                 150                 155                 160
   Pro Thr Ala Asp Gly Lys Leu Val Trp Lys Ile Asp Arg Leu Gly Gln
                   165                 170                 175
   Gly Glu Lys Ser Lys Ile Thr Val Trp Val Lys Pro Leu Lys Glu Gly
               180                 185                 190
   Cys Cys Phe Thr Ala Ala Thr Val Cys Ala Cys Pro Glu Ile Arg Ser
           195                 200                 205
   Val Thr Lys Cys Gly Gln Pro Ala Ile Cys Val Lys Gln Glu Gly Pro
       210                 215                 220
   Glu Asn Ala Cys Leu Arg Cys Pro Val Val Tyr Lys Ile Asn Ile Val
   225                 230                 235                 240
   Asn Gln Gly Thr Ala Thr Ala Arg Asn Val Val Val Glu Asn Pro Val
                   245                 250                 255
   Pro Asp Gly Tyr Ala His Ser Ser Gly Gln Arg Val Leu Thr Phe Thr
               260                 265                 270
   Leu Gly Asp Met Gln Pro Gly Glu His Arg Thr Ile Thr Val Glu Phe
           275                 280                 285
   Cys Pro Leu Lys Arg Gly Arg Ala Thr Asn Ile Ala Thr Val Ser Tyr
       290                 295                 300
   Cys Gly Gly His Lys Asn Thr Ala Ser Val Thr Thr Val Ile Asn Glu
   305                 310                 315                 320
   Pro Cys Val Gln Val Ser Ile Ala Gly Ala Asp Trp Ser Tyr Val Cys
                   325                 330                 335
   Lys Pro Val Glu Tyr Val Ile Ser Val Ser Asn Pro Gly Asp Leu Val
               340                 345                 350
   Leu Arg Asp Val Val Val Glu Asp Thr Leu Ser Pro Gly Val Thr Val
           355                 360                 365
   Leu Glu Ala Ala Gly Ala Gln Ile Ser Cys Asn Lys Val Val Trp Thr
       370                 375                 380
   Val Lys Glu Leu Asn Pro Gly Glu Ser Leu Gln Tyr Lys Val Leu Val
   385                 390                 395                 400
   Arg Ala Gln Thr Pro Gly Gln Phe Thr Asn Asn Val Val Val Lys Ser
                   405                 410                 415
   Cys Ser Asp Cys Gly Thr Cys Thr Ser Cys Ala Glu Ala Thr Thr Tyr
               420                 425                 430
   Trp Lys Gly Val Ala Ala Thr His Met Cys Val Val Asp Thr Cys Asp
                   435                 440                 445
   Pro Val Cys Val Gly Glu Asn Thr Val Tyr Arg Ile Cys Val Thr Asn
       450                 455                 460
   Arg Gly Ser Ala Glu Asp Thr Asn Val Ser Leu Met Leu Lys Phe Ser
   465                 470                 475                 480
   Lys Glu Leu Gln Pro Val Ser Phe Ser Gly Pro Thr Lys Gly Thr Ile
                   485                 490                 495
   Thr Gly Asn Thr Val Val Phe Asp Ser Leu Pro Arg Leu Gly Ser Lys
               500                 505                 510
   Glu Thr Val Glu Phe Ser Val Thr Leu Lys Ala Val Ser Ala Gly Asp
           515                 520                 525
   Ala Arg Gly Glu Ala Ile Leu Ser Ser Asp Thr Leu Thr Val Pro Val
       530                 535                 540
   Ser Asp Thr Glu Asn Thr His Ile Tyr
   545                 550
<210> 44
   <211> 1662
   <212> DNA
   <213> Chlamydia trachomatis
<400> 44
   atgcgaatag gagatcctat gaacaaactc atcagacgag cagtgacgat cttcgcggtg 60
   actagtgtgg cgagtttatt tgctagcggg gtgttagaga cctctatggc agagtctctc 120
   tctacaaacg ttattagctt agctgacacc aaagcgaaag acaacacttc tcataaaagc 180
   aaaaaagcaa gaaaaaacca cagcaaagag actcccgtag accgtaaaga ggttgctccg 240
   gttcatgagt ctaaagctac aggacctaaa caggattctt gctttggcag aatgtataca 300
   gtcaaagtta atgatgatcg caatgttgaa atcacacaag ctgttcctga atatgctacg 360
   gtaggatctc cctatcctat tgaaattact gctacaggta aaagggattg tgttgatgtt 420
   atcattactc agcaattacc atgtgaagca gagttcgtac gcagtgatcc agcgacaact 480
   cctactgctg atggtaagct agtttggaaa attgaccgct taggacaagg cgaaaagagt 540
   aaaattactg tatgggtaaa acctcttaaa gaaggttgct gctttacagc tgcaacagta 600
   tgcgcttgtc cagagatccg ttcggttaca aaatgtggac aacctgctat ctgtgttaaa 660
   caagaaggcc cagagaatgc ttgtttgcgt tgcccagtag tttacaaaat taatatagtg 720
   aaccaaggaa cagcaacagc tcgtaacgtt gttgttgaaa atcctgttcc agatggttac 780
   gctcattctt ctggacagcg tgtactgacg tttactcttg gagatatgca acctggagag 840
   cacagaacaa ttactgtaga gttttgtccg cttaaacgtg gtcgtgctac caatatagca 900
   acggtttctt actgtggagg acataaaaat acagcaagcg taacaactgt gatcaacgag 960
   ccttgcgtac aagtaagtat tgcaggagca gattggtctt atgtttgtaa gcctgtagaa 1020
   tatgtgatct ccgtttccaa tcctggagat cttgtgttgc gagatgtcgt cgttgaagac 1080
   actctttctc ccggagtcac agttcttgaa gctgcaggag ctcaaatttc ttgtaataaa 1140
   gtagtttgga ctgtgaaaga actgaatcct ggagagtctc tacagtataa agttctagta 1200
   agagcacaaa ctcctggaca attcacaaat aatgttgttg tgaagagctg ctctgactgt 1260
   ggtacttgta cttcttgcgc agaagcgaca acttactgga aaggagttgc tgctactcat 1320
   atgtgcgtag tagatacttg tgaccctgtt tgtgtaggag aaaatactgt ttaccgtatt 1380
   tgtgtcacca acagaggttc tgcagaagat acaaatgttt ctttaatgct taaattctct 1440
   aaagaactgc aacctgtatc cttctctgga ccaactaaag gaacgattac aggcaataca 1500
   gtagtattcg attcgttacc tagattaggt tctaaagaaa ctgtagagtt ttctgtaaca 1560
   ttgaaagcag tatcagctgg agatgctcgt ggggaagcga ttctttcttc cgatacattg 1620
   actgttccag tttctgatac agagaataca cacatctatt aa                   1662
<210> 45
   <211> 1751
   <212> PRT
   <213> Chlamydia trachomatis
<400> 45
   Met Lys Trp Leu Ser Ala Thr Ala Val Phe Ala Ala Val Leu Pro Ser
    1               5                  10                  15
   Val Ser Gly Phe Cys Phe Pro Glu Pro Lys Glu Leu Asn Phe Ser Arg
               20                  25                  30
   Val Gly Thr Ser Ser Ser Thr Thr Phe Thr Glu Thr Val Gly Glu Ala
           35                  40                  45
   Gly Ala Glu Tyr Ile Val Ser Gly Asn Ala Ser Phe Thr Lys Phe Thr
       50                  55                  60
   Asn Ile Pro Thr Thr Asp Thr Thr Thr Pro Thr Asn Ser Asn Ser Ser
   65 70 75 80
   Ser Ser Asn Gly Glu Thr Ala Ser Val Ser Glu Asp Ser Asp Ser Thr
   85 90 95
   Thr Thr Thr Pro Asp Pro Lys Gly Gly Gly Ala Phe Tyr Asn Ala His
   100 105 110
   Ser Gly Val Leu Ser Phe Met Thr Arg Ser Gly Thr Glu Gly Ser Leu
   115 120 125
   Thr Leu Ser Glu Ile Lys Ile Thr Gly Glu Gly Gly Ala Ile Phe Ser
   130 135 140
   Gln Gly Glu Leu Leu Phe Thr Asp Leu Thr Gly Leu Thr Ile Gln Asn
   145 150 155 160
   Asn Leu Ser Gln Leu Ser Gly Gly Ala Ile Phe Gly Glu Ser Thr Ile
   165 170 175
   Ser Leu Ser Gly Ile Thr Lys Ala Thr Phe Ser Ser Asn Ser Ala Glu
   180 185 190
   Val Pro Ala Pro Val Lys Lys Pro Thr Glu Pro Lys Ala Gln Thr Ala
   195 200 205
   Ser Glu Thr Ser Gly Ser Ser Ser Ser Ser Gly Asn Asp Ser Val Ser
   210 215 220
   Ser Pro Ser Ser Ser Arg Ala Glu Pro Ala Ala Ala Asn Leu Gln Ser
   225 230 235 240
   His Phe Ile Cys Ala Thr Ala Thr Pro Ala Ala Gln Thr Asp Thr Glu
   245 250 255
   Thr Ser Thr Pro Ser His Lys Pro Gly Ser Gly Gly Ala Ile Tyr Ala
   260 265 270
   Lys Gly Asp Leu Thr Ile Ala Asp Ser Gln Glu Val Leu Phe Ser Ile
   275 280 285
   Asn Lys Ala Thr Lys Asp Gly Gly Ala Ile Phe Ala Glu Lys Asp Val
   290 295 300
   Ser Phe Glu Asn Ile Thr Ser Leu Lys Val Gln Thr Asn Gly Ala Glu
   305 310 315 320
   Glu Lys Gly Gly Ala Ile Tyr Ala Lys Gly Asp Leu Ser Ile Gln Ser
   325 330 335
   Ser Lys Gln Ser Leu Phe Asn Ser Asn Tyr Ser Lys Gln Gly Gly Gly
   340 345 350
   Ala Leu Tyr Val Glu Gly Asp Ile Asn Phe Gln Asp Leu Glu Glu Ile
   355 360 365
   Arg Ile Lys Tyr Asn Lys Ala Gly Thr Phe Glu Thr Lys Lys Ile Thr
   370 375 380
   Leu Pro Lys Ala Gln Ala Ser Ala Gly Asn Ala Asp Ala Trp Ala Ser
   385 390 395 400
   Ser Ser Pro Gln Ser Gly Ser Gly Ala Thr Thr Val Ser Asn Ser Gly
   405 410 415
   Asp Ser Ser Ser Gly Ser Asp Ser Asp Thr Ser Glu Thr Val Pro Ala
   420 425 430
   Thr Ala Lys Gly Gly Gly Leu Tyr Thr Asp Lys Asn Leu Ser Ile Thr
   435 440 445
   Asn Ile Thr Gly Ile Ile Glu Ile Ala Asn Asn Lys Ala Thr Asp Val
   450 455 460
   Gly Gly Gly Ala Tyr Val Lys Gly Thr Leu Thr Cys Glu Asn Ser His
   465 470 475 480
   Arg Leu Gln Phe Leu Lys Asn Ser Ser Asp Lys Gln Gly Gly Gly Ile
   485 490 495
   Tyr Gly Glu Asp Asn Ile Thr Leu Ser Asn Leu Thr Gly Lys Thr Leu
   500 505 510
   Phe Gln Glu Asn Thr Ala Lys Glu Glu Gly Gly Gly Leu Phe Ile Lys
   515 520 525
   Gly Thr Asp Lys Ala Leu Thr Met Thr Gly Leu Asp Ser Phe Cys Leu
   530 535 540
   Ile Asn Asn Thr Ser Glu Lys His Gly Gly Gly Ala Phe Val Thr Lys
   545 550 555 560
   Glu Ile Ser Gln Thr Tyr Thr Ser Asp Val Glu Thr Ile Pro Gly Ile
   565 570 575
   Thr Pro Val His Gly Glu Thr Val Ile Thr Gly Asn Lys Ser Thr Gly
   580 585 590
   Gly Asn Gly Gly Gly Val Cys Thr Lys Arg Leu Ala Leu Ser Asn Leu
   595 600 605
   Gln Ser Ile Ser Ile Ser Gly Asn Ser Ala Ala Glu Asn Gly Gly Gly
   610 615 620
   Ala His Thr Cys Pro Asp Ser Phe Pro Thr Ala Asp Thr Ala Glu Gln
   625 630 635 640
   Pro Ala Ala Ala Ser Ala Ala Thr Ser Thr Pro Glu Ser Ala Pro Val
   645 650 655
   Val Ser Thr Ala Leu Ser Thr Pro Ser Ser Ser Thr Val Ser Ser Leu
   660 665 670
   Thr Leu Leu Ala Ala Ser Ser Gln Ala Ser Pro Ala Thr Ser Asn Lys
   675 680 685
   Glu Thr Gln Asp Pro Asn Ala Asp Thr Asp Leu Leu Ile Asp Tyr Val
   690 695 700
   Val Asp Thr Thr Ile Ser Lys Asn Thr Ala Lys Lys Gly Gly Gly Ile
   705 710 715 720
   Tyr Ala Lys Lys Ala Lys Met Ser Arg Ile Asp Gln Leu Asn Ile Ser
   725 730 735
   Glu Asn Ser Ala Thr Glu Ile Gly Gly Gly Ile Cys Cys Lys Glu Ser
   740 745 750
   Leu Glu Leu Asp Ala Leu Val Ser Leu Ser Val Thr Glu Asn Leu Val
   755 760 765
   Gly Lys Glu Gly Gly Gly Leu His Ala Lys Thr Val Asn Ile Ser Asn
   770 775 780
   Leu Lys Ser Gly Phe Ser Phe Ser Asn Asn Lys Ala Asn Ser Ser Ser
   785 790 795 800
   Thr Gly Val Ala Thr Thr Ala Ser Ala Pro Ala Ala Ala Ala Ala Ser
   805 810 815
   Leu Gln Ala Ala Ala Ala Ala Val Pro Ser Ser Pro Ala Thr Pro Thr
   820 825 830
   Tyr Ser Gly Val Val Gly Gly Ala Ile Tyr Gly Glu Lys Val Thr Phe
   835 840 845
   Ser Gln Cys Ser Gly Thr Cys Gln Phe Ser Gly Asn Gln Ala Ile Asp
   850 855 860
   Asn Asn Pro Ser Gln Ser Ser Leu Asn Val Gln Gly Gly Ala Ile Tyr
   865 870 875 880
   Ala Lys Thr Ser Leu Ser Ile Gly Ser Ser Asp Ala Gly Thr Ser Tyr
   885 890 895
   Ile Phe Ser Gly Asn Ser Val Ser Thr Gly Lys Ser Gln Thr Thr Gly
   900 905 910
   Gln Ile Ala Gly Gly Ala Ile Tyr Ser Pro Thr Val Thr Leu Asn Cys
   915 920 925
   Pro Ala Thr Phe Ser Asn Asn Thr Ala Ser Met Ala Thr Pro Lys Thr
   930 935 940
   Ser Ser Glu Asp Gly Ser Ser Gly Asn Ser Ile Lys Asp Thr Ile Gly
   945 950 955 960
   Gly Ala Ile Ala Gly Thr Ala Ile Thr Leu Ser Gly Val Ser Arg Phe
   965 970 975
   Ser Gly Asn Thr Ala Asp Leu Gly Ala Ala Ile Gly Thr Leu Ala Asn
   980 985 990
   Ala Asn Thr Pro Ser Ala Thr Ser Gly Ser Gln Asn Ser Ile Thr Glu
   995 1000 1005
   Lys Ile Thr Leu Glu Asn Gly Ser Phe Ile Phe Glu Arg Asn Gln Ala
   1010 1015 1020
   Asn Lys Arg Gly Ala Ile Tyr Ser Pro Ser Val Ser Ile Lys Gly Asn
   1025 1030 1035 1040
   Asn Ile Thr Phe Asn Gln Asn Thr Ser Thr His Asp Gly Ser Ala Ile
   1045 1050 1055
   Tyr Phe Thr Lys Asp Ala Thr Ile Glu Ser Leu Gly Ser Val Leu Phe
   1060 1065 1070
   Thr Gly Asn Asn Val Thr Ala Thr Gln Ala Ser Ser Ala Thr Ser Gly
   1075 1080 1085
   Gln Asn Thr Asn Thr Ala Asn Tyr Gly Ala Ala Ile Phe Gly Asp Pro
   1090 1095 1100
   Gly Thr Thr Gln Ser Ser Gln Thr Asp Ala Ile Leu Thr Leu Leu Ala
   1105 1110 1115 1120
   Ser Ser Gly Asn Ile Thr Phe Ser Asn Asn Ser Leu Gln Asn Asn Gln
   1125 1130 1135
   Gly Asp Thr Pro Ala Ser Lys Phe Cys Ser Ile Ala Gly Tyr Val Lys
   1140 1145 1150
   Leu Ser Leu Gln Ala Ala Lys Gly Lys Thr Ile Ser Phe Phe Asp Cys
   1155 1160 1165
   Val His Thr Ser Thr Lys Lys Ile Gly Ser Thr Gln Asn Val Tyr Glu
   1170 1175 1180
   Thr Leu Asp Ile Asn Lys Glu Glu Asn Ser Asn Pro Tyr Thr Gly Thr
   1185 1190 1195 1200
   Ile Val Phe Ser Ser Glu Leu His Glu Asn Lys Ser Tyr Ile Pro Gln
   1205 1210 1215
   Asn Ala Ile Leu His Asn Gly Thr Leu Val Leu Lys Glu Lys Thr Glu
   1220 1225 1230
   Leu His Val Val Ser Phe Glu Gln Lys Glu Gly Ser Lys Leu Ile Met
   1235 1240 1245
   Lys Pro Gly Ala Val Leu Ser Asn Gln Asn Ile Ala Asn Gly Ala Leu
   1250 1255 1260
   Val Ile Asn Gly Leu Thr Ile Asp Leu Ser Ser Met Gly Thr Pro Gln
   1265 1270 1275 1280
   Ala Gly Glu Ile Phe Ser Pro Pro Glu Leu Arg Ile Val Ala Thr Thr
   1285 1290 1295
   Ser Ser Ala Ser Gly Gly Ser Gly Val Ser Ser Ser Ile Pro Thr Asn
   1300 1305 1310
   Pro Lys Arg Ile Ser Ala Ala Ala Pro Ser Gly Ser Ala Ala Thr Thr
   1315 1320 1325
   Pro Thr Met Ser Glu Asn Lys Val Phe Leu Thr Gly Asp Leu Thr Leu
   1330 1335 1340
   Ile Asp Pro Asn Gly Asn Phe Tyr Gln Asn Pro Met Leu Gly Ser Asp
   1345 1350 1355 1360
   Leu Asp Val Pro Leu Ile Lys Leu Pro Thr Asn Thr Ser Asp Val Gln
   1365 1370 1375
   Val Tyr Asp Leu Thr Leu Ser Gly Asp Leu Phe Pro Gln Lys Gly Tyr
   1380 1385 1390
   Met Gly Thr Trp Thr Leu Asp Ser Asn Pro Gln Thr Gly Lys Leu Gln
   1395 1400 1405
   Ala Arg Trp Thr Phe Asp Thr Tyr Arg Arg Trp Val Tyr Ile Pro Arg
   1410 1415 1420
   Asp Asn His Phe Tyr Ala Asn Ser Ile Leu Gly Ser Gln Asn Ser Met
   1425 1430 1435 1440
   Ile Val Val Lys Gln Gly Leu Ile Asn Asn Met Leu Asn Asn Ala Arg
   1445 1450 1455
   Phe Asp Asp Ile Ala Tyr Asn Asn Phe Trp Val Ser Gly Val Gly Thr
   1460 1465 1470
   Phe Leu Ala Gln Gln Gly Thr Pro Leu Ser Glu Glu Phe Ser Tyr Tyr
   1475 1480 1485
   Ser Arg Gly Thr Ser Val Ala Ile Asp Ala Lys Pro Arg Gln Asp Phe
   1490 1495 1500
   Ile Leu Gly Ala Ala Phe Ser Lys Met Val Gly Lys Thr Lys Ala Ile
   1505 1510 1515 1520
   Lys Lys Met His Asn Tyr Phe His Lys Gly Ser Glu Tyr Ser Tyr Gln
   1525 1530 1535
   Ala Ser Val Tyr Gly Gly Lys Phe Leu Tyr Phe Leu Leu Asn Lys Gln
   1540 1545 1550
   His Gly Trp Ala Leu Pro Phe Leu Ile Gln Gly Val Val Ser Tyr Gly
   1555 1560 1565
   His Ile Lys His Asp Thr Thr Thr Leu Tyr Pro Ser Ile His Glu Arg
   1570 1575 1580
   Asn Lys Gly Asp Trp Glu Asp Leu Gly Trp Leu Ala Asp Leu Arg Ile
   1585 1590 1595 1600
   Ser Met Asp Leu Lys Glu Pro Ser Lys Asp Ser Ser Lys Arg Ile Thr
   1605 1610 1615
   Val Tyr Gly Glu Leu Glu Tyr Ser Ser Ile Arg Gln Lys Gln Phe Thr
   1620 1625 1630
   Glu Ile Asp Tyr Asp Pro Arg His Phe Asp Asp Cys Ala Tyr Arg Asn
   1635 1640 1645
   Leu Ser Leu Pro Val Gly Cys Ala Val Glu Gly Ala Ile Met Asn Cys
       1650                1655                1660
   Asn Ile Leu Met Tyr Asn Lys Leu Ala Leu Ala Tyr Met Pro Ser Ile
   1665                1670                1675                1680
   Tyr Arg Asn Asn Pro Val Cys Lys Tyr Arg Val Leu Ser Ser Asn Glu
                   1685                1690                1695
   Ala Gly Gln Val Ile Cys Gly Val Pro Thr Arg Thr Ser Ala Arg Ala
               1700                1705                1710
   Glu Tyr Ser Thr Gln Leu Tyr Leu Gly Pro Phe Trp Thr Leu Tyr Gly
           1715                1720                1725
   Asn Tyr Thr Ile Asp Val Gly Met Tyr Thr Leu Ser Gln Met Thr Ser
       1730                1735                1740
   Cys Gly Ala Arg Met Ile Phe
   1745                1750
<210> 46
   <211> 5256
   <212> DNA
   <213> Chlamydia trachomatis
<400> 46
   atgaaatggc tgtcagctac tgcggtgttt gctgctgttc tcccctcagt ttcagggttt 60
   tgcttcccag aacctaaaga attaaatttc tctcgcgtag gaacttcttc ctctaccact 120
   tttactgaaa cagttggaga agctggggca gaatatatcg tctctggtaa cgcatctttc 180
   acaaaattta ccaacattcc tactaccgat acaacaactc ccacgaactc aaactcctct 240
   agctctaacg gagagactgc ttccgtttct gaggatagtg actctacaac aacgactcct 300
   gatcctaaag gtggcggcgc cttttataac gcgcactccg gagttttatc ctttatgaca 360
   cgatcaggaa cagaaggttc cttaactctg tctgagataa aaataactgg tgaaggcggt 420
   gctatcttct ctcaaggaga gctgctattt acagatctga caggtctaac catccaaaat 480
   aacttatccc agctatccgg aggagcgatt tttggagaat ctacaatctc cctatcaggg 540
   attactaaag cgactttctc ctccaactct gcagaagttc ctgctcctgt taagaaacct 600
   acagaaccta aagctcaaac agcaagcgaa acgtcgggtt ctagtagttc tagcggaaat 660
   gattcggtgt cttcccccag ttccagtaga gctgaacccg cagcagctaa tcttcaaagt 720
   cactttattt gtgctacagc tactcctgct gctcaaaccg atacagaaac atcaactccc 780
   tctcataagc caggatctgg gggagctatc tatgctaaag gcgaccttac tatcgcagac 840
   tctcaagagg tactattctc aataaataaa gctactaaag atggaggagc gatctttgct 900
   gagaaagatg tttctttcga gaatattaca tcattaaaag tacaaactaa cggtgctgaa 960
   gaaaagggag gagctatcta tgctaaaggt gacctctcaa ttcaatcttc taaacagagt 1020
   ctttttaatt ctaactacag taaacaaggt ggtggggctc tatatgttga aggagatata 1080
   aacttccaag atcttgaaga aattcgcatt aagtacaata aagctggaac gttcgaaaca 1140
   aaaaaaatca ctttaccaaa agctcaagca tctgcaggaa atgcagatgc ttgggcctct 1200
   tcctctcctc aatctggttc tggagcaact acagtctcca actcaggaga ctctagctct 1260
   ggctcagact cggatacctc agaaacagtt ccagccacag ctaaaggcgg tgggctttat 1320
   actgataaga atctttcgat tactaacatc acaggaatta tcgaaattgc aaataacaaa 1380
   gcgacagatg ttggaggtgg tgcttacgta aaaggaaccc ttacttgtga aaactctcac 1440
   cgtctacaat ttttgaaaaa ctcttccgat aaacaaggtg gaggaatcta cggagaagac 1500
   aacatcaccc tatctaattt gacagggaag actctattcc aagagaatac tgccaaagaa 1560
   gagggcggtg gactcttcat aaaaggtaca gataaagctc ttacaatgac aggactggat 1620
   agtttctgtt taattaataa cacatcagaa aaacatggtg gtggagcctt tgttaccaaa 1680
   gaaatctctc agacttacac ctctgatgtg gaaacaattc caggaatcac gcctgtacat 1740
   ggtgaaacag tcattactgg caataaatct acaggaggta atggtggagg cgtgtgtaca 1800
   aaacgtcttg ccttatctaa ccttcaaagc atttctatat ccgggaattc tgcagctgaa 1860
   aatggtggtg gagcccacac atgcccagat agcttcccaa cggcggatac tgcagaacag 1920
   cccgcagcag cttctgccgc gacgtctact cccgagtctg ccccagtggt ctcaactgct 1980
   ctaagcacac cttcatcttc taccgtctct tcattaacct tactagcagc ctcttcacaa 2040
   gcctctcctg caacctctaa taaggaaact caagatccta atgctgatac agacttattg 2100
   atcgattatg tagttgatac gactatcagc aaaaacactg ctaagaaagg cggtggaatc 2160
   tatgctaaaa aagccaagat gtcccgcata gaccaactga atatctctga gaactccgct 2220
   acagagatag gtggaggtat ctgctgtaaa gaatctttag aactagatgc cctagtctcc 2280
   ttatctgtaa cagagaacct tgttgggaaa gaaggtggag gcttacatgc taaaactgta 2340
   aatatttcta atctgaaatc aggcttctct ttctcgaaca acaaagcaaa ctcctcatcc 2400
   acaggagtcg caacaacagc ttcagcacct gctgcagctg ctgcttccct acaagcagcc 2460
   gcagcagccg taccatcatc tccagcaaca ccaacttatt caggtgtagt aggaggagct 2520
   atctatggag aaaaggttac attctctcaa tgtagcggga cttgtcagtt ctctgggaac 2580
   caagctatcg ataacaatcc ctcccaatca tcgttgaacg tacaaggagg agccatctat 2640
   gccaaaacct ctttgtctat tggatcttcc gatgctggaa cctcctatat tttctcgggg 2700
   aacagtgtct ccactgggaa atctcaaaca acagggcaaa tagcgggagg agcgatctac 2760
   tcccctactg ttacattgaa ttgtcctgcg acattctcta acaatacagc ctctatggct 2820
   acaccaaaga cttcttctga agatggatcc tcaggaaatt ctattaaaga taccattgga 2880
   ggagccattg cagggacagc cattacccta tctggagtct ctcgattttc agggaatacg 2940
   gctgatttag gagctgcaat aggaactcta gctaatgcaa atacacccag tgcaactagc 3000
   ggatctcaaa atagcattac agaaaaaatt actttagaaa acggttcttt tatttttgaa 3060
   agaaaccaag ctaataaacg tggagcgatt tactctccta gcgtttccat taaagggaat 3120
   aatattacct tcaatcaaaa tacatccact catgatggaa gtgctatcta ctttacaaaa 3180
   gatgctacga ttgagtcttt aggatctgtt ctttttacag gaaataacgt tacagctaca 3240
   caagctagtt ctgcaacatc tggacaaaat acaaatactg ccaactatgg ggcagccatc 3300
   tttggagatc caggaaccac tcaatcgtct caaacagatg ccattttaac ccttcttgct 3360
   tcttctggaa acattacttt tagcaacaac agtttacaga ataaccaagg tgatactccc 3420
   gctagcaagt tttgtagtat tgcaggatac gtcaaactct ctctacaagc cgctaaaggg 3480
   aagactatta gctttttcga ttgtgtgcac acctctacca aaaaaatagg ttcaacacaa 3540
   aacgtttatg aaactttaga tattaataaa gaagagaaca gtaatccata tacaggaact 3600
   attgtgttct cttctgaatt acatgaaaac aaatcttaca tcccacagaa tgcaatcctt 3660
   cacaacggaa ctttagttct taaagagaaa acagaactcc acgtagtctc ttttgagcag 3720
   aaagaagggt ctaaattaat tatgaaaccc ggagctgtgt tatctaacca aaacatagct 3780
   aacggagctc tagttatcaa tgggttaacg attgatcttt ccagtatggg gactcctcaa 3840
   gcaggggaaa tcttctctcc tccagaatta cgtatcgttg ccacgacctc tagtgcatcc 3900
   ggaggaagcg gggtcagcag tagtatacca acaaatccta aaaggatttc tgcagcagcg 3960
   ccttcaggtt ctgccgcaac tactccaact atgagcgaga acaaagtttt cctaacagga 4020
   gaccttactt taatagatcc taatggaaac ttttaccaaa accctatgtt aggaagcgat 4080
   ctagatgtac cactaattaa gcttccgact aacacaagtg acgtccaagt ctatgattta 4140
   actttatctg ggatcttttt ccctcagaaa gggtacatgg gaacctggac attagattct 4200
   aatccacaaa cagggaaact tcaagccaga tggacattcg atacctatcg tcgctgggta 4260
   tacataccta gggataatca tttttatgcg aactctatct taggctccca aaactcaatg 4320
   attgttgtga agcaagggct tatcaacaac atgttgaata atgcccgctt cgatgatatc 4380
   gcttacaata acttctgggt ttcaggagta ggaactttct tagctcaaca aggaactcct 4440
   ctttccgaag aattcagtta ctacagccgc ggaacttcag ttgccatcga tgccaaacct 4500
   agacaagatt ttatcctagg agctgcattt agtaagatgg tggggaaaac caaagccatc 4560
   aaaaaaatgc ataattactt ccataagggc tctgagtact cttaccaagc ttctgtctat 4620
   ggaggtaaat tcctgtattt cttgctcaat aagcaacatg gttgggcact tcctttccta 4680
   atacaaggag tcgtgtccta tggacatatt aaacatgata caacaacact ttacccttct 4740
   atccatgaaa gaaataaagg agattgggaa gatttaggat ggttagcgga tcttcgtatc 4800
   tctatggatc ttaaagaacc ttctaaagat tcttctaaac ggatcactgt ctatggggaa 4860
   cttgagtatt ccagcattcg ccagaaacag ttcacagaaa tcgattacga tccaagacac 4920
   ttcgatgatt gtgcttacag aaatctgtcg cttcctgtgg gatgcgctgt cgaaggagct 4980
   atcatgaact gtaatattct tatgtataat aagcttgcat tagcctacat gccttctatc 5040
   tacagaaata atcctgtctg taaatatcgg gtattgtctt cgaatgaagc tggtcaagtt 5100
   atctgcggag tgccaactag aacctctgct agagcagaat acagtactca actatatctt 5160
   ggtcccttct ggactctcta cggaaactat actatcgatg taggcatgta tacgctatcg 5220
   caaatgacta gctgcggtgc tcgcatgatc ttctaa 5256
<210> 47
   <211> 975
   <212> PRT
   <213> Chlamydia trachomatis
<400> 47
   Met Asn Arg Val Ile Glu Ile His Ala His Tyr Asp Gln Arg Gln Leu
   1 5 10 15
   Ser Gln Ser Pro Asn Thr Asn Phe Leu Val His His Pro Tyr Leu Thr
   20 25 30
   Leu Ile Pro Lys Phe Leu Leu Gly Ala Leu Ile Val Tyr Ala Pro Tyr
   35 40 45
   Ser Phe Ala Glu Met Glu Leu Ala Ile Ser Gly His Lys Gln Gly Lys
   50 55 60
   Asp Arg Asp Thr Phe Thr Met Ile Ser Ser Cys Pro Glu Gly Thr Asn
   65 70 75 80
   Tyr Ile Ile Asn Arg Lys Leu Ile Leu Ser Asp Phe Ser Leu Leu Asn
   85 90 95
   Lys Val Ser Ser Gly Gly Ala Phe Arg Asn Leu Ala Gly Lys Ile Ser
   100 105 110
   Phe Leu Gly Lys Asn Ser Ser Ala Ser Ile His Phe Lys His Ile Asn
   115 120 125
   Ile Asn Gly Phe Gly Ala Gly Val Phe Ser Glu Ser Ser Ile Glu Phe
   130 135 140
   Thr Asp Leu Arg Lys Leu Val Ala Phe Gly Ser Glu Ser Thr Gly Gly
   145 150 155 160
   Ile Phe Thr Ala Lys Glu Asp Ile Ser Phe Lys Asn Asn His His Ile
   165 170 175
   Ala Phe Arg Asn Asn Ile Thr Lys Gly Asn Gly Gly Val Ile Gln Leu
   180 185 190
   Gln Gly Asp Met Lys Gly Ser Val Ser Phe Val Asp Gln Arg Gly Ala
   195 200 205
   Ile Ile Phe Thr Asn Asn Gln Ala Val Thr Ser Ser Ser Met Lys His
   210 215 220
   Ser Gly Arg Gly Gly Ala Ile Ser Gly Asp Phe Ala Gly Ser Arg Ile
   225 230 235 240
   Leu Phe Leu Asn Asn Gln Gln Ile Thr Phe Glu Gly Asn Ser Ala Val
   245 250 255
   His Gly Gly Ala Ile Tyr Asn Lys Asn Gly Leu Val Glu Phe Leu Gly
   260 265 270
   Asn Ala Gly Pro Leu Ala Phe Lys Glu Asn Thr Thr Ile Ala Asn Gly
   275 280 285
   Gly Ala Ile Tyr Thr Ser Asn Phe Lys Ala Asn Gln Gln Thr Ser Pro
   290 295 300
   Ile Leu Phe Ser Gln Asn His Ala Asn Lys Lys Gly Gly Ala Ile Tyr
   305 310 315 320
   Ala Gln Tyr Val Asn Leu Glu Gln Asn Gln Asp Thr Ile Arg Phe Glu
   325 330 335
   Lys Asn Thr Ala Lys Glu Gly Gly Gly Ala Ile Thr Ser Ser Gln Cys
   340 345 350
   Ser Ile Thr Ala His Asn Thr Ile Ile Phe Ser Asp Asn Ala Ala Gly
   355 360 365
   Asp Leu Gly Gly Gly Ala Ile Leu Leu Glu Gly Lys Lys Pro Ser Leu
   370 375 380
   Thr Leu Ile Ala His Ser Gly Asn Ile Ala Phe Ser Gly Asn Thr Met
   385 390 395 400
   Leu His Ile Thr Lys Lys Ala Ser Leu Asp Arg His Asn Ser Ile Leu
   405 410 415
   Ile Lys Glu Ala Pro Tyr Lys Ile Gln Leu Ala Ala Asn Lys Asn His
   420 425 430
   Ser Ile His Phe Phe Asp Pro Val Met Ala Leu Ser Ala Ser Ser Ser
   435 440 445
   Pro Ile Gln Ile Asn Ala Pro Glu Tyr Glu Thr Pro Phe Phe Ser Pro
   450 455 460
   Lys Gly Met Ile Val Phe Ser Gly Ala Asn Leu Leu Asp Asp Ala Arg
   465 470 475 480
   Glu Asp Val Ala Asn Arg Thr Ser Ile Phe Asn Gln Pro Val His Leu
   485 490 495
   Tyr Asn Gly Thr Leu Ser Ile Glu Asn Gly Ala His Leu Ile Val Gln
   500 505 510
   Ser Phe Lys Gln Thr Gly Gly Arg Ile Ser Leu Ser Pro Gly Ser Ser
   515 520 525
   Leu Ala Leu Tyr Thr Met Asn Ser Phe Phe His Gly Asn Ile Ser Ser
   530 535 540
   Lys Glu Pro Leu Glu Ile Asn Gly Leu Ser Phe Gly Val Asp Ile Ser
   545 550 555 560
   Pro Ser Asn Leu Gln Ala Glu Ile Arg Ala Gly Asn Ala Pro Leu Arg
   565 570 575
   Leu Ser Gly Ser Pro Ser Ile His Asp Pro Glu Gly Leu Phe Tyr Glu
   580 585 590
   Asn Arg Asp Thr Ala Ala Ser Pro Tyr Gln Met Glu Ile Leu Leu Thr
   595 600 605
   Ser Asp Lys Ile Val Asp Ile Ser Lys Phe Thr Thr Asp Ser Leu Val
   610 615 620
   Thr Asn Lys Gln Ser Gly Phe Gln Gly Ala Trp His Phe Ser Trp Gln
   625 630 635 640
   Pro Asn Thr Ile Asn Asn Thr Lys Gln Lys Ile Leu Arg Ala Ser Trp
   645 650 655
   Leu Pro Thr Gly Glu Tyr Val Leu Glu Ser Asn Arg Val Gly Arg Ala
   660 665 670
   Val Pro Asn Ser Leu Trp Ser Thr Phe Leu Leu Leu Gln Thr Ala Ser
   675 680 685
   His Asn Leu Gly Asp His Leu Cys Asn Asn Arg Ser Leu Ile Pro Thr
   690 695 700
   Ser Tyr Phe Gly Val Leu Ile Gly Gly Thr Gly Ala Glu Met Ser Thr
   705 710 715 720
   His Ser Ser Glu Glu Glu Ser Phe Ile Ser Arg Leu Gly Ala Thr Gly
   725 730 735
   Thr Ser Ile Ile Arg Leu Thr Pro Ser Leu Thr Leu Ser Gly Gly Gly
   740 745 750
   Ser His Met Phe Gly Asp Ser Phe Val Ala Asp Leu Pro Glu His Ile
   755 760 765
   Thr Ser Glu Gly Ile Val Gln Asn Val Gly Leu Thr His Val Trp Gly
   770 775 780
   Pro Leu Thr Val Asn Ser Thr Leu Cys Ala Ala Leu Asp His Asn Ala
   785 790 795 800
   Met Val Arg Ile Cys Ser Lys Lys Asp His Thr Tyr Gly Lys Trp Asp
   805 810 815
   Thr Phe Gly Met Arg Gly Thr Leu Gly Ala Ser Tyr Thr Phe Leu Glu
   820 825 830
   Tyr Asp Gln Thr Met Arg Val Phe Ser Phe Ala Asn Ile Glu Ala Thr
   835 840 845
   Asn Ile Leu Gln Arg Ala Phe Thr Glu Thr Gly Tyr Asn Pro Arg Ser
   850 855 860
   Phe Ser Lys Thr Lys Leu Leu Asn Ile Ala Ile Pro Ile Gly Ile Gly
   865 870 875 880
   Tyr Glu Phe Cys Leu Gly Asn Ser Ser Phe Ala Leu Leu Gly Lys Gly
   885 890 895
   Ser Ile Gly Tyr Ser Arg Asp Ile Lys Arg Glu Asn Pro Ser Thr Leu
   900 905 910
   Ala His Leu Ala Met Asn Asp Phe Ala Trp Thr Thr Asn Gly Cys Ser
   915 920 925
   Val Pro Thr Ser Ala His Thr Leu Ala Asn Gln Leu Ile Leu Arg Tyr
   930 935 940
   Lys Ala Cys Ser Leu Tyr Ile Thr Ala Tyr Thr Ile Asn Arg Glu Gly
   945 950 955 960
   Lys Asn Leu Ser Asn Ser Leu Ser Cys Gly Gly Tyr Val Gly Phe
   965 970 975
<210> 48
   <211> 2928
   <212> DNA
   <213> Chlamydia trachomatis
<400> 48
   atgaatcgag ttatagaaat ccatgctcac tacgatcaaa gacaactttc tcaatctcca 60
   aatacaaact tcttagtaca tcatccttat cttactctta ttcccaagtt tctactagga 120
   gctctaatcg tctatgctcc ttattcgttt gcagaaatgg aattagctat ttctggacat 180
   aaacaaggta aagatcgaga tacctttacc atgatctctt cctgtcctga aggcactaat 240
   tacatcatca atcgcaaact catactcagt gatttctcgt tactaaataa agtttcatca 300
   gggggagcct ttcggaatct agcagggaaa atttccttct taggaaaaaa ttcttctgcg 360
   tccattcatt ttaaacacat taatatcaat ggttttggag ccggagtctt ttctgaatcc 420
   tctattgaat ttactgattt acgaaaactt gttgcttttg gatctgaaag cacaggagga 480
   atttttactg cgaaagagga catctctttt aaaaacaacc accacattgc cttccgcaat 540
   aatatcacca aagggaatgg tggcgttatc cagctccaag gagatatgaa aggaagcgta 600
   tcctttgtag atcaacgtgg agctatcatc tttaccaata accaagctgt aacttcttca 660
   tcaatgaaac atagtggtcg tggaggagca attagcggtg acttcgcagg atccagaatt 720
   ctttttctta ataaccaaca aattactttc gaaggcaata gcgctgtgca tggaggtgct 780
   atctacaata agaatggcct tgtcgagttc ttaggaaatg caggacctct tgcctttaaa 840
   gagaacacaa caatagctaa cgggggagct atatacacaa gtaatttcaa agcgaatcaa 900
   caaacatccc ccattctatt ctctcaaaat catgcgaata agaaaggcgg agcgatttac 960
   gcgcaatatg tgaacttaga acagaatcaa gatactattc gctttgaaaa aaataccgct 1020
   aaagaaggcg gtggagccat cacctcttct caatgctcaa ttactgctca taataccatc 1080
   attttttccg ataatgctgc cggagatctt ggaggaggag caattcttct agaagggaaa 1140
   aaaccttctc taaccttgat tgctcatagt ggtaatattg catttagcgg caataccatg 1200
   cttcatatca ccaaaaaagc ttccctagat cgacacaatt ctatcttaat caaagaagct 1260
   ccctataaaa tccaacttgc agcgaacaaa aaccattcta ttcatttctt tgatcctgtc 1320
   atggcattgt cagcatcatc ttcccctata caaatcaatg ctcctgagta tgaaactccc 1380
   ttcttctcac ctaagggtat gatcgttttc tcgggtgcga atcttttaga tgatgctagg 1440
   gaagatgttg caaatagaac atcgattttt aaccaacccg ttcatctata taatggcacc 1500
   ctatctatcg aaaatggagc ccatctgatt gtccaaagct tcaaacagac cggaggacgt 1560
   atcagtttat ctccaggatc ctccttggct ctatacacga tgaactcgtt cttccatggc 1620
   aacatatcca gcaaagaacc cctagaaatt aatggtttaa gctttggagt agatatctct 1680
   ccttctaatc ttcaagcaga gatccgtgcc ggcaacgctc ctttacgatt atccggatcc 1740
   ccatctatcc atgatcctga aggattattc tacgaaaatc gcgatactgc agcatcacca 1800
   taccaaatgg aaatcttgct cacctctgat aaaattgtag atatctccaa atttactact 1860
   gattctctag ttacgaacaa acaatcagga ttccaaggag cctggcattt tagctggcag 1920
   ccaaatacta taaacaatac taaacaaaaa atattaagag cttcttggct cccaacagga 1980
   gaatatgtcc ttgaatccaa tcgagtgggg cgtgccgttc ctaattcctt atggagcaca 2040
   tttttacttt tacagacagc ctctcataac ttaggcgatc atctatgtaa taatcgatct 2100
   cttattccta cttcatactt cggagtttta attggaggaa ctggagcaga aatgtctacc 2160
   cactcctcag aagaagaaag ctttatatct cgtttaggag ctacaggaac ctctatcata 2220
   cgcttaactc cctccctgac actctctgga ggaggctcac atatgttcgg agattcgttc 2280
   gttgcagact taccagaaca catcacttca gaaggaattg ttcagaatgt cggtttaacc 2340
   catgtctggg gaccccttac tgtcaattct acattatgtg cagccttaga tcacaacgcg 2400
   atggtccgca tatgctccaa aaaagatcac acctatggga aatgggatac attcggtatg 2460
   cgaggaacat taggagcctc ttatacattc ctagaatatg atcaaactat gcgcgtattc 2520
   tcattcgcca acatcgaagc cacaaatatc ttgcaaagag cttttactga aacaggctat 2580
   aacccaagaa gtttttccaa gacaaaactt ctaaacatcg ccatccccat agggattggt 2640
   tatgaattct gcttagggaa tagctctttt gctctactag gtaagggatc catcggttac 2700
   tctcgagata ttaaacgaga aaacccatcc actcttgctc acctggctat gaatgatttt 2760
   gcttggacta ccaatggctg ttcagttcca acctctgcac acacattggc aaatcaattg 2820
   attcttcgct ataaagcatg ttccttatac atcacggcat atactatcaa ccgtgaaggg 2880
   aagaacctct ccaatagctt atcctgcgga ggctatgttg gcttctaa 2928
<210> 49
   <211> 210
   <212> PRT
   <213> Chlamydia trachomatis
<400> 49
   Met Tyr Lys Tyr Phe Ile Val Asp Thr Ser Gly Ser Gln Pro Phe Leu
   1 5 10 15
   Ala Tyr Val Asp Cys Arg Asp Val Leu Glu Val Trp Ser Leu Pro Thr
   20 25 30
   Gly Pro Asp Gln Gly Val Val Leu Asn Phe Ile Phe Asn Ser Leu Asp
   35 40 45
   Leu Pro Phe Gln Gly Ile Gly Val Ser Val Gly Pro Gly Gly Phe Ser
   50 55 60
   Ala Thr Arg Val Gly Val Ala Phe Ala Gln Gly Leu Ser Leu Ala Lys
   65 70 75 80
   Asn Val Pro Leu Val Gly Tyr Ser Ser Leu Glu Gly Tyr Leu Ser Leu
   85 90 95
   Gly Gln Glu Glu Glu Ala Leu Leu Leu Pro Leu Gly Lys Lys Gly Gly
   100 105 110
   Ile Val Ala Leu Asn Ser Glu Leu Ser Leu Asp Gly Phe Leu Leu Thr
   115 120 125
   Asp Thr Thr Ser Thr Pro Gly Ile Leu Leu Ser Tyr Ser Glu Ala Leu
   130 135 140
   Glu Tyr Cys Leu Asp Lys Gly Cys Cys His Val Ile Ser Pro Asp Pro
   145 150 155 160
   Thr Tyr Phe Val Glu Leu Phe Ser Ser Arg Ile Ser Val Arg Lys Val
   165 170 175
   Val Pro Cys Ile Asp Arg Ile Arg Lys Tyr Val Val Ser Gln Phe Val
   180 185 190
   Leu Ser Gln Asn Leu Pro Leu Cys Leu Asp Tyr Arg Ser Ile Ser Ser
   195 200 205
   Phe Phe
   210
<210> 50
   <211> 633
   <212> DNA
   <213> Chlamydia trachomatis
<400> 50
   gtgtacaaat attttattgt agacacttcc ggttctcagc cgtttttggc ctatgtcgat 60
   tgtcgagatg ttttagaagt atggtctttg cctacagggc cagatcaagg agttgtgtta 120
   aatttcattt tcaacagcct ggatttgcct ttccaaggta ttggagtctc tgttggtcct 180
   gggggatttt ctgcaactag agtgggagtt gcttttgctc aagggctctc tctggctaaa 240
   aatgtccctt tagttggcta tagctcttta gaaggatatc tttctttggg tcaagaagag 300
   gaggctttgc ttttgcctct agggaaaaag ggtgggattg tagctttaaa ctcagagctt 360
   tctcttgatg gttttctgct tacagatact acttctactc cgggaatttt attgtcttat 420
   tctgaagctc tagagtattg tttagataag ggatgttgtc atgtgatctc tccggatcca 480
   acgtatttcg tagaactatt ttcttcgcgt atttcggtaa ggaaggtggt tccttgtatc 540
   gatcgaatcc gtaagtacgt tgtttcgcag tttgttctgt ctcaaaatct tccgttgtgt 600
   ttagattacc ggagcatctc ttcctttttt tag 633
<210> 51
   <211> 141
   <212> PRT
   <213> Chlamydia trachomatis
<400> 51
   Met Ile Asp Val Ser Val Val Gly Pro Val Leu Ala Met Ala Leu Ala
   1               5                   10                  15
   Met Ile Gly Ser Ala Val Gly Cys Gly Met Ala Gly Val Ala Ser His
               20                  25                  30
   Ala Val Met Ser Arg Ile Asp Glu Gly His Gly Lys Ile Ile Gly Leu
           35                  40                  45
   Ser Ala Met Pro Ser Ser Gln Ser Ile Tyr Gly Leu Ile Phe Met Leu
       50                  55                  60
   Leu Leu Asn Asp Ala Ile Lys Asp Gly Lys Val Ser Ala Val Ser Gly
   65                  70                  75                  80
   Ile Val Met Gly Ile Ala Val Gly Ser Ala Leu Leu Leu Ser Ala Phe
                   85                  90                  95
   Met Gln Gly Lys Cys Cys Val Ser Ala Ile Gln Ala Tyr Ala Arg Ser
               100                 105                 110
   Ser Ala Ile Tyr Gly Lys Ser Phe Ala Ser Ile Gly Ile Val Glu Ser
           115                 120                 125
   Phe Ala Leu Phe Ala Phe Val Phe Ala Leu Leu Leu Phe
       130                 135                 140
<210> 52
   <211> 426
   <212> DNA
   <213> Chlamydia trachomatis
<400> 52
   atgatagatg tatcagtagt ggggcctgta ttggctatgg ctttggcaat gattggtagc 60
   gctgttggat gtggaatggc tggagtcgct tctcacgcag tgatgtctcg aatcgatgaa 120
   ggacacggga agattattgg tctgtctgct atgccctcat cacaatccat ttacgggttg 180
   attttcatgt tactgctgaa tgatgcaatt aaggatggaa aagtctctgc agtcagtggt 240
   atcgtaatgg gtatagctgt aggatctgcg ttattgcttt ctgcttttat gcaagggaag 300
   tgctgtgtga gtgctattca agcctatgcg cgttcctctg caatatatgg taaatcattt 360
   gcttcgattg ggattgttga gtcttttgcg ttatttgctt tcgtttttgc gctattgtta 420
   ttctaa                                                            426
<210> 53
   <211> 101
   <212> PRT
   <213> Chlamydia trachomatis
<400> 53
   Met Ala Lys Lys Ser Ala Val Ala Arg Glu Val Lys Arg Arg Lys Leu
   1 5 10 15
   Val Glu Ala Asn Phe Gln Lys Arg Ala Glu Leu Arg Lys Leu Ala Lys
   20 25 30
   Ser Leu Ser Val Ser Glu Glu Glu Arg Glu Arg Ala Arg Glu Ala Leu
   35 40 45
   Asn Lys Met Arg Arg Asp Thr Ser Pro Ser Arg Leu His Asn Arg Cys
   50 55 60
   Leu Leu Thr Gly Arg Pro Arg Gly Tyr Leu Arg Lys Phe Ala Ile Ser
   65 70 75 80
   Arg Ile Cys Phe Arg Gln Met Ala Ser Met Gly Asp Ile Pro Gly Val
   85 90 95
   Val Lys Ala Ser Trp
   100
<210> 54
   <211> 306
   <212> DNA
   <213> Chlamydia trachomatis
<400> 54
   atggcgaaga agtcagcagt agctagagaa gttaaacgtc gaaagttagt agaagctaat 60
   tttcagaaga gagcagagct tcgaaaactt gcgaagagtt tatctgttag cgaggaagaa 120
   agagaaagag ctcgggaagc tctcaataaa atgagaagag atacttctcc ttctcgttta 180
   cataatagat gcctattaac aggccgtcct cgtggatacc ttagaaagtt tgctatctca 240
   agaatttgtt ttagacaaat ggcttctatg ggagatatcc caggcgttgt gaaagcaagt 300
   tggtag 306
<210> 55
   <211> 393
   <212> PRT
   <213> Chlamydia trachomatis
<400> 55
   Met Lys Lys Leu Leu Lys Ser Val Leu Val Phe Ala Ala Leu Ser Ser
   1 5 10 15
   Ala Ser Ser Leu Gln Ala Leu Pro Val Gly Asn Pro Ala Glu Pro Ser
   20 25 30
   Leu Met Ile Asp Gly Ile Leu Trp Glu Gly Phe Gly Gly Asp Pro Cys
   35 40 45
   Asp Pro Cys Ala Thr Trp Cys Asp Ala Ile Ser Met Arg Val Gly Tyr
   50 55 60
   Tyr Gly Asp Phe Val Phe Asp Arg Val Leu Lys Thr Asp Val Asn Lys
   65 70 75 80
   Glu Phe Gln Met Gly Ala Lys Pro Thr Thr Asp Thr Gly Asn Ser Ala
   85 90 95
   Ala Pro Ser Thr Leu Thr Ala Arg Glu Asn Pro Ala Tyr Gly Arg His
   100 105 110
   Met Gln Asp Ala Glu Met Phe Thr Asn Ala Ala Cys Met Ala Leu Asn
   115 120 125
   Ile Trp Asp Arg Phe Asp Val Phe Cys Thr Leu Gly Ala Thr Ser Gly
   130 135 140
   Tyr Leu Lys Gly Asn Ser Ala Ser Phe Asn Leu Val Gly Leu Phe Gly
   145 150 155 160
   Asp Asn Glu Asn Gln Lys Thr Val Lys Ala Glu Ser Val Pro Asn Met
   165 170 175
   Ser Phe Asp Gln Ser Val Val Glu Leu Tyr Thr Asp Thr Thr Phe Ala
   180 185 190
   Trp Ser Val Gly Ala Arg Ala Ala Leu Trp Glu Cys Gly Cys Ala Thr
   195 200 205
   Leu Gly Ala Ser Phe Gln Tyr Ala Gln Ser Lys Pro Lys Val Glu Glu
   210 215 220
   Leu Asn Val Leu Cys Asn Ala Ala Glu Phe Thr Ile Asn Lys Pro Lys
   225 230 235 240
   Gly Tyr Val Gly Lys Glu Phe Pro Leu Asp Leu Thr Ala Gly Thr Asp
   245 250 255
   Ala Ala Thr Gly Thr Lys Asp Ala Ser Ile Asp Tyr His Glu Trp Gln
   260 265 270
   Ala Ser Leu Ala Leu Ser Tyr Arg Leu Asn Met Phe Thr Pro Tyr Ile
   275 280 285
   Gly Val Lys Trp Ser Arg Ala Ser Phe Asp Ala Asp Thr Ile Arg Ile
   290 295 300
   Ala Gln Pro Lys Ser Ala Thr Ala Ile Phe Asp Thr Thr Thr Leu Asn
   305 310 315 320
   Pro Thr Ile Ala Gly Ala Gly Asp Val Lys Thr Gly Ala Glu Gly Gln
   325 330 335
   Leu Gly Asp Thr Met Gln Ile Val Ser Leu Gln Leu Asn Lys Met Lys
   340 345 350
   Ser Arg Lys Ser Cys Gly Ile Ala Val Gly Thr Thr Ile Val Asp Ala
   355 360 365
   Asp Lys Tyr Ala Val Thr Val Glu Thr Arg Leu Ile Asp Glu Arg Ala
   370 375 380
   Ala His Val Asn Ala Gln Phe Arg Phe
   385 390
<210> 56
   <211> 1182
   <212> DNA
   <213> Chlamydia trachomatis
<400> 56
   atgaaaaaac tcttgaaatc ggtattagta tttgccgctt tgagttctgc ttcctccttg 60
   caagctctgc ctgtggggaa tcctgctgaa ccaagcctta tgatcgacgg aattctgtgg 120
   gaaggtttcg gcggagatcc ttgcgatcct tgcgccactt ggtgtgacgc tatcagcatg 180
   cgtgttggtt actacggaga ctttgttttc gaccgtgttt tgaaaactga tgtgaataaa 240
   gaatttcaga tgggtgccaa gcctacaact gatacaggca atagtgcagc tccatccact 300
   cttacagcaa gagagaatcc tgcttacggc cgacatatgc aggatgctga gatgtttaca 360
   aatgccgctt gcatggcatt gaatatttgg gatcgttttg atgtattctg tacattagga 420
   gccaccagtg gatatcttaa aggaaactct gcttctttca atttagttgg attgtttgga 480
   gataatgaaa atcaaaaaac ggtcaaagcg gagtctgtac caaatatgag ctttgatcaa 540
   tctgttgttg agttgtatac agatactact tttgcgtgga gcgtcggcgc tcgcgcagct 600
   ttgtgggaat gtggatgtgc aactttagga gcttcattcc aatatgctca atctaaacct 660
   aaagtagaag aattaaacgt tctctgcaat gcagcagagt ttactattaa taaacctaaa 720
   gggtatgtag gtaaggagtt tcctcttgat cttacagcag gaacagatgc tgcgacagga 780
   actaaggatg cctctattga ttaccatgaa tggcaagcaa gtttagctct ctcttacaga 840
   ctgaatatgt tcactcccta cattggagtt aaatggtctc gagcaagctt tgatgccgat 900
   acgattcgta tagcccagcc aaaatcagct acagctattt ttgatactac cacgcttaac 960
   ccaactattg ctggagctgg cgatgtgaaa actggcgcag agggtcagct cggagacaca 1020
   atgcaaatcg tttccttgca attgaacaag atgaaatcta gaaaatcttg cggtattgca 1080
   gtaggaacaa ctattgtgga tgcagacaaa tacgcagtta cagttgagac tcgcttgatc 1140
   gatgagagag cagctcacgt aaatgcacaa ttccgcttct aa                    1182
<210> 57
   <211> 194
   <212> PRT
   <213> Chlamydia trachomatis
<400> 57
   Met Leu Lys Met Phe Trp Leu Asn Ser Leu Val Phe Phe Ser Leu Leu
   1               5                   10                  15
   Leu Ser Ala Cys Gly Tyr Thr Val Leu Ser Pro His Tyr Val Glu Lys
               20                  25                  30
   Lys Phe Ser Leu Ser Glu Gly Ile Tyr Val Cys Pro Ile Glu Gly Asp
           35                  40                  45
   Ser Leu Gly Asp Leu Val Ser Ser Leu Ser Tyr Glu Leu Glu Lys Arg
       50                  55                  60
   Gly Leu His Thr Arg Ser Gln Gly Thr Ser Ser Gly Tyr Val Leu Lys
   65                  70                  75                  80
   Val Ser Leu Phe Asn Glu Thr Asp Glu Asn Ile Gly Phe Ala Tyr Thr
                   85                  90                  95
   Pro Gln Lys Pro Asp Glu Lys Pro Val Lys His Phe Ile Val Ser Asn
               100                 105                 110
   Glu Gly Arg Leu Ala Leu Ser Ala Lys Val Gln Leu Ile Lys Asn Arg
           115                 120                 125
   Thr Gln Glu Ile Leu Val Glu Lys Cys Leu Arg Lys Ser Val Thr Phe
       130                 135                 140
   Asp Phe Gln Pro Asp Leu Gly Thr Ala Asn Ala His Gln Leu Ala Leu
   145                 150                 155                 160
   Gly Gln Phe Glu Met His Asn Glu Ala Ile Lys Ser Ala Ser Arg Ile
                   165                 170                 175
   Leu Tyr Ser Gln Leu Ala Glu Thr Ile Val Gln Gln Val Tyr Tyr Asp
               180                 185                 190
   Leu Phe
<210> 58
   <211> 585
   <212> DNA
   <213> Chlamydia trachomatis
<400> 58
   atgctgaaaa tgttttggtt gaatagcctc gttttcttct cgttactact atcagcctgc 60
   ggctatacag tgctctcccc ccactatgta gaaaagaaat tctcgctttc cgaaggcatc 120
   tatgtctgcc ctatcgaagg agattcatta ggagatctcg tatcctctct ttcttacgaa 180
   ttagaaaagc gaggactcca cacacgatct caaggaacct cttctggtta tgtactcaaa 240
   gtctctcttt tcaatgagac tgatgaaaat attggattcg catacactcc ccaaaaacct 300
   gatgaaaaac ctgtaaaaca cttcattgtc tctaatgaag ggcgcttagc gttatcagca 360
   aaagtccaac taatcaaaaa ccgcacacaa gaaatattag tggagaaatg cctgagaaaa 420
   tcggttactt ttgattttca acctgacctc ggaaccgcga atgctcatca gctagctctc 480
   ggacaatttg aaatgcataa tgaagcaata aaaagcgctt ctcgtatatt gtattcgcaa 540
   ttagcagaga ctattgtaca acaggtatac tatgaccttt tctga                585
<210> 59
   <211> 542
   <212> PRT
   <213> Chlamydia trachomatis
<400> 59
   Met Phe Lys Leu Val Ser Tyr Ile Ile Leu Ser Trp Val Leu Val Cys
   1 5 10 15
   Leu Ala Gln Pro Asp Val Ser Val Val Ala Ser Val Val Ser Cys Ile
   20 25 30
   Cys Gly Tyr Ser Leu Leu Trp Ala Gly Leu Phe Ala Leu Val Glu Gln
   35 40 45
   Leu Ser Trp Lys Lys Val Trp Cys Ile Ala Phe Ile Trp Thr Trp Thr
   50 55 60
   Val Glu Gly Ala His Phe Ser Trp Met Leu Glu Asp Leu Tyr Val Gly
   65 70 75 80
   Thr Ser Ile Tyr Phe Val Trp Gly Ile Leu Leu Ser Tyr Leu Ala Thr
   85 90 95
   Leu Phe Ala Ser Phe Ser Cys Leu Val Val Trp Cys Cys Arg Lys Gln
   100 105 110
   Tyr Arg Gly Ala Leu Val Trp Leu Pro Gly Val Trp Val Ala Ile Glu
   115 120 125
   Ala Ile Arg Tyr Tyr Gly Leu Leu Ser Gly Val Ser Phe Asp Phe Ile
   130 135 140
   Gly Trp Pro Leu Thr Ala Thr Ala Tyr Gly Arg Gln Phe Gly Ser Phe
   145 150 155 160
   Phe Gly Trp Ala Gly Gln Ser Phe Leu Val Ile Ala Ala Asn Ile Cys
   165 170 175
   Cys Phe Ala Val Cys Leu Leu Lys His Ser Phe Ser Lys Gly Leu Trp
   180 185 190
   Leu Thr Leu Cys Ala Phe Pro Tyr Leu Leu Gly Gly Ala His Tyr Glu
               195                 200                 205
   Tyr Leu Lys Lys His Phe Ser Asp Ser Glu Val Leu Arg Val Ala Ile
       210                 215                 220
   Val Gln Pro Gly Tyr Ser Pro His Met His Ala Gly Arg Thr Ala Ser
   225                 230                 235                 240
   Ala Ile Trp Arg Gly Leu Val Ser Leu Cys Gln Thr Ile Gln Thr Pro
                   245                 250                 255
   Val Asp Val Ile Val Phe Pro Glu Val Ser Val Pro Phe Gly Leu His
               260                 265                 270
   Arg Gln Ala Tyr Thr Leu His Glu Asn Gln Pro Val Leu Glu Ser Leu
           275                 280                 285
   Leu Pro Asn Lys Ser Trp Gly Glu Phe Phe Thr Asn Leu Asp Trp Ile
       290                 295                 300
   Gln Ala Ile Ala Glu Arg Tyr Gln Cys Thr Val Ile Met Gly Met Glu
   305                 310                 315                 320
   Arg Trp Glu Asn Lys Gly Gly Ile Leu His Leu Tyr Asn Ala Ala Glu
                   325                 330                 335
   Cys Val Ser Arg Glu Gly Glu Ile Thr Ser Tyr Asp Lys Arg Ile Leu
               340                 345                 350
   Val Pro Gly Gly Glu Tyr Ile Pro Gly Gly Lys Ile Gly Phe Ser Leu
           355                 360                 365
   Cys Gln Thr Phe Phe Pro Glu Phe Ala Leu Pro Phe Gln Arg Leu Pro
       370                 375                 380
   Gly Glu Phe Ser Gly Val Val Asn Ile Thr Glu Arg Ile Lys Ala Gly
   385                 390                 395                 400
   Ile Ser Ile Cys Tyr Glu Glu Thr Phe Gly Tyr Ala Ile Arg Pro Tyr
                   405                 410                 415
   Lys Arg Gln Gln Ala Asp Ile Leu Val Asn Leu Thr Asn Asp Gly Trp
               420                 425                 430
   Tyr Pro Arg Ser Arg Leu Pro Leu Val His Phe Tyr His Gly Met Leu
           435                 440                 445
   Arg Asn Gln Glu Leu Gly Ile Pro Cys Ile Arg Ala Cys Arg Thr Gly
       450                 455                 460
   Val Ser Ala Ala Val Asp Ser Leu Gly Arg Ile Val Gly Ile Leu Pro
   465                 470                 475                 480
   Trp Glu Ser Arg Thr Cys Pro Val Ser Thr Gly Val Leu Gln Val Ser
                   485                 490                 495
   Val Pro Leu Tyr Ser Tyr His Thr Val Tyr Ala Arg Leu Gly Asp Ala
               500                 505                 510
   Pro Leu Leu Leu Ile Ala Val Cys Ser Val Ile Gly Ala Ile Ala Tyr
           515                 520                 525
   Phe Tyr Arg Lys Lys Lys Glu Thr Pro Pro Gln Thr Phe Phe
       530                 535                 540
<210> 60
   <211> 1629
   <212> DNA
   <213> Chlamydia trachomatis
<400> 60
   gtgtttaaac ttgtgtcata catcatcctt tcttgggtgc tggtctgttt ggctcagccg 60
   gatgtaagtg ttgtagcttc tgttgttagt tgtatttgcg gttacagctt actttgggct 120
   gggctttttg ctttagtaga gcaattatct tggaagaaag tttggtgcat cgcttttatt 180
   tggacttgga ctgtcgaagg cgctcatttc tcttggatgc ttgaagatct ttatgtaggg 240
   acaagcatct attttgtttg gggtatactg ctttcttatc tcgccaccct atttgctagt 300
   ttttcttgtt tggttgtgtg gtgttgtcgc aagcaatata ggggagctct tgtttggctt 360
   ccaggggttt gggtggcgat agaagcaata cgctattatg ggttgctttc aggagtttct 420
   tttgatttta ttggctggcc tcttacagcg acagcctatg gccggcaatt cggcagcttt 480
   tttggatggg ctggacaaag ctttctagtt attgctgcca atatatgctg ttttgcagta 540
   tgtttattaa aacactcttt ttccaaaggt ttgtggttga cgttgtgcgc gttcccttat 600
   ctgttaggcg gagcgcatta cgaataccta aagaagcatt tttccgactc tgaagtgctt 660
   cgagttgcca tcgtgcagcc tggatatagt cctcatatgc atgcagggag gacggctagt 720
   gctatttgga gaggtttggt ttctttgtgc cagactattc aaactcctgt agatgtgatc 780
   gttttcccag aagtaagtgt tccttttggc ttacatagac aagcctatac tcttcatgaa 840
   aatcagcctg tattagaaag tttgcttcct aacaaatctt ggggcgagtt tttcacaaat 900
   ttggattgga tccaagcgat agctgaacgt tatcaatgca ccgttatcat gggaatggaa 960
   cgatgggaaa ataaaggggg aatactgcat ttgtataatg ctgctgaatg cgtatcgcga 1020
   gaaggggaaa taactagcta tgataagcgg attcttgttc ctggaggtga gtacatccct 1080
   ggagggaaaa taggtttttc cttgtgtcaa acctttttcc cagaatttgc tcttcccttt 1140
   caacgtttgc caggagagtt ttctggagtt gtgaatataa cagagcgaat aaaagctggg 1200
   atctctattt gttatgagga gacatttggg tatgcaattc gcccttacaa aaggcaacaa 1260
   gccgatattt tagtaaatct tactaatgac ggttggtatc cgcgttcaag gctgcctcta 1320
   gtacattttt atcatggcat gttacgtaat caagagttgg gtataccttg tattcgcgcc 1380
   tgtcgcacag gagtttctgc tgcagtggat tctttgggta gaattgtcgg catacttccc 1440
   tgggaatcga gaacttgccc agtttctaca ggagtactcc aagtttccgt ccctctttac 1500
   agttatcata ctgtatatgc aaggctgggt gatgctcctc tgttactgat tgcagtttgt 1560
   tcggttatcg gagcgattgc ctatttttat aggaaaaaga aagagacccc accacaaaca 1620
   tttttttga                                                        1629
<210> 61
   <211> 553
   <212> PRT
   <213> Chlamydia trachomatis
<400> 61
   Met Ser Ser Asn Lys His Ala Ser Leu Cys Gln Lys Thr Pro Ser Leu
   1 5 10 15
   Cys Arg Glu Leu Gln Lys Ala Pro Ala Leu Leu Leu Thr Glu Asp Ile
   20 25 30
   Arg Phe Lys Ala Leu Leu Asn Glu Arg Ile Asp Ser Val Ala Glu Leu
   35 40 45
   Phe Pro Cys Thr Tyr Asn Ser Pro Tyr Tyr Lys Phe Ile Ser Lys Ser
   50 55 60
   Asp Leu Ser Ala Glu Thr Ser Pro Leu Lys Val Gly Val Met Leu Ser
   65 70 75 80
   Gly Gly Pro Ala Pro Gly Gly His Asn Val Ile Leu Gly Leu Leu His
   85 90 95
   Ser Ile Lys Lys Leu His Pro Asn Ser Gln Leu Leu Gly Phe Ile Arg
   100 105 110
   Asn Gly Glu Gly Leu Leu Asn Asn Asn Thr Val Glu Ile Thr Asp Glu
   115 120 125
   Phe Ile Glu Glu Phe Arg Asn Ser Gly Gly Phe Asn Cys Ile Gly Thr
   130 135 140
   Gly Arg Thr Asn Ile Ile Thr Glu Glu Asn Lys Ala Arg Cys Leu Gln
   145 150 155 160
   Thr Ala Asn Glu Leu Asp Leu Asp Gly Leu Val Ile Ile Gly Gly Asp
   165 170 175
   Gly Ser Asn Thr Ala Thr Ala Ile Leu Ala Glu Tyr Phe Ala Lys His
   180 185 190
   Gln Ala Lys Thr Val Leu Val Gly Val Pro Lys Thr Ile Asp Gly Asp
   195 200 205
   Leu Gln His Leu Phe Leu Asp Leu Thr Phe Gly Phe Asp Thr Ala Thr
   210 215 220
   Lys Phe Tyr Ser Ser Ile Ile Ser Asn Ile Ser Arg Asp Ala Leu Ser
   225 230 235 240
   Cys Lys Gly His Tyr His Phe Ile Lys Leu Met Gly Arg Ser Ser Ser
   245 250 255
   His Ile Thr Leu Glu Cys Ala Leu Gln Thr His Pro Asn Ile Ala Leu
   260 265 270
   Ile Gly Glu Glu Ile Ala Glu Lys Ser Ile Ser Leu Glu Thr Leu Ile
   275 280 285
   His Asp Ile Cys Glu Thr Ile Ala Asp Arg Ala Ala Met Gly Lys Tyr
   290 295 300
   His Gly Val Ile Leu Ile Pro Glu Gly Val Ile Glu Phe Ile Pro Glu
   305 310 315 320
   Ile Gln Ser Leu Val Lys Glu Ile Glu Ser Ile Pro Glu Gln Glu Asn
   325 330 335
   Leu Tyr Gln Ala Leu Ser Leu Ser Ser Gln Gln Leu Leu Cys Gln Phe
   340 345 350
   Pro Glu Asp Ile Cys His Gln Leu Leu Tyr Asn Arg Asp Ala His Gly
   355 360 365
   Asn Val Tyr Val Ser Lys Ile Ser Val Asp Lys Leu Leu Ile His Leu
   370 375 380
   Val Arg Gln His Leu Glu Thr His Phe Arg Gln Val Pro Phe Asn Ala
   385 390 395 400
   Ile Ser His Phe Leu Gly Tyr Glu Gly Arg Ser Gly Thr Pro Thr His
   405 410 415
   Phe Asp Asn Val Tyr Ser Tyr Asn Leu Gly Tyr Gly Ala Gly Val Leu
   420 425 430
   Val Phe Asn Arg Cys Asn Gly Tyr Leu Ser Thr Ile Glu Gly Leu Thr
   435 440 445
   Ser Pro Ile Glu Lys Trp Arg Leu Arg Ala Leu Pro Ile Val Arg Met
   450 455 460
   Leu Thr Thr Lys Gln Gly Lys Asp Ser Lys His Tyr Pro Leu Ile Lys
   465 470 475 480
   Lys Arg Leu Val Asp Ile Ala Ser Pro Val Phe Asn Lys Phe Ser Leu
   485 490 495
   Tyr Arg Lys Ile Trp Ala Leu Glu Asp Ser Tyr Arg Phe Val Gly Pro
   500 505 510
   Leu Gln Ile His Ser Pro Glu Asp Ala His Ser Asp Asp Phe Pro Pro
   515 520 525
   Leu Ile Leu Phe Leu Asn His Asn Glu Trp Gln Lys Arg Cys Ser Ile
   530 535 540
   Cys Leu Glu Ile Pro Asp Gln Asp Tyr
   545 550
<210> 62
   <211> 1662
   <212> DNA
   <213> Chlamydia trachomatis
<400> 62
   atgtcgtcga ataaacatgc ttctctttgt caaaagacgc cttctttgtg tcgggagctt 60
   caaaaagctc ctgctcttct attaacagaa gacataaggt ttaaagctct tcttaatgaa 120
   cgcattgact ctgttgcaga actatttcca tgcacttata actctcccta ctacaaattt 180
   atttcgaagt ccgatctttc cgctgagacc tctcccctta aagtgggcgt tatgctttct 240
   ggaggcccag ctcctggtgg gcacaatgtc atcttaggat tgctacacag tattaaaaag 300
   ctccatccga atagtcagct tttaggattt attcgcaatg gagaaggact tctcaataat 360
   aatactgtag aaatcacaga tgaattcatt gaagagtttc gtaactctgg aggctttaat 420
   tgcataggaa caggtcgcac taatatcata accgaagaaa ataaagcgcg ctgtttacaa 480
   acagcaaatg aactcgattt agatggatta gtgattattg gaggcgatgg ttcgaataca 540
   gccacggcga ttcttgctga atattttgct aagcatcaag caaaaacggt attagttggt 600
   gttcccaaaa ctattgatgg agatttgcag cacctatttt tagacctcac atttgggttt 660
   gatactgcta ctaaatttta ttcatccatc atcagcaaca tttctagaga cgcattatcg 720
   tgtaaaggcc actatcattt tattaaacta atgggccggt cttcttctca tatcacgcta 780
   gaatgcgcac tacagactca cccaaatatt gctcttatag gcgaagagat tgcagaaaaa 840
   agcatctcct tagaaacatt aatccatgat atttgtgaaa caatagcaga tcgagctgct 900
   atggggaaat accatggcgt tattctcatc cctgaaggag tcattgagtt tattcctgaa 960
   atacagtctc tggttaaaga aattgaatcc attccagagc aggagaatct ttaccaagct 1020
   ttatccttat cttctcagca acttttatgc caatttccgg aagatatttg ccatcagctc 1080
   ttgtataata gagatgctca tggcaacgtc tatgtatcaa aaattagtgt tgataaactt 1140
   ctgattcatc tagttcgtca acatttagaa acacatttta gacaagttcc cttcaatgca 1200
   atctcccatt ttttaggtta tgaagggcgt tcaggaactc ctacacattt tgataatgtg 1260
   tatagctata acttaggata tggtgctggg gttctcgttt ttaaccgctg taatgggtat 1320
   ttatccacga tcgaaggtct aactagccct attgaaaaat ggcgattgcg cgctttaccc 1380
   attgttcgaa tgttgacgac caagcagggg aaagacagta aacattatcc tctgataaaa 1440
   aaaagattgg tagatattgc tagtcctgtt tttaataagt tctcactgta tcggaaaatc 1500
   tgggctttag aagactccta tcgctttgta gggccattac aaatacattc tccggaggat 1560
   gctcattctg atgattttcc tcctttaatt ttgtttttga atcataatga atggcaaaaa 1620
   cgctgttcta tttgtttaga aatccccgat caggattatt aa 1662
<210> 63
   <211> 167
   <212> PRT
   <213> Chlamydia trachomatis
<400> 63
   Met Ile Cys Cys Asp Lys Val Leu Ser Ser Val Gln Ser Met Pro Val
    1               5                  10                  15
   Ile Asp Lys Cys Ser Val Thr Lys Cys Leu Gln Thr Ala Lys Gln Ala
               20                  25                  30
   Ala Val Leu Ala Leu Ser Leu Phe Ala Val Phe Ala Ser Gly Ser Leu
           35                  40                  45
   Ser Ile Leu Ser Ala Ala Val Leu Phe Ser Gly Thr Ala Ala Val Leu
       50                  55                  60
   Pro Tyr Leu Leu Ile Leu Thr Thr Ala Leu Leu Gly Phe Val Cys Ala
   65                  70                  75                  80
   Val Ile Val Leu Leu Arg Asn Leu Ser Ala Val Val Gln Ser Cys Lys
                   85                  90                  95
   Lys Arg Ser Pro Glu Glu Ile Glu Gly Ala Ala Arg Pro Ser Asp Gln
               100                 105                 110
   Gln Glu Ser Gly Gly Arg Leu Ser Glu Glu Ser Ala Ser Pro Gln Ala
           115                 120                 125
   Ser Pro Thr Ser Ser Thr Phe Gly Leu Glu Ser Ala Leu Arg Ser Ile
       130                 135                 140
   Gly Asp Ser Val Ser Gly Ala Phe Asp Asp Ile Asn Lys Asp Asn Ser
   145                 150                 155                 160
   Arg Ser Arg Ser His Ser Phe
                   165
<210> 64
   <211> 439
   <212> DNA
   <213> Chlamydia trachomatis
<400> 64
   atgatctgct gtgacaaagt cttgtcgagc gtacaatcaa tgcctgttat agataaatgc 60
   tctgtaacga aatgcttaca aacggctaag caagcagctg ttcttgcgtt gtctttgttt 120
   gcggtgtttg cttcaggaag tttatccata ttatcagcgg cggtactgtt tagtggcact 180
   gctgctgttc ttccatatct gctgatatta acaacagctc ttctaggatt tgtttgtgct 240
   gttattgtgc ttttaagaaa tttatcagca gttgttcaga gttgtaaaaa gagatcacct 300
   gaagaaattg aaggggctgc tcgtccctct gatcagcagg aatcaggagg acgtttgtcc 360
   gaggagagcg cttcaccaca agcatctcct acttcgtcta cttttggtct tgaatccgct 420
   ttgcgctcaa taggagata                                              439
<210> 65
   <211> 89
   <212> PRT
   <213> Chlamydia trachomatis
<400> 65
   Met Ser Lys Lys Cys Ala Leu Thr Gly Arg Lys Pro Arg Arg Gly Tyr
   1 5 10 15
   Ser Tyr Ala Ile Arg Gly Ile Ser Lys Lys Lys Lys Gly Ile Gly Leu
   20 25 30
   Lys Val Thr Gly Arg Thr Lys Arg Arg Phe Phe Pro Asn Met Met Thr
   35 40 45
   Lys Arg Leu Trp Ser Thr Glu Glu Asn Arg Phe Leu Lys Leu Lys Ile
   50 55 60
   Ser Ala Ala Ala Leu Arg Leu Val Asp Lys Leu Gly Leu Asp Gln Val
   65 70 75 80
   Val Ala Arg Ala Lys Ser Lys Gly Phe
   85
<210> 66
   <211> 270
   <212> DNA
   <213> Chlamydia trachomatis
<400> 66
   atgtcgaaaa aatgtgcgct tacaggaaga aagcctcgtc gcggttatag ctatgctatc 60
   cgagggattt ctaaaaagaa aaaagggatc ggtttgaaag ttacaggaag aacaaaacgt 120
   cgattcttcc ctaatatgat gactaagaga ctatggtcta ctgaggaaaa tcgcttcctc 180
   aaactcaaaa tttctgcagc agctttacgc cttgttgata aactagggtt agatcaggtt 240
   gttgctagag ctaaaagcaa gggtttttag 270
<210> 67
   <211> 157
   <212> PRT
   <213> Chlamydia trachomatis
<400> 67
   Met Leu Phe Gly Tyr Leu Val Gly Phe Leu Ala Ala Asp Pro Glu Glu
   1 5 10 15
   Arg Met Thr Ser Gly Gly Lys Arg Val Val Val Leu Arg Leu Gly Val
   20 25 30
   Lys Ser Arg Val Gly Ser Lys Asp Glu Thr Val Trp Cys Arg Cys Asn
   35 40 45
   Ile Trp Asn Asn Arg Tyr Asp Lys Met Leu Pro Tyr Leu Lys Lys Gly
   50 55 60
   Ser Ser Val Ile Val Ala Gly Glu Leu Ser Leu Glu Ser Tyr Val Gly
   65 70 75 80
   Arg Asp Gly Ser Pro Gln Ala Ser Ile Ser Val Ser Val Asp Thr Leu
   85 90 95
   Lys Phe Asn Ser Gly Ser Ser Arg Pro Asp Ala Arg Gly Ser Asp Glu
   100 105 110
   Gly Arg Gln Arg Ala Asn Asp Asn Val Ser Ile Gly Phe Asp Gly Glu
   115 120 125
   Ser Leu Asp Thr Asp Ser Ala Leu Asp Lys Glu Val Tyr Ala Gly Phe
   130 135 140
   Gly Glu Asp Gln Gln Tyr Ala Ser Glu Asp Val Pro Phe
   145 150 155
<210> 68
   <211> 474
   <212> DNA
   <213> Chlamydia trachomatis
<400> 68
   atgttgttcg gatatttggt aggatttcta gctgccgatc ctgaagaaag aatgacatcc 60
   ggaggtaaac gggttgttgt tttacgtttg ggtgtaaaat ctcgtgtagg atctaaagat 120
   gaaacagtgt ggtgcagatg caatatctgg aacaaccgtt atgataagat gcttccttat 180
   ttgaagaaag gttcttcagt cattgttgct ggagagcttt ctttagaaag ctatgtaggt 240
   agagacggtt ctccacaagc ttctatttct gtaagcgtag atacattaaa atttaattcc 300
   ggatcttctc gtcctgatgc tagaggttca gatgaaggtc gtcagagagc taatgataat 360
   gtctctattg gatttgatgg agaaagttta gatacagact ctgcgcttga taaggaagtc 420
   tatgcagggt ttggagaaga ccaacagtat gctagtgagg atgttccttt ttag       474
<210> 69
   <211> 247
   <212> PRT
   <213> Chlamydia trachomatis
<400> 69
   Met Lys Lys Gln Glu Lys Met His Pro Gln Asn Leu Leu Lys Val Phe
   1               5                   10                  15
   Ile Phe Phe Leu Ala Phe Phe Ile Ser Tyr Pro Ser Ala Glu Ala His
               20                  25                  30
   Ser Pro Leu Gln Ser Ser Ile Gln Glu Lys Ile Leu Thr Ala Arg Pro
           35                  40                  45
   Gly Asp Tyr Ala Val Leu Ser Arg Gly Ser Gln Lys Phe Phe Phe Leu
       50                  55                  60
   Ile Arg Gln Ser Ser Ser Glu Ala Thr Trp Val Glu Met Ser Glu Phe
   65                  70                  75                  80
   Ala Ser Leu Thr Gln Gln Glu Lys Lys Leu Val Glu Gln Ser Ser Trp
                   85                  90                  95
   Lys Asn Ala Phe His Gln Leu Gln Ser Ser Lys Lys Val Tyr Leu Leu
               100                 105                 110
   Arg Ile Ser Lys Asn Pro Leu Met Ile Phe Val Leu Lys Asn Ala Gln
           115                 120                 125
   Trp Met Pro Leu Ser Glu Lys Asp Pro Leu Pro Phe Phe Val Lys Ile
       130                 135                 140
   Leu Arg Leu Pro Leu Ser Pro Ala Pro Ser His Leu Ile Lys Tyr Lys
   145                 150                 155                 160
   Gly Lys Glu Arg Thr Pro Trp Ser Pro Arg Thr Ser Leu Asn Gly Glu
                   165                 170                 175
   Leu Ile Thr Leu Pro Ser Ser Ala Trp Ile Ser Val Trp Pro Lys Asp
               180                 185                 190
   Ser Ser Pro Leu Ser Glu Lys Asn Ile Leu Ile Tyr Phe Ser Asn Asn
           195                 200                 205
   Glu Arg Leu Ala Phe Pro Leu Trp Thr Ser Ile Asp Thr Pro Thr Gly
       210                 215                 220
   Thr Val Ile Ile Lys Thr Ile Glu Met Gly His Gln Ala Ala Ser Ser
   225                 230                 235                 240
   Tyr Pro Ala Leu Pro Asn Phe
                   245
<210> 70
   <211> 744
   <212> DNA
   <213> Chlamydia trachomatis
<400> 70
   atgaaaaagc aagaaaaaat gcaccctcaa aaccttctta aagtttttat ttttttcttg 60
   gcatttttca tatcctatcc ctcggctgaa gcccattctc ctctccaatc atcaatccaa 120
   gaaaaaattc taactgcccg ccccggagac tatgccgtct taagccgagg atctcaaaaa 180
   tttttctttt taattcgcca aagttcttcg gaagcgactt gggtcgaaat gtctgaattt 240
   gcctccctaa cacagcaaga aaaaaaatta gtagaacagt cttcctggaa gaatgccttc 300
   catcaactcc aatcttcaaa aaaagtgtac ttgttacgaa tttccaaaaa tcctcttatg 360
   atttttgttc tcaaaaatgc gcaatggatg cctctctcag aaaaagatcc tttgcctttc 420
   tttgtaaaaa tccttcgact ccctttatct ccagccccct ctcacttaat taaatacaaa 480
   gggaaagaac gcaccccctg gtctccgcga acatctttga atggagaact cataaccctt 540
   ccttccagtg cttggatttc tgtttggcca aaagattctt ctcctctatc agaaaaaaat 600
   attctcatat atttttctaa caatgaacgt ttagcgtttc ctctatggac tagtattgat 660
   actcctacag ggacagtgat tattaagact attgaaatgg ggcaccaagc cgcctcctcc 720
   tatccagctc ttcccaattt ctag 744
<210> 71
   <211> 456
   <212> DNA
   <213> Chlamydia trachomatis
<400> 71
   aatatgagca ctgtacccgt tgttcaagga gctggatctt ccaattcggc acaggatatt 60
   tccactagac cattaacact gaaagagcgt atatcgaatc ttctatcttc cactgcattt 120
   aaggtgggat tagtggtgat aggactactt ttagtgattg ctactttgat attcctagtt 180
   tcggcagctt cgtttgtaaa tgccatctat ctagtagcta ttcctgctat tttgggatgc 240
   gtgaatatct gcgtaggaat tttatccatg gaaggacact gttctccgga gagatggatc 300
   ttatgtaaga aggtattaaa gacttcagaa gatatcatcg atgatgggca gataaacaac 360
   tctaataaag tgtttactga tgagaggttg aatgccatag gtggggtagt ggaatctcta 420
   tctagaagaa atagtctggt ggatcagacc caatga 456
<210> 72
   <211> 151
   <212> PRT
   <213> Chlamydia trachomatis
<400> 72
   Asn Met Ser Thr Val Pro Val Val Gln Gly Ala Gly Ser Ser Asn Ser
   1               5                   10                  15
   Ala Gln Asp Ile Ser Thr Arg Pro Leu Thr Leu Lys Glu Arg Ile Ser
               20                  25                  30
   Asn Leu Leu Ser Ser Thr Ala Phe Lys Val Gly Leu Val Val Ile Gly
           35                  40                  45
   Leu Leu Leu Val Ile Ala Thr Leu Ile Phe Leu Val Ser Ala Ala Ser
       50                  55                  60
   Phe Val Asn Ala Ile Tyr Leu Val Ala Ile Pro Ala Ile Leu Gly Cys
   65                  70                  75                  80
   Val Asn Ile Cys Val Gly Ile Leu Ser Met Glu Gly His Cys Ser Pro
                   85                  90                  95
   Glu Arg Trp Ile Leu Cys Lys Lys Val Leu Lys Thr Ser Glu Asp Ile
               100                 105                 110
   Ile Asp Asp Gly Gln Ile Asn Asn Ser Asn Lys Val Phe Thr Asp Glu
           115                 120                 125
   Arg Leu Asn Ala Ile Gly Gly Val Val Glu Ser Leu Ser Arg Arg Asn
       130                 135                 140
   Ser Leu Val Asp Gln Thr Gln
   145                 150
<210> 73
   <211> 354
   <212> DNA
   <213> Chlamydia trachomatis
<400> 73
   gggctagttt cttttattgt taaaagaatt gcttttatcg ataaaagaaa cttcaagagc 60
   ccttttctag aaaggagtct ggaagttatg aaaaaaactg ctttactcgc tgctttatgt 120
   agtgttgttt ctttaagtag ttgttgtcgt atcgttgact gttgcttcga agatccatgc 180
   gcacctatcc aatgttcacc ttgtgaatct aagaagaaag acgtagacgg tggttgcaac 240
   tcttgtaacg ggtatgtccc agcttgcaaa ccttgcggag gggatacgca ccaagatgct 300
   aaacatggcc ctcaagctag aggaattcca gttgacggca aatgcagaca atag       354
<210> 74
   <211> 117
   <212> PRT
   <213> Chlamydia trachomatis
<400> 74
   Gly Leu Val Ser Phe Ile Val Lys Arg Ile Ala Phe Ile Asp Lys Arg
    1               5                  10                  15
   Asn Phe Lys Ser Pro Phe Leu Glu Arg Ser Leu Glu Val Met Lys Lys
               20                  25                  30
   Thr Ala Leu Leu Ala Ala Leu Cys Ser Val Val Ser Leu Ser Ser Cys
           35                  40                  45
   Cys Arg Ile Val Asp Cys Cys Phe Glu Asp Pro Cys Ala Pro Ile Gln
       50                  55                  60
   Cys Ser Pro Cys Glu Ser Lys Lys Lys Asp Val Asp Gly Gly Cys Asn
   65                  70                  75                  80
   Ser Cys Asn Gly Tyr Val Pro Ala Cys Lys Pro Cys Gly Gly Asp Thr
                   85                  90                  95
   His Gln Asp Ala Lys His Gly Pro Gln Ala Arg Gly Ile Pro Val Asp
               100                 105                 110
   Gly Lys Cys Arg Gln
           115
<210> 75
   <211> 1650
   <212> DNA
   <213> Chlamydia trachomatis
<400> 75
   gctagtaagg gagccccttt agtgtttaaa cttgtgtcat acatcatcct ttcttgggtg 60
   ctggtctgtt tggctcagcc ggatgtaagt gttgtagctt ctgttgttag ttgtatttgc 120
   ggttacagct tactttgggc tgggcttttt gctttagtag agcaattatc ttggaagaaa 180
   gtttggtgca tcgcttttat ttggacttgg actgtcgaag gcgctcattt ctcttggatg 240
   cttgaagatc tttatgtagg gacaagcatc tattttgttt ggggtatact gctttcttat 300
   ctcgccaccc tatttgctag tttttcttgt ttggttgtgt ggtgttgtcg caagcaatat 360
   aggggagctc ttgtttggct tccaggggtt tgggtggcga tagaagcaat acgctattat 420
   gggttgcttt caggagtttc ttttgatttt attggctggc ctcttacagc gacagcctat 480
   ggccggcaat tcggcagctt ttttggatgg gctggacaaa gctttctagt tattgctgcc 540
   aatatatgct gttttgcagt atgtttatta aaacactctt tttccaaagg tttgtggttg 600
   acgttgtgcg cgttccctta tctgttaggc ggagcgcatt acgaatacct aaagaagcat 660
   ttttccgact ctgaagtgct tcgagttgcc atcgtgcagc ctggatatag tcctcatatg 720
   catgcaggga ggacggctag tgctatttgg agaggtttgg tttctttgtg ccagactatt 780
   caaactcctg tagatgtgat cgttttccca gaagtaagtg ttccttttgg cttacataga 840
   caagcctata ctcttcatga aaatcagcct gtattagaaa gtttgcttcc taacaaatct 900
   tggggcgagt ttttcacaaa tttggattgg atccaagcga tagctgaacg ttatcaatgc 960
   accgttatca tgggaatgga acgatgggaa aataaagggg gaatactgca tttgtataat 1020
   gctgctgaat gcgtatcgcg agaaggggaa ataactagct atgataagcg gattcttgtt 1080
   cctggaggtg agtacatccc tggagggaaa ataggttttt ccttgtgtca aacctttttc 1140
   ccagaatttg ctcttccctt tcaacgtttg ccaggagagt tttctggagt tgtgaatata 1200
   acagagcgaa taaaagctgg gatctctatt tgttatgagg agacatttgg gtatgcaatt 1260
   cgcccttaca aaaggcaaca agccgatatt ttagtaaatc ttactaatga cggttggtat 1320
   ccgcgttcaa ggctgcctct agtacatttt tatcatggca tgttacgtaa tcaagagttg 1380
   ggtatacctt gtattcgcgc ctgtcgcaca ggagtttctg ctgcagtgga ttctttgggt 1440
   agaattgtcg gcatacttcc ctgggaatcg agaacttgcc cagtttctac aggagtactc 1500
   caagtttccg tccctcttta cagttatcat actgtatatg caaggctggg tgatgctcct 1560
   ctgttactga ttgcagtttg ttcggttatc ggagcgattg cctattttta taggaaaaag 1620
   aaagagaccc caccacaaac atttttttga                                  1650
<210> 76
   <211> 549
   <212> PRT
   <213> Chlamydia trachomatis
<400> 76
   Ala Ser Lys Gly Ala Pro Leu Val Phe Lys Leu Val Ser Tyr Ile Ile
   1               5                   10                  15
   Leu Ser Trp Val Leu Val Cys Leu Ala Gln Pro Asp Val Ser Val Val
               20                  25                  30
   Ala Ser Val Val Ser Cys Ile Cys Gly Tyr Ser Leu Leu Trp Ala Gly
           35                  40                  45
   Leu Phe Ala Leu Val Glu Gln Leu Ser Trp Lys Lys Val Trp Cys Ile
       50                  55                  60
   Ala Phe Ile Trp Thr Trp Thr Val Glu Gly Ala His Phe Ser Trp Met
   65                  70                  75                  80
   Leu Glu Asp Leu Tyr Val Gly Thr Ser Ile Tyr Phe Val Trp Gly Ile
                   85                  90                  95
   Leu Leu Ser Tyr Leu Ala Thr Leu Phe Ala Ser Phe Ser Cys Leu Val
               100                 105                 110
   Val Trp Cys Cys Arg Lys Gln Tyr Arg Gly Ala Leu Val Trp Leu Pro
           115                 120                 125
   Gly Val Trp Val Ala Ile Glu Ala Ile Arg Tyr Tyr Gly Leu Leu Ser
       130                 135                 140
   Gly Val Ser Phe Asp Phe Ile Gly Trp Pro Leu Thr Ala Thr Ala Tyr
   145                 150                 155                 160
   Gly Arg Gln Phe Gly Ser Phe Phe Gly Trp Ala Gly Gln Ser Phe Leu
                   165                 170                 175
   Val Ile Ala Ala Asn Ile Cys Cys Phe Ala Val Cys Leu Leu Lys His
               180                 185                 190
   Ser Phe Ser Lys Gly Leu Trp Leu Thr Leu Cys Ala Phe Pro Tyr Leu
           195                 200                 205
   Leu Gly Gly Ala His Tyr Glu Tyr Leu Lys Lys His Phe Ser Asp Ser
       210                 215                 220
   Glu Val Leu Arg Val Ala Ile Val Gln Pro Gly Tyr Ser Pro His Met
   225                 230                 235                 240
   His Ala Gly Arg Thr Ala Ser Ala Ile Trp Arg Gly Leu Val Ser Leu
                   245                 250                 255
   Cys Gln Thr Ile Gln Thr Pro Val Asp Val Ile Val Phe Pro Glu Val
               260                 265                 270
   Ser Val Pro Phe Gly Leu His Arg Gln Ala Tyr Thr Leu His Glu Asn
           275                 280                 285
   Gln Pro Val Leu Glu Ser Leu Leu Pro Asn Lys Ser Trp Gly Glu Phe
       290                 295                 300
   Phe Thr Asn Leu Asp Trp Ile Gln Ala Ile Ala Glu Arg Tyr Gln Cys
   305                 310                 315                 320
   Thr Val Ile Met Gly Met Glu Arg Trp Glu Asn Lys Gly Gly Ile Leu
                   325                 330                 335
   His Leu Tyr Asn Ala Ala Glu Cys Val Ser Arg Glu Gly Glu Ile Thr
               340                 345                 350
   Ser Tyr Asp Lys Arg Ile Leu Val Pro Gly Gly Glu Tyr Ile Pro Gly
           355                 360                 365
   Gly Lys Ile Gly Phe Ser Leu Cys Gln Thr Phe Phe Pro Glu Phe Ala
       370                 375                 380
   Leu Pro Phe Gln Arg Leu Pro Gly Glu Phe Ser Gly Val Val Asn Ile
   385                 390                 395                 400
   Thr Glu Arg Ile Lys Ala Gly Ile Ser Ile Cys Tyr Glu Glu Thr Phe
                   405                 410                 415
   Gly Tyr Ala Ile Arg Pro Tyr Lys Arg Gln Gln Ala Asp Ile Leu Val
               420                 425                 430
   Asn Leu Thr Asn Asp Gly Trp Tyr Pro Arg Ser Arg Leu Pro Leu Val
           435                 440                 445
   His Phe Tyr His Gly Met Leu Arg Asn Gln Glu Leu Gly Ile Pro Cys
       450                 455                 460
   Ile Arg Ala Cys Arg Thr Gly Val Ser Ala Ala Val Asp Ser Leu Gly
   465                 470                 475                 480
   Arg Ile Val Gly Ile Leu Pro Trp Glu Ser Arg Thr Cys Pro Val Ser
                   485                 490                 495
   Thr Gly Val Leu Gln Val Ser Val Pro Leu Tyr Ser Tyr His Thr Val
               500                 505                 510
   Tyr Ala Arg Leu Gly Asp Ala Pro Leu Leu Leu Ile Ala Val Cys Ser
           515                 520                 525
   Val Ile Gly Ala Ile Ala Tyr Phe Tyr Arg Lys Lys Lys Glu Thr Pro
       530                 535                 540
   Pro Gln Thr Phe Phe
   545
<210> 77
   <211> 645
   <212> DNA
   <213> Chlamydia trachomatis
<400> 77
   gaaaattgtt ataggatcag gagagaaacg tttcccatgc tggggagcat ttccttcaca 60
   acatataaag aaaacctcat gagaaagact atttttaaag cgtttaattt attattctcc 120
   cttctttttc tttcttcatg ctcttatcct tgcagagatt gggaatgcca tggttgcgac 180
   tccgcaagac ctcgtaaatc ctcttttgga ttcgtacctt tctactccga tgaagaaatt 240
   caacaagctt ttgttgaaga ttttgattcc aaagaagagc agctgtacaa aacgagcgca 300
   cagagtacct ctttccgaaa tatcactttc gctacagata gttattctat taaaggagag 360
   gataacctca cgattcttgc aagcttagtt cgtcatttgc ataaatctcc taaagctacg 420
   ctatatatag agggccatac agatgaacgt ggagctgcag cttataacct agctttagga 480
   gctcgtcgtg cgaatgctgt aaaacaatac ctcatcaaac agggaatcgc tgcagaccgc 540
   ttattcacta tttcttacgg aaaagaacat cctgttcatc caggccataa tgaattagct 600
   tggcaacaaa atcgtcgtac tgaatttaag atccatgctc gctaa                 645
<210> 78
   <211> 214
   <212> PRT
   <213> Chlamydia trachomatis
<400> 78
   Glu Asn Cys Tyr Arg Ile Arg Arg Glu Thr Phe Pro Met Leu Gly Ser
   1               5                   10                  15
   Ile Ser Phe Thr Thr Tyr Lys Glu Asn Leu Met Arg Lys Thr Ile Phe
               20                  25                  30
   Lys Ala Phe Asn Leu Leu Phe Ser Leu Leu Phe Leu Ser Ser Cys Ser
           35                  40                  45
   Tyr Pro Cys Arg Asp Trp Glu Cys His Gly Cys Asp Ser Ala Arg Pro
       50                  55                  60
   Arg Lys Ser Ser Phe Gly Phe Val Pro Phe Tyr Ser Asp Glu Glu Ile
   65                  70                  75                  80
   Gln Gln Ala Phe Val Glu Asp Phe Asp Ser Lys Glu Glu Gln Leu Tyr
                   85                  90                  95
   Lys Thr Ser Ala Gln Ser Thr Ser Phe Arg Asn Ile Thr Phe Ala Thr
               100                 105                 110
   Asp Ser Tyr Ser Ile Lys Gly Glu Asp Asn Leu Thr Ile Leu Ala Ser
           115                 120                 125
   Leu Val Arg His Leu His Lys Ser Pro Lys Ala Thr Leu Tyr Ile Glu
       130                 135                 140
   Gly His Thr Asp Glu Arg Gly Ala Ala Ala Tyr Asn Leu Ala Leu Gly
   145                 150                 155                 160
   Ala Arg Arg Ala Asn Ala Val Lys Gln Tyr Leu Ile Lys Gln Gly Ile
                   165                 170                 175
   Ala Ala Asp Arg Leu Phe Thr Ile Ser Tyr Gly Lys Glu His Pro Val
               180                 185                 190
   His Pro Gly His Asn Glu Leu Ala Trp Gln Gln Asn Arg Arg Thr Glu
           195                 200                 205
   Phe Lys Ile His Ala Arg
       210
<210> 79
   <211> 943
   <212> PRT
   <213> Neisseria gonorrhoeae
<400> 79
   Met Asn Lys Lys His Gly Phe Pro Leu Thr Leu Thr Ala Leu Ala Ile
   1               5                   10                  15
   Ala Thr Ala Phe Pro Ala Tyr Ala Ala Gln Ala Gly Ala Ala Ala Leu
               20                  25                  30
   Asp Ala Ala Gln Ser Gln Ser Leu Lys Glu Val Thr Val Arg Ala Ala
           35                  40                  45
   Lys Val Gly Arg Arg Ser Lys Glu Ala Thr Gly Leu Gly Lys Ile Val
       50                  55                  60
   Lys Thr Ser Glu Thr Leu Asn Lys Glu Gln Val Leu Gly Ile Arg Asp
   65                  70                  75                  80
   Leu Thr Arg Tyr Asp Pro Gly Val Ala Val Val Glu Gln Gly Asn Gly
                   85                  90                  95
   Ala Ser Gly Gly Tyr Ser Ile Arg Gly Val Asp Lys Asn Arg Val Ala
               100                 105                 110
   Val Ser Val Asp Gly Val Ala Gln Ile Gln Ala Phe Thr Val Gln Gly
           115                 120                 125
   Ser Leu Ser Gly Tyr Gly Gly Arg Gly Gly Ser Gly Ala Ile Asn Glu
       130                 135                 140
   Ile Glu Tyr Glu Asn Ile Ser Thr Val Glu Ile Asp Lys Gly Ala Gly
   145                 150                 155                 160
   Ser Ser Asp His Gly Ser Gly Ala Leu Gly Gly Ala Val Ala Phe Arg
                   165                 170                 175
   Thr Lys Glu Ala Ala Asp Leu Ile Ser Asp Gly Lys Ser Trp Gly Ile
               180                 185                 190
   Gln Ala Lys Thr Ala Tyr Gly Ser Lys Asn Arg Gln Phe Met Lys Ser
           195                 200                 205
   Leu Gly Ala Gly Phe Ser Lys Asp Gly Trp Glu Gly Leu Leu Ile Arg
       210                 215                 220
   Thr Glu Arg Gln Gly Arg Glu Thr Arg Pro His Gly Asp Ile Ala Asp
   225                 230                 235                 240
   Gly Val Glu Tyr Gly Ile Asp Arg Leu Asp Ala Phe Arg Gln Thr Tyr
                   245                 250                 255
   Asp Ile Lys Arg Lys Thr Thr Glu Pro Phe Phe Leu Val Glu Gly Glu
               260                 265                 270
   Asn Thr Leu Lys Pro Val Ala Lys Leu Ala Gly Tyr Gly Ile Tyr Leu
   275 280 285
   Asn Arg Gln Leu Asn Arg Trp Val Lys Glu Arg Ile Glu Gln Asn Gln
   290 295 300
   Pro Leu Ser Ala Glu Glu Glu Ala Gln Val Arg Glu Ala Gln Ala Arg
   305 310 315 320
   His Glu Asn Leu Ser Ala Gln Ala Tyr Thr Gly Gly Gly Arg Ile Leu
   325 330 335
   Pro Asp Pro Met Asp Tyr Arg Ser Gly Ser Trp Leu Ala Lys Leu Gly
   340 345 350
   Tyr Arg Phe Gly Gly Arg His Tyr Val Gly Gly Val Phe Glu Asp Thr
   355 360 365
   Lys Gln Arg Tyr Asp Ile Arg Asp Met Thr Glu Lys Gln Tyr Tyr Gly
   370 375 380
   Thr Asp Glu Ala Glu Lys Phe Arg Asp Lys Ser Gly Val Tyr Asp Gly
   385 390 395 400
   Asp Asp Phe Arg Asp Gly Leu Tyr Phe Val Pro Asn Ile Glu Glu Trp
   405 410 415
   Lys Gly Asp Lys Asn Leu Val Lys Gly Ile Gly Leu Lys Tyr Ser Arg
   420 425 430
   Thr Lys Phe Ile Asp Glu His His Arg Arg Arg Arg Met Gly Leu Leu
   435 440 445
   Tyr Arg Tyr Glu Asn Glu Lys Tyr Ser Asp Asn Trp Ala Asp Lys Ala
   450 455 460
   Val Leu Ser Phe Asp Lys Gln Gly Val Ala Thr Asp Asn Asn Thr Leu
   465 470 475 480
   Lys Leu Asn Cys Ala Val Tyr Pro Ala Val Asp Lys Ser Cys Arg Ala
   485 490 495
   Ser Ala Asp Lys Pro Tyr Ser Tyr Asp Ser Ser Asp Arg Phe His Tyr
   500 505 510
   Arg Glu Gln His Asn Val Leu Asn Ala Ser Phe Glu Lys Ser Leu Lys
   515 520 525
   Asn Lys Trp Thr Lys His His Leu Thr Leu Gly Phe Gly Tyr Asp Ala
   530 535 540
   Ser Lys Ala Val Ser Arg Pro Glu Gln Leu Ser His Asn Ala Ala Arg
   545 550 555 560
   Ile Ser Glu Ser Thr Gly Phe Asp Glu Lys Asn Gln Asp Lys Tyr Arg
   565 570 575
   Leu Gly Lys Pro Glu Val Val Glu Gly Ser Val Cys Gly Tyr Ile Glu
   580 585 590
   Thr Leu Arg Ser Arg Lys Cys Val Pro Arg Lys Ile Asn Gly Ser Asn
   595 600 605
   Ile His Ile Ser Leu Asn Asp Arg Phe Ser Ile Gly Lys Tyr Phe Asp
   610 615 620
   Phe Ser Leu Gly Gly Arg Tyr Asp Arg Lys Asn Phe Thr Thr Ser Glu
   625 630 635 640
   Glu Leu Val Arg Ser Gly Arg Tyr Ala Asp Arg Ser Trp Asn Ser Gly
   645 650 655
   Ile Val Phe Lys Pro Asn Arg His Phe Ser Val Ser Tyr Arg Ala Ser
   660 665 670
   Ser Gly Phe Arg Thr Pro Ser Phe Gln Glu Leu Phe Gly Ile Asp Ile
   675 680 685
   Tyr His Asp Tyr Pro Lys Gly Trp Gln Arg Pro Ala Leu Lys Ser Glu
   690 695 700
   Lys Ala Ala Asn Arg Glu Ile Gly Leu Gln Trp Lys Gly Asp Phe Gly
   705 710 715 720
   Phe Leu Glu Ile Ser Ser Phe Arg Asn Arg Tyr Thr Asp Met Ile Ala
   725 730 735
   Val Ala Asp Gln Lys Thr Lys Leu Pro Asp Ser Ala Gly Arg Leu Thr
   740 745 750
   Glu Ile Asp Ile Arg Asp Tyr Tyr Asn Ala Gln Asn Met Ser Leu Gln
   755 760 765
   Gly Ile Asn Ile Leu Gly Lys Ile Asp Trp Asn Gly Val Tyr Gly Lys
   770 775 780
   Leu Pro Glu Gly Leu Tyr Thr Thr Leu Ala Tyr Asn Arg Ile Lys Pro
   785 790 795 800
   Lys Ser Val Ser Asn Arg Pro Asp Leu Ser Leu Arg Ser Tyr Ala Leu
   805 810 815
   Asp Ala Val Gln Pro Ser Arg Tyr Val Leu Gly Phe Gly Tyr Asp Gln
   820 825 830
   Pro Glu Gly Lys Trp Gly Ala Asn Ile Met Leu Thr Tyr Ser Lys Gly
   835 840 845
   Lys Asn Pro Asp Glu Leu Ala Tyr Leu Ala Gly Asp Gln Lys Arg Tyr
   850 855 860
   Ser Ala Gly Arg Val Thr Ser Ser Trp Lys Thr Ala Asp Val Ser Ala
   865 870 875 880
   Tyr Leu Asn Leu Lys Lys Arg Leu Thr Leu Arg Ala Ala Ile Tyr Asn
   885 890 895
   Ile Gly Asn Tyr Arg Tyr Val Thr Trp Glu Ser Leu Arg Gln Thr Ala
   900 905 910
   Glu Ser Thr Ala Asn Arg His Gly Gly Asp Ser Asn Tyr Gly Arg Tyr
   915 920 925
   Ala Ala Pro Gly Arg Asn Phe Ser Leu Ala Leu Glu Met Lys Phe
   930 935 940
<210> 80
   <211> 3300
   <212> DNA
   <213> Neisseria gonorrhoeae
<400> 80
   ctcgggataa cggcatcaat ctttcgggaa atggttcgac taatcctcaa agtttcaaag 60
   ccgacaatct tcttgtaacg ggcggctttt acggcccgca ggcggcggaa ttgggcggca 120
   ctattttcaa taaggatggg aaatctcttg gtataactga agatattgaa aatgaagttg 180
   aaaatgaagc tgatgttggc gaacagttag aacctgaagt taaaccccaa ttcggcgtgg 240
   tattcggtgc gaagaaagat aataaagagg tggaaaaatg aataagaaac acggttttcc 300
   gctgactttg acggcgttgg ccattgcaac cgcttttccg gcttatgctg cccaagcggg 360
   ggcggcggca cttgatgcgg cgcaaagtca atcattgaaa gaggttaccg tccgtgccgc 420
   caaagtggga cggcgatcga aagaggcgac aggtttgggc aaaatcgtca aaacgtcgga 480
   aacgttgaac aaagaacagg tactcggtat ccgcgacctg acgcgctacg atccgggcgt 540
   ggcggttgtc gaacagggca acggcgcgag cggcggctac tcgatacgcg gcgtagataa 600
   aaaccgtgtg gcggtttcgg ttgacggcgt tgcccaaata caggcgttta ccgtgcaggg 660
   atcgttgagc ggatacggcg gacgcggcgg cagcggcgca atcaacgaaa tcgaatatga 720
   aaacatcagc acggtggaaa tcgacaaagg cgccggttcg tccgatcacg gcagcggcgc 780
   actcggcggc gcggtcgcct tccgcaccaa agaggcggca gacctgattt cagacggcaa 840
   aagctggggg atacaggcaa aaaccgctta cggcagtaaa aaccgccaat ttatgaagtc 900
   gctcggcgcg gggttcagca aagacggttg ggaagggctg ctaatccgaa ccgaacgcca 960
   agggcgggaa acgcgcccgc acggcgatat tgcggacggg gtggaatacg gcatagaccg 1020
   tttggacgcg ttccgccaga catacgatat taaacgcaag acaacagagc catttttctt 1080
   agtagagggc gagaatacac tcaagcccgt ggcaaaattg gcgggctacg ggatatattt 1140
   gaaccgccag ctcaaccgct gggtaaaaga acgtattgaa caaaatcagc ctttaagtgc 1200
   tgaagaagag gcgcaggtgc gggaggcgca ggcgcgccac gaaaacctgt ccgcccaagc 1260
   ctacacgggc ggcggcagga tattgcccga tccgatggat taccgcagcg gctcttggct 1320
   tgccaagctg ggctaccgct tcggcggcag gcattatgtc ggcggcgtgt ttgaggatac 1380
   caaacagcgt tacgacatcc gcgatatgac ggaaaaacag tattacggta cggacgaggc 1440
   ggaaaagttt agagacaaga gcggggtgta cgacggcgac gatttccgcg acggcttgta 1500
   ttttgtgccg aatatagaag agtggaaggg cgataaaaat ttggtcaagg gcataggttt 1560
   gaaatattcc cgcaccaaat ttattgacga acatcaccgc cgccgccgta tgggtttgct 1620
   gtaccgttat gaaaatgaga aatactcgga caactgggcg gataaggcgg tgttgtcgtt 1680
   tgacaaacag ggcgtggcaa ccgacaacaa cacgctgaag ctgaattgcg ccgtgtatcc 1740
   tgccgtggac aaatcctgcc gcgcgtcggc ggacaaaccg tattcctacg acagcagcga 1800
   ccgtttccac taccgcgaac agcacaatgt tttgaatgcc tcgtttgaga agtcgctgaa 1860
   aaacaaatgg acgaaacacc atctgacttt gggcttcggt tacgatgctt ccaaagcagt 1920
   atcccgccca gaacagcttt cccacaatgc ggcaaggatt tcggaatcca cgggattcga 1980
   tgaaaagaat caagataagt accgtttggg taagcccgaa gtcgtcgaag ggtcggtctg 2040
   cggctatatc gaaaccctgc gttcccgcaa atgcgtgcca agaaaaatca acggcagcaa 2100
   tatccacatt tctttgaacg accgtttttc aatcggcaaa tattttgatt tcagcttggg 2160
   cggcaggtac gaccggaaaa acttcaccac gtcggaagaa ctcgtccgca gcgggcggta 2220
   tgccgaccgt tcgtggaaca gcggcatcgt gttcaaaccg aaccggcatt tttccgtgtc 2280
   ttaccgcgcc tccagcggct tcagaacgcc ttccttccaa gaacttttcg ggatagacat 2340
   ttatcacgat tatccgaaag gctggcagcg tcccgccctg aaatcggaaa aggcagccaa 2400
   ccgggaaatc ggtttgcagt ggaagggcga tttcggcttt ttggaaatca gcagtttccg 2460
   caaccgttat accgatatga ttgccgttgc cgatcaaaaa accaaattgc cggattcagc 2520
   aggacgattg acagagattg atatacgcga ttattacaat gcccaaaata tgtcgcttca 2580
   aggcatcaac atcttgggga aaatcgactg gaacggcgta tacggcaaac tgcccgaagg 2640
   cctgtacacc acattggcgt acaaccgtat caaaccgaaa tcggtatcca accggccgga 2700
   cttgtccctc cgcagctatg ctttggatgc ggtacagccg tcgcgttatg ttttggggtt 2760
   cggatacgac cagcccgagg ggaaatgggg cgcaaacatt atgctgacct attccaaagg 2820
   gaaaaaccct gacgagcttg cttatctggc aggcgatcaa aaacgatatt cggcaggaag 2880
   ggttacgtct tcttggaaaa cggcagatgt ttccgcttat ctgaatctga aaaaacggct 2940
   gaccttgagg gcggctatct acaatatcgg caactaccgc tacgttactt gggaatcctt 3000
   gcgccagact gcggaaagca cggcaaaccg gcacggcggc gacagcaact atggaaggta 3060
   tgccgcaccg ggcaggaact tcagcctcgc gctcgaaatg aagttttaaa ggaaatgccg 3120
   tctggaagct tgatctgcac cccaaaagtc ggactaaacc gccaactgat taaggtgcag 3180
   gtttttttga ttcaatataa acaagatttc cgccgtcatt cccgcgcagg cgggaatccg 3240
   gacattcaat gctaaggcaa tttatcggaa atgactgaaa ctcaaaaaac cggattccca 3300
<210> 81
   <211> 728
   <212> PRT
   <213> Neisseria gonorrhoeae
<400> 81
   Met Arg Lys Leu Asn Tyr Tyr Gly Ile Ala Leu Leu Pro Leu Met Leu
   1               5                   10                  15
   Ala Ser Cys Gly Gly Asn Phe Gly Val Gln Pro Val Val Glu Ser Thr
               20                  25                  30
   Pro Thr Ala Tyr Pro Val Thr Phe Lys Ser Lys Asp Val Pro Thr Ser
           35                  40                  45
   Pro Pro Pro Ala Glu Pro Ser Val Glu Thr Thr Pro Val Asn Arg Pro
       50                  55                  60
   Ala Val Gly Ala Ala Met Arg Leu Leu Arg Arg Asn Thr Ala Phe His
   65                  70                  75                  80
   Arg Glu Asp Gly Thr Ala Ile Pro Asp Ser Lys Gln Ala Glu Glu Lys
                   85                  90                  95
   Leu Ser Phe Lys Glu Gly Asp Val Leu Phe Leu Tyr Gly Ser Lys Gly
               100                 105                 110
   Asn Lys Leu Gln Gln Leu Lys Ser Glu Ile His Lys Arg Asp Ser Asp
           115                 120                 125
   Val Glu Ile Arg Thr Ser Glu Lys Glu Asn Lys Lys Tyr Gly Tyr Glu
       130                 135                 140
   Phe Val Asp Ala Gly Tyr Val Tyr Thr Lys Asn Gly Lys Asp Glu Ile
   145                 150                 155                 160
   Glu Gln Asn Ser Gly Gly Lys Arg Phe Thr His Arg Phe Gly Tyr Asp
                   165                 170                 175
   Gly Phe Val Tyr Tyr Ser Gly Glu Arg Pro Ser Gln Ser Leu Pro Ser
               180                 185                 190
   Ala Gly Thr Val Lys Tyr Phe Gly Asn Trp Gln Tyr Met Thr Asp Ala
           195                 200                 205
   Lys Arg His Arg Thr Gly Lys Ala Val Ala Ser Asp Asp Leu Gly Tyr
       210                 215                 220
   Ile Thr Phe Tyr Gly Asn Asp Ile Gly Ala Thr Ser Tyr Ala Ala Lys
   225                 230                 235                 240
   Asp Ala Asp Asp Arg Glu Lys His Pro Ala Glu Tyr Thr Val Asp Phe
                   245                 250                 255
   Asp Lys Lys Ile Leu Lys Gly Glu Leu Ile Lys Asn Gln Tyr Val Gln
               260                 265                 270
   Lys Lys Asn Asp Pro Lys Lys Pro Leu Thr Ile Tyr Asn Ile Thr Ala
           275                 280                 285
   Asp Leu Asn Gly Asn Arg Phe Thr Gly Ser Ala Lys Val Asn Thr Glu
       290                 295                 300
   Val Lys Thr Arg His Ala Asp Lys Glu Tyr Leu Phe Phe His Thr Asp
   305                 310                 315                 320
   Ala Asp Gln Arg Leu Glu Gly Gly Phe Phe Gly Asp Asn Gly Glu Glu
                   325                 330                 335
   Leu Ala Gly Arg Phe Ile Ser Asn Asp Asn Gly Val Phe Gly Val Phe
                   340                 345                 350
   Ala Gly Lys Gln Lys Thr Asn Ala Ser Gly Thr Asn Pro Ala Met Pro
               355                 360                 365
   Phe Gly Lys His Thr Lys Ile Leu Asp Ser Leu Lys Ile Ser Val Asp
       370                 375                 380
   Glu Ala Thr Asp Glu Asn Pro Arg Pro Phe Glu Val Ser Thr Met Pro
   385                 390                 395                 400
   Asp Phe Gly His Pro Asp Lys Leu Leu Val Glu Gly Arg Glu Ile Pro
                   405                 410                 415
   Leu Val Ser Lys Glu Lys Thr Ile Asp Leu Ala Asp Gly Arg Lys Met
               420                 425                 430
   Thr Val Ser Ala Cys Cys Asp Phe Leu Thr Tyr Val Lys Leu Gly Arg
           435                 440                 445
   Ile Lys Thr Glu Arg Pro Ala Val Lys Pro Lys Ala Gln Asp Glu Glu
       450                 455                 460
   Asp Ser Gly Ile Asn Asn Gly Glu Glu Ser Glu Asp Glu Glu Glu Ile
   465                 470                 475                 480
   Ala Glu Glu Ser Glu Asp Glu Val Ser Glu Asp Asp Asn Gly Glu Asp
                   485                 490                 495
   Glu Asp Glu Ile Val Glu Glu Glu Ala Asp Glu Ala Glu Glu Ile Glu
               500                 505                 510
   Glu Glu Ala Glu Glu Glu Glu Pro Glu Glu Glu Ser Pro Glu Glu Gly
           515                 520                 525
   Asn Gly Val Ser Asp Gly Ile Pro Pro Ala Pro Glu Ala Leu Lys Gly
       530                 535                 540
   Arg Asp Ile Asp Leu Phe Leu Lys Gly Ile Arg Thr Ala Glu Ala Asp
   545                 550                 555                 560
   Ile Pro Lys Thr Gly Thr Ala His Tyr Thr Gly Thr Trp Glu Ala Arg
                   565                 570                 575
   Ile Gly Glu Pro Ile Gln Trp Asp Asn Lys Ala Asp Lys Ala Ala Lys
               580                 585                 590
   Ala Glu Phe Asp Val Asp Phe Gly Asn Lys Ser Ile Ser Gly Thr Leu
           595                 600                 605
   Thr Glu Gln Asn Gly Val Glu Pro Ala Phe Arg Ile Glu Asn Gly Val
       610                 615                 620
   Ile Glu Gly Asn Gly Phe His Pro Thr Ala Arg Thr Arg Asp Asn Gly
   625                 630                 635                 640
   Ile Asn Leu Ser Gly Asn Gly Ser Thr Asn Pro Gln Ser Phe Lys Ala
                   645                 650                 655
   Asp Asn Leu Leu Val Thr Gly Gly Phe Tyr Gly Pro Gln Ala Ala Glu
               660                 665                 670
   Leu Gly Gly Thr Ile Phe Asn Lys Asp Gly Lys Ser Leu Gly Ile Thr
           675                 680                 685
   Glu Asp Ile Glu Asn Glu Val Glu Asn Glu Ala Asp Val Gly Glu Gln
       690                 695                 700
   Leu Glu Pro Glu Val Lys Pro Gln Phe Gly Val Val Phe Gly Ala Lys
   705                 710                 715                 720
   Lys Asp Asn Lys Glu Val Glu Lys
                   725
<210> 82
   <211> 907
   <212> PRT
   <213> Neisseria gonorrhoeae
<400> 82
   Met Gln Gln Gln His Leu Phe Arg Phe Asn Ile Leu Cys Leu Ser Leu
   1 5 10 15
   Met Thr Ala Leu Pro Ala Tyr Ala Glu Asn Val Gln Ala Gly Gln Ala
   20 25 30
   Gln Glu Lys Gln Leu Asp Thr Ile Gln Val Lys Ala Lys Lys Gln Lys
   35 40 45
   Thr Arg Arg Asp Asn Glu Val Thr Gly Leu Gly Lys Leu Val Lys Thr
   50 55 60
   Ala Asp Thr Leu Ser Lys Glu Gln Val Leu Asp Ile Arg Asp Leu Thr
   65                  70                  75                  80
   Arg Tyr Asp Pro Gly Ile Ala Val Val Glu Gln Gly Arg Gly Ala Ser
                   85                  90                  95
   Ser Gly Tyr Ser Ile Arg Gly Met Asp Lys Asn Arg Val Ser Leu Thr
               100                 105                 110
   Val Asp Gly Leu Ala Gln Ile Gln Ser Tyr Thr Ala Gln Ala Ala Leu
           115                 120                 125
   Gly Gly Thr Arg Thr Ala Gly Ser Ser Gly Ala Ile Asn Glu Ile Glu
       130                 135                 140
   Tyr Glu Asn Val Lys Ala Val Glu Ile Ser Lys Gly Ser Asn Ser Val
   145                 150                 155                 160
   Glu Gln Gly Ser Gly Ala Leu Ala Gly Ser Val Ala Phe Gln Thr Lys
                   165                 170                 175
   Thr Ala Asp Asp Val Ile Gly Glu Gly Arg Gln Trp Gly Ile Gln Ser
               180                 185                 190
   Lys Thr Ala Tyr Ser Gly Lys Asn Arg Gly Leu Thr Gln Ser Ile Ala
           195                 200                 205
   Leu Ala Gly Arg Ile Gly Gly Ala Glu Ala Leu Leu Ile Arg Thr Gly
       210                 215                 220
   Arg His Ala Gly Glu Ile Arg Ala His Glu Ala Ala Gly Arg Gly Val
   225                 230                 235                 240
   Gln Ser Phe Asn Arg Leu Ala Pro Val Glu Asp Gly Ser Asp Tyr Ala
                   245                 250                 255
   Tyr Phe Val Val Glu Gly Glu Cys Pro Asp Gly Tyr Ala Ala Cys Lys
               260                 265                 270
   Asp Lys Pro Lys Lys Asp Val Val Gly Glu Asp Lys Arg Gln Thr Val
           275                 280                 285
   Ser Thr Arg Asp Tyr Thr Gly Pro Asn Arg Phe Leu Ala Asp Pro Leu
       290                 295                 300
   Ser Tyr Glu Ser Arg Ser Trp Leu Phe Arg Pro Gly Phe Arg Phe Glu
   305                 310                 315                 320
   Asn Lys Arg His Tyr Ile Gly Gly Ile Leu Glu Arg Thr Gln Gln Thr
                   325                 330                 335
   Phe Asp Thr Arg Asp Met Thr Val Pro Ala Phe Leu Thr Lys Ala Val
               340                 345                 350
   Phe Asp Ala Asn Ser Lys Gln Ala Gly Ser Leu Arg Gly Asn Gly Lys
           355                 360                 365
   Tyr Ala Gly Asn His Lys Tyr Gly Gly Leu Phe Thr Asn Gly Glu Asn
       370                 375                 380
   Asn Ala Pro Val Gly Ala Glu Tyr Gly Thr Gly Val Phe Tyr Asp Glu
   385                 390                 395                 400
   Thr His Thr Lys Ser Arg Tyr Gly Leu Glu Tyr Val Tyr Thr Asn Ala
                   405                 410                 415
   Asp Lys Asp Thr Trp Ala Asp Tyr Ala Arg Leu Ser Tyr Asp Arg Gln
               420                 425                 430
   Gly Ile Gly Leu Asp Asn His Phe Gln Gln Thr His Cys Ser Ala Asp
           435                 440                 445
   Gly Ser Asp Lys Tyr Cys Arg Pro Ser Ala Asp Lys Pro Phe Ser Tyr
       450                 455                 460
   Tyr Lys Ser Asp Arg Val Ile Tyr Gly Glu Ser His Arg Leu Leu Gln
   465                 470                 475                 480
   Ala Ala Phe Lys Lys Ser Phe Asp Thr Ala Lys Ile Arg His Asn Leu
                   485                 490                 495
   Ser Val Asn Leu Gly Tyr Asp Arg Phe Gly Ser Asn Leu Arg His Gln
               500                 505                 510
   Asp Tyr Tyr Tyr Gln Ser Ala Asn Arg Ala Tyr Ser Leu Lys Thr Pro
           515                 520                 525
   Pro Gln Asn Asn Gly Lys Lys Thr Ser Pro Tyr Trp Val Ser Ile Gly
       530                 535                 540
   Arg Gly Asn Val Val Thr Gly Gln Ile Cys Arg Ser Gly Asn Asn Thr
   545                 550                 555                 560
   Tyr Thr Asp Cys Thr Pro Arg Ser Ile Asn Gly Lys Ser Tyr Tyr Ala
                   565                 570                 575
   Ala Val Arg Asp Asn Val Arg Leu Gly Arg Trp Ala Asp Val Gly Ala
               580                 585                 590
   Gly Leu Arg Tyr Asp Tyr Arg Ser Thr His Ser Asp Asp Gly Ser Val
               595                 600                 605
   Ser Thr Gly Thr His Arg Thr Leu Ser Trp Asn Thr Gly Ile Val Leu
       610                 615                 620
   Lys Pro Ala Asp Trp Leu Asp Leu Thr Tyr Arg Thr Ser Thr Gly Phe
   625                 630                 635                 640
   Arg Leu Pro Ser Phe Ala Glu Met Tyr Gly Trp Arg Ser Gly Asp Lys
                   645                 650                 655
   Ile Lys Ala Val Lys Ile Asp Pro Glu Lys Ser Phe Asn Lys Glu Ala
               660                 665                 670
   Gly Ile Val Phe Lys Gly Asp Phe Gly Asn Leu Glu Ala Ser Trp Phe
           675                 680                 685
   Asp Asn Ala Tyr Arg Asp Leu Ile Val Arg Gly Tyr Glu Ala Glu Ile
       690                 695                 700
   Lys Asn Gly Lys Glu Gln Ala Lys Gly Ala Pro Ala Tyr Leu Asn Ala
   705                 710                 715                 720
   Gln Ser Ala Arg Ile Thr Gly Ile Asn Ile Leu Gly Lys Ile Asp Trp
                   725                 730                 735
   Asn Gly Val Trp Asp Lys Leu Pro Glu Gly Trp Tyr Ser Thr Phe Ala
               740                 745                 750
   Tyr Asn Arg Val Arg Val Arg Asp Ile Lys Lys Arg Ala Asp Arg Thr
           755                 760                 765
   Asp Ile Gln Ser His Leu Phe Asp Ala Ile Gln Pro Ser Arg Tyr Val
       770                 775                 780
   Val Gly Ser Gly Tyr Asp Gln Pro Glu Gly Lys Trp Gly Val Asn Gly
   785                 790                 795                 800
   Met Leu Thr Tyr Ser Lys Ala Lys Glu Ile Thr Glu Leu Leu Gly Ser
                   805                 810                 815
   Arg Ala Leu Leu Asn Gly Asn Ser Arg Asn Thr Lys Ala Thr Ala Arg
               820                 825                 830
   Arg Thr Arg Pro Trp Tyr Ile Val Asp Val Ser Gly Tyr Tyr Thr Val
           835                 840                 845
   Lys Lys His Phe Thr Leu Arg Ala Gly Val Tyr Asn Leu Leu Asn His
       850                 855                 860
   Arg Tyr Val Thr Trp Glu Asn Val Arg Gln Thr Ala Ala Gly Ala Val
   865                 870                 875                 880
   Asn Gln His Lys Asn Val Gly Val Tyr Asn Arg Tyr Ala Ala Pro Gly
                   885                 890                 895
   Arg Asn Tyr Thr Phe Ser Leu Glu Met Lys Phe
               900                 905
<210> 83
   <211> 3035
   <212> DNA
   <213> Neisseria gonorrhoeae
<400> 83
   cgaagagttg ggcggatggt ttgcctatcc gggcaatgaa caaacgaaaa atgcgcaagc 60
   ttcatccggc aatggaaatt cagcaggcag cgcgaccgtg gtattcggtg cgaaacgcca 120
   aaagcttgtg caataagcac ggctgccgaa caatcgagaa taaggcttca gacggcatcg 180
   ttcctgccga ttccgtctga aagcgaagat tagggaaaca ctatgcaaca gcaacatttg 240
   ttccgattca atattttatg cctgtcttta atgactgcgc tgcccgctta tgcagaaaat 300
   gtgcaagccg gacaagcaca ggaaaaacag ttggacacca tacaggtaaa agccaaaaaa 360
   cagaaaaccc gccgcgataa cgaagtaacc ggtttgggca aattggtcaa aaccgccgac 420
   acactcagca aagaacaggt actcgacatc cgcgacctga cgcgttacga ccccggcatc 480
   gccgtcgtcg aacaggggcg cggcgcaagc tcgggctact cgatacgcgg tatggacaaa 540
   aaccgcgtct ccttgacggt ggacggcttg gcgcaaatac agtcctacac cgcgcaggcg 600
   gcattgggcg ggacgaggac ggcgggcagc agcggcgcaa tcaatgaaat cgagtatgaa 660
   aacgttaagg ctgtcgaaat cagcaaaggc tcaaactcgg tcgaacaagg cagcggcgca 720
   ttggcgggtt cggtcgcatt tcaaaccaaa accgcagacg atgttatcgg ggaaggcagg 780
   cagtggggca ttcagagtaa aaccgcctat tccggcaaaa accgggggct tacccaatcc 840
   atcgcgctgg cggggcgcat cggcggtgcg gaggctttgc tgatccgcac cggccggcac 900
   gcgggggaaa tccgcgccca cgaagccgcc ggacgcggcg ttcagagctt taacaggctg 960
   gcgccggttg aagacggcag tgactatgcc tattttgtgg tcgaaggaga atgccctgat 1020
   ggatatgcgg cttgtaaaga caaaccgaaa aaagatgttg tcggcgaaga caaacgtcaa 1080
   acggtttcca cccgagacta cacgggcccc aaccgcttcc ttgccgatcc gctttcatac 1140
   gaaagccggt cgtggctgtt ccgcccgggt tttcgttttg agaataagcg gcactacatc 1200
   ggcggcatac tcgaacgcac gcaacaaact ttcgacacgc gcgatatgac ggttccggca 1260
   ttcctgacca aggcggtttt tgatgcaaat tcaaaacagg cgggttcttt gcgcggcaac 1320
   ggcaaatacg cgggcaacca caaatacggc gggctgttta ccaacggcga aaacaatgcg 1380
   ccggtgggcg cggaatacgg tacgggcgtg ttttacgacg agacgcacac caaaagccgc 1440
   tacggtttgg aatatgtcta taccaatgcc gataaagaca cttgggcgga ttatgcccgc 1500
   ctctcttacg accggcaggg catcggtttg gacaaccatt ttcagcagac gcactgttct 1560
   gccgacggtt cggacaaata ttgccgcccg agtgccgaca agccgttttc ctattacaaa 1620
   tccgaccgcg tgatttacgg ggaaagccac aggctcttgc aggcggcatt caaaaaatcc 1680
   ttcgataccg ccaaaatccg ccacaacctg agcgtgaatc tcggttacga ccgcttcggc 1740
   tctaatctgc gccatcagga ttattattat caaagtgcca accgcgccta ttcgttgaaa 1800
   acgccccctc aaaacaacgg caaaaaaacc agcccctatt gggtcagcat aggcagggga 1860
   aatgtcgtta cggggcaaat ctgccgctcg ggcaacaata cttatacgga ctgcacgccg 1920
   cgcagcatca acggcaaaag ctattacgcg gcggtccggg acaatgtccg tttgggcagg 1980
   tgggcggatg tcggcgcggg cttgcgctac gactaccgca gcacgcattc ggacgacggc 2040
   agcgtttcca ccggcacgca ccgcaccctg tcctggaaca ccggcatcgt cctcaaacct 2100
   gccgactggc tggatttgac ttaccgcact tcaaccggct tccgcctgcc ctcgtttgcg 2160
   gaaatgtacg gctggcggtc gggcgataaa ataaaagccg tcaaaatcga tccggaaaaa 2220
   tcgttcaaca aagaagccgg catcgtgttt aaaggcgatt tcggcaactt ggaggcaagt 2280
   tggttcgaca atgcctaccg cgatttgatt gtccggggtt atgaagcgga aattaaaaac 2340
   ggcaaagaac aagccaaagg cgccccggct tacctcaatg cccaaagcgc gcggattacc 2400
   ggcatcaata ttttgggcaa aatcgattgg aacggcgtat gggataaatt gcccgaaggt 2460
   tggtattcta catttgccta taatcgtgtc cgtgtccgcg acatcaaaaa acgcgcagac 2520
   cgcaccgata ttcaatcaca cctgtttgat gccatccaac cctcgcgcta tgtcgtcggc 2580
   tcgggctatg accaaccgga aggcaaatgg ggcgtgaacg gtatgctgac ttattccaaa 2640
   gccaaggaaa tcacagagtt gttgggcagc cgggctttgc tcaacggcaa cagccgcaat 2700
   acaaaagcca ccgcgcgccg tacccgccct tggtatattg tggatgtgtc cggttattac 2760
   acggttaaaa aacacttcac cctccgtgcg ggcgtgtaca acctcctcaa ccaccgctat 2820
   gttacttggg aaaatgtgcg gcaaactgcc gccggcgcag tcaaccaaca caaaaatgtc 2880
   ggcgtttaca accgatatgc cgcccccggc cgcaactaca catttagctt ggaaatgaag 2940
   ttctaaacgt ccgaacgccg caaatgccgt ctgaaaggct tcagacggcg ttttttacac 3000
   aatccccacc gtttcccatc cttcccgata caccg 3035
<210> 84
   <211> 683
   <212> PRT
   <213> Neisseria gonorrhoeae
<400> 84
   Met Asn Asn Pro Leu Val Asn Gln Ala Ala Met Val Leu Pro Val Phe
    1               5                  10                  15
   Leu Leu Ser Ala Cys Leu Gly Gly Gly Gly Ser Phe Asp Leu Asp Ser
               20                  25                  30
   Val Asp Thr Glu Ala Pro Arg Ala Ala Pro Lys Tyr Gln Asp Val Pro
           35                  40                  45
   Ser Lys Lys Pro Glu Ala Arg Lys Asp Gln Gly Gly Tyr Gly Phe Ala
       50                  55                  60
   Met Arg Phe Lys Arg Arg Asn Trp Tyr Arg Ala Ala Asn Glu Asn Glu
   65                  70                  75                  80
   Val Lys Leu Lys Glu Ser Asp Trp Glu Gln Thr Asp Asp Asp Glu Ile
                   85                  90                  95
   Lys Asn Pro Phe Lys Gln Lys Asn Ile Ile Asn Ala Leu Pro Gly Asn
               100                 105                 110
   Glu Gly Glu Leu Leu Gln Asp Ser Ser Gln Gln Gly Lys Gly Thr Ser
           115                 120                 125
   Lys Val Arg Asp His His Asp Phe Lys Tyr Val Trp Ser Gly Phe Phe
       130                 135                 140
   Tyr Lys Arg Ile Lys Ile Thr Thr Lys Lys Asp Glu Ser His Lys Ile
   145                 150                 155                 160
   Ile Glu Ala Arg Ser Gly Pro Asp Gly Tyr Ile Phe Tyr Lys Gly Arg
                   165                 170                 175
   Asn Pro Ser Arg Lys Leu Pro Val Ser Gly Glu Val Thr Tyr Lys Gly
               180                 185                 190
   Thr Trp Asp Phe Leu Thr Asp Val Lys Ala Asn Gln Lys Phe Thr Asp
           195                 200                 205
   Leu Gly Asn Ala Ser Thr Lys Ser Gly Asp Gln Tyr Ser Ala Phe Ser
       210                 215                 220
   Gly Glu Leu Asp Tyr Ile Val Lys Lys Glu Glu Asp Lys Lys Glu Lys
   225                 230                 235                 240
   His Lys Gly Leu Gly Leu Thr Thr Glu Ile Thr Val Asp Phe Glu Lys
                   245                 250                 255
   Lys Thr Leu Ile Gly Lys Leu Ile Lys Asn Asn Met Leu Ile Asn Asn
               260                 265                 270
   Asn Thr Lys Pro Thr Thr Gln Tyr Tyr Ser Leu Glu Ala Gln Val Thr
           275                 280                 285
   Gly Asn Arg Phe Ser Gly Lys Ala Met Ala Thr Glu Lys Gly Glu Asn
       290                 295                 300
   Lys Gln His Pro Phe Val Ser Asp Ser Ser Ser Leu Ser Gly Gly Phe
   305                 310                 315                 320
   Phe Gly Pro Gln Gly Glu Glu Leu Gly Phe Arg Phe Leu Ser Asp Asp
                   325                 330                 335
   Gly Lys Val Ala Val Val Gly Ser Ala Lys Thr Lys Asp Glu Thr Ala
               340                 345                 350
   Ser Ser Gly Gly Thr Ser Gly Gly Ala Ser Val Ser Thr Ser Asn Gly
           355                 360                 365
   Ala Ala Gly Thr Ser Ser Glu Asn Lys Leu Thr Thr Val Leu Asp Ala
       370                 375                 380
   Val Glu Leu Thr Pro Asn Gly Lys Lys Ile Lys Asp Leu Asp Asn Phe
   385                 390                 395                 400
   Ser Asn Ala Ala Gln Leu Val Val Asp Gly Ile Met Ile Pro Leu Leu
                   405                 410                 415
   Pro Lys Asp Ser Glu Ser Gly Gly Ser His Thr Asp Lys Gly Glu Asn
               420                 425                 430
   Gly Lys Thr Ala Phe Ile Tyr Glu Thr Thr Tyr Thr Pro Glu Ser Asp
           435                 440                 445
   Lys Glu Asp Ala Gln Thr Gly Met Ala Thr Asn Gly Val Gln Thr Val
       450                 455                 460
   Ser Asn Thr Ala Gly Gly Thr Ser Gly Lys Thr Lys Thr His Tyr Glu
   465                 470                 475                 480
   Val Gln Ala Cys Cys Ser Asn Leu Asn Tyr Leu Lys Tyr Gly Leu Leu
                   485                 490                 495
   Thr Arg Lys Asn Ser Ser Gln Ala Asp Ala Lys Met Gly Gln Val Glu
               500                 505                 510
   Gln Ser Met Phe Leu Gln Gly Glu Arg Thr Asp Glu Lys Glu Ile Pro
           515                 520                 525
   Gln Glu Gln Asn Val Val Tyr Ser Gly Thr Trp Tyr Gly His Ile Ala
       530                 535                 540
   Thr Asn Gly Thr Ser Trp Thr Arg Glu Ala Ser Asp Gln Glu Asn Gly
   545                 550                 555                 560
   Asn Arg Ala Asn Phe Asp Val Asn Phe Lys Asp Lys Arg Ile Thr Gly
                   565                 570                 575
   Thr Leu Thr Ala Glu Asn Arg Ser Glu Ala Thr Phe Thr Ile Glu Ala
               580                 585                 590
   Met Ile Glu Gly Asn Gly Phe Lys Gly Thr Ala Lys Thr Gly Asn Gly
           595                 600                 605
   Gly Phe Ala Pro Asp Gln Asn Ser Ser Thr Gly Thr His Lys Val His
       610                 615                 620
   Ile Thr Asn Ala Ala Val Gln Gly Gly Phe Tyr Gly Pro Asn Ala Glu
   625                 630                 635                 640
   Glu Leu Gly Gly Trp Phe Ala Tyr Pro Gly Asn Gly Gln Thr Lys Asn
                   645                 650                 655
   Ala Gln Thr Ser Ser Gly Asn Gly Asn Ser Ala Gly Ser Ala Thr Val
               660                 665                 670
   Val Phe Gly Ala Lys Arg Gln Gln Leu Val Lys
           675                 680
<210> 85
   <211> 2052
   <212> DNA
   <213> Neisseria gonorrhoeae
<400> 85
   atgaacaatc cattggtgaa tcaggctgct atggtgctgc ccgtgttttt gttgagcgct 60
   tgtctgggcg gaggcggcag tttcgatctt gattctgtcg ataccgaagc cccgcgtgcc 120
   gcgccaaagt atcaagatgt tccttccaaa aaaccggaag cccgaaaaga ccaaggcgga 180
   tacggtttcg cgatgcgctt caagcggcgg aattggtatc gggcggcaaa cgaaaacgag 240
   gttaaactga aagagagtga ttgggaacaa acggatgatg atgagatcaa aaaccctttc 300
   aaacaaaaaa atattattaa tgccttacct ggaaatgagg gggaattatt gcaagattcc 360
   agtcaacaag gtaagggtac atctaaggtt agggaccatc acgattttaa atacgtatgg 420
   tcgggttttt tttataaacg gattaagatt acaactaaaa aagacgaatc tcataaaata 480
   atcgaagcca gaagcggtcc tgacggttat attttttata aaggcagaaa tccctcgaga 540
   aaacttcctg tttcagggga ggttacgtac aaaggtactt gggatttttt aactgatgtg 600
   aaagcaaatc agaaatttac agatttagga aatgcttcta cgaaatccgg agaccaatat 660
   agtgcttttt ccggggagtt ggattatata gtcaaaaaag aggaggataa aaaagaaaag 720
   cacaaaggtt tgggattaac aacggaaata acggttgatt ttgagaaaaa aaccctgatc 780
   ggaaaattaa ttaaaaacaa catgttaatc aataataaca ctaaacccac cacccaatat 840
   tacagccttg aggctcaagt aacaggcaac cgcttcagcg gcaaggcgat ggcaaccgaa 900
   aaaggcgaaa acaaacaaca tccctttgtt tccgactcgt cttctctgag cggcggcttt 960
   ttcggcccgc agggtgagga attgggtttc cgctttttga gcgacgatgg aaaagttgcc 1020
   gttgtcggca gcgcgaaaac caaagacgaa accgcaagca gtggcggcac ttcgggcggt 1080
   gcaagcgttt ccacatcaaa cggtgcggca ggcacgtcgt ctgaaaacaa gctgaccacg 1140
   gttttggatg cggttgaatt gacaccaaac ggcaagaaaa tcaaagatct cgacaacttc 1200
   agcaacgccg cccaactggt tgtcgacggc attatgattc cgctcctgcc caaggattcc 1260
   gaaagcgggg gcagtcatac agataaaggt gaaaacggca aaacagcctt tatctacgaa 1320
   acaacctaca cgccggaaag tgataaagaa gacgctcaaa caggtatggc gaccaatggc 1380
   gtgcaaaccg tttcaaatac ggcaggcggc acaagtggca aaacaaaaac ccattatgaa 1440
   gtccaagcct gctgttccaa cctcaattat ctgaaatacg ggttgctgac gcgtaaaaac 1500
   agtagtcaag ctgacgctaa aatgggacaa gttgaacaaa gtatgttcct ccaaggcgag 1560
   cgcaccgatg aaaaagaaat tccacaagaa caaaatgtcg tttattcagg cacttggtac 1620
   gggcatattg ccaccaacgg cacaagttgg acccgcgaag cctccgatca ggaaaatggt 1680
   aatcgggcaa attttgacgt gaatttcaaa gacaaaagaa ttaccggcac gttaaccgct 1740
   gaaaacaggt cggaggcaac ctttaccatt gaagccatga ttgagggcaa cggctttaaa 1800
   ggtacggcga aaaccggtaa tggcggcttt gcgccggatc aaaacagcag caccggtaca 1860
   cataaagtgc acatcacaaa tgccgcggtg cagggcggtt tttacgggcc taacgccgaa 1920
   gagttgggcg gttggtttgc ctatccgggc aatggacaaa cgaaaaatgc gcaaacttca 1980
   tccggcaatg gaaattcagc aggcagcgcg accgtggtat tcggtgcgaa acgccaacag 2040
   cttgtgaaat aa                                                    2052
<210> 86
   <211> 215
   <212> PRT
   <213> Neisseria gonorrhoeae
<400> 86
   Met Ala Ser Lys Ser Ser Lys Thr Asn Leu Asp Leu Asn Asn Leu His
   1 5 10 15
   Leu Leu Asn Leu Pro Ala Arg Leu Phe Ile Ala Leu Leu Val Val Ala
   20 25 30
   Ala Val Leu Gly Leu Gly Tyr Ala Gly Leu Phe Lys Ser Gln Met Glu
   35 40 45
   Ser Leu Glu Glu Tyr Glu Ala Lys Glu Thr Glu Leu Lys Asn Thr Tyr
   50 55 60
   Lys Gln Lys Ser Ile Asp Ala Ala Ser Leu Asn Asn Leu Arg Asp Glu
   65 70 75 80
   Leu Ala Ser Ile Arg Ser Ala Phe Asp Ile Met Leu Lys Gln Leu Pro
   85 90 95
   Thr Asp Ala Glu Ile Pro Asn Leu Val Gln Glu Leu His Gln Ala Gly
   100 105 110
   Ser Ser Asn Gly Leu Arg Leu Asp Ser Val Met Pro Gln Pro Pro Val
   115 120 125
   Asp Asp Gly Pro Ile Lys Lys Leu Pro Tyr Ser Ile Ser Ile Thr Gly
   130 135 140
   Asn Tyr Glu Gln Ile Ser Gln Phe Thr Arg Asp Val Gly Ser Leu Ser
   145 150 155 160
   Arg Ile Ile Thr Leu Glu Ser Leu Lys Ile Ala Gln Ser Pro Glu Asn
   165 170 175
   Gly Gly Asn Pro Asp Gly Lys Ser Ser Ile Leu Asn Leu Ser Ala Ile
   180 185 190
   Ala Thr Thr Tyr Gln Ala Lys Ser Ile Glu Glu Leu Ala Ala Glu Ala
   195 200 205
   Ala Gln Asn Ala Glu Gln Lys
   210 215
<210> 87
   <211> 180
   <212> PRT
   <213> Neisseria gonorrhoeae
<400> 87
   Met Lys His Tyr Ala Leu Leu Ile Ser Phe Leu Ala Leu Ser Ala Cys
   1 5 10 15
   Ser Gln Ser Ser Glu Asp Leu Asn Glu Trp Met Ala Gln Thr Arg Arg
   20 25 30
   Glu Ala Lys Ala Glu Ile Ile Pro Phe Gln Ala Pro Thr Leu Pro Val
   35 40 45
   Ala Pro Val Tyr Ser Pro Pro Gln Leu Thr Gly Pro Asn Ala Phe Asp
   50 55 60
   Phe Arg Arg Met Glu Thr Ala Lys Lys Gly Glu Asn Ala Pro Asp Thr
   65 70 75 80
   Lys Arg Ile Lys Glu Thr Leu Glu Lys Phe Ser Leu Glu Asn Met Arg
   85 90 95
   Tyr Val Gly Ile Leu Lys Ser Gly Gln Lys Val Ser Gly Phe Ile Glu
   100 105 110
   Ala Glu Gly Tyr Val Tyr Thr Val Gly Val Gly Asn Tyr Leu Gly Gln
   115 120 125
   Asn Tyr Gly Arg Ile Glu Ser Ile Thr Asp Asp Ser Ile Ile Leu Asn
   130 135 140
   Glu Leu Ile Glu Asp Ser Thr Gly Asn Trp Val Ser Arg Lys Ala Glu
   145 150 155 160
   Leu Leu Leu Asn Ser Ser Asp Lys Asn Thr Glu Gln Ala Ala Gln Pro
   165 170 175
   Glu Glu Gln Asn
   180
<210> 88
   <211> 720
   <212> PRT
   <213> Neisseria gonorrhoeae
<400> 88
   Met Asn Thr Lys Leu Thr Lys Ile Ile Ser Gly Leu Phe Val Ala Thr
   1 5 10 15
   Ala Ala Phe Gln Thr Ala Ser Ala Gly Asn Ile Thr Asp Ile Lys Val
   20 25 30
   Ser Ser Leu Pro Asn Lys Gln Lys Ile Val Lys Val Ser Phe Asp Lys
   35 40 45
   Glu Ile Val Asn Pro Thr Gly Phe Val Thr Ser Ser Pro Ala Arg Ile
   50 55 60
   Ala Leu Asp Phe Glu Gln Thr Gly Ile Ser Met Asp Gln Gln Val Leu
   65 70 75 80
   Glu Tyr Ala Asp Pro Leu Leu Ser Lys Ile Ser Ala Ala Gln Asn Ser
   85 90 95
   Ser Arg Ala Arg Leu Val Leu Asn Leu Asn Lys Pro Gly Gln Tyr Asn
   100 105 110
   Thr Glu Val Arg Gly Asn Lys Val Trp Ile Phe Ile Asn Glu Ser Asp
   115 120 125
   Asp Thr Val Ser Ala Pro Ala Arg Pro Ala Val Lys Ala Ala Pro Ala
   130 135 140
   Ala Pro Ala Lys Gln Gln Ala Ala Ala Pro Phe Thr Glu Ser Val Val
   145 150 155 160
   Ser Val Ser Ala Pro Phe Ser Pro Ala Lys Gln Gln Ala Ala Ala Ser
   165 170 175
   Ala Lys Gln Gln Ala Ala Thr Pro Ala Lys Gln Thr Asn Ile Asp Phe
   180 185 190
   Arg Lys Asp Gly Lys Asn Ala Gly Ile Ile Glu Leu Ala Ala Leu Gly
   195 200 205
   Phe Ala Gly Gln Pro Asp Ile Ser Gln Gln His Asp His Ile Ile Val
   210 215 220
   Thr Leu Lys Asn His Thr Leu Pro Thr Ala Leu Gln Arg Ser Leu Asp
   225 230 235 240
   Val Ala Asp Phe Lys Thr Pro Val Gln Lys Val Thr Leu Lys Arg Leu
   245 250 255
   Asn Asn Asp Thr Gln Leu Ile Ile Thr Thr Thr Gly Asn Trp Glu Leu
   260 265 270
   Val Asn Lys Ser Ala Ala Pro Gly Tyr Phe Thr Phe Gln Val Leu Pro
   275 280 285
   Lys Lys Gln Asn Leu Glu Ser Gly Gly Val Asn Asn Ala Pro Lys Thr
   290 295 300
   Phe Thr Gly Arg Lys Ile Ser Leu Asp Phe Gln Asp Val Glu Ile Arg
   305 310 315 320
   Thr Ile Leu Gln Ile Leu Ala Lys Glu Ser Gly Met Asn Ile Val Ala
   325 330 335
   Ser Asp Ser Val Ser Gly Lys Met Thr Leu Ser Leu Lys Asp Val Pro
   340 345 350
   Trp Asp Gln Ala Leu Asp Leu Val Met Gln Ala Arg Asn Leu Asp Met
   355 360 365
   Arg Gln Gln Gly Asn Ile Val Asn Met Ala Pro Arg Arg Ala Ala Cys
   370 375 380
   Gln Arg Gln Ser Leu Leu Thr Ser Gly Lys Arg His Cys Arg Ser Gly
   385 390 395 400
   Arg Ala Val Phe Pro Lys Leu Pro Ile Glu Ile Gln Lys Cys Gly Arg
   405 410 415
   Ile Pro Gln His Pro Ala Leu Asp Asn Ala Asp Thr Thr Gly Asn Arg
   420 425 430
   Asn Thr Leu Val Ser Gly Arg Gly Ser Val Leu Ile Asp Pro Ala Thr
   435 440 445
   Asn Thr Leu Ile Val Thr Asp Thr Arg Ser Val Ile Glu Lys Phe Arg
   450 455 460
   Lys Leu Ile Asp Glu Leu Asp Val Pro Ala Gln Gln Val Met Ile Glu
   465 470 475 480
   Ala Arg Ile Val Glu Ala Ala Asp Gly Phe Ser Arg Asp Leu Gly Val
   485 490 495
   Lys Phe Gly Ala Thr Gly Arg Lys Lys Leu Lys Asn Glu Thr Ser Ala
   500 505 510
   Phe Gly Trp Gly Val Asn Ser Gly Phe Gly Gly Gly Asp Lys Trp Glu
   515 520 525
   Ala Lys Pro Lys Ser Thr Cys Arg Leu Pro Cys Arg Lys Gln His Phe
   530 535 540
   Ala Gly Ala Arg Asp Phe Ser Gly Ala Leu Asn Leu Glu Leu Ser Ala
   545 550 555 560
   Ser Glu Ser Leu Ser Lys Thr Lys Thr Leu Ala Asn Pro Arg Val Leu
   565 570 575
   Thr Gln Asn Arg Lys Glu Ala Lys Ile Glu Ser Gly Tyr Glu Ile Pro
   580 585 590
   Phe Thr Val Thr Thr Arg Ser Gly Gly Gly Asn Ser Thr Asn Thr Glu
   595 600 605
   Leu Lys Lys Ala Val Leu Gly Leu Thr Val Thr Ala Asn Ile Thr Pro
   610 615 620
   Asp Gly Gln Ile Ile Met Thr Val Lys Ile Asn Lys Asp Ser Pro Arg
   625 630 635 640
   Gln Cys Ala Ser Gly Asn Asn Thr Ile Leu Cys Ile Ser Thr Lys Ser
   645 650 655
   Leu Asn Thr Gln Ala Met Val Glu Asn Gly Gly Thr Leu Ile Val Gly
   660 665 670
   Gly Ile Tyr Glu Glu Asn Asn Gly Asn Thr Leu Thr Lys Val Pro Leu
   675 680 685
   Leu Ala Thr Ser Pro Leu Ser Ala Thr Ser Leu Lys His Ser Gly Lys
   690 695 700
   Asn Arg Pro Pro Arg Thr Ala Asp Phe Gln Leu Pro Pro Arg Glu Leu
   705 710 715 720
<210> 89
   <211> 3829
   <212> DNA
   <213> Neisseria gonorrhoeae
<400> 89
   aaacgcacag gaggaaactg aatggcttct aaatcatcta aaaccaactt ggatctcaac 60
   aaccttcacc tgctcaacct tcctgccagg ctttttatcg ccctgctggt cgttgccgcc 120
   gtgctggggc tcggttatgc cggattgttc aaaagccaga tggaatccct tgaggaatat 180
   gaagcaaaag aaaccgaact gaaaaacacc tacaaacaga aaagtatcga cgcggccagc 240
   ctgaacaacc ttagggacga acttgcctca atccgctctg ccttcgatat catgttgaaa 300
   cagctgccga cagatgcaga aattcccaat ttggttcaag agcttcatca ggcgggttcg 360
   agcaacggtc tgcgcttgga cagcgttatg ccccaacctc ccgtagatga cggtcccatc 420
   aaaaaattac cctattccat ttccattacc ggaaattacg aacagatcag ccaatttacc 480
   cgcgatgtcg gcagtctctc ccgaatcatt acccttgagt cgctgaaaat cgcccaatct 540
   ccggaaaacg gcggcaatcc tgacggcaag agcagtatcc tgaacctcag cgccattgcc 600
   accacctacc aagcaaaatc catagaagag cttgccgcag aagcggcaca aaatgccgag 660
   caaaaataac ttacgttagg gaaaccatga aacactatgc cttactcatc agctttctgg 720
   ctctctccgc gtgttcccaa agttctgaag acctaaacga atggatggca caaacgcgac 780
   gcgaagccaa agcagaaatc atacctttcc aagcacctac cctgccggtt gcgccggtat 840
   acagcccgcc gcagcttaca gggccgaacg cattcgactt ccgccgcatg gaaaccgcca 900
   aaaaagggga aaatgccccc gacaccaagc gtattaaaga aacgctggaa aaattcagtt 960
   tggaaaatat gcgttatgtc ggcattttga agtccggaca gaaagtctcc ggcttcatcg 1020
   aggctgaagg ttatgtctac actgtcggtg tcggcaacta tttgggacaa aactacggta 1080
   gaatcgaaag cattaccgac gacagcatca tcctgaacga gctgatagaa gacagcacgg 1140
   gcaactgggt ttcccgtaaa gcagaactgc tgttgaattc ttccgacaaa aacaccgaac 1200
   aagcggcaca gcctgaggaa caaaattaag aagaggatta ctccattatg aataccaaac 1260
   tgacaaaaat catttccggt ctctttgtcg caaccgccgc ctttcagacg gcatcggcag 1320
   gaaacattac agacatcaaa gtttcctccc tgcccaacaa acagaaaatc gtcaaagtca 1380
   gctttgacaa agagattgtc aacccgaccg gcttcgtaac ctcctcaccg gcccgcatcg 1440
   ccttggactt tgaacaaacc ggcatttcca tggatcaaca ggtactcgaa tatgccgatc 1500
   ctctgttgag caaaatcagt gccgcacaaa acagcagccg tgcgcgtctg gttctgaatt 1560
   tgaacaaacc gggccaatac aataccgaag tacgcgggaa caaagtttgg atattcatta 1620
   acgaatcgga cgataccgtg tccgcccccg cccgcccagc cgtaaaagcc gcgcctgccg 1680
   caccggcaaa acaacaggct gccgcaccgt ttaccgagtc cgtagtatcc gtatccgcac 1740
   cgttcagccc ggcaaaacaa caggcagcgg catcggcaaa acaacaggcg gcgacaccgg 1800
   caaaacaaac caatatcgat ttccgcaaag acggcaaaaa tgccggcatt atcgaattgg 1860
   cggcattggg ctttgccggg cagcccgaca tcagccaaca gcacgaccac atcatcgtta 1920
   cgctgaaaaa ccataccctg ccgaccgcgc tccaacgcag tttggatgtg gcagacttca 1980
   aaacaccggt tcaaaaggtt acgctgaaac gcctcaataa cgacacccag ctgattatca 2040
   caacaaccgg caactgggaa ctcgtcaaca aatccgccgc gcccggatac tttaccttcc 2100
   aagtcctgcc gaaaaaacaa aacctcgagt caggcggcgt gaacaatgcg cccaaaacct 2160
   tcacaggccg gaaaatctcc cttgacttcc aagatgtcga aatccgcacc atcctgcaga 2220
   ttttggcaaa agaatccggg atgaacattg ttgccagcga ctccgtcagc ggcaaaatga 2280
   ccctctccct caaagacgta ccttgggatc aggctttgga tttggttatg caggcgcgca 2340
   acctcgatat gcgccagcaa gggaacatcg tcaacatggc cccgcgacga gctgcttgcc 2400
   aaagacaaag ccttcttaca agcggaaaaa gacattgccg atctgggcgc gctgtattcc 2460
   caaaacttcc aattgaaata caaaaatgtg gaagaattcc gcagcatcct gctttggaca 2520
   atgccgacac gaccggaaac cgcaacacgc ttgtcagcgg caggggcagc gtgctgatcg 2580
   atcccgccac caacaccctg attgttaccg atacccgcag cgtcatcgaa aaattccgca 2640
   aactgattga cgaattggac gtacccgcgc aacaagtgat gattgaggcg cgtatcgtcg 2700
   aagcggcaga cggcttctcg cgcgatttgg gcgttaagtt cggcgcgaca ggcaggaaaa 2760
   aactgaaaaa tgagacgagc gcattcggct ggggcgtgaa ctccggcttc gggggcggcg 2820
   ataaatggga ggccaaacca aaatcaacct gccggttgcc gtgccgcaaa cagcatttcg 2880
   ctggtgcgcg cgatttctcc ggcgcgttga atttggaatt gtccgcatcc gagtcgcttt 2940
   caaaaaccaa aacgcttgcc aatccgcgcg tgctgaccca aaaccgcaaa gaggccaaaa 3000
   tcgaatccgg ttacgaaatt ccttttaccg taactacacg ctcgggcggc ggcaactcta 3060
   ccaacacgga actcaaaaaa gccgtcttgg ggctgaccgt tacggcgaac atcacgcccg 3120
   acggacaaat catcatgacc gtcaaaatca acaaagactc gcctcgacaa tgtgcttcag 3180
   gcaacaacac aatcctatgt atttcgacca aaagcctgaa tacgcaggct atggttgaaa 3240
   acggcggcac tttgattgtc ggcggtattt atgaagaaaa caacggcaat acgctgacca 3300
   aagtccccct gttggctaca tccccgttat cggcaacctc tttaaaacac tcgggaaaaa 3360
   accgaccgcc gcgaactgct gattttcaat tacccccgag ggaattatag atacggcgca 3420
   acagcctgcg ctattgatgc gtcaaaataa gggcatatgt tttacagcat atgccctttc 3480
   tttatgcttt ttgccgcgac cgaaatgccg tcattcccgc gagcgaatcc aacttgtccg 3540
   gtttcggttg tttttcgtct cgtaactttt gagccgtcat tcccgcgaaa tcggaaatcc 3600
   agtccgttca gtttcggtca tttccgataa attcctgttg cttttcattt ctagattccc 3660
   actttcgtgg aataacggcg gaagggataa atcctcgcaa tccaaagcct gctcatttcc 3720
   acaaaaaaca gcaacccgaa acaccccgtc attcccgagc aggcggaatc tagaaccgca 3780
   acgccaggaa tctgtcggat acggctgaaa ccgaacgact ggattcccg            3829
<210> 90
   <211> 174
   <212> PRT
   <213> Neisseria gonorrhoeae
<400> 90
   Met Lys Lys Ala Leu Ala Ala Leu Ile Ala Leu Ala Leu Pro Ala Ala
   1               5                   10                  15
   Ala Leu Ala Glu Gly Ala Ser Gly Phe Tyr Val Gln Ala Asp Ala Ala
               20                  25                  30
   His Ala Lys Ala Ser Ser Ser Leu Gly Ser Ala Lys Gly Phe Ser Pro
           35                  40                  45
   Arg Ile Ser Ala Gly Tyr Arg Ile Asn Asp Leu Arg Phe Ala Val Asp
       50                  55                  60
   Tyr Thr Arg Tyr Lys Asn Tyr Lys Ala Pro Ser Thr Asp Phe Lys Leu
   65                  70                  75                  80
   Tyr Ser Ile Gly Ala Ser Val Ile Tyr Asp Phe Asp Thr Gln Ser Pro
                   85                  90                  95
   Val Lys Pro Tyr Phe Gly Ala Arg Leu Ser Leu Asn Arg Ala Ser Ala
               100                 105                 110
   His Leu Gly Gly Ser Asp Ser Phe Ser Lys Thr Ser Ala Gly Leu Gly
           115                 120                 125
   Val Leu Ala Gly Val Ser Tyr Ala Val Thr Pro Asn Val Asp Leu Asp
       130                 135                 140
   Ala Gly Tyr Arg Tyr Asn Tyr Val Gly Lys Val Asn Thr Val Lys Asn
   145                 150                 155                 160
   Val Arg Ser Gly Glu Leu Ser Ala Gly Val Arg Val Lys Phe
                   165                 170
<210> 91
   <211> 710
   <212> DNA
   <213> Neisseria gonorrhoeae
<400> 91
   cggcaaagca gccggatgcc gccgcgtatc ttgaggcatt gaaaatatta cgatgcaaaa 60
   agaaaatttc agtataatac ggcaggattc tttaacggat tattaacaat ttttctccct 120
   gaccataaag gaaccaaaat atgaaaaaag cacttgccgc actgattgcc ctcgcactcc 180
   cggccgccgc actggcggaa ggcgcatccg gcttttacgt ccaagccgat gccgcacacg 240
   ccaaagcctc aagctcttta ggttctgcca aaggcttcag cccgcgcatc tccgcaggct 300
   accgcatcaa cgacctccgc ttcgccgtcg attacacgcg ctacaaaaac tataaagccc 360
   catccaccga tttcaaactt tacagcatcg gcgcgtccgt catttacgac ttcgacaccc 420
   aatcgcccgt caaaccgtat ttcggcgcgc gcttgagcct caaccgcgct tccgcccact 480
   tgggcggcag cgacagcttc agcaaaacct ccgccggcct cggcgtattg gcgggcgtaa 540
   gctatgccgt taccccgaat gtcgatttgg atgccggcta ccgctacaac tacgtcggca 600
   aagtcaacac tgtcaaaaac gtccgttccg gcgaactgtc cgccggcgtg cgcgtcaaat 660
   tctgatatac gcgttattcc gcaaaccgcc gagccttcgg cggttttttg           710
<210> 92
   <211> 792
   <212> PRT
   <213> Neisseria gonorrhoeae
<400> 92
   Met Lys Leu Lys Gln Ile Ala Ser Ala Leu Met Met Leu Gly Ile Ser
   1 5 10 15
   Pro Leu Ala Phe Ala Asp Phe Thr Ile Gln Asp Ile Arg Val Glu Gly
   20 25 30
   Leu Gln Arg Thr Glu Pro Ser Thr Val Phe Asn Tyr Leu Pro Val Lys
   35 40 45
   Val Gly Asp Thr Tyr Asn Asp Thr His Gly Ser Ala Ile Ile Lys Ser
   50 55 60
   Leu Tyr Ala Thr Gly Phe Phe Asp Asp Val Arg Val Glu Thr Ala Asp
   65 70 75 80
   Gly Leu Leu Leu Leu Thr Val Ile Val Cys Pro Thr Ile Gly Ser Leu
   85 90 95
   Asn Ile Thr Gly Ala Lys Met Leu Gln Asn Asp Ala Ile Lys Lys Asn
   100 105 110
   Leu Glu Ser Phe Gly Leu Ala Gln Ser Gln Tyr Phe Asn Gln Ala Thr
   115 120 125
   Leu Asn Gln Ala Val Ala Gly Leu Lys Glu Glu Tyr Leu Gly Arg Gly
   130 135 140
   Lys Leu Asn Ile Gln Ile Thr Pro Lys Val Thr Lys Leu Ala Arg Asn
   145 150 155 160
   Arg Val Asp Ile Asp Ile Thr Ile Asp Glu Gly Lys Ser Ala Lys Ile
   165 170 175
   Thr Asp Ile Glu Phe Glu Gly Asn Gln Val Tyr Ser Asp Arg Lys Leu
   180 185 190
   Met Arg Gln Met Ser Leu Thr Glu Gly Gly Ile Trp Thr Trp Leu Thr
   195 200 205
   Arg Ser Asp Arg Phe Asp Arg Gln Lys Phe Ala Gln Asp Met Glu Lys
   210 215 220
   Val Thr Asp Phe Tyr Gln Asn Asn Gly Tyr Phe Asp Phe Arg Ile Leu
   225 230 235 240
   Asp Thr Asp Ile Gln Thr Asn Glu Asp Lys Thr Arg Gln Thr Ile Lys
   245 250 255
   Ile Thr Val His Glu Gly Gly Arg Phe Arg Trp Gly Lys Val Ser Ile
   260 265 270
   Glu Gly Asp Thr Asn Glu Val Pro Lys Ala Glu Leu Glu Lys Leu Leu
   275 280 285
   Thr Met Lys Pro Gly Lys Trp Tyr Glu Arg Gln Gln Met Thr Ala Val
   290 295 300
   Leu Gly Glu Ile Gln Asn Arg Met Gly Ser Ala Gly Tyr Ala Tyr Ser
   305 310 315 320
   Glu Ile Ser Val Gln Pro Leu Pro Asn Ala Gly Thr Lys Thr Val Asp
   325 330 335
   Phe Val Leu His Ile Glu Pro Gly Arg Lys Ile Tyr Val Asn Glu Ile
   340 345 350
   His Ile Thr Gly Asn Asn Lys Thr Arg Asp Glu Val Val Arg Arg Glu
   355 360 365
   Leu Arg Gln Met Glu Ser Ala Pro Tyr Asp Thr Ser Lys Leu Gln Arg
   370 375 380
   Ser Lys Glu Arg Val Glu Leu Leu Gly Tyr Phe Asp Asn Val Gln Phe
   385 390 395 400
   Asp Ala Val Pro Leu Ala Gly Thr Pro Asp Lys Val Asp Leu Asn Met
   405 410 415
   Ser Leu Thr Glu Arg Ser Thr Gly Ser Leu Asp Leu Ser Ala Gly Trp
   420 425 430
   Val Gln Asp Thr Gly Leu Val Met Ser Ala Gly Val Ser Gln Asp Asn
   435 440 445
   Leu Phe Gly Thr Gly Lys Ser Ala Ala Leu Arg Ala Ser Arg Ser Lys
   450 455 460
   Thr Thr Leu Asn Gly Ser Leu Ser Phe Thr Asp Pro Tyr Phe Thr Ala
   465 470 475 480
   Asp Gly Val Ser Leu Gly Tyr Asp Ile Tyr Gly Lys Ala Phe Asp Pro
   485 490 495
   Arg Lys Ala Ser Thr Ser Val Lys Gln Tyr Lys Thr Thr Thr Ala Gly
   500 505 510
   Gly Gly Val Arg Met Gly Ile Pro Val Thr Glu Tyr Asp Arg Val Asn
   515 520 525
   Phe Gly Leu Ala Ala Glu His Leu Thr Val Asn Thr Tyr Asn Lys Ala
   530 535 540
   Pro Lys Arg Tyr Ala Asp Phe Ile Lys Gln Tyr Gly Lys Thr Asp Gly
   545 550 555 560
   Ala Asp Gly Ser Phe Lys Gly Leu Leu Tyr Lys Gly Thr Val Gly Trp
   565 570 575
   Gly Arg Asn Lys Thr Asp Ser Ala Leu Trp Pro Thr Arg Gly Tyr Leu
   580 585 590
   Thr Gly Val Asn Ala Glu Ile Ala Leu Pro Gly Ser Lys Leu Gln Tyr
   595 600 605
   Tyr Ser Ala Thr His Asn Gln Thr Trp Phe Phe Pro Leu Ser Lys Thr
   610 615 620
   Phe Thr Leu Met Leu Gly Gly Glu Val Gly Ile Ala Gly Gly Tyr Gly
   625 630 635 640
   Arg Thr Lys Glu Ile Pro Phe Phe Glu Asn Phe Tyr Gly Gly Gly Leu
   645 650 655
   Gly Ser Val Arg Gly Tyr Glu Ser Gly Thr Leu Gly Pro Lys Val Tyr
   660 665 670
   Asp Glu Tyr Gly Glu Lys Ile Ser Tyr Gly Gly Asn Lys Lys Ala Asn
   675 680 685
   Val Ser Ala Glu Leu Leu Phe Pro Met Pro Gly Ala Lys Asp Ala Arg
   690 695 700
   Thr Val Arg Leu Ser Leu Phe Ala Asp Ala Gly Ser Val Trp Asp Gly
   705 710 715 720
   Arg Thr Tyr Thr Ala Ala Glu Asn Gly Asn Asn Lys Ser Val Tyr Ser
   725 730 735
   Glu Asn Ala His Lys Ser Thr Phe Thr Asn Glu Leu Arg Tyr Ser Ala
   740 745 750
   Gly Gly Ala Val Thr Trp Leu Ser Pro Leu Gly Pro Met Lys Phe Ile
   755 760 765
   Tyr Ala Tyr Pro Leu Lys Lys Lys Pro Glu Asp Glu Ile Gln Arg Phe
   770 775 780
   Gln Phe Gln Leu Gly Thr Thr Phe
   785 790
<210> 93
   <211> 2379
   <212> DNA
   <213> Neisseria gonorrhoeae
<400> 93
   atgaaactga aacagattgc ctccgcactg atgatgttgg gcatatcgcc tttggcattt 60
   gccgacttca ccatccaaga catccgtgtc gaaggcttgc agcgtaccga gccgagcacc 120
   gtattcaact acctgcccgt caaagtcggc gacacctaca acgacacaca cggcagtgcc 180
   atcatcaaaa gcctgtacgc caccggtttc tttgacgacg tacgagtcga aactgcggac 240
   gggctgcttc tgctgaccgt tatcgtatgc cctaccatcg gctcgctcaa catcaccggc 300
   gccaaaatgc tgcagaacga cgccatcaag aaaaacctcg aatcgttcgg gctggcgcag 360
   tcgcaatact ttaatcaggc gacactcaac caggcagtcg ccggcctgaa agaagaatat 420
   ctcgggcgcg gcaaactcaa tatccaaatc acgcccaaag taaccaaact cgcccgcaac 480
   cgcgtcgaca tcgacatcac gattgacgag ggcaaatccg ccaaaatcac cgacatcgaa 540
   tttgaaggca accaagtcta ttccgaccgc aaactgatgc ggcagatgtc gctgaccgaa 600
   ggcggcattt ggacatggct gacacgaagc gaccggttcg accgccagaa attcgcccaa 660
   gacatggaaa aagtaaccga cttctaccag aacaacggct acttcgattt ccgtatcctc 720
   gataccgaca tccaaaccaa cgaagacaaa accaggcaga ccatcaaaat caccgtccac 780
   gaaggcggac gtttccgctg gggcaaagtg tcgattgaag gcgacaccaa cgaagtcccc 840
   aaggccgaac tggaaaaact gctgaccatg aagcccggca aatggtacga acgccagcag 900
   atgaccgccg ttttgggtga gattcagaac cgcatgggct cggcaggcta cgcatacagc 960
   gaaatcagcg tacagccgct gccgaacgcc ggaaccaaaa ccgtcgattt cgtcctgcac 1020
   atcgaaccgg gcagaaaaat ctacgtcaac gaaatccaca tcaccggcaa caacaaaacc 1080
   cgcgacgaag tcgtgcgccg cgaattgcgc caaatggaat ccgcgcctta cgacacctcc 1140
   aagctgcaac gctccaaaga gcgcgtcgag cttttggcct acttcgacaa cgtacagttt 1200
   gatgccgtcc cgcttgccgg tacgcccgac aaagtcgatt tgaacatgag cctgaccgaa 1260
   cgttccaccg gctcgctcga cttgagcgcg ggctgggttc aggataccgg cttggtcatg 1320
   tccgccggcg tatcgcagga caacctgttc ggtacgggca agtcggccgc cctgcgcgcc 1380
   tcgcgaagca aaaccacgct caacggctcg ctgtcgttta ccgacccgta cttcacggca 1440
   gacggggtca gcctgggcta cgatatttac ggaaaagcct tcgacccgcg caaagcatcg 1500
   accagcgtca aacaatataa aaccaccacc gccggcggcg gcgtaaggat gggtatcccc 1560
   gttaccgaat acgaccgcgt caatttcggg ctggcggcgg aacacctgac cgtcaacacc 1620
   tacaacaaag cacccaaacg ctatgccgac tttatcaaac aatacggcaa aaccgacggc 1680
   gcagacggca gcttcaaagg cctgctgtac aaaggcactg tcggctgggg gcgcaacaag 1740
   accgacagcg ccttatggcc gacgcgcggc tacctgaccg gcgtaaatgc cgaaatcgcc 1800
   ctgcccggca gcaaactgca atactactcc gccacccaca accaaacctg gttcttcccc 1860
   ttaagcaaaa ccttcacgct gatgctcggc ggcgaagtcg gcattgcggg cggctacggc 1920
   agaaccaaag aaatcccctt ctttgaaaac ttctacggcg gcggcctggg ttcggtgcgc 1980
   ggctacgaaa gcggcacgct cggcccgaaa gtgtatgacg aatacggcga aaaaatcagc 2040
   tacggcggca acaaaaaagc caacgtctcc gccgagctgc tcttcccgat gcccggtgcg 2100
   aaagacgcac gcaccgtccg cctgagcctg tttgccgacg caggcagcgt gtgggacggc 2160
   agaacctata ccgccgccga aaacggtaac aacaaatcgg tttactcgga aaacgcgcat 2220
   aaatccacct ttaccaacga attgcgctat tccgccggcg gcgcggttac ctggctctcg 2280
   cctttgggcc cgatgaaatt catctacgcc tacccgctga agaaaaaacc ggaagacgaa 2340
   atccaacgct tccaattcca gctcggcacg acgttctaa                       2379
<210> 94
   <211> 1128
   <212> DNA
   <213> Neisseria gonorrhoeae
<400> 94
   atgaatacac gaaatatgcg ctatattctt ttgacaggac tgttgccgac ggcatccgct 60
   tttggagaga ccgcgctgca atgcgccgct ttgacggaca atgttacgcg tttggcgtgt 120
   tacgacagga tttttgcggc acagcttccg tcttcggcag ggcaggaagg gcaggagtcg 180
   aaagccgtac tcaatctgac ggaaaccgtc cgcagcagct tggataaggg cgaggcggtc 240
   attgttgttg aaaaaggcgg ggatgcgctt cctgccgaca gtgcgggcga aaccgccgat 300
   atctatacgc ctttgagcct gatgtacgac ttggacaaaa acgatttgcg cgggctgttg 360
   ggcgtacgcg aacacaatcc gatgtacctt atgccgtttt ggtataacaa ttcgcccaac 420
   tatgccccga gttcgccgac gcgcggtacg actgtacagg aaaaattcgg acagcagaaa 480
   cgtgcggaaa ccaaattgca ggtttcgttc aaaagcaaaa ttgccgaaaa tttgtttaaa 540
   acccgcgcgg atctgtggtt cggctacacc caaagatccg attggcagat ttacaaccaa 600
   ggcaggaaat ccgcgccgtt ccgcaatacg gattacaaac ctgaaatttt cctgacccag 660
   cctgtgaagg cggatttgcc gttcggcggc aggctgcgta tgctcggtgc gggttttgtc 720
   caccagtcca acggacagag ccgtcccgaa tcgcgttcgt ggaacaggat ttatgccatg 780
   gcaggcatgg aatggggcaa attgacggtg attccgcgcg tgtgggtgcg tgcgttcgat 840
   cagagcggcg ataaaaacga caatcccgat attgccgact atatggggta tggcgacgtg 900
   aagctgcagt accgcctgaa cgacaggcag aatgtgtatt ccgtattgcg ctacaacccc 960
   aaaacgggct acggcgcgat tgaagccgcc tacacgtttc cgattaaggg caaactcaaa 1020
   ggcgtggtac gcggattcca cggttacggc gagagcctga tcgactacaa ccacaagcag 1080
   aacggtatcg gtatcgggtt gatgttcaac gactgggacg gcatctga              1128
<210> 95
   <211> 375
   <212> PRT
   <213> Neisseria gonorrhoeae
<400> 95
   Met Asn Thr Arg Asn Met Arg Tyr Ile Leu Leu Thr Gly Leu Leu Pro
   1               5                   10                  15
   Thr Ala Ser Ala Phe Gly Glu Thr Ala Leu Gln Cys Ala Ala Leu Thr
               20                  25                  30
   Asp Asn Val Thr Arg Leu Ala Cys Tyr Asp Arg Ile Phe Ala Ala Gln
           35                  40                  45
   Leu Pro Ser Ser Ala Gly Gln Glu Gly Gln Glu Ser Lys Ala Val Leu
       50                  55                  60
   Asn Leu Thr Glu Thr Val Arg Ser Ser Leu Asp Lys Gly Glu Ala Val
   65                  70                  75                  80
   Ile Val Val Glu Lys Gly Gly Asp Ala Leu Pro Ala Asp Ser Ala Gly
                   85                  90                  95
   Glu Thr Ala Asp Ile Tyr Thr Pro Leu Ser Leu Met Tyr Asp Leu Asp
               100                 105                 110
   Lys Asn Asp Leu Arg Gly Leu Leu Gly Val Arg Glu His Asn Pro Met
           115                 120                 125
   Tyr Leu Met Pro Phe Trp Tyr Asn Asn Ser Pro Asn Tyr Ala Pro Ser
       130                 135                 140
   Ser Pro Thr Arg Gly Thr Thr Val Gln Glu Lys Phe Gly Gln Gln Lys
   145 150 155 160
   Arg Ala Glu Thr Lys Leu Gln Val Ser Phe Lys Ser Lys Ile Ala Glu
   165 170 175
   Asn Leu Phe Lys Thr Arg Ala Asp Leu Trp Phe Gly Tyr Thr Gln Arg
   180 185 190
   Ser Asp Trp Gln Ile Tyr Asn Gln Gly Arg Lys Ser Ala Pro Phe Arg
   195 200 205
   Asn Thr Asp Tyr Lys Pro Glu Ile Phe Leu Thr Gln Pro Val Lys Ala
   210 215 220
   Asp Leu Pro Phe Gly Gly Arg Leu Arg Met Leu Gly Ala Gly Phe Val
   225 230 235 240
   His Gln Ser Asn Gly Gln Ser Arg Pro Glu Ser Arg Ser Trp Asn Arg
   245 250 255
   Ile Tyr Ala Met Ala Gly Met Glu Trp Gly Lys Leu Thr Val Ile Pro
   260 265 270
   Arg Val Trp Val Arg Ala Phe Asp Gln Ser Gly Asp Lys Asn Asp Asn
   275 280 285
   Pro Asp Ile Ala Asp Tyr Met Gly Tyr Gly Asp Val Lys Leu Gln Tyr
   290 295 300
   Arg Leu Asn Asp Arg Gln Asn Val Tyr Ser Val Leu Arg Tyr Asn Pro
   305 310 315 320
   Lys Thr Gly Tyr Gly Ala Ile Glu Ala Ala Tyr Thr Phe Pro Ile Lys
   325 330 335
   Gly Lys Leu Lys Gly Val Val Arg Gly Phe His Gly Tyr Gly Glu Ser
   340 345 350
   Leu Ile Asp Tyr Asn His Lys Gln Asn Gly Ile Gly Ile Gly Leu Met
   355 360 365
   Phe Asn Asp Trp Asp Gly Ile
   370 375
<210> 96
   <211> 1000
   <212> DNA
   <213> Neisseria gonorrhoeae
<400> 96
   ttttggcttc cagcgtttcg ttgttttcgt acaagtcgta agtcagcttc agattgttgg 60
   cttttttaaa gtcttcgacc gtactctcgt caacataatt cgaccagttg tagatgttca 120
   gagtatcggt ggcagcggct tcggcattgg cagcaggtgc gctgcctgct tgaggctgca 180
   cggcgttttt ttcgctgccg ccgcaggctg ccagagacag cgcggccaaa acggctaata 240
   cggatttttt catacgggca gattcctgat gaaagaggtt ggaaaaaaag aaaaccccgc 300
   gccccataaa caccccggcg caaggtttgg gtattgtaaa gtaaatttgt gcaaactcaa 360
   agcgatattg gcctgatttt cctaaaaaat taccctgttt ccaaaaaagg gggggaaacg 420
   gccgcccgat tttgccgttt ttttgcgccg tcagggtgtc cgacgggcgg atagagaaaa 480
   aaggcttgca tataatgtaa acccccttta aaattgcgcg tttacagaat ttatttttct 540
   ttcaggagat tccaatatgg caaacagcgc acaagcacgc aaacgtgccc gccagtccgt 600
   caaacaacgc gcccacaacg ctagcctgcg taccgcattc cgcaccgcag tgaaaaaagt 660
   attgaaagca gtcgaagcag gcgataaagc tgccgcacaa gcggtttacc aagagtccgt 720
   caaagtcatc gaccgcatcg ccgacaaagg cgtgtttcat aaaaacaaag cggctcgcca 780
   caaaagccgc ctgtctgcaa aagtaaaagc actggcttga tttttgcaaa accgccaagg 840
   cggttgatac gcgataagcg gaaaaccctg aagcccgacg gtttcggggt tttctgtatt 900
   tcgggggtaa agttcgaaat ggcggaaagg gtgcggtttt ttatccgaat ccgctataaa 960
   atgccgtttg aaaaccaata tgccgacaat gggggcggag 1000
<210> 97
   <211> 713
   <212> PRT
   <213> Neisseria gonorrhoeae
<400> 97
   Met Asn Ala Pro Phe Phe Arg Leu Ser Leu Leu Ser Leu Thr Leu Ala
   1 5 10 15
   Ala Gly Phe Ala His Ala Ala Glu Asn Asn Ala Asn Val Ala Leu Asp
   20 25 30
   Thr Val Thr Val Lys Gly Asp Arg Gln Gly Ser Lys Ile Arg Thr Asn
   35 40 45
   Ile Val Thr Leu Gln Gln Lys Asp Glu Ser Thr Ala Thr Asp Met Arg
   50 55 60
   Glu Leu Leu Lys Glu Glu Pro Ser Ile Asp Phe Gly Gly Gly Asn Gly
   65 70 75 80
   Thr Ser Gln Phe Leu Thr Leu Arg Gly Met Gly Gln Asn Ser Val Asp
   85 90 95
   Ile Lys Val Asp Asn Ala Tyr Ser Asp Ser Gln Ile Leu Tyr His Gln
   100 105 110
   Gly Arg Phe Ile Val Asp Pro Ala Leu Val Lys Val Val Ser Val Gln
   115 120 125
   Lys Gly Ala Gly Ser Ala Ser Ala Gly Ile Gly Ala Thr Asn Gly Ala
   130 135 140
   Ile Ile Ala Lys Thr Val Asp Ala Gln Asp Leu Leu Lys Gly Leu Asp
   145 150 155 160
   Lys Asn Trp Gly Val Arg Leu Asn Ser Gly Phe Ala Gly Asn Asn Gly
   165 170 175
   Ala Ser Tyr Gly Ala Ser Val Phe Gly Lys Glu Gly Asn Phe Asp Gly
   180 185 190
   Leu Phe Ser Tyr Asn Arg Asn Asp Glu Lys Asp Tyr Glu Ala Gly Lys
   195 200 205
   Gly Phe Arg Asn Asp Asn Gly Gly Lys Thr Val Pro Tyr Ser Ala Leu
   210 215 220
   Asp Lys Arg Ser Tyr Leu Ala Lys Ile Gly Thr Thr Phe Gly Asp Gly
   225 230 235 240
   Asp His Arg Ile Val Leu Ser His Met Lys Asp Gln His Arg Gly Ile
   245 250 255
   Arg Thr Val Arg Glu Glu Phe Ala Val Ser Glu Lys Asn Ser Arg Ile
   260 265 270
   Thr Ile Lys Arg Gln Ala Pro Ser Tyr Arg Glu Thr Thr Gln Ser Asn
   275 280 285
   Thr Asn Leu Ala Tyr Thr Gly Lys Asp Leu Gly Phe Val Glu Lys Leu
   290 295 300
   Asp Ala Asn Ala Tyr Val Leu Glu Lys Lys Arg Tyr Ser Ala Asp Asp
   305 310 315 320
   Lys Asp Asn Gly Tyr Ala Gly Asn Val Lys Gly Pro Asn His Thr Arg
   325 330 335
   Ile Ala Thr Arg Ser Met Asn Phe Asn Phe Asp Ser Arg Leu Ala Glu
   340 345 350
   Gln Thr Leu Leu Lys Tyr Gly Ile Asn Tyr Arg His Gln Glu Ile Lys
   355 360 365
   Pro Gln Ala Phe Leu Asn Ser Glu Phe Glu Ile Lys Asp Lys Glu Lys
   370 375 380
   Ala Thr Asn Glu Glu Lys Lys Lys Asn Arg Glu Asn Glu Lys Ile Ala
   385 390 395 400
   Lys Ala Tyr Arg Leu Thr Asn Pro Thr Lys Thr Asp Thr Gly Ala Tyr
   405 410 415
   Ile Glu Ala Ile His Glu Ile Asp Gly Phe Thr Leu Thr Gly Gly Leu
   420 425 430
   Arg Tyr Asp Arg Phe Lys Val Lys Thr His Asp Gly Lys Thr Val Ser
   435 440 445
   Ser Ser Ser Leu Asn Pro Ser Phe Gly Val Ile Trp Gln Pro Arg Glu
   450 455 460
   His Trp Ser Phe Ser Ala Ser His Asn Tyr Ala Gly Arg Ser Pro Arg
   465 470 475 480
   Leu Tyr Asp Ala Leu Gln Thr His Gly Lys Arg Gly Ile Ile Ser Ile
   485 490 495
   Ala Asp Gly Thr Lys Ala Glu Arg Ala Arg Asn Thr Glu Ile Gly Phe
   500 505 510
   Asn Tyr Asn Asp Gly Thr Phe Ala Ala Asn Gly Ser Tyr Phe Arg Gln
   515 520 525
   Thr Ile Lys Asp Ala Leu Ala Asn Pro Gln Asn Arg His Asp Ser Val
   530 535 540
   Ala Val Arg Glu Ala Val Asn Ala Gly Tyr Ile Lys Asn His Gly Tyr
   545 550 555 560
   Glu Leu Gly Ala Ser Tyr Arg Thr Gly Gly Leu Thr Ala Lys Val Gly
   565 570 575
   Val Ser His Ser Lys Pro Arg Phe Tyr Asp Thr His Lys Asp Lys Leu
   580 585 590
   Leu Ser Ala Asn Pro Glu Phe Gly Ala Gln Val Gly Arg Thr Trp Thr
   595 600 605
   Ala Ser Leu Ala Tyr Arg Phe Lys Asn Pro Asn Leu Glu Ile Gly Trp
   610 615 620
   Arg Gly Arg Tyr Val Gln Lys Ala Val Gly Ser Ile Leu Ala Ala Gly
   625 630 635 640
   Gln Lys Asp Arg Asp Gly Lys Leu Glu Asn Val Val Arg Gln Gly Phe
   645 650 655
   Gly Val Asn Asp Val Phe Ala Asn Trp Lys Pro Leu Gly Lys Asp Thr
   660 665 670
   Leu Asn Val Asn Leu Ser Val Asn Asn Val Phe Asp Lys Phe Tyr Tyr
   675 680 685
   Pro His Ser Gln Arg Trp Thr Asn Thr Leu Pro Gly Val Gly Arg Asp
   690 695 700
   Val Arg Leu Gly Val Asn Tyr Lys Phe
   705 710
<210> 98
   <211> 2600
   <212> DNA
   <213> Neisseria gonorrhoeae
<400> 98
   aaaccggtac ggcgttgccc cgccttagct caaagagaac gattccctaa ggtgctgaag 60
   caccgagtga atcggttccg tactatttgt actgtctgcg gcttcgccgc cttgtcctga 120
   tttttgttag tccacatata catttccgac aaaacctgtc aacaaaaaac aacgcttcgc 180
   aaataaaaac gataatcagc tttacacaac ccccccccgc taatataaac aaaaataatt 240
   attattattt tttcttatcc tgccaaacct taacggtttg gcttaacttc ccttcataca 300
   ctcaaaagga cgaacaaatg aacgccccgt ttttccgcct cagcctgctc tcgctcacac 360
   ttgccgccgg ctttgcccac gcggcagaaa ataatgccaa tgtcgcattg gataccgtta 420
   ccgtaaaagg cgaccgccaa ggcagcaaaa tccgtaccaa catcgttacg cttcaacaaa 480
   aagacgaaag caccgcaacc gatatgcgcg aactcttaaa agaagagccc tccatcgatt 540
   tcggcggcgg caacggcacg tcccaattcc tgacgctgcg cggtatgggt cagaactctg 600
   tcgacatcaa ggtggacaac gcctattccg acagccaaat cctttaccac caaggcagat 660
   ttattgtcga tcccgctttg gttaaagtcg tttccgtaca gaaaggcgcg ggttccgcct 720
   ctgccggtat cggcgcgacc aacggcgcga tcatcgccaa aaccgtcgat gcccaagacc 780
   tgctcaaagg cttggataaa aactggggcg tgcgcctcaa cagcggcttt gccggcaaca 840
   acggcgcaag ctacggcgca agcgtattcg gaaaagaggg caacttcgac ggtttgttct 900
   cttacaaccg caacgatgaa aaagattacg aagccggcaa aggtttccgc aatgacaacg 960
   gcggcaaaac cgtaccgtac agcgcgctgg acaaacgcag ctacctcgcc aaaatcggaa 1020
   caaccttcgg cgacggcgac caccgcatcg tgttgagcca tatgaaagac caacaccggg 1080
   gcatccgcac tgtgcgtgaa gagtttgccg tcagcgaaaa aaattcacgg ataactatta 1140
   aacgccaagc cccatcctac cgcgaaacca ctcaatccaa caccaacttg gcgtacaccg 1200
   gcaaagattt gggctttgtc gaaaaactgg atgccaacgc ctatgtgttg gaaaagaaac 1260
   gctattccgc cgatgacaaa gataacggct acgcaggcaa tgtaaaaggc cccaaccata 1320
   cccgaatcgc cactcggagt atgaacttca acttcgacag ccgccttgcc gaacaaaccc 1380
   tgttgaaata cggcatcaac taccgccatc aggaaatcaa accgcaagcg tttttgaact 1440
   cggaatttga aataaaagat aaagaaaaag caactaatga agagaaaaag aagaaccgtg 1500
   aaaatgaaaa aattgccaaa gcctaccgcc tgaccaaccc gaccaaaacc gataccggcg 1560
   cgtatatcga agccattcac gagattgacg gctttaccct gaccggcggg ctgcgttacg 1620
   accgcttcaa ggtgaaaacc cacgacggca aaaccgtttc aagcagcagc ctcaacccga 1680
   gtttcggcgt gatttggcag ccgcgcgaac actggagctt cagcgcgagc cacaactacg 1740
   ccggccgcag cccgcgcctg tatgacgctc tgcaaaccca cggcaagcgc ggcatcatct 1800
   cgattgccga cggcacgaaa gccgaacgcg cgcgcaatac cgaaatcggc ttcaactaca 1860
   acgacggcac gtttgccgca aacggcagct acttccggca gaccatcaaa gacgcgcttg 1920
   ccaatccgca aaaccgccac gactccgtcg ccgtccgcga agccgtcaac gccggctaca 1980
   tcaaaaacca cggttacgaa ttgggcgcgt cctaccgcac cggcggcctg accgccaaag 2040
   tcggcgtaag ccacagcaaa ccgcgctttt acgatacgca caaagacaag ctgttgagcg 2100
   cgaaccctga atttggcgca caagtcggcc gcacttggac ggcctccctt gcctaccgct 2160
   tcaaaaaccc gaatctggaa atcggctggc gcggtcgtta tgttcaaaaa gccgtgggtt 2220
   cgatattggc ggcaggtcaa aaagaccgcg acggcaaatt ggaaaacgtt gtacgccaag 2280
   gtttcggtgt gaacgatgtc ttcgccaact ggaaaccgct gggcaaagac acgctcaatg 2340
   ttaatctttc ggttaacaac gtgttcgaca agttctacta tccgcacagc caacgctgga 2400
   ccaataccct gccgggcgtg ggacgtgatg tacgcctggg cgtgaactac aagttctaaa 2460
   acgcacatcc cgaaaaaatg ccgtctgaaa gcctttcaga cggcatctgt cctgataatt 2520
   tgatatatag tggattaaca aaaaccggta cggcgttgcc ccgccttagc tcaaagggaa 2580
   cgattcccta aggtgctgaa                                            2600
<210> 99
   <211> 236
   <212> PRT
   <213> Neisseria gonorrhoeae
<400> 99
   Met Thr Lys Gln Leu Lys Leu Ser Ala Leu Phe Val Ala Leu Leu Ala
   1               5                   10                  15
   Ser Gly Thr Ala Val Ala Gly Glu Ala Ser Val Gln Gly Tyr Thr Val
               20                  25                  30
   Ser Gly Gln Ser Asn Glu Ile Val Arg Asn Asn Tyr Gly Glu Cys Trp
           35                  40                  45
   Lys Asn Ala Tyr Phe Asp Lys Ala Ser Gln Gly Arg Val Glu Cys Gly
       50                  55                  60
   Asp Ala Val Ala Val Pro Glu Pro Glu Pro Ala Pro Val Ala Val Val
   65                  70                  75                  80
   Glu Gln Ala Pro Gln Tyr Val Asp Glu Thr Ile Ser Leu Ser Ala Lys
                   85                  90                  95
   Thr Leu Phe Gly Phe Asp Lys Asp Ser Leu Arg Ala Glu Ala Gln Asp
               100                 105                 110
   Asn Leu Lys Val Leu Ala Gln Arg Leu Ser Arg Thr Asn Val Gln Ser
           115                 120                 125
   Val Arg Val Glu Gly His Thr Asp Phe Met Gly Ser Glu Lys Tyr Asn
       130                 135                 140
   Gln Ala Leu Ser Glu Arg Arg Ala Tyr Val Val Ala Asn Asn Leu Val
   145                 150                 155                 160
   Ser Asn Gly Val Pro Ala Ser Arg Ile Ser Ala Val Gly Leu Gly Glu
                   165                 170                 175
   Ser Gln Ala Gln Met Thr Gln Val Cys Gln Ala Glu Val Ala Lys Leu
               180                 185                 190
   Gly Ala Lys Ala Ser Lys Ala Lys Lys Arg Glu Ala Leu Ile Ala Cys
           195                 200                 205
   Ile Glu Pro Asp Arg Arg Val Asp Val Lys Ile Arg Ser Ile Val Thr
       210                 215                 220
   Arg Gln Val Val Pro Ala Arg Asn His His Gln His
   225                 230                 235
<210> 100
   <211> 1349
   <212> DNA
   <213> Neisseria gonorrhoeae
<400> 100
   gaattcctat ccgatttgcc gccatgtttc tacagcggcc tgtatgttgg caattcagca 60
   gttgcttctg tatctgctgt acaaatctaa tgagggaata aaatgaccaa acagctgaaa 120
   ttaagcgcat tattcgttgc attgctcgct tccggcactg ctgttgcggg cgaggcgtcc 180
   gttcagggtt acaccgtaag cggccagtcg aacgaaatcg tacgcaacaa ctatggagaa 240
   tgctggaaaa acgcctactt tgataaagca agccaaggtc gcgtagaatg cggcgatgcg 300
   gttgccgtcc ccgagcccga acccgcgcct gtcgccgttg tggagcaggc tcctcaatat 360
   gttgatgaaa ccatttccct gtctgccaaa accctgttcg gtttcgataa ggattcattg 420
   cgcgccgaag ctcaagacaa cctgaaagta ttggcgcaac gcctgagtcg aaccaatgtc 480
   caatctgtcc gcgtcgaagg ccataccgac tttatgggtt ctgaaaaata caatcaggct 540
   ctgtccgaac gccgcgcata cgtagtggca aacaacctgg tcagcaacgg cgtacctgct 600
   tctagaattt ctgctgtcgg cttgggcgaa tctcaagcgc aaatgactca agtttgtcaa 660
   gccgaagttg ccaaactggg tgcgaaagcc tctaaagcca aaaaacgtga ggctctgatt 720
   gcatgtatcg aacctgaccg ccgcgtagat gtgaaaatcc gcagcatcgt aacccgtcag 780
   gttgtgccgg cacgcaatca tcaccaacac taaggctagg taatatcttg ccgatgcatg 840
   aggttagcgg attttgtacc gggtactgtt gcaatattcg tgaaacgtcg gccggtatcg 900
   atgatgtgaa acaaaccccg cttttgcggg gtttgttttt ttgggtggtt ttctgaaacg 960
   gctatcgtca gaatcggggt gcaggttcgg attcggattc agattcatgt ttgtgtccca 1020
   ttgccgcgct ttatagtgga ttaacaaaaa tcaggacaag gcgacgaagc cgcagacagt 1080
   acaatagtac ggcaaggcga ggcaacgccg taccggttta aatttaatcc actatatcgg 1140
   ttgaaactct gattttaagg cggtaggatg tgggtttgcc catagcaagg gaatcctttc 1200
   tgtatcaagc cccgaaaggg ataattcata caaattcacg cctttccccc tcattgggaa 1260
   atggatggaa tcgtgcccga tgtgtgcggc actgtatgcc ggatatggtt ttatcatcat 1320
   cccttttcgg ttgaaacccc gcggaattc 1349
<210> 101
   <211> 32
   <212> DNA
   <213> Artificial Sequence
<220>
   <223> Primer
<400> 101
   gggaatccat atgaaaaaac tcttgaaatc gg 32
<210> 102
   <211> 29
   <212> DNA
   <213> Artificial Sequence
<220>
   <223> Primer
<400> 102
   ggaagatctt tagaagcgga attgtgcat 29
<210> 103
   <211> 36
   <212> DNA
   <213> Artificial Sequence
<220>
   <223> Primer
<400> 103
   ctgcagaatc gcgaatgaaa aaactcttga aatcgg 36
<210> 104
   <211> 35
   <212> DNA
   <213> Artificial Sequence
<220>
   <223> Primer
<400> 104
   ctgcagaatc gcgaatgagt agcaagctag tgaac 35
<210> 105
   <211> 29
   <212> DNA
   <213> Artificial Sequence
<220>
   <223> Primer
<400> 105
   aggagatctt tagaattgga atcctccgg 29
<210> 106
   <211> 1194
<212> DNA
   <213> Chlamydia trachomatis
<400> 106
   atgaaaaaac tcttgaaatc ggtattagta tttgccgctt tgagttctgc ttcctccttg 60
   caagctctgc ctgtggggaa tcctgctgaa ccaagcctta tgatcgacgg aattctgtgg 120
   gaaggtttcg gcggagatcc ttgcgatcct tgcaccactt ggtgtgacgc tatcagcatg 180
   cgcgttggtt actacggaga ctttgttttc gaccgtgttt tgaaaactga tgtgaataaa 240
   gaatttcaga tgggagcggc gcctactacc agcgatgtag aaggcttaca aaacgatcca 300
   acaacaaatg ttgctcgtcc aaatcccgct tatggcaaac acatgcaaga tgctgaaatg 360
   tttacgaacg ctgcttacat ggcattaaat atctgggatc gttttgatgt attttgtaca 420
   ttgggagcaa ctaccggtta tttaagagga aactccgctt ccttcaactt agttggatta 480
   ttcggaacaa aaacacaata ttctaagttt aatacagcga atcttgttcc taacactgct 540
   ttggatcgag ctgtggttga gctttataca gacaccacct ttgcttggag cgtaggtgct 600
   cgtgcagctc tctgggaatg tgggtgtgca acgttaggag cttctttcca atatgctcaa 660
   tctaaaccta aagtagaaga gttaaatgtt ctttgtaatg catccgaatt tactattaat 720
   aagccgaaag gatatgttgg ggtggaattt ccacttgata ttaccgcagg aacagaagct 780
   gcgacaggga ctaaggatgc ctctattgac taccatgagt ggcaagcaag tttagccctt 840
   tcttacagat taaatatgtt cactccttac attggagtta aatggtctag agtaagtttt 900
   gatgccgaca cgatccgtat cgctcagcct aaattggctg aagcaatctt ggatgtcact 960
   actctaaacc cgaccatcgc tggtaaagga gctgtggtct cttccggaag cgataacgaa 1020
   ctggctgata caatgcaaat cgtttccttg cagttgaaca agctgaaatc tagaaaatct 1080
   tgcggtattg cagtaggaac gactattgta gatgcagata aatacgcagt tacagttgag 1140
   actcgcttga tcgatgagag agcagctcac gtaaatgcac aattccgctt ctaa 1194
<210> 107
   <211> 1182
   <212> DNA
   <213> Chlamydia trachomatis
<400> 107
   atgaaaaaac tcttgaaatc ggtattagta tttgccgctt tgagttctgc ttcctccttg 60
   caagctctgc ctgtggggaa tcctgctgaa ccaagcctta tgatcgacgg aattctgtgg 120
   gaaggtttcg gcggagatcc ttgcgatcct tgcgccactt ggtgtgacgc tatcagcatg 180
   cgtgttggtt actacggaga ctttgttttc gaccgtgttt tgaaaactga tgtgaataaa 240
   gaatttcaga tgggtgccaa gcctacaact gatacaggca atagtgcagc tccatccact 300
   cttacagcaa gagagaatcc tgcttacggc cgacatatgc aggatgctga gatgtttaca 360
   aatgccgctt gcatggcatt gaatatttgg gatcgttttg atgtattctg tacattagga 420
   gccaccagtg gatatcttaa aggaaactct gcttctttca atttagttgg attgtttgga 480
   gataatgaaa atcaaaaaac ggtcaaagcg gagtctgtac caaatatgag ctttgatcaa 540
   tctgttgttg agttgtatac agatactact tttgcgtgga gcgtcggcgc tcgcgcagct 600
   ttgtgggaat gtggatgtgc aactttagga gcttcattcc aatatgctca atctaaacct 660
   aaagtagaag aattaaacgt tctctgcaat gcagcagagt ttactattaa taaacctaaa 720
   gggtatgtag gtaaggagtt tcctcttgat cttacagcag gaacagatgc tgcgacagga 780
   actaaggatg cctctattga ttaccatgaa tggcaagcaa gtttagctct ctcttacaga 840
   ctgaatatgt tcactcccta cattggagtt aaatggtctc gagcaagctt tgatgccgat 900
   acgattcgta tagcccagcc aaaatcagct acagctattt ttgatactac cacgcttaac 960
   ccaactattg ctggagctgg cgatgtgaaa actggcgcag agggtcagct cggagacaca 1020
   atgcaaatcg tttccttgca attgaacaag atgaaatcta gaaaatcttg cggtattgca 1080
   gtaggaacaa ctattgtgga tgcagacaaa tacgcagtta cagttgagac tcgcttgatc 1140
   gatgagagag cagctcacgt aaatgcacaa ttccgcttct aa                   1182
<210> 108
   <211> 1023
   <212> DNA
   <213> Chlamydia trachomatis
<400> 108
   atgagtagca agctagtgaa ctatctccgt ttgactttcc tatctttttt agggatcgca 60
   tctacttcat tagacgctat gcctgcgggg aatccggcgt ttccagtcat cccggggatt 120
   aatattgaac agaaaaatgc ctgttctttc gatttatgta attcttatga tgtactatcc 180
   gcactgtccg gtaacctgaa gctctgcttc tgcggagatt atatcttttc agaagaagct 240
   caggtaaaag atgtccctgt cgttacctct gtgacaacag ctggggttgg tccttctcct 300
   gatattactt cgacaaccaa aacgcgaaat ttcgatctcg tgaactgtaa tctcaataca 360
   aactgtgtag ctgtagcttt ttcccttcct gatcgttcgc tgagcgcgat tcctctgttt 420
   gatgtgagtt tcgaagtgaa agtaggagga ctgaaacaat actaccgcct tcccatgaat 480
   gcctatcgag acttcacctc ggaacctctc aattctgaat cagaagttac ggacgggatg 540
   attgaagtac agtccaatta cggatttgtt tgggatgtta gcttgaaaaa agtcatatgg 600
   aaagatggcg tttcctttgt aggcgtcggt gcagactatc gccatgcttc ttgccctatt 660
   gactacatca ttgcaaacag tcaagctaat ccagaagtat tcatcgctga ctcggatggg 720
   aaactgaact tcaaggagtg gagtgtctgc gtaggtctta ctacctatgt gaatgactac 780
   gttcttcctt acttagcgtt ttctataggg agtgtttctc gccaagctcc ggacgacagc 840
   ttcaaaaaat tagaagatcg cttcactaac ctcaaattta aagttcgtaa aattaccagc 900
   tctcatcgtg gaaacatctg catcggagcg acaaactatg tcgccgataa cttcttctac 960
   aacgtagaag gaagatgggg aagccagcgc gctgtgaacg tctccggagg attccaattc 1020
   taa                                                               1023